# EUROPEAN PATENT APPLICATION

(11) **EP 2 397 479 A1**
(43) Date of publication of application: **21.12.2011**
(21) Application number: 10741239.7
(22) Date of filing: 10.02.2010
(51) Int. Cl.: C07D 403/04, A61K 31/5377, A61K 31/541, A61K 31/55, A61P 1/04, A61P 11/06, A61P 17/00, A61P 17/06, A61P 19/02, A61P 29/00, A61P 35/02, A61P 37/02, A61P 37/06, A61P 37/08, A61P 43/00, C07D 401/14, C07D 403/14, C07D 405/14, C07D 413/14, C07D 417/14

(54) **HETERO RING DERIVATIVE**

(30) Priority: 12.02.2009 JP 2009029759
(71) Applicant: Astellas Pharma Inc., Tokyo 103-8411 (JP)
(72) Inventor: TAKAHASHI, Fumie, Tokyo 103-8411 (JP); IMADA, Sunao, Tokyo 103-8411 (JP); SHIWAKU, Masahiko, deceased (JP); KATO, Koji, Tokyo 103-8411 (JP); FUKAHORI, Hidehiko, Tokyo 103-8411 (JP)
(74) Representative: HOFFMANN EITLE
(86) International application number: PCT/JP2010/051910
(87) International publication number: WO 2010/092962

(57) **Abstract**

[Object]

A novel and excellent method for preventing or treating rejection in the transplantation of various organs, allergy diseases, autoimmune diseases, hematologic tumor, or the like, based on a PI3Kδ-selective inhibitory action and/or an IL-2 production inhibitory action, and/or a B cell proliferation inhibitory action (including an activation inhibitory action), is provided

[Means for Solution]

It was found that a 3-substituted triazine or 3-substituted pyrimidine derivative exhibits a PI3Kδ-selective inhibitory action, and/or an IL-2 production inhibitory action, and/or a B cell proliferation inhibitory action (including an activation inhibitory action), and can be an agent for preventing or treating rejection in the transplantation of various organs, allergy diseases (asthma, atopic dermatitis, etc.), autoimmune diseases (rheumatoid arthritis, psoriasis, ulcerative colitis, Crohn's disease, systemic lupus erytheznatosus, etc.), hematologic tumor (leukemia etc.), or the like, thereby completing the present invention.

## Description

### Technical Field

The present invention relates to a hetero ring derivative and/or a salt thereof, which has a pharmacological activity. Further, the present invention relates to a pharmaceutical or a pharmaceutical composition, which contains the hetero ring derivative above and/or a salt thereof as an active ingredient.

### Background Art

Phosphatidylinositol-3-kinase (PI3K) is a lipid signaling kinase, which is present universally throughout all species, ranging from plants or yeasts to mammals including humans. PI3K is an enzyme for phosphorylating the hydroxyl group at the 3-position of phosphatidylinositol, phosphatidylinositol-4-phosphate, and phosphatidylinositol-4,5-diphosphate, which are cell membrane phospholipids, and from each of the substrates, phosphatidylinositol-3-phosphate, phosphatidylinasitol-3,4-diphosphate, and phosphatidylinasitol-3,4,5-triphosphate (PIP3) are produced. These phosphorylated phosphatidylinositol thus produced act as an intracellular second messenger. Particularly, PIP3 causes migration of various molecules having pleckstrin homology (PH) domains to a position near the cell membrane, and thus induces activation of the molecules, and thus it is considered to be the most important phosphorylated phosphatidylinositol ("The Journal of Biological Chemistry", 1999, Vol. 274, p. 8347-8350).

PI3K is divided into three classes, Classes I, II, and III, according to various characteristics, and from the viewpoints that the only enzyme producing PIP3 in vivo is Class I PI3K, the Class I PI3K is considered to be the most important class ("Biochimica et Biophysica Acta", 2008, Vol. 1784, p. 159-185). The Class I PI3K is subdivided into IA and IB. The Class IA PI3K consists of heterodimers including a combination of a 110-kDa catalytic subunit (p110α, β, or δ) and a 50 to 85-kDa regulatory subunit (p85α, p85β, p55α, p55γ, or p50α), and the Class IB PI3K is a heterodimer of a 110-kDa catalytic subunit (p110γ) and a 101-kDa regulatory subunit (p101) ("Nature Immunology", 2003, No. 4, p. 313-319). Hereinafter, the respective names of PI3K are referred to as PI3Kα, β, δ, and γ, corresponding to catalytic subunits included therein, respectively.

PI3Kα and β are widely present in vivo and deficiency of PI3Kα and β in mice has been reported to be fetally lethal in both cases ("The Journal of Biological Chemistry", 1999, Vol. 274, p. 10963-10968; and "Mammalian Genome", 2002, Vol. 13, p. 169-172). As a result of the studies using subtype-selective compounds, it has been reported that PI3Kα plays an important role in insulin signaling and a PI3Kα inhibitor causes insulin resistance ("Cell", 2006, Vol. 125, p. 733-747). Further, it has been reported that PI3Kβ is involved in platelet aggregation and a PI3Kβ inhibitor has an antithrombotic effect ("Nature Medicine", 2005, Vol. 11, p. 507-514). With this regard, mice deficient in PI3Kδ or γ are all born normal, and no problem in growth, life span, reproduction, or the like has been found ("Science", 2000, Vol. 287, p. 1040-1046; and "Molecular and Cellular Biology", 2002, Vol. 22, p. 8580-8591). In particular, PI3Kδ is significantly limited to hemocytes and lymphoid tissues in term of its expression, and mice deficient in PI3Kδ were found to have significant damage in activation of lymphocytes. A close relationship between the activation of lymphocytes and immunity/inflammation is well known, and compounds selectively inhibiting the PI3Kδ have a potential to be immunity/inflammatory inhibitors having both of a potent inhibitory action on the activation of lymphocytes and safety.

Interleukin-2 (IL-2) is a kind of cytokine which is mainly produced from activated T cells. IL-2 induces proliferation and activation of lymphocytes via an IL-2 receptor which is a receptor for IL-2. IL-2 is a very important molecule in signaling the activation of an immune system, and its production inhibitors (for example, Tacrolimus and Cyclosporin A) have been used clinically as immunosuppressants. In addition, anti-IL-2 receptor monoclonal antibodies such as Basiliximab and Daclizumab have been used clinically as immunosuppressants.

B cells are one of the main subsets of lymphocytes, along with T cells, and are cells which form a main form of humoral immunity. It is known that humoral immunity plays an extremely important role in preventing infection from pathogens or the like, but in autoimmune diseases such as rheumatoid arthritis and the like, abnormal activation of humoral immunity occurs, which is deeply involved in the pathogenesis. In fact, an anti-CD20 antibody, Rituximab, has been used clinically as a drug for treating rheumatoid arthritis.

As a PI3Kδ-selective inhibitor, a quinazolin-4-one derivative (Patent Documents 1 to 3) has been reported and its usefulness against inflammation, immune diseases, hematologic tumor (leukemia, etc.), and the like has been disclosed. As another PI3Kδ-selective inhibitor, a thiazolyl urea derivative (Patent Document 4) has been reported, and its usefulness against inflammation, immune diseases, or the like has been disclosed.

As triazine and pyrimidine derivatives, the following compounds have been reported. In Patent Documents 5 to 9, it is disclosed that a compound of the formula (A) has an anti-tumor activity. In Patent Document 10 and Non-Patent Document 1, the PI3K inhibitory action of the compound of the formula (A) in the immune system cells has been reported and the usefulness of the compound of the formula (A) as an immunosuppressant was disclosed. However, there is no disclosure of the compound described in the present application and there is no specific description of a PI3Kδ-selective inhibitory action.
(In the formula, R³ represents H, a difluoromethyl group, or the like, and R⁶ represents a ring group such as a morpholino group, a piperidino group, and the like, an amino group which may be substituted with C₁₋₆ alkyl, hydroxy-C₁₋₆ alkyl, morpholino-C₁₋₆ alkyl, or the like. For the other symbols, reference may be made to the publications.)

In Patent Documents 11 to 22, it is disclosed that the compounds of the formulae (B-1) to (B-4) have a PI3K inhibitory action. However, there is no disclosure of the compound described in the present application and there is no description of a PI3Kδ-selective inhibitory action.

### (For the symbols in the formula, reference may be made to the publications.)

In Patent Documents 23 and 24, it is disclosed that a compound represented by the formula (C) has a PI3K inhibitory action. However, there is no disclosure of the compound described in the present application.

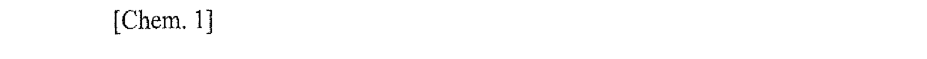

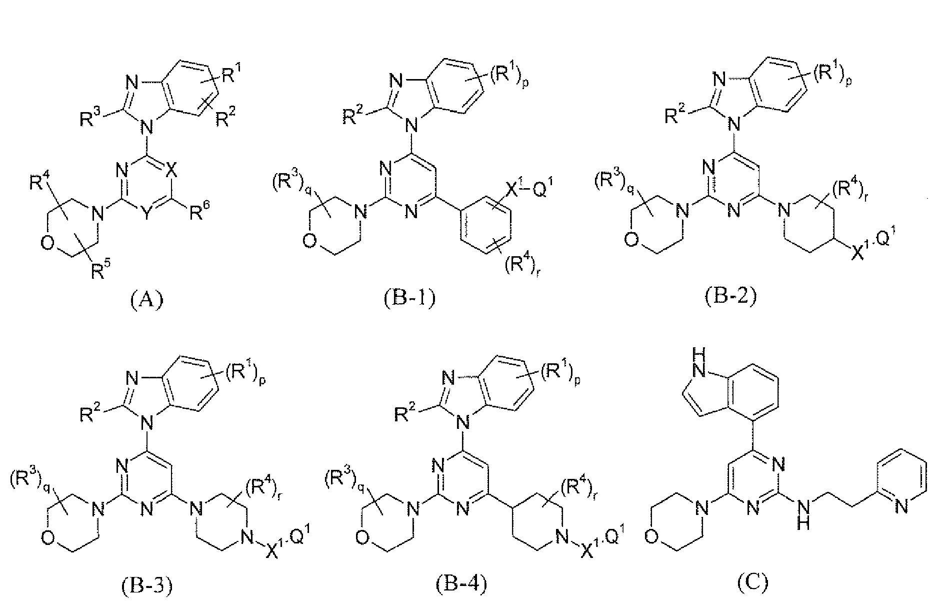

In Non-Patent Document 2, it is suggested that a secondary amine compound of the formula (D) has an Lck inhibitory action and an IL-2 production inhibitory action, and has applications in autoimmune diseases and rejection in organ transplantation. However, there is no description of a PI3K inhibitory action. 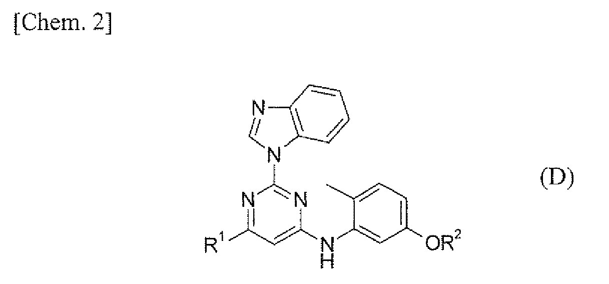 (In the formula, R¹ represents a morpholino group or the like, and R² represents H or methyl.)

### List of the Documents

### Patent Documents

Patent Document 1: Pamphlet of International Publication WO 01/81346
Patent Document 2: Pamphlet of International Publication WO 03/035075
Patent Document 3: Pamphlet of International Publication WO 2005/113556
Patent Document 4: Pamphlet of International Publication WO 2008/000421
Patent Document 5: Specification of European Patent Application Publication No. 1020462
Patent Document 6: Pamphlet of International Publication WO 00/43385
Patent Document 7: Specification of European Patent Application Publication No. 1389617
Patent Document 8: Specification of European Patent Application Publication No. 1557415
Patent Document 9: Specification of European Patent Application Publication No. 1741714
Patent Document 10: Specification of European Patent Application Publication No.1864665
Patent Document 11; Pamphlet of International Publication WO 2008/032027
Patent Document 12: Pamphlet of International Publication WO 2008/032028
Patent Document 13: Pamphlet of International Publication WO 2008/032033
Patent Document 14: Pamphlet of International Publication WO 2008/032036
Patent Document 15: Pamphlet of International Publication WO 2008/032041
Patent Document 16: Pamphlet of International Publication WO 2008/032060
Patent Document 17: Pamphlet of International Publication WO 2008/032064
Patent Document 18: Pamphlet of International Publication WO 2008/032072
Patent Document 19: Pamphlet of International Publication WO 2008/032077
Patent Document 20: Pamphlet of International Publication WO 2008/032086
Patent Document 21: Pamphlet of International Publication WO 2008/032089
Patent Document 22: Pamphlet of International Publication WO 2008/032091
Patent Document 23: Pamphlet of International Publication WO 2007/042810
Patent Document 24: Pamphlet of International Publication WO 2008/125839

### Non-Patent Documents

Non-Patent Document 1: "Journal of the National Cancer Institute", 2006, Vol. 98, p. 545-556
Non-Patent Document 2: "Bioorganic & Medicinal Chemistry Letters", 2006, Vol. 16, p. 5973-5977

### Summary of the Invention

### Problem that the Invention is to Solve

An object of the present invention is to provide a novel compound useful as a pharmaceutical, which can be an agent for preventing or treating rejection in the transplantation of various organs, allergy diseases, autoimmune diseases, hematologic tumor, and the like.

### Means for Solving the Problem

The present inventors have conducted extensive studies on a compound having a PI3Kδ-selective inhibitory action, and/or an IL-2 production inhibitory action, and/or a B cell proliferation inhibitory action (including an activation inhibitory action), and as a result, have found that a novel triazine or pyrimidine derivative has an excellent PI3Kδ-selective inhibitory action, and/or an IL-2 production inhibitory action, and/or a B cell proliferation inhibitory action (including an activation inhibitory action), and can be an agent for preventing or treating rejection in the transplantation of various organs, allergy diseases, autoimmune diseases, hematologic tumor, and the like, thereby completed the present invention.

That is, the present invention relates to the compound of the formula (I) or a salt thereof, and a pharmaceutical composition containing the compound of the formula (I) or a salt thereof and an excipient. 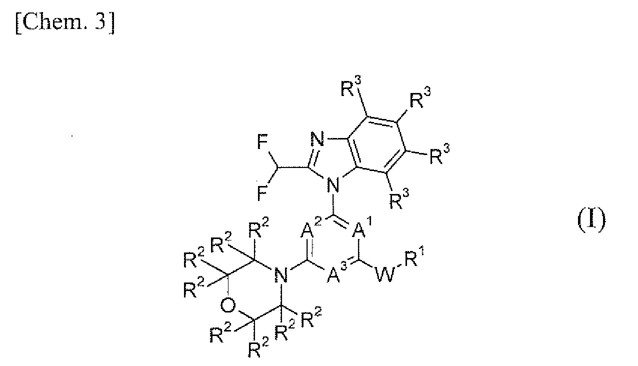 [wherein
A¹ A², and A³: the same as or different from each other, each representing CH or N, provided that at least two of A¹ to A³ are N;
W: NH or O;
R¹: 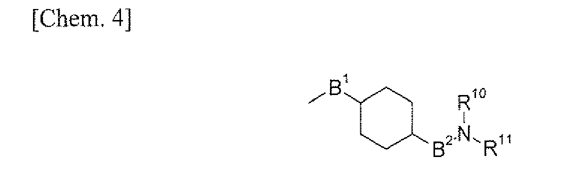 or 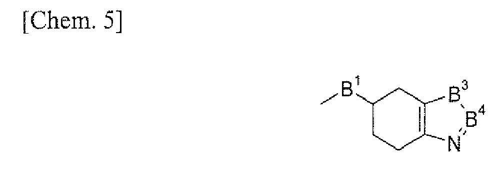
R²: the same as or different from each other, each representing H, or lower alkyl which may be substituted with halogen or -OH;
R³: the same as or different from each other, each representing H or halogen;
B¹: a bond or C₁₋₄ alkylene;
B²: a bond or C₁₋₄ alkylene;
B³: O, S, or NR⁰;
B⁴: CR¹² or N;
R⁰: the same as or different from each other, each representing H or lower alkyl;
R¹⁰: H; lower alkyl, in which the lower alkyl may be substituted with halogen, -C(O)O-lower alkyl, -OH, or -O-lower alkyl; lower alkenyl; lower alkynyl; -lower alkylene-phenyl, in which the phenyl may be substituted with -O-lower alkyl; -lower alkylene-O-lower alkylene-phenyl;
R¹¹: H, R¹⁰⁰, -C(O)R¹⁰¹, -C(O)OR¹⁰², -C(O)NR¹⁰³R¹⁰⁴, or -S(O)2R¹⁰⁵; or R¹⁰ and R¹¹ are combined with the N to which they are bonded to form a 3- to 8-membered monocyclic hetero ring group containing 1 to 4 hetero atoms selected from O, S, and N, and the monocyclic hetero ring may be substituted with lower alkyl which may be substituted with halogen, OH, -O-lower alkyl, or a hetero ring, oxo, -C(O)O-lower alkyl, N(R⁰)₂, halogen, -CN, -OH, -O-lower alkyl, -O-C(O)-lower alkyl, -O-lower alkylene-phenyl, or a hetero ring group;
R¹²: R⁰ or amino;
R¹⁰⁰: lower alkyl, in which the lower alkyl may be substituted with group(s) selected from halogen, -C(O)N(R⁰)₂, -C(O)O-lower alkyl, -CN, -OH, -O-lower alkyl, -O-lower alkylene-phenyl, -NHC(O)O-lower alkylene-phenyl, and -S(O)₂-lower alkyl; lower alkenyl; lower alkynyl;
-X-cycloalkyl, in which the cycloalkyl may be substituted with group(s) selected from lower alkyl, phenyl, -lower alkylene-O-lower alkyl, -O-lower alkyl, and -lower alkylene-phenyl, in which the phenyl may be substituted with -O-lower alkyl;
-X-aryl, in which the aryl may be substituted with group(s) selected from lower alkyl, halogen, halogeno-lower alkyl, phenyl, -CN, -OH, -O-lower alkyl, -O-halogeno-lower alkyl, -O-lower alkylene-OH, -O-lower alkylene-phenyl, -S(O)₂-lower alkyl, -N(R⁰)₂, pyrrolidinyl, piperidyl which may be substituted with OH, morpholinyl, and triazolyl; or
-X-hetero ring group, in which the hetero ring group may be substituted with group(s) selected from lower alkyl, halogen, halogeno-lower alkyl, phenyl, morpholinyl, -C(O)O-lower alkylene-phenyl, -OH, -lower alkylene-phenyl, and -lower alkylene-OH;
R¹⁰¹; lower alkyl, in which the lower alkyl may be substituted with group(s) selected from halogen; -C(O)N(R⁰)₂; -C(O)-piperazinyl, in which the piperazinyl may be substituted with -lower alkylene-OH; -CN; -OH; -O-lower alkyl; -O-lower alkylene-phenyl; -O-lower alkylene-O-lower alkyl; -O-(phenyl which may be substituted with -CN); -S(O)₂-lower alkyl; -S(O)₂-phenyl; -N(R⁰)₂; -N(R⁰)-lower alkyl, in which the lower alkyl may be substituted with -O-lower alkyl; -NH-phenyl; -NHC(O)-lower alkyl; -NHC(O)-phenyl; -NHC(O)-(pyridyl which may be substituted with -OH); -N(R⁰)C(O)O-lower alkyl; -NHC(O)O-low alkylene-phenyl; -NHS(O)₂-phenyl, in which the phenyl may be substituted with group(s) selected from lower alkyl and halogen; and -NHS(O)₂-thicnyl;
-X-cycloalkyl, in which the cycloalkyl may be substituted with group(s) selected from phenyl, -CN, -OH, -O-lower alkyl, and -lower alkylene-OH;
-X-phenyl, in which the phenyl may be substituted with group(s) selected from lower alkyl, halogen, halogeno-lower alkyl, -C(O)O-lower alkyl, -CN, -OH, -O-lower alkyl, -N(R⁰)₂, -N(R⁰)-lower alkylene-OH, -N(-lower alkylene-OH)₂, -NHC(O)-lower alkyl, -N(R⁰)C(O)N(R⁰)₂, -S(O)₂-lower alkyl, -S(O)₂N(lower alkyl)₂, -lower alkylene-OH, -lower alkylene-O-lower alkyl, -X-piperidyl, -X-morpholinyl, and -X-(piperazinyl which may be substituted with lower alkyl);
-X-hetero ring group, in which the hetero ring group may be substituted with group(s) selected from lower alkyl, halogen, -OH, halogeno-lower alkyl, phenyl, -C(O)O-lower alkyl, -C(O)O-lower alkylene-phenyl, -C(O)-(pyridyl which may be substituted with -OH), -C(O)-lower alkyl, oxo, -N(R⁰)₂, -N(R⁰)C(O)O-lower alkyl, -S(O)₂-phenyl, piperidyl which may be substituted with lower alkyl, -X-pyridyl, -lower alkylene-phenyl, -lower alkylene-OH, -lower alkylene-O-lower alkyl, and -lower alkylene-(pyrazolyl which may be substituted with lower alkyl); or
-C(O)N(R⁰)₂;
R¹⁰²_{:} lower alkyl;
R¹⁰³:H or lower alkyl;
R¹⁰⁴: lower alkyl, in which the lower alkyl may be substituted with group(s) selected from -CN, -OH, -O-lower alkyl, or -N(R⁰)₂
-X-phenyl, in which the phenyl may be substituted with group(s) selected from lower alkyl, halogen, halogeno-lower alkyl, -CN, -O-lower alkyl, -O-halogeno-lower alkyl, and -N(R⁰)₂; or
-X-hetero ring group;
or R¹⁰³ and R¹⁰⁴ are combined with the N to which they are bonded to form a morpholinyl group;
R¹⁰⁵: lower alkyl, in which the lower alkyl may be substituted with group(s) selected from halogen, and -O-phenyl, in which the phenyl may be substituted with -O-lower alkyl; or hetero ring group;
lower alkenyl;
-X-cycloalkyl;
-X-aryl, in which the aryl may be substituted with group(s) selected from lower alkyl, halogen, halogeno-lower alkyl, phenyl, -C(O)O-lower
   alkyl, -C(O)N(R⁰)₂, -CN, -C(O)-lower alkyl, -C(O)-pyridyl, -O-lower alkyl, -O-halogeno-lower alkyl, -O-cycloalkyl, -O-phenyl, -O-lower alkylene-CN, -X-NHC(O)-lower alkyl, -NHC(O)-morpholinyl, -S(O)₂-lower alkyl, -N(R⁰)C(O)N(R⁰)₂, -S(O)₂N(R⁰)₂,
   and -S(O)₂-morpholinyl;
-X-hetero ring group, in which the hetero ring group may be substituted with lower alkyl, halogen, halogeno-lower alkyl, phenyl, -C(O)-lower alkyl, -C(O)-halogeno-lower alkyl, -C(O)-cyclaalkyl, -O-lower alkyl, -O-phenyl, oxo, -NHC(O)-lower alkyl, morpholinyl, and isoxozolyl; or
-N(R⁰)₂; and
X: a bond or lower alkylene].
In the present specification, the symbols defined above are used with the same meanings unless otherwise specifically mentioned.

Further, the present invention relates to a pharmaceutical composition for preventing or treating rejection in the transplantation of various organs, an allergy disease, an autoimmune disease, a hematologic tumor, or the like, containing the compound of the formula (I) or a salt thereof, that is, an agent for preventing or an agent for treating rejection in the transplantation of various organs, an allergy disease, an autoimmune disease, a hematologic tumor, or the like, containing the compound of the formula (I) or a salt thereof.

In addition, the present invention relates to use of the compound of the formula (I) or a salt thereof for the manufacture of a pharmaceutical composition for preventing or treating rejection in the transplantation of various organs, an allergy disease, an autoimmune disease, a hematologic tumor, or the like.

Further, the present invention relates to a method for preventing or treating rejection in the transplantation of various organs, an allergy disease, an autoimmune disease, a hematologic tumor, or the like, containing administering to a patient an effective amount of the compound of the formula (I) or a salt thereof.

In addition, the present invention relates to a PI3Kδ-selective inhibitor and/or a IL-2 production inhibitor containing the compound of the formula (I) or a salt thereof.

Furthermore, the present invention relates to a method for preparing a pharmaceutical composition for preventing or treating rejection in the transplantation of various organs, an allergy disease, an autoimmune disease, a hematologic tumor, or the like, including mixing a compound of the formula (I) or a salt thereof, and a pharmaceutically acceptable carrier, solvent, or excipient.

Moreover, the present invention relates to a commercial package including a pharmaceutical composition containing the compound of the formula (I) or a salt thereof, and a description that the compound of the formula (I) or a salt thereof can be used or should be used for treating or preventing rejection in the transplantation of various organs, an allergy disease, an autoimmune disease, a hematologic tumor, or the like.

### Effects of the Invention

Since the compound of the formula (I) has a PI3Kδ-selective inhibitory action, and/or an IL-2 production inhibitory action, and/or a B cell proliferation inhibitory action (including an activation inhibitory action), it can be used as an agent for preventing or treating rejection in the transplantation of various organs, allergy diseases, autoimmune diseases, hematologic tumor, or the like.

### Best Mode for Carrying out the Invention

Hereinafter, the present invention will be described in more detail.
In the definition of the present specification, "alkyl", "alkenyl", "alkynyl", and "alkylene" mean linear or branched hydrocarbon chains, unless otherwise specifically mentioned.

The "lower alkyl" refers to alkyl having 1 to 7 carbon atoms (hereinafter referred to as C₁₋₇), in another embodiment, alkyl having 1 to 6 carbon atoms (hereinafter referred to as C₁₋₆), for example, a methyl group, an ethyl group, an n-propyl group, an isopropyl group, an n-butyl group, an isobutyl group, a sec-butyl group, a tert-butyl group, an n-pentyl group, an n-hexyl group, or the like. In a further embodiment, it is C₁₋₄ alkyl, and in a further embodiment, a methyl group, an ethyl group, an n-propyl group, an isopropyl group, or a tert-butyl group.

The "lower alkenyl" refers to linear or branched C₂₋₆ alkenyl, for example, vinyl, propenyl, butenyl, pentenyl, 1-methylvinyl, 1-methyl-2-propenyl, 1,1-dimethyl-2-propenyl, 1,3-butadienyl, 1,3-pentadienyl, or the like. In another embodiment, it is C₂₋₄ alkenyl, and in a further embodiment, vinyl, propenyl, butenyl, pentenyl, 1-methylvinyl, 1 -methyl-2-propenyl, or 1,1-dimethyl-2-propenyl.

The "lower alkynyl" refers to-linear or branched C₂₋₆ alkynyl, for example, ethynyl, propynyl, butynyl, pentynyl, 1-methyl-2-propynyl, 1,3-butadiynyl, 1,3-pentadiynyl, or the like. In another embodiment, it is C₂₋₄ alkynyl, and in a further embodiment, a propynyl group, a butynyl group, a pentynyl group, or a 1-methyl-2-propynyl group.

The "lower alkylene" refers to C₁₋₆ alkylene, for example, a methylene group, an ethylene group, a trimethylene group, a tetramethylene group, a pentamethylene group, a hexamethylene group, a propylene group, a methylmethylene group, an ethylethylene group, a 1,2-dimethylethylene group, a 1,1,2,2-tetramethylethylene group, or the like. In another embodiment, it is C₁₋₅ alkylene, and in a further embodiment group, a methylene group, an ethylene group, a trimethylene group, a tetramethylene group, or a pentamethylene group.

The "halogen" means F, Cl, Br, or I.

The "halageno-lower alkyl" refers to lower alkyl substituted with one or more halogen atoms. In another embodiment, it is lower alkyl substituted with 1 to 5 halogen atoms, and in a further embodiment, a trifluoromethyl group.

The "cycloalkane" refers to a C₃₋₁₀ saturated hydrocarbon ring, which may have a bridge.

The "cycloalkyl" refers to a C₃₋₁₀ saturated hydrocarbon ring group formed by removal of one hydrogen atom from cycloalkane, which may have a bridge. Examples thereof include a cyclopropyl group, a cyclobutyl group, a cyclopentyl group, a cyclohexyl group, a cycloheptyl group, a cyclooctyl group, a bicyclo[2.2.1]heptyl group, a bicyclo[2.2.2]octyl group, an adamantyl group, and the like. In another embodiment, it is C₃₋₈ cycloalkyl, and in a further embodiment, a cyclopropyl group, a cyclobutyl group, a cyclopentyl group, and a cyclohexyl group.

The "cycloalkene" refers to C₄₋₁₅ cycloalkene.

The "cycloalkenyl" refers to C₄₋₁₅ cycloalkenyl formed by removal of one hydrogen atom from cycloalkene.

The "aryl" is a C₆₋₁₄ monocyclic to tricyclic aromatic hydrocarbon ring group, and includes a ring group condensed with C₅₋₈ cycloalkene at a site of a double bond thereof. For example, it is a phenyl group, a naphthyl group, a tetrahydronaphthalenyl group, an indanyl group, an indenyl group, a fluorenyl group, and the like. In another embodiment, it is a phenyl group, a naphthyl group, and an indanyl group, and in a further embodiment, a phenyl group.

The "hetero ring" group means a ring group selected from i) a monocyclic 3- to 8-membered, and in another embodiment, a 5- to 7-membered, hetero ring containing 1 to 4 hetero atoms selected from O, S, and N, and ii) a bicyclic to tricyclic hetero ring containing 1 to 5 hetero atoms selected from O, S, and N, which is formed by the condensation of the monocyclic hetero ring and one or two rings selected from the group consisting of a monocyclic hetero ring, a benzene ring, C₅₋₈ cycloalkane, and C₅₋₈ cycloalkene. The ring atom S or N may be oxidized to form an oxide or a dioxide, may have a bridge, or may form a spiro ring.
Examples of the "hetero ring" group include an aziridinyl group, an azetidyl group, a pyrrolidinyl group, a piperidyl group, an azepanyl group, an azocanyl group, a piperazinyl group, a homopiperazinyl group, an oxiranyl group, an oxetanyl group, a tetrahydrofuranyl group, a tetrahydropyranyl group, a tetrahydrothiofuranyl group, a tetrahydrothiopyranyl group, a morpholinyl group, a homomorpholinyl group, an isothiazolidinyl group, a thiomorpholinyl group, a pyrrolyl group, an indolyl group, an imidazolyl group, a pyrazolyl group, a pyridyl group, a pyrimidinyl group, a pyrazinyl group, a triazolyl group, a tetrazolyl group, a furyl group, a thienyl group, an oxozolyl group, an isoxazolyl group, an oxadiazolyl group, a thiazolyl group, a thiadiazolyl group, a dihydrobenzothiophenyl group, a benzimidazolyl group, a tetrahydrobenzimidazolyl group, a dihydrobenzoxazolyl group, a benzoisoxazolyl group, a quinolyl group, a tetrahydroquinolinyl group, a tetrahydroisoquinolinyl group, a quinazolyl group, a quinoxalinyl group, a benzofuranyl group, a benzothiophenyl group, a benzoxazolyl group, a benzothiazolyl group, a dihydrobenzothiazolyl group, a tetrahydrobenzothiazolyl group, a carbazolyl group, an indolyl group, an indolinyl group, a tetrahydroquinolinyl group, a tetrahydroisoquinolinyl group, a quinuclidinyl group, a dibenzofuranyl group, a dibenzofuranyl group, a 1,3-benzodioxol-5-yl group, a chromanyl group, a dihydrobenzoxadinyl group, and 1,4-benzodioxinyl group. In another embodiment, it is a 5- to 10-membered monocyclic to bicyclic hetero ring group. In a further embodiment, azetidyl, pyrrolidinyl, piperidyl, azepanyl, azocanyl, piperazinyl, tetrahydrofuranyl, tetrahydropyranyl, tetrahydrothiofuranyl, tetrahydrothiopyranyl, morpholinyl, homomorpholinyl, isothiazolidinyl, thiomorpholinyl, imidazolyl, pyrazolyl, pyridyl, pyrimidinyl, pyrazinyl, triazolyl, tetrazolyl, furyl, thienyl, oxazolyl, isoxazolyl; thiazolyl, thiadiazolyl, indolyl, indolinyl, dihydrobenzothiophenyl, benzimidazolyl, tetrahydrobenzimidazolyl, dihydrobenzoxazolyl, benzoisoxazolyl, benzothiazolyl, dihydrobenthiazolyl, tetrahydrobenzothiazolyl, quinolyl, tetrahydroquinolinyl, tetrahydroisoquinolinyl, chromanyl, dihydrobenzoxadinyl, and 1,4-benzodioxinyl.

The "saturated hetero ring" group means a group of the "hetero ring" group above, in which the bonds constituting the ring include only single bond.
Examples of the "saturated hetero ring" group include an azetidyl group, a pyrrolidinyl group, a piperidyl group, an azepanyl group, an azocanyl group, a piperazinyl group, a tetrahydrofuranyl group, a tetrahydropyranyl group, a tetrahydrothiofuranyl group, a tetrahydrothiopyranyl group, a morpholinyl group, an isothiazolidinyl group, and a thiomorpholinyl group.

In the present specification, the expression "which may be substituted" represents unsubstituted or substituted with 1 to 5 substituents. Further, if it has a plurality of substituents, the substituents may be the same as or different from each other.

The "PI3Kδ-selective inhibitor" means an inhibitor having a PI3Kα inhibitory activity showing an IC₅₀ value which is 10-fold or more higher, in another embodiment, 30-fold or more higher, and in a further embodiment, 100-fold or more higher than that of a PI3Kδ inhibitory activity.

Embodiments of the compound of the formula (I) of the present invention are presented below.
(1) The compound, wherein A³ is N, in another embodiment, the compound, wherein A¹, A², and A³ are N, in a further embodiment, the compound, wherein A¹ is CH and A² and A³ are N, or wherein A² is CH and A¹ and A³ are N, in a further embodiment, the compound, wherein A¹ is CH and A² and A³ are N, and in a further embodiment, the compound, wherein A² is CH and A¹ and A³ are N.
(2) The compound, wherein W is NH, and in another embodiment, the compound, wherein W is O.
(3) The compound, wherein R¹ is: 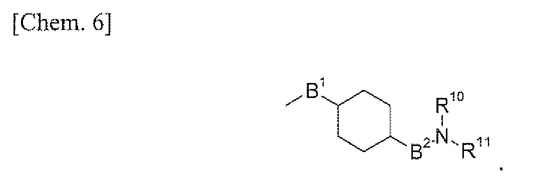
(4) The compound, wherein R² are the same as or different from each other and represent H, or lower alkyl which may be substituted with halogen or -OH, in another embodiment, the compound, wherein R² are the same as or different from each other and represent H or lower alkyl, in a further embodiment, the compound, wherein R² is H, in a further embodiment, the compound, wherein R² is lower alkyl, and in a further embodiment, the compound, wherein R² is lower alkyl which may be substituted with halogen or -OH.
(5) The compound, wherein R³ is H.
(6) The compound, wherein B¹ is a bond, in another embodiment, the compound, wherein B¹ is C₁₋₄ alkylene, in a further embodiment, the compound, wherein B¹ is methylene, and in a further embodiment, the compound, wherein B¹ is a bond or methylene.
(7) The compound, wherein B² is a bond, in another embodiment, the compound, wherein B² is C₁₋₄ alkylene, in a further embodiment, the compound, wherein B² is methylene, and in a further embodiment, the compound, wherein B² is a bond or methylene.
(8) The compound, wherein R¹⁰ is H, lower alkyl which may be substituted with halogen or -OH, -lower alkylene-O-lower alkyl, lower alkenyl, lower alkynyl, -lower alkylene-phenyl, or -lower alkylene-O-lower alkylene-phenyl, in which the phenyl may be substituted with -O-lower alkyl, in another embodiment, the compound, wherein R¹⁰ is H, lower alkyl, or -lower alkylene-O-lower alkyl, in a further embodiment, the compound, wherein R¹⁰ is H, in a further embodiment, the compound, wherein R¹⁰ is lower alkyl, and in a further embodiment, the compound, wherein R¹⁰ is -lower alkylene-O-lower alkyl.
(9) The compound, wherein R¹¹ is R¹⁰⁰ or -C(C)R¹⁰¹, in another embodiment, the compound, wherein R¹¹ is R¹⁰⁰, in a further embodiment, the compound, wherein R¹¹ is -C(O)R¹⁰¹, in a further embodiment, the compound, wherein R¹¹ is -C(O)OR¹⁰², in a further embodiment, the compound, wherein R¹¹ is -C(O)NR¹⁰³R¹⁰⁴, and in a further embodiment, the compound, wherein R¹¹ is -S(O)₂R¹⁰⁵.
(10) The compound, wherein R¹⁰ and R¹¹ are combined with the N to which they are bonded to form a 3- to 8-membered monocyclic hetero ring group containing 1 to 4 hetero atoms selected from O, S, and N, and the monocyclic hetero ring may be substituted with lower alkyl, oxo, halogeno-lower alkyl, -lower alkylene-OH, -C(O)O-lower alkyl, -C(O)NR¹⁰³R¹⁰⁴, N(R⁰)₂, halogen, -CN, -OH, -O-lower alkyl, -lower alkylene-O-lower alkyl, or a hetero ring group, and in another embodiment, the compound, wherein R¹⁰ and R¹¹ are combined with the N to which they are bonded to form a 3- to 8-membered monocyclic hetero ring group containing 1 to 4 heteroatoms selected from O, S, and N, and the monocyclic hetero ring is lower alkyl or oxo.
(11) The compound, wherein R¹⁰⁰ is lower alkyl which may be substituted with group(s) selected from the group consisting of -OH, halogen, and -O-lower alkyl, and in another embodiment, the compound, wherein R¹⁰⁰ is lower alkyl which may be substituted with group(s) selected from the group consisting of halogen, and -O-lower alkyl.
(12) The compound, wherein R¹⁰¹ is lower alkyl which may be substituted with group(s) selected from the group consisting of halogen, -OH, -O-lower alkyl, and -N(R⁰)₂.
(13) The compound, which is a combination of two or more groups as described in (1) to (12), or a pharmaceutically acceptable salt thereof.

Specific examples of the compound of (13) above include the following compounds.
(14) The compound as described in (3), wherein A¹ is CH and A² and A³ are N, or wherein A² is CH and A¹ and A³ are N.
(15) The compound as described in (14), wherein B¹ is a bond or methylene, and B² is a bond.
(16) The compound as described in (15), wherein R² are the same as or different from each other and represent H or lower alkyl.
(17) The compound as described in (16), wherein R³ is H.
(18) The compound as described in (17), wherein R¹⁰ is H, lower alkyl which may be substituted with halogen or -OH, -lower alkylene-O-lower alkyl, lower alkenyl, lower alkynyl, -lower alkylene-phenyl, or -lower alkylene-O-lower alkylene-phenyl, in which the phenyl may be substituted with -O-lower alkyl.
(19) The compound as described in (17), wherein R¹⁰ is H, lower alkyl, or -lower alkylene-O-lower alkyl.
(20) The compound as described in (18) or (19), wherein R¹¹ is R¹⁰⁰ or -C(O) R¹⁰¹.
(21) The compound as described in (20), wherein R¹⁰⁰ is lower alkyl which may be substituted with group(s) selected from the group consisting of -OH, halogen, and -O-lower alkyl.
(22) The compound as described in (20), wherein R¹⁰⁰ is lower alkyl which may be substituted with group(s) selected from the group consisting of halogen and -O-lower alkyl.
(23) The compound as described in (20), wherein R¹⁰¹ is lower alkyl which may be substituted with group(s) selected from the group consisting of halogen, -OH, -O-lower alkyl, and -N(R⁰)₂.
(24) The compound as described in (17), wherein R¹⁰ and R¹¹ are combined with the N to which they are bonded to form a 3- to 8-membered monocyclic hetero ring group containing 1 to 4 hetero atoms selected from O, S, and N, and the monneyclic hetero ring may be substituted with lower alkyl, oxo, halogeno-lower alkyl, -lower alkylene-OH, -C(O)O-lower alkyl, -C(O)NR¹⁰³R¹⁰⁴, N(R⁰)₂, halogen, -CN, -OH, -O-lower alkyl, -lower alkylene-O-lower alkyl, or a hetero ring group.
(25) The compound as described in (17), wherein R¹⁰ and R¹¹ are combined with the N to which they are bonded to form a 3- to 8-membered monocyclic hetero ring group containing 1 to 4 hetero atoms selected from O, S, and N, and the monocyclic hetero ring may be substituted with lower alkyl or oxo.
(26) The compound represented by the formula (I) or a pharmaceutically acceptable salt thereof, which is selected from the group consisting of:
   N-[trans-4-({6-[2-(difluoromethyl)-1H-benzimidazol-1-yl]-2-morpholin-4-ylpyrimidin-4-yl}oxy)cyclohexyl]-N,N-dimethylglycinamide,
   N-[trans-4-({6-[2-(difluoromethyl)-1H-benzimidazol-1-yl]-2-morpholin-4-ylpyrimidin-4-yl}amino)cyclohexyl]-N,N-dimethylglycinamide,
   4-[2-(difluoromethyl)-1H-benzimidazol-yl]-N-({trans-4-[(2-fluoroethyl)(methyl)amino]cyclohexyl}mcthyl)-6-morpholin-4-ylpyrimidn-2-amine,
   4-[2-(difluoromethyl)-1H-benzimidazol-1-yl]-N-({trans-4-[(2-methoxyethyl)(methyl)amino]cylohexyl}methyl)-6-morpholin-4-ylpyrimidin-2-amine,
   6-[2-(difluoromethyl)-1H-benzimidazol-1-yl]-2-morpholin-4-yl-N-[(trans-4-morpholin-4-ylcyclohexyl)methyl]pyrimidin-4-amine,
   1-[{trans-4-[({4-[2-(difluoromethyl)-1H-benzimidazol-1-yl]-6-[(3S)-3-mcthylmorpholin-4-yl]pyrimidin-2-yl}amino)methyl]cyclohexyl}(methyl)amino]-2-methylpropan-2-ol,
   1-[{trans-4-[({4-[2-(difluoromethyl)-1H-benzimidazol-1-yl]-6-[(3S)-3-methylmorpholin-4-yl]pyrimidin-2-yl}amino)methyl]cyclohexyl}(ethyl)amino]-2-methylpropan-2-ol,
   4-[2-(difluoromethyl)-1H-benzimidazol-1-yl]-N-({trans-4-[ethyl(1-methoxypropan-2-yl)amino]cyclohexy}methyl)-6-(morpholin-4-yl)pyrimidin-2-amine,
   4-[2-(difluoromethyl)-1H-benzimidazol-1-yl]-N-{[trans-4-(dipropylamino)cyclohexyl]methyl}-6-(morpholin-4-yl)pyrimidin-2-amine,
   3-[{trans-4-[({4-[2-(difluoromethyl)-1H-benzimidazol-1-yl]-6-(morpholin-4-yl)pyrimidin-2-yl}amino)methyl]cyclohexyl}(methyl)amino]-2-methylbutan-2-ol,
   6- [2-(difluoromethyl)-1H-benzimidazol-1-yl]-N-({trans-4-[(3S)-3-fluoropyrrolidin-1-yl]cyclohcxyl}methyl)-2-(morpholin-4-yl)pyrimidin-4-amine,
   3-[{trans-4-[({4-[2-(difluoromethyl)-1H-benzimidazol-1-yl]-6-[(3S)-3-methylmorpholin-4-yl]pyrimidin-2-yl}amino)methyl]cyclohexyl}(methyl)amino]-2-methylbutan-2-ol,
   3-[{trans-4-[({4-[2-(difluoromethyl)-1H-benzimidazol-1-yl]-6-[(3R)-3-methylmorpholin-4-yl]pyrimidin-2-yl}amino)methyl]cyclohexyl}(methyl)amino]-2-methylbutan-2-ol,
   4-[2-(difluoromethyl)-1H-benzimidazol-1-yl]-N-({trans-4-[(1-methoxypropan-2-yl)(methyl)amino]cyclohexyl}methyl)-6-[(3R)-3-methylmorpholin-4-yl]pyrimidin-2-amine,
   4-[2-(dinuoromethyl)-1H-benzimidazol-1-yl]-N-({trans-4-[ethyl(1-methoxypropan-2-yl)amino]cyclohexyl}methyl)-6-[(3R)-3-methylmorpholin-4-yl]pyrimidin-2-amine,
   4-[2-(difluoromethyl)-1H-benzimidazol-1-yl]-N-({trans-4-[(2S)-2-(fluoramethyl)pyrrolidin-1-yl]cyelohexyl}methyl)-6-(morpholin-4-yl)pyrimidin-2-amine, and
   6-[2-(difluoromethyl)-1H-benzimidazol-1-yl]-N-({trans-4-[(2S)-2-(fluoromethyl)azetidin-1-yl]cyclohexyl)methyl)-2-(morpholin-4-yl)pyrimidin-4-amine.

The compound of the formula (I) may in some cases exist in the form of tautamers or geometrical isomers, depending on the kind of substituents. In the present specification, the compound of the formula (I) may be described only in one form of the isomers, but the present invention includes other isomers as well as isolated forms or mixtures thereof.

Further, the compound of the formula (I) may have asymmetric carbon atoms or axial asymmetries in some cases, and correspondingly, it may exist in the form of optical isomers. The present invention also includes isolates or mixtures of optical isomers of the compound of the formula (I).

Further, the present invention includes a pharmaceutically acceptable prodrug of the compound of the formula (I). The pharmaceutically acceptable prodrug is a compound having a group which can be converted into an amino group, a hydroxyl group, a carboxyl group or the like by solvolysis or under a physiological condition. Examples of the group which forms a prodrug include the groups as described, for example, in Prog. Med., 5, 2157-2161 (1985) or "Pharmaceutical Research and Development" (Hirokawa Publishing Company, 1990), Vol. 7, "Drug Design", pp. 163-198.

In addition, in some cases, the compound of the formula (I) may form an acid addition salt or salt with a base, depending on the kind of substituents, and the salt is included in the present invention as long as it is a pharmaceutically acceptable salt. Specifically, examples thereof include acid addition salts with inorganic acids such as hydrochloric acid, hydrobromic acid, hydroiodic acid, sulfuric acid, nitric acid, and phosphoric acid, or with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, and glutamic acid, salts with inorganic bases such as sodium, potassium, magnesium, calcium, and aluminum, or with organic bases such as methylamine, ethylamine, ethanolamine, lysine, and ornithine, salts with various amino acids and amino acid derivatives such as acetylleucine, ammonium salts, and the like.

Further, the present invention also includes various hydrates or solvates, and polymorphic crystal substances of the compound of the formula (I) and a pharmaceutically acceptable salt thereof. Further, the present invention also includes compounds labeled with various radioactive or non-radioactive isotopes.

### (Production Processes)

The compound of the formula (I) and a pharmaceutically acceptable salt thereof can be produced by utilizing the characteristics based on the types of its basic skeleton or substituents and by applying various known synthetic methods. At this time, it is in some cases effective, in terms of production techniques, that the functional group is replaced with an appropriate protecting group (a group that can be easily converted into the functional group) in the stage of a starting material to intermediate depending on the type of the functional group. Examples of such functional groups include an amino group, a hydroxyl group, a carboxyl group, and the like, and examples of such protecting groups include protecting groups described for example in "Protective Groups in Organic Synthesis (the third edition, 1999)" edited by Greene and Wuts, or the like, which may be appropriately selected and used depending on the reaction conditions. In these methods, a desired compound can be obtained by introducing the protecting group and carrying out the reaction, and then removing the protecting group, if desired.
In addition, the prodrug of the compound of the formula (I) can be produced in the same manner as the case of the protecting groups, by carrying out the reaction after introducing a specific group at the stage of starting materials to intermediates or using the compound of the formula (I) obtained. The reaction can be carried out by applying methods known to those skilled in the art, such as the usual esterification, amidation, dehydration and the like.
Hereinbelow, representative production processes of the compound of the formula (I) are explained. Each production process may be carried out with reference to the references attached to this description. In this regard, the production processes of the present invention are not limited to the following examples.

### (Production Process 1)

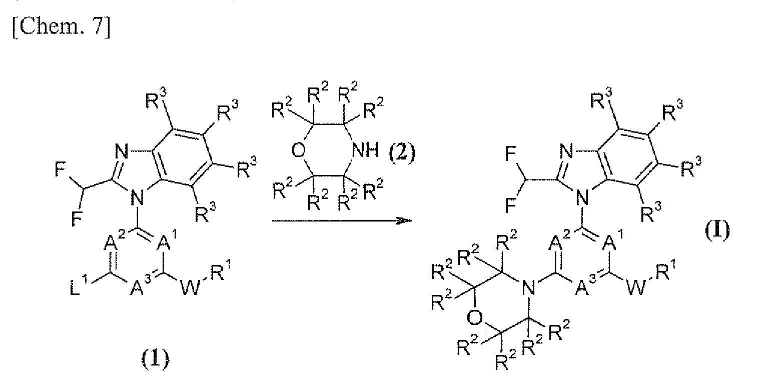

(In the formula, L¹ represents a leaving group. The same shall apply hereinafter).
The compound of the formula (I) can be obtained by the reaction of a compound (1) with a compound (2). Examples of the leaving group include halogen, methylsulfinyl, and methylsulfonyl groups.
In this reaction, the compound (1) and the compound (2) are used in an equivalent amount, or with either thereof in an excess amount, and a mixture thereof is stirred under from cooling to heating and refluxing, preferably at 0°C to 100°C, usually for 0.1 hour to 5 days, in a solvent inert to the reaction or without a solvent. The solvent used herein is not particularly limited, but examples thereof include aromatic hydrocarbons such as benzene, toluene, xylene, and the like, ethers such as diethylether, tetrahydrofuran, dioxane, dimethoxyethane, and the like, halogenated hydrocarbons such as dichloromethane, 1,2-dichloroethane, chloroform, and the like, N,N-dimethylformamide, N,N-dimethylacetamide, dimethylsulfoxide, ethyl acetate, acetonitrile, and a mixture thereof. It may be advantageous in some cases for the smooth progress of the reaction to carry out the reaction in the presence of an organic base such as triethylamine, N,N-diisopropylethylamine, N-methylmorpholine, and the like, or an inorganic base such as potassium carbonate, sodium carbonate, cesium carbonate, potassium hydroxide, and the like.

### [References]

"Organic Functional Group Preparations" by S. R. Sandler and W. Karo, 2nd Ed., Vol. 1, Academic Press Inc., 1991
"The 5th Ed., Jikken Kagaku Koza (Courses in Experimental Chemistry) (Vol. 14)", edited by The Chemical Society of Japan, Maruzen, 2005

### (Production Process 2)

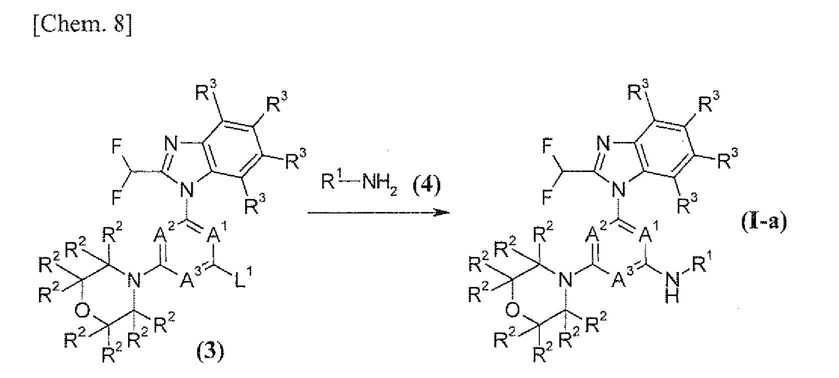

A compound of the formula (I-a) can be obtained by the reaction of a compound (3) with a compound (4). The reaction conditions are the same as in Production Process 1.

Various substituents of R¹ and R² groups in the compound of the formula (I) can be easily converted to other functional groups by using the compound of the formula (I) as a starting material by means of the reactions described in Examples as described later, reactions apparent to a person skilled in the art, or modified methods thereof. For example, processes that can be usually employed by a person skilled in the art, such as O-alkylation, N-alkylation, reduction, hydrolysis, amidation, and the like can be arbitrarily combined and performed.

### (Preparation of Starting Compounds)

The starting compound in the production processes above can be prepared by, for example, the following method, the method described in Preparation Examples as described later, known methods, or modified methods thereof.

### (Starting Material Synthesis 1)

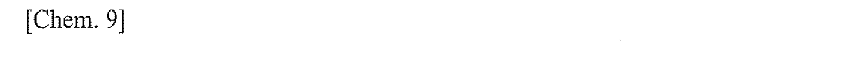

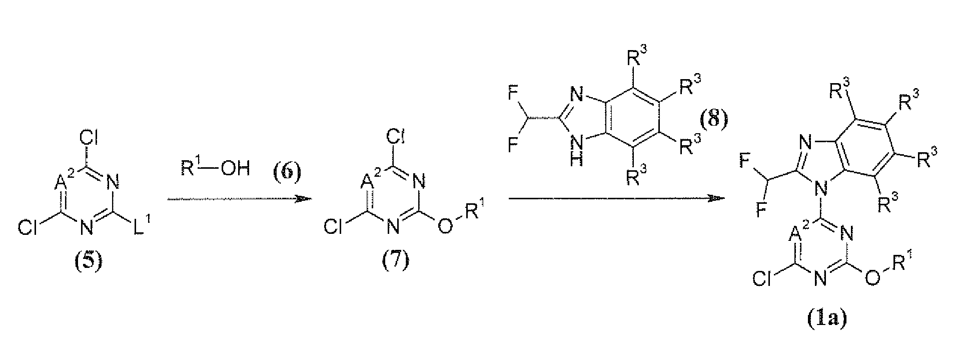

A compound of the formula (7) can be obtained by the reaction of a compound (5) with a compound (6). In this reaction, the compound (5) and the compound (6) are used in an equivalent amount, or with either thereof in an excess amount, and a mixture thereof is stirred under from cooling to heating and refluxing, usually for 0.1 hour to 5 days, in a solvent inert to the reaction or without a solvent, in the presence of a base. The solvent used herein is not particularly limited, but examples thereof include aromatic hydrocarbons such as benzene, toluene, xylene, and the like, ethers such as diethylether, tetrahydrofuran, dioxane, dimethoxyethane, and the like, halogenated hydrocarbons such as dichloromethane, 1,2-dichloroethane, chloroform, and the like, N,N-dimethylformamide, N,N-dimethylacetamide, dimethylsulfoxide, ethyl acetate, acetonitrile, and a mixture thereof. Examples of the base include organic bases such as triethylamine, N,N-diisopropylethylamine, N-methylmorpholine, and the like, or inorganic bases such as potassium carbonate, sodium carbonate, cesium carbonate, sodium hydrogen carbonate, potassium hydroxide, sodium hydride and the like. It may be advantageous in some cases for the smooth progress of the reaction to add a crown ether and the like.
A compound (1a) can be obtained by the reaction of the compound (7) with a compound (8) in the presence of a base.

### (Starting Material Synthesis 2)

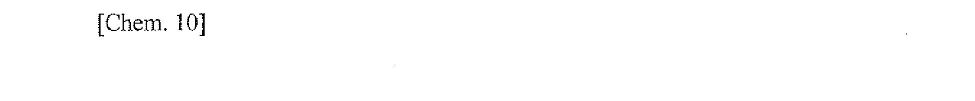

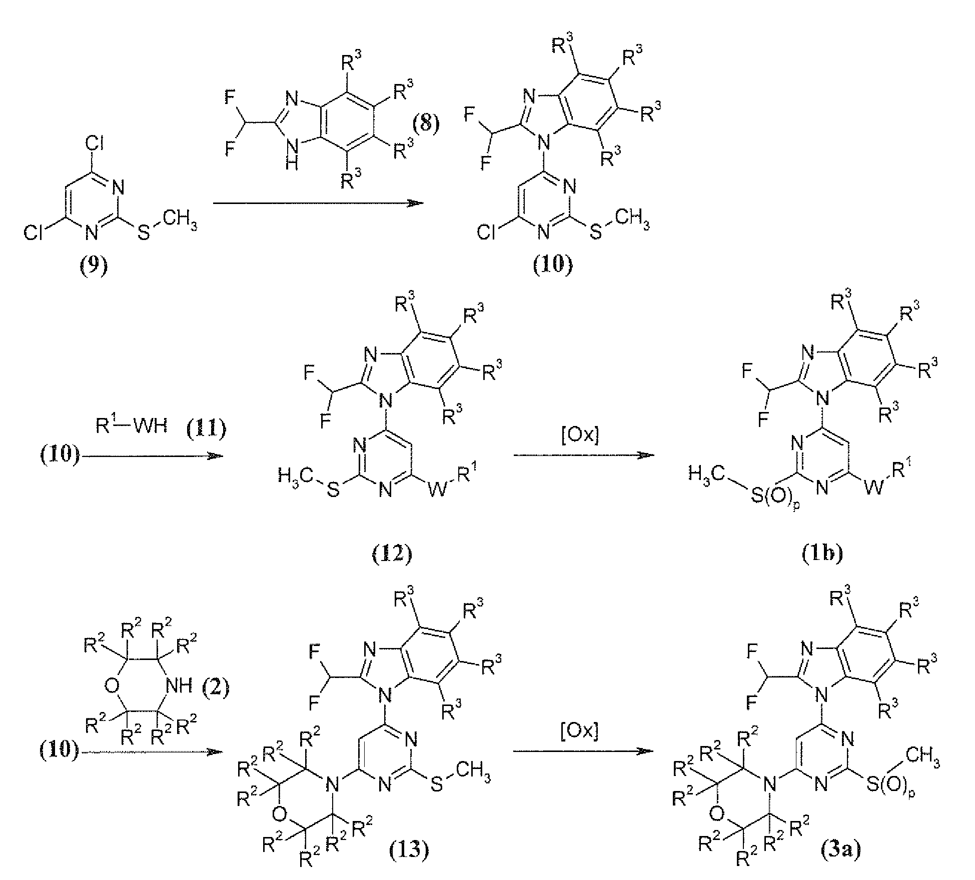

(In the formula, Ox represents an oxidant and p represents an integer of 1 or 2. The same shall apply hereinafter).
A compound (10) can be obtained by the reaction of a compound (9) with the compound (8) in the presence of a base.
A compound (12) can be obtained by the reaction of the compound (10) with a compound (11) in the presence of a base. It may be advantageous in some cases for the smooth progress of the reaction to heat the reaction mixture by radiation with microwaves.
A compound (1b) can be obtained by the oxidation reaction of the compound (12). The oxidation reaction can be carried out using the compound (12) and an oxidant such as m-chloropcrbenzoic acid, peracetic acid, aqueous hydrogen peroxide, and the like, in an equivalent amount, or with either thereof in an excess amount, under from cooling to heating and refluxing. As the solvent, solvents such as aromatic hydrocarbons, halogenated hydrocarbons, and the like can be used singly or in a combination of two or more kinds thereof.
Further, a compound (13) can be obtained by the reaction of the compound (10) with the compound (2) under the same reaction condition as above, and subsequently, a compound (3a) can be obtained therefrom.

A further starting compound (3) can be prepared, for example, with reference to the method described in the following documents: WO2002/088112, EP1389617, WO2008/032033, WO2008/032036, WO2008/032041, or WO2008/032060

The compound of the formula (I) can be isolated and purified as its free compound, pharmaceutically acceptable salt, hydrate, solvate, or polymorphic substance. The pharmaceutically acceptable salt of the compound of the formula (I) can also be prepared by carrying out a conventional salt formation reaction.
Isolation and purification are carried out by employing general chemical operations such as extraction, fractional crystallization, various types of fractionation chromatography, and the like.
Various isomers can be prepared by selecting an appropriate starting compound or separated by making use of the difference in the physicochemical properties between the isomers. For example, the optical isomers are obtained by means of general optical resolution methods of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) was confirmed by the tests shown below.

### 1. PI3Kδ Enzyme Inhibitory Activity

For the experiment, a PI3-Kinase HTRF Assay kit (Millipore Corporation, Catalogue No. 33-016) and a human PI3Kδ enzyme (Millipore Corporation, Catalogue No. 14-604) were used. The measurement method was in accordance with the appended instructions. The overview thereof is as follows.
PI3Kδ (10 ng/well), phosphatidylinositol-4,5-bisphosphate (10 µM), ATP (30 µM), and the test compound were mixed in a 384-well plate (total 20 µL), and incubated at room temperature for 30 minutes. EDTA and biotinylated phosphatidylinositol-3,4,5-triphosphate were added thereto to stop the reaction. Thereafter, a Europium labeled anti-GST antibody, a GST bond GRP1 PH domain, and streptavidin-APC were added thereto, followed by incubation overnight. An HTRF ratio was measured using an HTRF plate reader. The IC₅₀ value of the compound was calculated, taking the inhibition rate without addition of the enzyme as 100% and the inhibition rate without addition of the test compound as 0%, by means of a logistic method.

### 2. PI3Kα Enzyme Inhibitory Activity

Human PI3Kα (12 ng/well, Millipore Corporation, Catalogue No. 14-602), phosphatidylinositol (0.2 µg/well), and the test compound were mixed in a 384-well plate in a reaction buffer (50 mM Hepes, 10 mM NaCl, 10 mM MgCl₂, 2 mM EGTA, 2 mM DTT, pH 7.3) (total 10 µL), and incubated at 37°C for 3 hours. After the reaction, 10 µL of a Kinase-Glo Plus reagent (Promega, Catalogue No. V3772) was added thereto, and a luminescence was measured with a luxninometer. The IC₅₀ value of the compound was calculated, taking the inhibition rate without addition of the enzyme as 100% and the inhibition rate without addition of the test compound as 0%, by a logistic method.

The results of several compounds are shown in Tables 1 and 2. In the Table, Ex represents Example Compound No. as described later, PI3Kδ represents the IC₅₀ value (nM) of a PI3Kδ enzyme inhibitory activity, and PI3Kα represents the IC₅₀ value (nM) of a PI3Kα enzyme inhibitory activity.

**[Table 1]**

| Ex | PI3Kδ | PI3Kα | | Ex | PI3Kδ | PI3Kα |
|---|---|---|---|---|---|---|
| 4 | 33 | 2000 | | 210 | 22 | 5100 |
| 5 | 11 | 210 | | 216 | 5.6 | >3000 |
| 6 | 4.6 | 330 | | 219 | 14 | 6000 |
| 10 | 7.1 | 1500 | | 223 | 6.2 | >10000 |
| 11 | 14 | 930 | | 229 | 10 | 6700 |
| 14 | 4.4 | 1400 | | 231 | 8.2 | 1500 |
| 18 | 5.0 | 2900 | | 246 | 5.5 | 1700 |
| 24 | 5.2 | >3000 | | 271 | 2.6 | 2500 |
| 27 | 20 | 990 | | 274 | 9.7 | >3000 |
| 39 | 9.5 | 470 | | 280 | 4.9 | 2500 |
| 40 | 3.6 | 1200 | | 330 | 8.6 | 5500 |
| 46 | 44 | >3000 | | 344 | 14 | 1000 |
| 47 | 16 | 900 | | 363 | 18 | 1400 |
| 52 | 3.4 | 2700 | | 374 | 6.4 | 1200 |
| 53 | 4.6 | 2700 | | 375 | 12 | 1600 |
| 95 | 4.9 | 2500 | | 384 | 16 | 1600 |
| 104 | 2.1 | 810 | | 385 | 21 | 3000 |
| 107 | 8.8 | 3000 | | 393 | 7.7 | 780 |
| 108 | 2.8 | 2200 | | 396 | 13 | 2300 |
| 112 | 4.6 | 1400 | | 397 | 23 | 2900 |
| 116 | 5.2 | 180 | | 398 | 32 | 3400 |
| 123 | 0.85 | 460 | | 399 | 22 | 3200 |
| 125 | 1.8 | >3000 | | 401 | 15 | 3500 |
| 154 | 3.8 | 1800 | | 402 | 3.6 | 610 |
| 174 | 5.9 | 2400 | | 403 | 4.9 | 700 |
| 177 | 4.8 | >3000 | | 422 | 6.0 | 5800 |
| 185 | 4.3 | >3000 | | 423 | 11 | 4200 |
| 187 | 9.1 | 3000 | | 424 | 6.0 | 3600 |
| 190 | 4.1 | >3000 | | 430 | 2.3 | 2300 |
| 193 | 23 | 550 | | | | |
| 206 | 4.4 | 3300 | | | | |
| 208 | 8.6 | 2300 | | | | |
| 209 | 20 | 2800 | | | | |

**[Table 2]**

| Ex | PI3Kδ | PI3Kα | | Ex | PI3Kδ | PI3Kα |
|---|---|---|---|---|---|---|
| 434 | 4.3 | 1900 | | 487 | 7.9 | 4400 |
| 435 | 2.8 | 950 | | 488 | 30 | 5600 |
| 437 | 7.1 | 2200 | | 490 | 9.4 | 1600 |
| 438 | 3.6 | 2400 | | 491 | 4.5 | 570 |
| 441 | 15 | 5900 | | 495 | 14 | 6700 |
| 442 | 10 | 2700 | | 496 | 17 | 7600 |
| 445 | 7.0 | 3400 | | 497 | 16 | 10000 |
| 446 | 8.9 | 1700 | | 499 | 11 | 1500 |
| 447 | 5.3 | 2900 | | 500 | 14 | >10000 |
| 449 | 14 | 1300 | | 505 | 4.1 | 450 |
| 450 | 14 | 3500 | | 506 | 4.3 | 590 |
| 456 | 13 | 1800 | | 507 | 4.9 | 490 |
| 461 | 16 | 3800 | | 508 | 4.6 | 620 |
| 471 | 8.1 | 1700 | | 510 | 36 | >10000 |
| 473 | 30 | 9900 | | 511 | 4.7 | 2000 |
| 481 | 8.9 | 1100 | | 512 | 23 | >10000 |
| 482 | 4.6 | 3400 | | 513 | 3.5 | 3100 |
| 483 | 15 | 8700 | | 515 | 6.0 | 1200 |
| 484 | 1.5 | 2600 | | 527 | 14 | 5700 |
| 485 | 31 | >10000 | | 539 | 7.6 | 2200 |
| 486 | 9.8 | 3600 | | 546 | 50 | 5500 |

### 3. Rat In vivo IL-2 Production Inhibition Test

For the experiment, male LEW/CrlCrlj rats (Charles River Laboratories, Japan, Inc.) (6-week old, body weight 130 to 180 g) were used. The test compound was suspended in a 0.5% methyl cellulose solution and orally administered at 5 mL/kg. IL-2 production was induced by tail vein injection of Concanavalin A (Funalcoshi Corporation, Catalogue No. L-1000) at a dose of 15 mg/kg.
The test was carried out according to the protocol shown below. At 2 hours or 16 hours before administration of Concanavalin A, the test compound was orally administered to rats. At 3 hours after administration of Concanavalin A, blood was collected. The IL-2 concentration in blood was quantified using an ELISA kit (R&D Systems, Inc., Catalogue No. DY502E). An inhibition rate was calculated from the amount of IL-2 produced in a group administered with the test compound with respect to the amount of the IL-2 produced of a control group administered with a vehicle.

As a result, it was confirmed that the compound of the formula (I) has an excellent IL-2 production inhibition activity. For example, when the test compound (10 mg/kg) was administered at 2 hours before administration of Concanavalin A, the compounds of Examples 4, 11, 24, 40, 46, 194, 201, 202, 206, and 219 showed inhibition activities of 83%,80%, 79%, 94%, 71%, 89%, 76%, 80%, 83%, and 78%, respectively.

### 4. Rat B Cell Proliferation Inhibition Test

Spleen cells (1.0 × 10⁵ cells/well) prepared from male LEW/CrlCrlj rats (Charles River Laboratories, Japan, Inc.), mouse F(ab')₂ fragment anti-rat IgM (3 µg/well, SouthernBiotech Associates, Inc., Catalogue No. 3082-14) and the test compound dissolved in DMSO (final DMSO concentration 0.1 %) were mixed in a 96-well plate using a 10% FCS-containing RPMI-1640 culture medium (total 200 µL). They were cultured in a CO₂ incubator for 48 hours and [³H]thyrnidine (925 GBq/mmol, Moravek Biochemicals, Inc., Catalogue No. MT6038) was added thereto at 0.037 MBq/well at 4 hours before completion of culture. Cells were harvested in a GF/C glass filter using a cell harvester, and a radioactivity on the filter was measured using a liquid scintillation counter. The IC₅₀ value of the compound was calculated, taking the dpm (disintegration per minute) without addition of IgM as an inhibition rate of 100 % and the dpm without addition of the test compound as an inhibition rate of 0%, by a logistic method.

The results of several compounds are shown in Table 3. In the Table, Ex represents Example Compound No. below, and the IC₅₀ value (nM) represent a B cell proliferation inhibition activity.

**[Table 3]**

| Ex | IC₅₀(nM) | | Ex | IC₅₀(nM) | | Ex | IC₅₀(nM) |
|---|---|---|---|---|---|---|---|
| 4 | 1.8 | | 424 | 1.7 | | 491 | 1.8 |
| 11 | 6.1 | | 430 | 1.8 | | 495 | 1.7 |
| 24 | 4.2 | | 434 | 1.3 | | 496 | 1.7 |
| 40 | 0.62 | | 435 | 0.58 | | 497 | 2.1 |
| 46 | 5.2 | | 437 | 3.6 | | 500 | 2.7 |
| 174 | 2.4 | | 438 | 3.0 | | 505 | 0.46 |
| 177 | 1.1 | | 441 | 4.2 | | 506 | 0.75 |
| 206 | 4.1 | | 442 | 1.2 | | 507 | 0.38 |
| 219 | 3.5 | | 445 | 0.72 | | 508 | 0.37 |
| 223 | 1.5 | | 446 | 1.7 | | 510 | 18 |
| 246 | 2.1 | | 447 | 0.77 | | 511 | 1.2 |
| 271 | 5.5 | | 449 | 2.2 | | 512 | 10 |
| 274 | 5.5 | | 450 | 2.1 | | 513 | 0.64 |
| 280 | 3.8 | | 456 | 1.1 | | 515 | 4.1 |
| 363 | 1.4 | | 461 | 1.1 | | 527 | 2.8 |
| 374 | 2.0 | | 471 | 1.3 | | 539 | 0.84 |
| 375 | 1.2 | | 473 | 4.0 | | 546 | 2.2 |
| 385 | 1.9 | | 482 | 4.6 | | | |
| 393 | 0.70 | | 484 | 2.2 | | | |
| 398 | 2.3 | | 486 | 3.6 | | | |
| 399 | 3.4 | | 487 | 1.4 | | | |
| 403 | 0.82 | | 488 | 2.8 | | | |
| 422 | 4.9 | | 490 | 0.76 | | | |

As a result of the test above, it was confirmed that the compound of the formula (I) has excellent PI3Kδ-selective inhibitory action, and/or IL-2 production inhibitory action, and/or B cell proliferation inhibitory action (including an activation inhibitory action). Accordingly, it can be used as an agent for preventing or treating rejection in the transplantation of various organs, allergy diseases, autoimmune diseases, hematologic tumor, or the like.

Various types of organ transplantation as above represent, for example, transplantation of the kidney, liver, heart, and the like. Examples of the rejection include T cell-related rejection which is related to T cells, and an antibody-related rejection which is related to B cells. The allergy diseases above refer to asthma, atopic dermatitis, or the like. The autoimmune diseases above refer to rheumatoid arthritis, psoriasis, ulcerative colitis, Crohn's disease, systemic lupus erythematosus, or the like. The hematologic tumor refers to, leukemia or the like.

Furthermore, since the compound of the formula (I) has a significantly stronger PI3Kδ inhibitory action than a PI3Kα inhibitory action, it can be an excellent immunosuppressant which does not cause insulin resistance based on the PI3Kα inhibitory action.

A pharmaceutical composition containing one or two or more kinds of the compound of the formula (I) or a salt thereof as an active ingredient can be prepared in accordance with a generally used method, using an excipient, that is, a pharmaceutical excipient, a pharmaceutical carrier, or the like, that is usually used in the art.
Administration may be carried out in any form of oral administration via tablets, pills, capsules, granules, powders, liquid preparations, or the like, or of parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

As solid compositions for oral administration, tablets, powders, granules, or the like are used. In such a solid composition, one or two or more kinds of active ingredients are mixed with at least one inert excipient such as lactose, mannitol, glucose, hydroxypropylcellulose, microcrystalline cellulose, starch, polyvinyl pyrrolidone, and/or magnesium aluminometasilicate. According to a conventional method, the composition may contain inert additives such as a lubricant such as magnesium stearate, a disintegrator such as sodium carboxymethyl starch, a stabilizing agent, and a solubilizing agent. As occasion demands, the tablets or the pills may be coated with a sugar coating, or a film of gastric or enteric materials.
Liquid compositions for oral administration include pharmaceutically acceptable emulsions, solutions, suspensions, syrups, elixirs, or the like, and contain a generally used inert diluent such as purified water or ethanol. In addition to the inert diluent, the liquid composition may contain an adjuvant such as a solubilizing agent, a moistening agent, and a suspending agent, a sweetener, a flavor, an aroma, and an antiseptic.

Injections for parenteral administration include sterile aqueous or non-aqueous solutions, suspensions or emulsions. The aqueous solvent includes, for example, distilled water for injection or physiological saline. Examples of the non-aqueous solvent include propylene glycol, polyethylene glycol, vegetable oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent. These are sterilized, for example, by filtration through a bacteria-retaining filter, blending of a sterilizing agent, or irradiation. In addition, these can also be used by preparing a sterile solid composition, and dissolving or suspending it in sterile water or a sterile solvent for injection prior to use.

External preparations include ointments, plasters, creams, jellies, cataplasms, sprays, lotions, eye drops, eye ointments, and the like. Generally used ointment bases, lotion bases, aqueous or non-aqueous liquids, suspensions, emulsions, and the like are included. Examples of the ointment or lotion bases include polyethylene glycol, propylene glycol, white Vaseline, bleached beewax, polyoxyethylene hydrogenated castor oil, glyceryl monostearate, stearyl alcohol, cetyl alcohol, lauromacrogol, sorbitan sesquioleate, and the like.

As the transmucosal preparations such as inhalations and transnasal preparations, a solid, liquid or semi-solid form are used, and can be prepared in accordance with a conventionally known method. For example, a known excipient, and also a pH-adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device. The dry powder inhalation devices or the like may be for single or multiple administration use, and a dry powder or a powder-containing capsule can be used. Alternatively, it may be in a form such as a pressurized aerosol spray or the like which uses an appropriate propellant, for example, a suitable gas such as chlorofluoroalkane, hydrofluoroalkane, or carbon dioxide and the like.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compounds of the formula (I) can be used in combination with various agents for treating or preventing the aforementioned diseases for which the compound of the formula (I) are considered to be effective. The combined preparation may be administered simultaneously, or separately and continuously or at a desired time interval. The preparations to be co-administered may be a blend, or may be prepared individually.

### (Examples)

The production processes for the compounds of the formula (I) and the starting compounds thereof will be described in detail below based on Examples. In this connenction, the present invention is not limited to the compounds described in the following Examples. In addition, production processes for the starting compounds are described as Preparation Examples. The production processes for the compounds of the formula (I) are not limited to the production processes in specific Examples shown below, and the compound of the formula (I) can be produced by a combination of these production processes or methods apparent to one skilled in the art.

Furthermore, the following abbreviations are used in the Preparation Examples, Examples, and Tables below.
PEx: Preparation Example No., Ex: Example No., Syn: Example No. prepared in the same method, PSyn: Preparation Example No. prepared in the same method, No: Compound No., Str: Structural formula, DATA: Physicochemical Data, EI+: m/z values in mass spectroscopy (Ionization EI, representing (M)⁺ unless otherwise specified), ESI+: m/z values in mass spectroscopy (Ionization ESI, representing (M+H)⁺ unless otherwise specified), ESI-: m/z values (Ionization ESI, representing (M-H)⁻ unless otherwise specified), FAB+: m/z values in mass spectroscopy (representing (M+H)⁺ unless otherwise specified), NMR1: δ (ppm) in ¹H NMR in DMSO-d₆, NMR2: δ (ppm) in ¹H NMR in CDCl₃, NMR3: δ (ppm) in ¹H NMR in CD₃OD, s: singlet (spectrum), d: doublet (spectrum), t: triplet (spectrum), q: quartet (spectrum), br: broad line (spectrum) (e.g.: br-s), and RT: Retention time (min.) in HPLC. Further, HCl in the structural formula represents hydrochloride, and a numeral prefixed to HCl represents a molar ratio. For example, 2HCl represents dihydrochloride.

### Preparation Example 1

To a solution of 4,6-dichloro-2-(methylsulfanyl)pyrimidine (5 g) in N,N-dimethylformamide (50 mL) were added potassium carbonate (5.3 g) and 2-(difluoromethyl)-1H-benzimidazole (3.9 g), and the mixture was stirred at room temperature for 5 hours. To the reaction mixture was added water (300 mL), followed by extraction with ethyl acetate (300 mL). The organic layer was washed with saturated brine and dried over anhydrous magnesium sulfate. The solvent was evaporated under reduced pressure, and then the residue was purified by silica gel column chromatography (hexane:ethyl acetate=90:10 to 40:60) to obtain 1-[6-chloro-2-(methylsulfanyl)pyrimidin-4-yl]-2-(difluoromethyl)-1H-benzimidazole (5.49 g) as a white powder.

### Preparation Example 2

To a solution of 1-[6-chloro-2-(methylsulfanyl)pyrimidin-4-yl]-2-(difluoromethyl)-1H-benzimidazole (2.2 g) in N,N-dimethylformamide (11 mL) were added potassium carbonate (1.4 g) and morpholine (0.88 mL), and the mixture was stirred at room temperature for 1 hour. To the reaction mixture was added water (150 mL), followed by extraction with ethyl acetate (150 mL). The organic layer was washed with saturated brine (150 mL) and dried over anhydrous magnesium sulfate. The solvent was evaporated under reduced pressure, and then the residue was purified by silica gel column chromatography (hexane:ethyl acetate=70:30 to 50:50) to obtain 2-(difluoromethyl)-1-[2-(methylsulfanyl)-6-morpholin-4-ylpyrimidin-4-yl]-1H-benzimidazole (2.1 g) as a white powder.

### Preparation Example 3

To a solution of 2-(difluoromethyl)-1-[2-(methylsulfanyl)-6-morpholin-4-ylpyrimidin-4-yl]-1H-benzimidazole (3 g) in dichloromethane (60 mL) was added m-chloroperbenzoic acid (75%, containing water) (1.9 g) under ice-cooling, and the mixture was stirred at 0°C for 15 minutes. To the reaction mixture was added saturated aqueous sodium bicarbonate, followed by extraction with dichloromethane (200 mL). The organic layer was washed with water (200 mL) and saturated brine (200 mL), and dried over anhydrous magnesium sulfate. The solvent was evaporated under reduced pressure, and then the residue was purified by silica gel column chromatography (chloroform:methanol=100:0 to 98:2) to obtain 2-(difluoromethyl)-1-[2-(methylsulfinyl)-6-morpholin-4-ylpyrimidin-4-yl]-1H-benzimidazole (2.8 g) as a pale yellow amorphous substance. The Rf value of the present compound in the silica gel TLC (chloroform:methanol=10:1) was 0.56.

### Preparation Example 4

To a solution of 2-(difluoromethyl)-1-[2-(methylsulfanyl)-6-morpholin-4-ylpyrimidin-4-yl]-1H-benzimidazole (2.1 g) in dichloromethane (21 mL) was added m-chloroperbenzoic acid (75%, containing water) (2.7 g) under ice-cooling, and the mixture was stirred at 0°C for 15 minutes. To the reaction mixture was added saturated aqueous sodium bicarbonate, followed by extraction with dichloromethane (200 mL). The organic layer was washed with water (200 mL) and saturated brine (200 mL), and dried over anhydrous magnesium sulfate. The solvent was evaporated under reduced pressure, and then the residue was purified by silica gel column chromatography (chloroform:methanol=100:0 to 98:2) to obtain 2-(difluoromethyl)-1-[2-(methylsulfonyl)-6-morpholin-4-ylpyrimidin-4-yl]-1H-benzimidazole (2.27 g) as a pale yellow amorphous substance. The Rf value of the present compound in the silica gel TLC (chloroform:methanol=10:1) was 0.67.

### Preparation Example 5

To a solution of 1-[6-chloro-2-(methylsulfanyl)pyrimidin-4-yl]-2-(difluoromethyl)-1H-benzimidazole (150 mg) in N,N-dimethylacetamide (2 mL) were added tert-butyl(trans-4-hydroxycyclohexyl)carbamate (125 mg) and cesium carbonate (225 mg), and the mixture was stirred at room temperature for 1 hour, at 60°C for 1 hour, and at 120°C for 3 hours. Water (20 mL) was poured into the reaction mixture, followed by extraction with hexane:ethyl acetate (1:1, 100 mL). The organic layer was washed with water and saturated brine, and dried over anhydrous magnesium sulfate. The solvent was evaporated under reduced pressure, and then the residue was purified by silica gel column chromatography (hexane:ethyl acetate=90:10 to 60:40) to obtain tert-butyl[trans-4-({6-[2-(difluoromethyl)-1H-benzimidazol-1-yl]-2-(methylsulfanyl)pyrimidn-4-yl}oxy)cyclohexyl]carbamate (129 mg) as a white amorphous substance.

### Preparation Example 6

To 4-fluorobenzene-1,2-diamine (1.00 g) was added difluoroacetic acid (1 mL), and the mixture was stirred at 90°C for 6 hours. The reaction mixture was poured into water (20 mL), followed by addition of ethyl acetate (20 mL). The mixture was alkalified by the addition of a 1 M aqueous sodium hydroxide solution, followed by extraction with ethyl acetate (50 mL). The organic layer was washed with water and saturated brine, and dried over anhydrous magnesium sulfate. The solvent was evaporated under reduced pressure, and then purified by silica gel column chromatography (hexane:ethyl acetate=70:30 to 0:100) to obtain 2-(difluoromethyl)-5-fluoro-1H-benzimidazole (1.22 g) as a white powder.

### Preparation Example 7

To a solution of 4,6-dichloro-2-(methylsulfanyl)pyrimidine (1.4 g) and 2-(difluoromethyl)-5-fluoro-1H-benzimidazole (1.2 g) in N,N-dimethylformamide (28 mL) was added potassium carbonate (1.48 g), and the mixture was stirred at room temperature overnight. To the reaction mixture was added water (100 mL), followed by extraction with ethyl acetate (200 mL). The organic layer was washed with water and saturated brine, and dried over anhydrous magnesium sulfate. The solvent was evaporated under reduced pressure, and then purified by silica gel column chromatography (hexane:ethyl acetate=95:5 to 70:30) to obtain two kinds of compound as a white powder, respectively.
1-[6-chloro-2-(methylsulfanyl)pyrimidin-4-yl] -2-(difluoromethyl)-5-fluoro- 1H-benzimidazole: 319 mg, the Rf value in silica gel TLC (hexane:ethyl acetate=5:1) was 0.51.
1-[6-chloro-2-(methylsulfanyl)pyrimidin-4-yl]-2-(difluoromethyl)-6-fluoro-1H-benzimidazole: 438 mg, the Rf value in silica gel TLC(hexane:ethyl acetate=5:1) was 0.46.

### Preparation Example 8

60% sodium hydride (417 mg) was suspended in tetrahydrofuran (24 mL), and tert-butyl(trans-4-hydroxycyclohexyl)carbamate (1.87 g) and 15-crown-5 (1.73 mL) were added thereto. The mixture was stirred at room temperature for 30 minutes. To the reaction mixture was added 4,6-dichloro-2-(methylsulfonyl)pyrimidine (1.97 g), followed by stirring at 60°C overnight. The reaction mixture was poured into a saturated aqueous ammonium chloride solution (100 mL), followed by extraction with ethyl acetate (200 mL). The organic layer was washed with water and saturated brine, and dried over anhydrous magnesium sulfate. The solvent was evaporated under reduced pressure, and then purified by silica gel column chromatography (hexane:ethyl acetate=93:7 to 70:30) to obtain tert-butyl{trans-4-[(4,6-dichloropyrimidin-2-yl)oxy]cyclohexyl}carbamate (598 mg) as a white powder.

### Preparation Example 16

60% sodium hydride (288 mg) was suspended in dimethoxyethan (15 mL), and tert-butyl[trans-4-(hydroxymethyl)cyclohexyl]carbamate (750 mg) and 1,4,7,10,13-pentaoxacyclopentadecane were added thereto, followed by stirring at room temperature for 30 minutes. Subsequently, 4,6-dichloro-2-(methylsulfonyl)pyrimidine (743 mg) was added thereto, followed by stirring at 80°C overnight. The reaction mixture was added to a saturated aqueous ammonium chloride solution (50 mL), followed by extraction with ethyl acetate (200 mL) and washing with water and saturated brine. The organic layer was dried over anhydrous magnesium sulfate, and then the solvent was evaporated under reduced pressure. Purification using silica gel column chromatography (hexane:ethyl acetate=95:5 to 85:15) was performed to obtain a desired compound, tert-butyl(trans-4-{[(4,6-dichloropyrimidin-2-yl)oxy]methyl}cyclohexyl)carbamate (290 mg), as a white powder.

### Preparation Example 23

2-(Difluoromethyl)-1-[6-(1,4-dioxaspiro[4.5]dec-8-ylmethoxy)-2-(methylsulfanyl)pyrimidin-4-yl]-1H-benzimidazole (1.3 g) was dissolved in dichloromethane (20 mL), and m-chloroperbenzoic acid (75%, containing water) (712 mg) was added thereto at 0°C, followed by stirring for 30 minutes. To the reaction mixture was added saturated aqueous sodium bicarbonate (30 mL), followed by extraction with chloroform (100 mL) and washing with water and saturated brine. The organic layer was dried over anhydrous magnesium sulfate, and then the solvent was evaporated under reduced pressure. The residue was dissolved in dimethylformamide (10 mL), and morpholine (1.22 mL) was added thereto, followed by stirring at room temperature for 2 hours. The reaction mixture was poured into water (50 mL), followed by extraction with ethyl acetate (200 mL), and washing with water and saturated brine. The organic layer was dried over anhydrous magnesium sulfate, and then the solvent was evaporated under reduced pressure. Purification using silica gel column chromatography (hexane:ethyl acetate=95:5 to 80:20) was performed to obtain a desired compound, 2-(difluoromethyl)-1-[6-(1,4-dioxaspiro[4.5]dec-8-ylmethoxy)-2-(morpholin-4-yl)pyrimidin-4-yl]-1H-benzimidazole (1.21 g), as a white powder.

### Preparation Example 24

2-(Difluoromethyl)-1-[6-(1,4-dioxospiro[4.5]dec-8-ylmethoxy)-2-(morpholin-4-yl)pyrimidin-4-yl]-1H-benzimidazole (1.2 g) was dissolved in tetrahydrofuran (12 mL)-water (12 mL), and 4-methylbenzenesulfonic acid monohydrate (2.27 g) was added thereto, followed by stirring at room temperature for 3 hours. To the reaction mixture was added saturated aqueous sodium bicarbonate (30 mL) followed by extraction with ethyl acetate (100 mL), and washing with water and saturated brine. The organic layer was dried over anhydrous magnesium sulfate, and then the solvent was evaporated under reduced pressure. Purification using silica gel column chromatography (hexane:ethyl acetate=80:20 to 40:60) was performed to obtain a desired compound, 4-[({6-[2-(difluoromethyl)-1H-benzimidazol-1-yl]-2-(morpholin-4-yl)pyrimidin-4-yl}oxy)methyl]cyclohexanone (941 mg), as a white powder.

### Example 1

To a solution of 2-(difluoromethyl)-1-[2-(methylsulfonyl)-6-morpholin-4-ylpyrimidin-4-yl]-1H-benzimidazole (2.27 g) in N,N-dimethylacetamide (57 mL) were added trans-cyclohexane-1,4-diamine (5.45 g) and potassium carbonate (1.15 g), and the mixture was stirred at 100°C for 1 hour. The reaction mixture was cooled to room temperature, and water (300 mL) was added thereto, followed by extraction with ethyl acetate (300 mL). The organic layer was washed with saturated brine (200 mL) and dried over anhydrous magnesium sulfate. The solvent was evaporated under reduced pressure, and then the residue was purified by amino silica gel column chromatography (hexane:ethyl acetate=25:75 to 0:100, and subsequently chloroform:methanol=100:0 to 97:3) to obtain trans-N-{4-[2-(difluoromethyl)-1H-benzimidazol-1-yl]-6-morpholin-4-ylpyrimidin-2-yl}cyclohexane-1,4-diamine (2.21 g) as a pale yellow powder.

### Example 2

To a solution of trans-N-{4-[2-(difluoromethyl)-1H-benzimidazol-1-yl]-6-morpholin-4-yl-1,3,5-triazin-2-yl}cyclohexane-1,4-diamine (50 mg) in N,N-dimethylformamide were added methoxyacetic acid (9 µL), 1-hydroxybenzotriazole (15 mg), and N-[3-(dimethylamino)propyl]-N'-ethylcarbodiimide hydrochloride (22 mg), and the mixture was stirred at room temperature overnight. To the reaction mixture was added water (50 mL), followed by extraction with ethyl acetate (50 mL). The organic layer was washed with saturated brine and dried over anhydrous magnesium sulfate. The solvent was evaporated under reduced pressure, and then the resulting solid was collected by filtration and washed with ethyl acetate to obtain N-[trans-4-({4-[2-(difluoromethyl)-1H-benzimidazol-1-yl]-6-morpholin-4-yl-1,3,5-triazin-2-yl}amino)cyclohexyl]-2-methoxyacetamide (32 mg) as a white powder.

### Example 3

To a solution of trans-N-{4-[2-(difluoromethyl)-1H-benzimidazol-1-yl]-6-morpholin-4-yl-1,3,5-triazin-2-yl}cyclohexane-1,4-diamine (50 mg) in dichloromethane (1.25 mL) were added triethylamine (47 µL) and propane-1-sulfonyl chloride (12 µL), and the mixture was stirred at room temperature overnight. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the residue was purified by silica gel column chromatography (hexane:ethyl acetate=50:50 to 0:100) to obtain N-[trans-4-({4-[2-(difluoromethyl)-1H-benzimidazol-1-yl]-6-morpholin-4-yl-1,3,5-triazin-2-yl}amino)cyclohexyl]propane-1-sulfonamide (46 mg) as a white powder.

### Example 4

To a solution of trans-N-{4-[2-(difluoromethyl)-1H-benzimidazol-1-yl]-6-morpholin-4-yl-1,3,5-triazin-2-yl}cyclohexane-1,4-diamine (250 mg) in dichloromethane (4.5 mL) were added a 37% aqueous formaldehyde solution (0.443 mL) and sodium triacetoxyborohydride (476 mg), and the mixture was stirred at room temperature for 1 hour. To the reaction mixture was added water (30 mL), followed by extraction with chloroform (100 mL). The organic layer was washed with saturated brine and dried over anhydrous sodium sulfate. The solvent was evaporated under reduced pressure, and then the residue was purified by amino silica gel column chromatography (hexane:ethyl acetate=50:50 to 90:10) to obtain trans-N'-{4-[2-(difluoromethyl)-1H-benzimidazol-1-yl]-6-morpholin-4-yl-1,3,5-triazin-2-yl}-N,N-dimethylcyclohexane-1,4-diamine (216 mg) as a white powder.

### Example 5

To a solution of trans-N-{4-[2-(difluoromethyl)-1H-benzimidazol-1-yl]-6-morpholin-4-yl-1,3,5-triazin-2-yl}cyclohexane-1,4-diamine (30 mg) in dichloromethane (1.2 mL) was added methyl isocyanate (4.2 µL), and the mixture was stirred at room temperature for 0.5 hours. The reaction mixture was concentrated under reduced pressure, and then purified by silica gel column chromatography (chloroform) to obtain 1-[trans-4-({4-[2-(difluoromethyl)-1H-benzimidazol-1-yl]-6-morpholin-4-yl-1,3,5-triazin-2-yl}amino)cyclohexyl]-3-methylurea (28 mg) as a white powder.

### Example 6

To a solution of trans-N-{4-[2-(difluoromethyl)-1H-benzimidazol-1-yl]-6-morpholin-4-yl-1,3,5-triazin-2-yl}cyclohexane-1,4-diamine (70 mg) in N,N-dimethylformamide (1.4 mL) were added triethylamine (44 µL) and 1,1'-carbonylbis(1H-imidazole) (26 mg), and the mixture was stirred at room temperature for 1 hour. After confirming the progress of the reaction by a mass spectrum, to the reaction mixture was added 2-(morpholin-4-yl)ethaneamine (25 µL), followed by stirring at room temperature for 3 hours. To the reaction mixture was added water (20 mL), followed by extraction with chloroform (10 mL). The organic layer was dried over anhydrous magnesium sulfate. The solvent was evaporated under reduced pressure, and then the residue was purified by silica gel column chromatography (chloroform:methanol=20:80) to obtain 1-[trans-4-({4-[2-(difluoromethyl)-1H-benzimidazol-1-yl]-6-morpholin-4-yl-1,3,5-triazin-2-yl}amino)cyclohexyl]-3-(2-morpholin-4-ylethyl)urea (62 mg) as a white powder.

### Example 7

To a solution of trans-N-{4-[2-(difluoromethyl)-1H-benzimidazol-1-yl]-6-morpholin-4-yl-1,3,5-triazin-2-yl}cyclohexane-1,4-diamine (1.86 g) in dichloromethane (3 7 mL) were added triethylamine (1.46 mL) and di-tert-butyl dicarbonate (1.1 g), and the mixture was stirred at room temperature for 1 hour. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the residue was purified by silica gel column chromatography (hexane:ethyl acetate=80:20 to 50:50) to obtain tert-butyl[trans-4-({4-[2-(difluoromethyl)-1H-benzimidazol-1-yl]-6-morpholin-4-yl-1,3,5-triazin-2-yl}amino)cyclohexyl]carbamate (2.04 g) as a white powder.

### Example 8

To a solution of tert-butyl (2-{[trans-4-({4-[2-(difluoromethyl)-1H-benzimidazol-1-yl]-6-morpholin-4-yl-1,3,5-triazin-2-yl}amino)cyclohexane]amino}-2-oxoethyl)carbamate (138 mg) in 1,4-dioxane (1.4 mL) was added a 4 M hydrogen choride/1,4-dioxane solution (574 µL), and the mixture was stirred at room temperature for 2 hours. To the reaction mixture was added a 2 M ammonia/ethanol solution (2 mL), followed by concentration under reduced pressure, and the residue was purified by amino silica gel column chromatography (hexane:ethyl acetate=50:50 to 0:100, and subsequently chloroform:methanol=100:0 to 98:2) to obtain N-[trans-4-({4-[2-(difluoromethyl)-1H-benztriazol-1-yl]-6-morpholin-4-yl-1,3,5-triazin-2-yl}amino)cyclohexyl]glycinamide (74 mg) as a white powder.

### Example 9

To a solution of trans-N-{4-[2-(difluoromethyl)-1H-benzimidazol-1-yl]-6-morpholin-4-yl-1,3,5-triazin-2-yl}cyclohexane-1,4-diamine (200 mg) in dichloromethane (2 mL) were added triethylamine (63 µL) and bromoacetylchloride (37 µL) under ice-cooling, and the mixture was stirred at 0°C for 30 minutes. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the resulting solid was collected by filtration and washed with diisopropylether to obtain 2-bromo-N-[trans-4-({4-[2-(difluoromethyl)-1H-benzimidazol-1-yl]-6-morpholin-4-yl-1,3,5-triazin-2-yl}amino)cyclohexyl]acetamide (207 mg) as a white powder.

### Example 10

To a solution of trans-N-{4-[2-(difluoromethyl)-1H-benzimidazol-1-yl]-6-morpholin-4-yl-1,3,5-triazin-2-yl}cyclohexane-1,4-diamine (300 mg) in N,N-dimethylacetamide (6 mL) were added triethylamine (0.261 mL) and bis(2-bromo ethyl)ether (0.12 mL), and the mixture was stirred at 70°C overnight. To the reaction mixture were added triethylamine (0.261 mL) and bis(2-bromoethyl)ether (0.12 mL), followed by stirring at 70°C overnight. To the reaction mixture was added water (30 mL), followed by extraction with ethyl acetate (100 mL). The organic layer was washed with water and saturated brine, and dried over anhydrous sodium sulfate. The solvent was evaporated under reduced pressure, and then the residue was purified by amino silica gel column chromatography (hexane:ethyl acetate=50:50 to 80:20) to obtain 4-[2-(difluoromethyl)-1H-benzimidazol-1-yl]-6-morpholin-4-yl-N-(trans-4-morpholin-4-ylcyclohexyl)-1,3,5-triazin-2-amine (231 mg) as a white powder.

### Example 11

To a solution of trans-N-{4-[2-(difluoromethyl)-1H-benzimidazol-1-yl]-6-morpholin-4-ylpyrimidin-2-yl}cyclohexane-1,4-diamine (50 mg) in dichloromethane (1 mL) were added triethylamine (0.047 mL), 4-chlorobutanoyl chloride (0.014 mL), and the mixture was stirred for 1 hour under ice-cooling. The reaction mixture was concentrated, and then the residue was dissolved in N,N-dimethylformamide (5 mL). 60% sodium hydride (13.5 mg) was added thereto, followed by stirring at 0°C for 30 minutes and at room temperature for 1 hour. To the reaction mixture was added water (30 mL), followed by extraction with ethyl acetate (100 mL). The organic layer was washed with water and saturated brine, and dried over anhydrous magnesium sulfate. The solvent was evaporated under reduced pressure, and then the residue was purified by amino silica gel column chromatography (hexane:ethyl acetate=50:50 to 100:0) to obtain 1-[trans-4-({4-[2-(difluoromethyl)-1H-benzimidazol-1-yl]-6-morpholin-4-ylpyrimidin-2-yl}amino)cyclohexyl]pyrrolidin-2-one (40 mg) as a white powder.

### Example 12

To a solution of 2-bromo-N-[trans-4-({4-[2-(difluoromethyl)-1H-benzimidazol-1-yl]-6-morpholin-4-yl-1,3,5-triazin-2-yl}amino)cylohexyl]acetamide (50 mg) in 1,3-dimethyl-2-imidazolidione (0.5 mL) were added potassium carbonate (24 mg) and pyrrolidine (15 µL), and the mixture was stirred at room temperature for 1 hour. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the residue was purified by silica gel column chromatography (hexane:ethyl acetate=50:50 to 0:100, and subsequently chloroform:methanol=100:0 to 90:10) to obtain N-[trans-4-({4-[2-(difluoromethyl)-1H-benzimidazol-1-yl]-6-morpholin-4-yl-1,3,5-triazin-2-yl}amino)cyclohexyl]-2-pyrrolidin-1-ylacetamide (49 mg) as a white powder.

### Example 13

To a suspension of 60% sodium hydride (2.8 mg) in N,N-dimethylformamide (1 mL) was added 3-chloro-N-[trans-4-({4-[2-(difluorornethyl)-1H-benzimidazol-1-yl]-6-morpholin-4-ylpyrimidin-2-yl}amino)cyclohexyl]propane-1-sulfonamide (35 mg), and the mixture was stirred at 0°C for 1 hour and at room temperature for 2 hours. To the reaction mixture was added water (20 mL), followed by extraction with ethyl acetate (100 mL). The organic layer was washed with water and saturated brine, and dried over anhydrous sodium sulfate. The solvent was evaporated under reduced pressure, and then the residue was purified by silica gel column chromatography (hexane:ethyl acetate=60:50 to 100:0) to obtain 4-[2-(difluoromethyl)-1H-benzimidazol-1-yl]-N-[trans-4-(1,1-dioxidoisothiazolin-2-yl)cyclohexyl]-6-morpholin-4-ylpyrimidin-2-amine (31.6 mg) as a white powder.

### Example 14

To a solution of trans-N-{4-[2-(difluoromethyl)-1H-benzimidazol-1-yl]-6-morpholin-4-yl-1,3,5-triazin-2-yl}-N-methylcyclohexane-1,4-diamine (53 mg) in pyridine (468 µL) was added acetic anhydride (14 µL), and the mixture was stirred at room temperature for 1 hour. To the reaction solution was added water (50 mL), followed by extraction with ethyl acetate (50 mL). The organic layer was washed with saturated brine (50 mL) and dried over anhydrous magnesium sulfate. The solvent was evaporated under reduced pressure, and then the resulting solid was collected by filtration and washed with diisopropylether to obtain N-[trans-4-({4-[2-(difluoromethyl)-1H-benzimidazol-1-yl]-6-morpholin-4-yl-1,3,5-triazin-2-yl}amino)cylohexyl]-N-methylacetamide (55 mg) as a white powder.

### Example 15

To a solution of trans-N-{4-[2-(difluoromethyl)-1H-benzimidazol-1-yl]-6-morpholin-4-yl-1,3,5-triazin-2-yl}cyclohexane-1,4-diamine (1 g) in ethanol (20 mL) was added 1H-1,2,3-benzotriazol-1-ylmethanol (336 mg), and the mixture was stirred at room temperature for 1 hour. To the reaction mixture was added sodium tetrahydroborate (170 mg), followed by stirring at room temperature for 1 hour. To the reaction mixture was added saturated aqueous sodium bicarbonate (200 mL), followed by extraction with ethyl acetate (200 mL). The organic layer was washed with saturated brine (200 mL) and dried over anhydrous magnesium sulfate. The solvent was evaporated under reduced pressure, and then the residue was purified by amino silica gel column chromatography (hexane:ethyl acetate=50:50 to 0:100, and subsequently chloroform:methanol=100:0 to 98:2) to obtain trans-N-{4-[2-(difluoromethyl)-1H-benzimidazol-1-yl]-6-morpholin-4-yl-1,3,5-triazin-2-yl}-N'-methylcyclohexane-1,4-diamine (890 mg) as a white powder.

### Example 16

N-[trans-4-({6-[2-(Difluoromethyl)-1H-benzimidazol-1-yl]-2-morpholin-4-ylpyrimidin-4-yl}amino)cyclohexyl]-N,N-dimethylglycinamide (100 mg) was dissolved in a mixed solvent of dichloromethane (20 mL)-methanol (4 mL), and a 2 M hydrogen choride/ethanol solution (0.3 mL) was added thereto, followed by stirring at room temperature for 10 minutes. The solvent was evaporated under reduced pressure and to the residue was added methanol (30 mL). The solvent was evaporated again under reduced pressure. The resulting solid was collected by filtration and washed with diisopropylether to obtain N-[trans-4-({6-[2-(difluoromethyl)-1H-benzimidazol-1-yl]-2-morpholin-4-ylpyrimidin-4-yl}amino)cyclohexyl]-N,N-dimethylglycinamide hydrochloride (93 mg) as a white powder.

### Example 17

To a solution of trans-N-{4-[2-(difluoromethyl)-1H-benzimidazol-1-yl]-6-morpholin-4-ylpyrimidin-2-yl}cyclohexane-1,4-diamine (65 mg) in dichloromethane (1.3 mL) were added triethylamine (20 µL) and 3-bromopropionyl chloride (25 mg), and the mixture was stirred at room temperature for 30 minutes. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the resulting solid was collected by filtration and washed with ethyl acetate.
This solid was dissolved in 1,3-dimethyl-2-imidazolidione (1.3 mL), and potassium carbonate (61 mg) and pyrrolidine (18 µL) were added thereto, followed by stirring at room temperature for 2 hours. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the residue was purified by silica gel column chromatography (hexane:ethyl acetate=50:50 to 0:100, and subsequently chloroform:methanol=100:0 to 90:10) to obtain N-[trans-4-({4-[2-(difluoromethyl)-1H-benzimidazol-1-yl]-6-morpholin-4-ylpyrimidin-2-yl}amino)cyclohexyl]-3-pyrrolidin-1-yl propionamide (13 mg) as a white powder.

### Example 18

To a mixture of trans-N-{6-[2-(difluoromethyl)-1H-benzimidazol-1-yl]-2-morpholin-4-ylpyrimidin-4-yl}cyclohexane-1,4-diamine (100 mg), pyridine (0.04 mL), and dichloromethane (1 mL) was added 3-chloropropane-1-sulfonyl chloride (0.04 mL) under ice-cooling, followed by stirring at room temperature overnight. The reaction mixture was concentrated and the residue was purified by amino silica gel column chromatography (hexane:ethyl acetate=50:50 to 0:100) and a desired fraction was collected and concentrated. The residue was dissolved in N,N-dimethylformamide (1 mL), and 60% sodium hydride (27 mg) was added thereto, followed by stirring at 0°C for 1 hour and at room temperature for 2 hours. The reaction mixture was poured into a saturated aqueous ammonium chloride solution, followed by extraction with ethyl acetate (100 mL). The organic layer was washed with water and saturated brine, and dried over anhydrous sodium sulfate. The solvent was evaporated under reduced pressure, and then the residue was purified by silica gel column chromatography (hexane:ethyl acetate=60:40 to 0:100) to obtain 6-[2-(difluoromethyl)-1H-benzimidazol-1-yl]-N-[trans-4-(1,1-dioxidoisothiazolin-2-yl)cyclohexyl]-2-morpholin-4-ylpyrimidin-4-amine (27 mg) as a pale brown powder.

### Example 19

To a solution of tert-butyl[(1R)-3-(benzyloxy)-1-{[trans-4-({6-[2-(difluoromethyl)-1H]-benzimidazol-1-yl]-2-morpholin-4-ylpyrimidin-4-yl}amino)cyclohexyl]carbamayl}propyl]carbamate (180 mg) in methanol (3 mL) was added 10% palladium-carbon (50%, containing water), followed by stirring for 9 hours under a hydrogen atmosphere of 1 atm. The reaction mixture was filtered using Celite, and then concentrated. The residue was purified by silica gel column chromatography (hexane:ethyl acetate=40:60 to 0: 100) to obtain tert-butyl[(1R)-1-{[trans-4-({6-[2-(difluoromethyl)-1H-benzimidazol-1-yl]-2-morpholin-4-ylpyrimidin-4-yl}amino)cyclohexyl]carbamoyl}-3-hydroxypropyl]carbamate (143 mg) as a white powder.

### Example 20

To a solution of trans-N-{6-[2-(difluoromethyl)-1H-benzimidazol-1-yl]-2-morpholin-4-ylpyrimidin-4-yl}cyclohexane-1,4-diamine (40 mg) in N,N-dimethylformamide (0.4 mL) was added N-(tert-butoxycarbonyl)-2-methylalanine (21 mg), 1-[bis(dimethylamino)methylene]-1H-1,2,3-triazolo[4,5-b]pyridinium-3-oxide hexafluorophosphate (HATU) (51 mg), and N,N-diisopropylethylamine (0.079 mL), and the mixture was stirred at room temperature for 3 hours. To the reaction mixture was added water (20 mL). The resulting powder was collected by filtration, washed with isopropylether, and dried under reduced pressure to obtain tert-butyl(2-{[trans-4-({6-[2-(difluoromethyl)-1H-benzimidazol-1-yl]-2-morpholin-4-ylpyrimidin-4-yl}amino)cyclohexyl]amino}-1,1-dimethyl-2-oxoethyl)carbamate (56 mg) as a white powder.

### Example 21

To a solution of (3R)-N-[trans-4-({6-[2-(difluoromethyl)-1H-benzimidazol-1-yl]-2-morpholin-4-ylpyrimidin-4-yl}amino)cyclohexyl]piperidine-3-carboxamide (50 mg) in dichloromethane (1 mL) was added a 37% aqueous formaldehyde solution (0.022 mL), followed by stirring at room temperature for 30 minutes. To the reaction mixture was added sodium triacetoxyborohydride (57 mg), followed by stirring at room temperature for 2 hours. To the reaction mixture were added saturated aqueous sodium bicarbonate (5 mL) and water (5 mL), followed by extraction with chloroform (100 mL). The organic layer was washed with water and saturated brine, and dried over anhydrous sodium sulfate. The solvent was evaporated under reduced pressure, and then the residue was purified by amino silica gel column chromatography (hexane:ethyl acetate=50:50). A desired fraction was collected and concentrated. The residue was dissolved in methanol, and a 4 M hydrogen choride/1,4-dioxane solution was added thereto, followed by stirring at room temperature for 10 minutes and then concentrating, to obtain (3R)-N-[trans-4-({6-[2-(difluoromethyl)-1H-benzimidazol-1-yl]-2-morpholin-4-ylpyrimidin-4-yl}amino)cyclohexyl]-1-methylpiperidine-3-carboxamide hydrochloride (41 mg) as a white powder.

### Example 22

To a solution of tert-butyl[trans-4-({6-[2-(difluoromethyl)-1H-benzimidazol-1-yl]-2-(methylsulfanyl)pyrimidin-4-yl}amino)cyclohexyl]carbamate (7.6 g) in chloroform (76 mL) was added m-chloroperbenzoic acid (75%, containing water) (3.81 g) at 0°C, followed by stirring for 20 minutes. To the reaction mixture was added saturated aqueous sodium bicarbonate (50 mL), followed by extraction with chloroform (200 mL). The organic layer was washed with water and saturated brine, and dried over anhydrous sodium sulfate. The solvent was evaporated under reduced pressure, and then the residue was dissolved in N,N-dimethylacetamide (40 mL). Morpholine (6.57 mL) was added thereto, followed by stirring at 100°C for 3 hours. The reaction mixture was poured into water (200 mL), followed by extraction with ethyl acetate (500 mL). The organic layer was washed with water and saturated brine, and dried over anhydrous magnesium sulfate. The solvent was evaporated under reduced pressure, and then the residue was purified by silica gel column chromatography (hexane:ethyl acetate=70:30 to 0:100) to obtain tert-butyl[trans-4-({6-[2-(difluoromethyl)-1H-benzimidazol-1-yl]-2-morpholin-4-ylpyrimidin-4-yl}amino)cyclohexyl]carbamate (7.88 g) as a white powder.

### Example 23

To a solution of trans-4-({6-[2-(difluoromethyl)-1H-benzimidazol-1-yl]-2-morpholin-4-ylpyrimidin-4-yl}oxy)cyclohexanamine (40 mg) in N,N-dimethylacetamide (1 mL) were added triethylamine (0.05 mL) and bis(2-bromoethyl)ether (0.025 mL), and the mixture was heated by radiation with microwaves and stirred at 120°C for 1.5 hours. To the reaction mixture was added water (30 mL), followed by extraction with ethyl acetate (100 mL). The organic layer was washed with water and saturated brine, and dried over anhydrous sodium sulfate. The solvent was evaporated under reduced pressure, and then the residue was purified by amino silica gel column chromatography (hexane:ethyl acetate=60:40 to 20:80). A desired fraction was collected and concentrated. The residue was dissolved in methanol, and a 4 M hydrogen choride/1,4-dioxane solution was added thereto, followed by stirring at room temperature for 10 minutes and then concentrating, to obtain 2-(difluoromethyl)-1-{2-morpholin-4-yl-6-[(trans-4-morpholin-4-ylcyclohexyl)oxy]pyrimidin-4-yl}-1H-benzimidazole hydrochloride (36 mg) as a white powder.

### Example 24

To a solution of trans-4-({6-[2-(difluoromethyl)-1H-benzimidazol-1-yl]-2-morpholin-4-ylpyrimidin-4-yl}oxy)cyclohexanamine (50 mg) in N,N-dimethylformamide (0.5 mL) was added N,N-dimethylglycine (13 mg), 1-hydroxybenzotriazole (17 mg), and N-[3-(dimethylamino)propyl]-N'-ethylcarbodiimide hydrochloride (24 mg), and the mixture was stirred at room temperature overnight. To the reaction mixture was added water (50 mL), followed by extraction with ethyl acetate (100 mL). The organic layer was washed with saturated brine and dried over anhydrous sodium sulfate. The solvent was evaporated under reduced pressure, and then the residue was purified by amino silica gel column chromatography (hexane:ethyl acetate=50:50 to 0:100). A desired fraction was collected and concentrated. The residue was dissolved in chloroform (1 mL)-methanol (0.5 mL), and 4 M hydrogen choridc/1,4-dioxane solution (0.2 mL) was added thereto, followed by stirring at room temperature for 10 minutes and then concentrating, to obtain N-[trans-4-({6-[2-(difluoromethyl)-1H-benzimidazol-1-yl]-2-morpholin-4-ylpyrimidin-4-yl}oxy)cyclohexyl]-N,N-dimethylglycinamide hydrochloride (41 mg) as a white powder.

### Example 25

To a solution of trans-4-[({6-[2-(difluoromethyl)-1H-benzimidazol-1-yl]-2-morpholin-4-ylpyrimidin-4-yl}oxy)methyl]cyclohexanamine (55 mg) in methanol (1.65 mL) was added divinylsulfone (0.012 mL), and the mixture was stirred at room temperature overnight. The reaction mixture was concentrated and the residue was purified by silica gel column chromatography (chloroform:methanol=100:0 to 98:2). A desired fraction was collected and concentrated. The residue was dissolved in chloroform (1 mL)-methanol (0.5 mL), and 4 M hydrogen choride/1,4-dioxane solution (0.2 mL) was added thereto, followed by stirring at room temperature for 10 minutes, and then concentrating, to obtain 2-(difluoromethyl)-1-(6-{[trans-4-(1,1-dioxidethiomorpholin-4-yl)cyclohexyl]methoxy}-2-morpholin-4-ylpyrimidin-4-yl)-1H-benzimidazole hydrochloride (71 mg) as a white powder.

### Example 26

To a solution of 4-[2-(difluoromethyl)-1H-benzimidazol-1-yl]-N-{[trans-4-(methylamino)cyclohexyl]methyl}-6-morpholin-4-ylpyrimidin-2-amine (80 mg) in N,N-dimethylacetamide (0.8 mL) were added 1-bromo-2-fluoroethane (26 mg) and potassium carbonate (52 mg), and the mixture was heated by radiation with microwaves, followed by stirring at 100°C for 1 hour. To the reaction mixture was added water (50 mL), followed by extraction with ethyl acetate (50 mL). The organic layer was washed with saturated brine (50 mL) and dried over anhydrous magnesium sulfate. The solvent was evaporated under reduced pressure, and then the residue was purified by silica gel column chromatography (hexane:ethyl acetate=50:50) to obtain 4-[2-(difluoromethyl)-1H-benzimidazol-1-yl]-N-({trans-4-[(2-fluoroethyl)(methyl)amino]cyclohexyl} methyl)-6-morpholin-4-ylpyrimidin-2-amine (34 mg) as a colorless oily substance.

### Example 225

N-[trans-4-({4-[2-(difluoromethyl)-1H-benzimidazol-1-yl]-6-morpholin-4-ylpyrimidin-2-yl}amino)cyclohexyl]-3-(4-iodophenyl)propanamide (80 mg) was dissolved in dimethylformamide (800 µl), and zinc cyanide (40 mg), tris(dibenzylideneacetone) dipalladium (0) (16 mg), and 1,1'-bis(diphenylphosphino)ferrocene (13 mg) were added thereto, followed by stirring at 120°C for 4 hours. Filtration was performed through Celite to remove Pd. To the filtrate was added water (10 mL), followed by extraction with ethyl acetate (15 mL) and then washing with saturated brine. The organic layer was dried over anhydrous magnesium sulfate, the solvent was evaporated under reduced pressure, and then the residue was purified by amino silica gel column chromatography (hexane:ethyl acetate=40:60) to obtain 3-(4-cyanophenyl)-N-[trans-4-({4-[2-(difluoromethyl)-1H-benzimidazol-1-yl]-6-morpholin-4-ylpyrimidin-2-yl}amino) cyanohexyl]propanamide (41 mg) as a brown oily substance.

### Example 239

A mixture of N-[trans-4-({6-[2-(difluoromethyl)-1H-benzimidazol-1-yl]-2-morpholin-4-ylpyrimidin-4-yl}amino)cyclohexyl]ethenesulfoneamide (100 mg) and pyrrolidine (155 µl) was dissolved in isopropanol (1.6 mL), followed by stirring using a microwave reaction device at 170°C for 7 minutes. The reaction mixture was concentrated and the residue was dissolved in ethyl acetate (10 mL). Saturated ammonium chloride (10 mL) was added thereto, followed by extraction with ethyl acetate (100 mL). The organic layer was washed with water and saturated brine, and dried over anhydrous sodium sulfate. The solvent was evaporated under reduced pressure, and then purified by amino silica gel column chromatography (chloroform:methanol=100:0 to 99:1). A desired fraction was concentrated and the residue was dissolved in methanol (1 mL). A 4 M hydrogen choride/1,4-dioxane solution (0.05 mL) was added thereto, followed by stirring at room temperature for 10 minutes and then concentrating. N-[trans-4-({6-[2-(difluoromethyl)-1H-benzimidazol-1-yl]-2-morpholin-4-ylpyrimidin-4-yl}amino)cyclohexyl]-2-pyrrolidin-1-ylethanesulfonamide hydrochloride (105 mg) was obtained as a white powder.

### Example 245

4-[2-(Difluoromethyl)-1H-benzimidazol-1-yl)-6-morpholin-4-yl-N-{[trans-4-(tetrahydro-2H-thiopyran-4-ylamino)cyclohexyl]methyl}pyrimidin-2-amine (64 mg) was dissolved in methylene chloride (1.3 mL), and m-chloroperbenzoic acid (75%, containing water) (83 mg) was added thereto at 0°C, followed by stirring at room temperature for 1.5 hours. To the reaction mixture was added saturated aqueous sodium bicarbonate (10 mL), followed by extraction with chloroform (15 mL), and washing with water and saturated brine. The organic layer was dried over anhydrous magnesium sulfate. The solvent was evaporated under reduced pressure and the residue was purified by amino silica gel column chromatography (chloroform: methanol=1000:0 to 0:100) to obtain 4-[2-(difluoromethyl)-1H-benzimidazol-1-yl]-6-morpholin-4-yl-N-{[trans-4-(1-oxidetetrahydro-2H-thiopyran-4-ylamino)cyclohexyl]methyl}pyrimidin-2-amine (27 mg) as a white powder.

### Example 258

N-{[trans-4-(Aminomethyl)cyclohexyl]methyl}-4-[2-(difluoromethyl)-1H-benzimidazol-1-yl]-6-morpholin-4-ylpyridin-2-amine (80 mg) was dissolved in methanol (2.4 mL), and divinyl sulfone (17 µl) was added thereto, followed by stirring at room temperature for 3 hours. The reaction mixture was concentrated and the residue was purified by amino silica gel column chromatography (ethyl acetate alone) to obtain 4-[2-(difluoromethyl)-1H-benzimidazol-1-yl]-N-({trans-4-[(1,1-dioxidethiomorpholin-4-yl)methyl]cyclohexylmethyl)-6-morpholin-4-ylpyridin-2-amine (40 mg) as a white powder.

### Example 275

4-[2-(Difluoromethyl)-1H-benzimidazol-1-yl]-N-({trans-4-[(2-methoxyethyl)amino]cyclohexyl}methyl)-6-morpholin-4-ylpyridin-2-amine (70 mg) was dissolved in methanol (1.4 mL), and Molecular Sieve 4A (100 mg), [(1-ethoxy cyclopropyl)oxy](trimethyl)silane (163 µl), sodium tricyanoborohydride (54 mg), and acetic acid (78 µl) were added thereto, followed by stirring under heating and refluxing for 5 hours under a nitrogen atmosphere. Saturated aqueous sodium bicarbonate (10 mL) was added thereto, followed by extraction with ethyl acetate (15 mL). The organic layer was dried over anhydrous magnesium sulfate, and then the solvent was evaporated under reduced pressure. The residue was purified by amino silica gel column chromatography (hexane:ethyl acetate=90:10 to 40:60) to obtain N-(trans-4-[cyclopropyl(2-methoxyethyl)amino]cyclohexyl}methyl)-4-(2-(difluoromethyl)-1H-benzimidazol-1-yl]-6-morpholin-4-ylpyridin-2-amine (9.7 mg) as a white powder.

### Example 279

4-[2-(Difluoromethyl)-1H-dibenzimidazol-1-yl]-N-{[trans-4-(methylamino)cyclohexyl]methyl}-6-morpholin-4-ylpyridin-2-amine (80 mg) was dissolved in N-methyl pyrrolidone (800 µl), and potassium carbonate (130 mg) and 2,2-difluoroethyl trifluoromethanesulfonate (109 mg) were added thereto, followed by stirring at 200°C for 1 hour using a microwave reaction device. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane:ethyl acetate=30:70 to 60:40), and then silica gel column chromatography (ethyl acetate alone) to obtain N-({trans-4-[(2,2-difluoroethyl)(methyl)amino]cyclohexyl}methyl)-4-[2-(difluoromethyl)-1H-benzimidazol-1-yl]-6-morpholin-4-ylpyridin-2-amine (24 mg) as a colorless oily substance.

### Example 289

tert-Butyl{trans-4-[({4-[2-(difluoromethyl)-1H-benzimidazol-1-yl]-6-[cis-2,6-dimethylmorpholin-4-yl]pyrimidin-2-yl}amino)methy]cyclohexyl}carbamate (180 mg) was dissolved in 1,4-dioxane (1.8 mL), and a 4 M hydrogen choride/1,4-dioxane solution was added thereto, followed by stirring at room temperature for 4 hours. To the reaction mixture was added diisopropylether (5 mL), and the resulting solid was collected by filtration, washed with diisopropylether, and then dried under reduced pressure to obtain N-[(trans-4-aminocyclohexyl)methyl]-4-[2-(difluoromethyl)-1H-benzimidazol-1-yl]-6-[cis-2,6-dimethylmorpholin-4-yl]pyrimidin-2-amine dihydrochloride (131 mg) as a white powder.

### Example 295

4-[({4-[2-(Difluoromethyl)-1H-benzimidazol-1-yl]-6-morpholin-4-ylpyrimidin-2-yl}amino)methyl]cyclohexanone (550 mg) was dissolved in dichloroethane (11 mL), and tert-butyl piperazine-1-earboxylate (673 mg) was added thereto, followed by stirring for 10 minutes. Then, sodium triacetoxyborohydride (766 mg) was added thereto, followed by stirring at room temperature for 3 hours. Water (50 mL) was added thereto, followed by extraction with chloroform (200 mL) and washing with saturated brine. The organic layer was dried over anhydrous sodium sulfate, and then the solvent was evaporated under reduced pressure. The residue was purified by amino silica gel column chromatography (hexane:ethyl acctate=90:10 to 70:30) to obtain two kinds of compound as a white powder, respectively.
tert-butyl 4-{cis-4-[({4-[2-(difluoromethyl)-1H-benzimidazol-1-yl]-6-morpholin-4-ylpyrimidin-2-yl}amino)methyl]cyclohexyl}piperazine-1-carboxylate: 472 mg, the Rf value in amino silica gel TLC (hexane:ethyl acetate=1:1) was 0.42.
tert-butyl 4-{trans-4-[({4-[2-(difluoromethyl)-1H-benzimidazol-1-yl]-6-morpholin-4-ylpyrimidin-2-yl}amino)methyl]cyclohexyl}piperazine-1-carboxylate: 290 mg, the Rf value in amino silica gel TLC (hexane:ethyl acetate=1:1) was 0.30.

### Example 325

N-[(trans-4-Aminocyclohexyl)methyl]-4-[2-(difluoromethyl)-1H-benzimidazol-1-yl]-6-morpholin-4-ylpyrimidin-2-amine (250 mg) was dissolved in dimethylacetamide (2.5 mL), and 2-fluoropropyl-4-methylbenzenesulfonate (165 mg) and potassium carbonate (168 mg) were added thereto, followed by stirring at 100°C for 1 hour and then at 120°C for 1.5 hours using a microwave reaction device. Water (10 mL) was added thereto, followed by extraction with ethyl acetate (15 mL) and then washing with saturated brine. The organic layer was dried over anhydrous magnesium sulfate and the solvent was evaporated under reduced pressure. The residue was purified by amino silica gel column chromatography (hexane:ethyl acetate=40:60) to obtain 4-[2-(difluoromethyl)-1H-benzimidazol-1-yl]-N-({trans-4-[(2-fluoropropyl)amino]cyclohexyl}methyl)-6-morpholin-4-ylpyrimidin-2-amine (183 mg) as a white powder.

### Example 3 26

4-[2-(Difluoromethyl)-1H-benzimidazol-1-yl]-N-({trans-4-[(2-fluoroethyl)amino]cyclohexyl}methyl)-6-morpholin-4-ylpyridin-2-amine (50 mg) was dissolved in dimethylacetamide (500 µl), and potassium phosphate (140 mg) and 3-bromo propan-1-ol (26 µl) were added thereto, followed by stirring at 120°C for 2 hours using a microwave reaction device. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane:ethyl acetate=60:40) to obtain 3-[{trans-4-[({4-[2-(difluoromethyl)-1H-benzimidazol-1-yl]-6-morpholin-4-ylpyridin-2-yl} amino)methyl]cyclohexyl} (2-fluoroethyl)amino]propan-1-ol (21 mg) as a white powder.

### Example 328

N-[(trans-4-Aminocyclohexyl)methyl]-4-[2-(difluoromethyl)-1H-benzimidazol-1-yl]-6-morpholin-4-ylpyrimidin-2-amine (100 mg) was dissolved in dimethylacetamide (1 mL), and 2-fluoropropyl-4-methylbenzenesulfonate (127 mg) and potassium carbonate (101 mg) were added thereto, followed by stirring at 160°C for 1 hour using a microwave reaction device. Water (10 mL) was added thereto, followed by extraction with ethyl acetate (15 mL) and washing with saturated brine. The organic layer was dried over anhydrous magnesium sulfate, and then the solvent was evaporated under reduced pressure. The residue was purified by amino silica gel column chromatography (hexane:ethyl acetate=40:60) to obtain 3-{trans-4-[({4-[2-(difluoromethyl)-1H-benzimidazol-1-yl]-6-morpholin-4-ylpyrimidin-2-yl}amino)methyl]cyclohexyl}-5-methyl-1,3-oxazolidin-2-one (39 mg) as a white powder.

### Example 333

N-[(trans-4-Aminocyclohexyl)methyl]-4-[2-(difluoromethyl)-1H-benzimidazol-1-yl]-6-morpholin-4-ylpyrimidin-2-amine (50 mg) was dissolved in dimethylacetamide (500 µl), and 2-fluoropropyl-4-methylbenzenesulfonate (63 mg) and potassium phosphate (103 mg) were added thereto, followed by stirring at 200°C for 1 hour using a microwave reaction device. Water (10 mL) was added thereto, followed by extraction with ethyl acetate (15 mL) and then washing with saturated brine. The organic layer was dried over anhydrous magnesium sulfate and the solvent was evaporated under reduced pressure. The residue was purified by amino silica gel column chromatography (hexane:ethyl acetate=40:60) to obtain N-({trans-4-[bis(2-fluoropropyl)amino]cyclohexyl}methyl)-4-[2-(difluoromethyl)-1H-benzimidazol-1-yl]-6-morpholin-4-ylpyrimidin-2-amine (8 mg) as a white powder.

### Example 335

4-[2-(Difluoromethyl)-1H-benzimidazol-1-yl]-N-({trans-4-[(2-fluoro-1-methylethyl)amino]cyclohexyl}methyl)-6-morpholin-4-ylpyrimidin-2-amine (150 mg) was dissolved in dimethylacetamide (1.5 mL), and potassium carbonate (120 mg) and water (5 µl) were added thereto, followed by stirring at 160°C for 2 hours and at 180°C for 3 hours using a microwave reaction device. Water (10 mL) was added thereto, followed by extraction with ethyl acetate (15 mL) and then washing with saturated brine. The organic layer was dried over anhydrous magnesium sulfate and the solvent was evaporated under reduced pressure. The residue was purified by amino silica gel column chromatography (hexane:ethyl acetate=40:60), and then silica gel column chromatography (hexane:ethyl acetate=20:80) to obtain 3-{trans-4-[({4-[2-(difluoromethyl)-1H-benzimidazol-1-yl]-6-morpholin-4-ylpyrimidin-2-yl}amino)methyl]cyclohexyl}-4-methyl-1,3-oxazolidin-2-one (66 mg) as a white powder.

### Example 343

N-[(trans-4-Aminocyclohexyl)methyl]-4-[2-(difluoromethyl)-1H-benzimidazol-1-yl]-6-morpholin-4-ylpyrimidin-2-amine (200 mg) was dissolved in ethanol (4 mL), and 2-(fluoromethyl)oxirane (34 µl) and diisopropylethylamine (99 µl) were added thereto, followed by stirring at 80°C for 6 hours. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane:ethyl acetate=40:60) to obtain 1-({trans-4-[({4-[2-(difluoromethyl)-1H-benzimidazol-1-yl]-6-(morpholin-4-yl)pyrimidin-2-yl}ammo)methyl]cyclohcxyl}amino)-3-fluoropropan-2-ol (128 mg) as a white powder.

### Example 345

1-({trans-4-[({4-[2-(Difluoromethyl)-1H-benzimidazol-1-yl]-6-(morpholin-4-yl)pyrimidin-2-yl}amino)methyl]cyclohexyl}amino)-3-fluoropropan-2-ol (100 mg) was dissolved in dimethylacetamide (2 mL), and diethyl carbonate (34 µl) and sodium methoxide (30 mg) were added thereto, followed by stirring at room temperature for 2 hours. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane: ethyl acetate=40:60) to obtain 3-{trans-4-[({4-[2-(difluoromethyl)-1H-benzimidazol-1-yl]-6-(morpholin-4-yl)pyrimidin-2-yl}amino)methyl]cyclohexyl}-5-(fluoromethyl)-1,3-oxazolidin-2-one (50 mg) as a white powder.

### Example 353

1-({trans-4-[({4-[2-(Difluoromethyl)-1H-benzimidazol-1-yl]-6-(morpholin-4-yl)pyrimidin-2-yl}amino)methyl]cyclohexyl}amino)-3-butan-2-ol (40 mg) was dissolved in tetrahydrofuran (800 µl), and carbonyl diimidazole (73 mg) and triethylamine (32 µl) were added thereto, followed by stirring at room temperature for 3 hours. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane:ethyl acetate=40:60) to obtain 3-{trans-4-[({4-[2-(difluoromethyl)-1H-benzimidazol-1-yl]-6-(morpholin-4-yl)pyrimidin-2-yl}amino)methyl]cyclohexyl}-5-(ethyl)-1,3-oxazolidin-2-one (32 mg) as a white powder.

### Example 386

4-[2-(Difluoromethyl)-1H-benzimidazol-1-yl]-N-({trans-4-[(2-fluoro-1-methylethyl)amino]cyclohexyl}methyl)-6-morpholin-4-ylpyrimidin-2-amine (100 mg) was dissolved in dichloromethane (1.5 mL), and 1,4-dioxane-2,5-diol (28 mg) and sodium triacetoxyborohydride (61 mg) were added thereto, followed by stirring at room temperature for 3 hours. Water (10 mL) was added thereto, followed by extraction with chloroform (15 mL). The organic layer was dried over anhydrous sodium sulfate, and then the solvent was evaporated under reduced pressure. The residue was purified by amino silica gel column chromatography (hexane:ethyl acetate=60:40) to obtain 2-[{trans-4-[({4-[2-(difluoromethyl)-1H-benzimidazol-1-yl]-6-(morpholin-4-yl)pyrimidin-2-yl}amino)methyl]cyclohexyl}(1-fluoropropan-2-yl)amino]ethanol (80 mg) as a white powder.

### Example 417

trans-N-{4-[2-(Difluoromethyl)-1H-benzimidazol-1-yl]-6-[(3R)-3-methylmorpholin-4-yl]pyrimidin-2-yl}cyclohexane-1,4-diamine (100 mg) was dissolved in ethanol (2 mL), and 1H-1,2,3-benzotriazol-1-ylmethanol (17 mg) was added thereto, followed by stirring at room temperature for 5 hours. To the reaction mixture was added sodium tetrahydroborate (170 mg), followed by stirring at room temperature for 1 hour. Saturated aqueous sodium bicarbonate (100 mL) was added thereto, followed by extraction with ethyl acetate (100 mL) and washing with saturated brine (100 mL). The organic layer was dried over anhydrous magnesium sulfate and the solvent was evaporated under reduced pressure. The residue was purified by amino silica gel column chromatography (hexane:ethyl acetate=50:50 to 0:100) and chloroform:methanol (100:0 to 98:2) to obtain a free form (35 mg).
The free form was dissolved in dioxane (2 mL), and a 4 M hydrogen choride/1,4-dioxane solution (55 µl) and then diisopropylether (5 mL) were added thereto. The precipitated solid was collected by filtration and then washed with diisopropylether to obtain trans-N'-{4-[2-(difluoromethyl)-1H-benzimidazol-1-yl]-6-[(3R)-3-methylmorpholin-4-yl]pyrimidin-2-yl}-N,N-dimethylcyclohexane-1,4-diamine dihydrochloride (31 mg) as a white powder.

### Example 433

1-{trans-4-[({4-[2-(Difluoromethyl)-1H-benzimidazol-1-yl]-6-(morpholin-4-yl)pyrimidin-2-yl}amino)methyl]cyclohexyl}azetidin-3-ol (55 mg) was dissolved in dichloroethane (550 µl), and bis(2-methoxyethyl)amino sulfate fluoride (21 µl) was added thereto, followed by stirring at 0°C for 2 hours and at room temperature for 3 hours. Water (10 mL) was added thereto, followed by extraction with ethyl acetate (15 mL) and then washing with saturated brine. The organic layer was dried over anhydrous magnesium sulfate, and then the solvent was evaporated under reduced pressure. The residue was purified by amino silica gel column chromatography (hexane:ethyl acetate=60:40) to obtain 4-[2-(difluoromethyl)-1H-benzimidazol-1-yl]-N-{[trans-4-(3-fluoroazetidin-1-yl)cyclohexyl]methyl}-6-(morpholin-4-yl)pyrimidin-2-amine (8.7 mg) as a white powder.

### Example 436

N-[(trans-4-Aminocyclohexyl)methyl]-4-[2-(difluoromethyl)-1H-benzimidazol-1-yl]-6-morpholin-4-ylpyrimidin-2-amine (100 mg) was dissolved in chloroform (2 mL), and 1-chloro-2-isocyanatoethane (21 µl) and potassium carbonate (76 mg) were added thereto, followed by stirring at room temperature for 1 hour. After confirming the progress of urea formation, stirring was performed under heating and refluxing for 6 hours. Water (10 mL) was added thereto, followed by extraction with ethyl acetate (15 mL) and washing with saturated brine. The organic layer was dried over anhydrous magnesium sulfate, and then the solvent was evaporated under reduced pressure. The residue was purified by amino silica gel column chromatography (ethyl acetate) to obtain 1-{trans-4-[({4-[2-(difluoromethyl)-1H-benzimidazol-1-yl]-6-(morpholin-4-yl)pyrimidin-2-yl}amino)methyl]cyclohexyl}imidazolidin-2-one (35 mg) as a white powder.

### Example 439

1-({trans-4-[({4-[2-(Difluoromethyl)-1H-benzimidazol-1-yl]-6-[(3S)-3-methylmorpholin-4-yl]pyrimidin-2-yl} amino)methyl]cyclohexyl}amino)-2-methylpropan-2-ol (100 mg) was dissolved in ethanol (2 mL), and triethylamine (31 µl) and 1H-benzotriazal-1-ylmethanol (82 mg) were added thereto, followed by stirring at room temperature for 2 hours. To the reaction mixture was added lithium borohydride (4.8 mg), followed by further stirring at room temperature for 1 hour. Water (10mL) was added thereto, followed by extraction with ethyl acetate (15 mL) and washing with saturated brine. The organic layer was dried over anhydrous magnesium sulfate, and then the solvent was evaporated under reduced pressure. The residue was purified by amino silica gel column chromatography (hexane:ethyl acetate=70:30) to obtain 4-[2-(difluoromethyl)-1H-benzimidazol-1-yl]-N-{[trans-4-(5,5-dimethyl-1,3-oxazolidin-3-yl)cyclohexyl]methyl}-6-[(3S)-3-methylmorpholin-4-yl]pyrimidin-2-amine (68 mg) as a white powder.

### Example 540

1-({trans-4-[({4-[2-(Difluoromethyl)-1H-benzimidazol-1-yl)-6-(morpholin-4-yl)pyrimidin-2-yl}amino)methyl]cyclohexyl}amino)-2-methylpropan-2-ol (100 mg) was dissolved in dimethylacetamide (2 mL), and chloroacetyl chloride (36 µl) and potassium tert-butoxide (102 mg) were added thereto, followed by stirring at room temperature for 20 hours. Saturated brine (10 mL) was added thereto, followed by extraction with ethyl acetate (15 mL). The organic layer was dried over anhydrous sodium sulfate and the solvent was evaporated under reduced pressure. The residue was purified by amino silica gel column chromatography (hexane:ethyl acetate=60:40) to obtain 4-{trans-4-[({4-[2-(difluoromethyl)-1H-benzimidazol-1-yl]-6-(morpholin-4-yl)pyrimidin-2-yl}amino)methyl]cyclohexyl}-6,6-dimethylmorpholin-3-one (35 mg) as a white powder.

### Example 542

1-({trans-4-[({4-[2-(Difluoromethyl)-1H-benzimidazol-1-yl]-6-(morpholin-4-yl)pyrimidin-2-yl}amino)methyl]cyclohexyl}amino)-2-methylpropan-2-ol (100 mg) was dissolved in dimethylacetamide (2 mL), and methyl bromoacetate (22 µl) and triethylamine (35 µl) were added thereto, followed by stirring at 180°C for 3 hours using a microwave reaction device. Subsequently, 4-methylbenzenesulfonic acid (78 mg) was added thereto, followed by stirring at 100°C for 30 minutes using a microwave reaction device. The aqueous layer was alkalified with saturated aqueous sodium bicarbonate (10 mL), followed by extraction with ethyl acetate (15 mL). The organic layer was dried over anhydrous sodium sulfate, and then the solvent was evaporated under reduced pressure. The residue was purified by amino silica gel column chromatography (hexane:ethyl acetate=60:40) to obtain 4-{trans-4-[({4-[2-(difluoromethyl)-1H-benzimidazol-1-yl]-6-(morpholin-4-yl)pyrimidin-2-yl}amino)methyl]cyclohexyl}-6,6-dimethylmorpholin-2-one (20 mg) as a pale yellow powder.

### Example 554

Methyl-N-{trans-4-[({4-[2-(difluoromethyl)-1H-benzimidazol-1-yl]-6-(morpholin-4-yl)pyrimidin-2-yl}amino)methyl]cyclohexyl}glycinate (80 mg) was dissolved in tetrahydrofuran (1.6 mL), and a catalytic amount of zinc (II) chloride (2 mg) and ethyl magnesium bromide (1.06 M solution in tetrahydrofuran, 428 µl) were added thereto under ice-cooling, followed by stirring at 0°C for 1 hour. To the reaction mixture was added saturated brine (10 mL), followed by extraction with ethyl acetate (15 mL). The organic layer was dried over anhydrous magnesium sulfate, and then the solvent was evaporated under reduced pressure. The residue was purified by amino silica gel column chromatography (hexane:ethyl acetate=40.60) to obtain 3-[({trans-4-[({4-[2-(difluoromethyl)-1H-benzimidazol-1-yl]-6-(morpholin-4-yl)pyrimidin-2-yl}amino)methyl]cyclohexyl}amino)methyl}pentan-3-ol (17 mg) as a white powder.

### Example 555

1-{trans-4-[({4-[2-(Difluoromethyl)-1H-benzimidazol-1-yl]-6-(morpholin-4-yl)pyrimidin-2-yl}amino)methyl]cyclohexyl}piperidin-3-ylacetate (50 mg) was dissolved in methanol (500 µl), and potassium carbonate (36 mg) and water (5 µl) were added thereto, followed by stirring for 2 hours under heating and refluxing. To the reaction mixture was added saturated brine (10 mL), followed by extraction with ethyl acetate (15 mL). The organic layer was dried over anhydrous magnesium sulfate, and then the solvent was evaporated under reduced pressure. The residue was purified by amino silica gel column chromatography (hexane:ethyl acetate=40:60) to obtain 1-{trans-4-[({4-[2-(difluoromethyl)-1H-benzimidazol-1-yl]-6-(morpholin-4-yl)pyrimidin-2-yl}amino)methyl]cyclohexyl}piperidin-3-ol (26 mg) as a white powder.

### Example 570

trans-N-{6-[2-(Difluoromethyl)-1H-benzimidazol-1-yl]-2-morpholin-4-ylpyrimidin-4-yl}cyclohexane-1,4-diamine (100 mg) was dissolved in methylene chloride (1 mL), and triethylamine (47 µl) and 2,4-dibromobutanoyl chloride (45 µl) were added thereto under ice-cooling, followed by stirring at 0°C for 1 hour. To the reaction mixture was added water (30 mL), followed by extraction with ethyl acetate (30 mL) and washing with saturated brine. The organic layer was dried over anhydrous magnesium sulfate and the solvent was evaporated under reduced pressure. The residue was purified by silica gel column chromatography (hexane:ethyl acetate=70:30 to 50:50) to obtain a corresponding acylic form (80 mg 19%). The obtained acylic form was dissolved in tetrahydrofuran (1 mL), and potassium tert-butoxide (15 mg) was added thereto, followed by stirring at room temperature for 1 hour. To the reaction mixture was added water (30 mL), followed by extraction with ethyl acetate (30 mL) and washing with saturated brine. The organic layer was dried over anhydrous magnesium sulfate and the solvent was evaporated under reduced pressure. The residue was purified by silica gel column chromatography (hexane:ethyl acetate=50:50 to 0:100) to obtain 3-bromo-1-[trans-4-({6-[2-(difluoromethyl)-1H-benzimidazol-1-yl]-2-(morpholin-4-yl)pyrimidin-4-yl}amino)cyclohexyl]pyrrolidin-2-one (60 mg) as a pale yellow powder.

### Example A1

To a solution of trans-N-{4-[2-(difluoromethyl)-1H-benzimidazol-1-yl]-6-morpholin-4-yl-1,3,5-triazin-2-yl}cyclohexane-1,4-diamine (11.1 mg), propionic acid (1.9 mg), 1-hydroxybenzotriazole (3.4 mg), and triethylamine (3.5 µl) in N,N-dimethylformamide (1.0 mL) was added PS-Carbodiimide (Biotage Japan Ltd.) (100 mg) at room temperature, followed by stirring overnight. To the reaction mixture were added PS-Benzaldehyde (Biotage Japan Ltd.) (50 mg) and MP-Carbonate (Biotage Japan Ltd.) (50 mg) at room temperature, followed by stirring for 4 hours, and the insoluble materials were filtered. The filtrate was concentrated under reduced pressure to obtain N-[trans-4-({4-[2-(difluoromethyl)-1H-benzimidazol-1-yl]-6-morpholin-4-yl-1,3,5-triazin-2-yl}amino)cyclohexyl]propanamide (9.4 mg).
Here, the HPLC conditions used to determine RT were as shown below.
Column: Wakosil-II 5 C18AR (Wako Pure Chemical Industries, Ltd.) (Particle diameter: 5 µm, Internal diameter: 2.0 mm, and length: 30 mm)
Mobile Phase: A Solution, a 5 mM aqueous trifluoroacetic acid solution, B Solution, methanol
Flow rate: 1.2 mL/min; Detection wavelength: 254 nm; Column temperature: 35.0°C; Injection amount: 5 µL

**[Table 4]**

| Time (min) | A sol (%) | B sol (%) | Elution |
|---|---|---|---|
| 0 to 4 | 95→0 | 5→100 | Gradient |
| 4 to 4.5 | 0 | 100 | Isocratic |

### Example B 1

To ethanesulfonyl chloride (3.9 mg) was added a solution of trans-N-{4-[2-(difluoromethyl)-1H-benzimidazol-1-yl]-6-morpholin-4-yl-1,3,5-triazin-2-yl} cyclohexane-1,4-diamine (11.1 mg) and triethylamine (8.4 µl) in dichloromethane (0.5 mL), followed by stirring at room temperature overnight. To the reaction mixture were added PS-Benzaldehyde (Biotage Japan Ltd.) (50 mg), PS-Trisamine (Biotage Japan Ltd.) (50 mg), and dichloromethane (1.0 mL) at room temperature, followed by stirring for 4 hours, and the insoluble materials were filtered. The filtrate was concentrated under reduced pressure and the residue was purified by preparative HPLC to obtain N-[trans-4-({4-[2-(difluoromethyl)-1H-benzimidazol-1-yl]-6-morpholin-4-yl-1,3,5-triazin-2-yl}amino)cyclohexyl]ethanesulfonamide (8.0 mg).
Here, the HPLC conditions used to determine RT were as shown below.
Column: ACQUITY UPLC HSS T3 (Particle diameter: 1.8 µm, Internal diameter: 2.1 mm, and Length: 50 mm)
Mobile Phase: A Solution, a 0.1% aqueous formic acid solution, B Solution, a 0.1% formic acid-methanol solution
Flow rate: 0.70 mL/min; Detection wavelength: 254 nm; Column temperature: 40.0°C; Injection amount: 2 µL

**[Table 5]**

| Time (min) | A sol (%) | B sol (%) | Elution |
|---|---|---|---|
| 0 to 3 | 95→10 | 5→90 | Gradient |
| 3 to 4 | 10 | 90 | Isocratic |

### Example C 1

To a solution of trans-N-{4-[2-(difluoromethyl)-1H-benzimidazol-1-yl]-6-morpholin-4-yl-1,3,5-triazin-2-yl}cyclohexane-1,4-diamine (11.1 mg) and acetaldehyde (1.1 mg) in N,N-dimethylformamide (0.3 mL)/acetic acid (0.03 mL) was added MP-Triacetoxyborohydride (Biotage Japan Ltd.) (75 mg) at room temperature, followed by stirring overnight. To the reaction mixture were added PS-Benzaldehyde (Biotage Japan Ltd.) (50 mg) and N,N-dimethylformamide (0.3 mL) at room temperature, followed by stirring for 4 hours. Purification was performed by solid extraction using BondElut (registered trademark) SCX (eluent: concentrated aqueous ammonia/methanol=1/9). The filtrate was concentrated under reduced pressure and then the residue was purified by preparative HPLC to obtain trans-N-{4-[2-(difluoromethyl)-1H-benzimidazol-1-yl]-6-morpholin-4-yl-1,3,5-triazin-2-yl)-N'-ethylcyclohexane-1,4-diamine (0.6 mg).
The HPLC conditions used to determine RT were the same as in Example B1.

### Example D 1

To a solution of trans-N-{4-[2-(difluoromethyl)-1H-benzimidazol-1-yl]-6-morphalin-4-ylpyrimidin-2-yl}cyclohexane-1,4-diamine (11.1 mg), propionic acid (1.9 mg), 1-hydroxybenzotriazole (3.4 mg), and triethylamine (3.5 µl) in N,N-dimethylformamide (1.0 mL) was added PS-Carbodiimide (Biotage Japan Ltd.) (100 mg) at room temperature, followed by stirring overnight. To the reaction mixture were added PS-Benzaldehyde (Biotage Japan Ltd.) (50 mg) and MP-Carbonate (Biotage Japan Ltd.) (50 mg) at room temperature, followed by stirring for 4 hours, and the insoluble materials were filtered. The filtrate was concentrated under reduced pressure to obtain N-[trans-4-({4-[2-(difluoromethyl)-1H-benzimidazol-1-yl}-6-morpholin-4-ylpyrimidin-2-yl}amino)cyclohexyl]propanamide (6.7 mg).
The HPLC conditions used to determine RT were the same as in Example B1.

### Example E 1

To methanesulfonyl chloride (3.4 mg) was added a solution of trans-N-{4-[2-(difluoromethyl)-1H-benzimidazol-1-yl]-6-morpholin-4-ylpyrimidin-2-yl}cyclohexane-1,4-diamine (11.1 mg) and triethylamine (8.4 µl) in dichloromethane (0.5 mL), followed by stirring at room temperature overnight. To the reaction mixture were added PS-Benzaldehyde (Biotage Japan Ltd.) (50 mg), PS-Trisamine (Biotage Japan Ltd.) (50 mg), and dichloromethane (1.0 mL) at room temperature, followed by stirring for 4 hours, and the insoluble materials were filtered. The filtrate was concentrated under reduced pressure, and then the residue was purified by preparative HPLC to obtain N-[trans-4-({4-[2-(difluoromethyl)-1H-benzimidazol-1-yl]-6-morpholin-4-ylpyrimidin-2-yl }amino)cyclohexyl]methanesulfonamide (6.5 mg).
The HPLC conditions used to determine RT were the same as in Example B1.

### Example F1

To a solution of trans-N-{4-[2-(difluoromethyl)-1H-benzimidazol-1-yl]-6-morpholin-4-ylpyrimidin-2-yl}cyclohexane-1,4-diamine (11.1 mg) and acetaldehyde (1.1 mg) in N,N-dimethylformamide (0.3 mL)/acetic acid (0.03 mL) was added MP-Triacetoxyborohydride (Biotage Japan Ltd.) (75 mg) at room temperature, followed by stirring overnight. To the reaction mixture were added PS-Benzaldehyde (Biotage Japan Ltd.) (50 mg) and N,N-dimethylformamide (0.3 mL) at room temperature, followed by stirring for 4 hours. Purification was performed by solid extraction using BondElut (registered trademark) SCX (eluent: concentrated aqueous ammonia/methanol=1/9). The filtrate was concentrated under reduced pressure, and then the residue was purified by preparative HPLC to obtain trans-N-{4-[2-(difluoromethyl)-1H-benzimidazol-1-yl]-6-morpholin-4-ylpyrimidin-2-yl}-N'-ethylcyclohexane-1,4-diamine (0.5 mg).
The HPLC conditions used to determine RT were the same as in Example B 1.

### Example G1

To a solution of trans-N-{6-[2-(difluoromethyl)-1H-benzimidazol-1-yl]-2-morpholin-4-ylpyrimidin-4-yl}cyclohexane-1,4-diamine (11.1 mg), propionic acid (1.9 mg), 1-hydroxybenzotriazole (3.4 mg), and triethylamine (6.9 µl) in N,N-dimethylformamide (1.0 mL) was added PS-Carbodiimide (Biotage Japan Ltd.) (100 mg) at room temperature, followed by stirring overnight. To the reaction mixture were added PS-Benzaldehyde (Biotage Japan Ltd.) (50 mg) and MP-Carbonate (Biotage Japan Ltd.) (50 mg) at room temperature, followed by stirring for 4 hours, and the insoluble materials were filtered. The filtrate was concentrated under reduced pressure, and then the residue was purified by preparative HPLC to obtain N-[trans-4-({6-[2-(difluoromethyl)-1H-benzimidazol-1-yl]-2-morpholin-4-ylpyrimidin-4-yl}amino)cyclohexyl]propanamide (7.4 mg).
The HPLC conditions used to determine RT were the same as in Example B1.

### Example H1

To 3,3,3-trifluoropropane-1-sulfonyl chloride (5.9 mg) were added a solution of trans-N- (6-[2-(difluoromethyl)-1H-benzimidazol-1-yl]-2-morpholin-4-ylpyrimidin-4-yl}cyclohexane-1,4-diamine (11.1 mg) and triethylamine (8.4 µl) in dichloromethane (0.5 mL), followed by stirring at room temperature overnight. To the reaction mixture were added PS-Benzaldehyde (Biotage Japan Ltd.) (50 mg), PS-Trisamine (Biotage Japan Ltd.) (50 mg), and dichloromethane (1.0 mL) at room temperature, followed by stirring for 4 hours, and the insoluble materials were filtered. The filtrate was concentrated under reduced pressure, and then the residue was purified by preparative HPLC to obtain N-[trans-4-({6-[2-(difluoromethyl)-1H-benzimidazol-1-yl]-2-morpholin-4-ylpyrimidin-4-yl}ammo)cyclohexyl]-3,3,3-trifluoropropane-1-sulfonamide (8.9 mg).
The HPLC conditions used to determine RT were the same as in Example B1.

### Example J1

To a solution of trans-N-{6-[2-(difluoromethyl)-1H-benzimidazol-1-yl]-2-morpholin-4-ylpyrimidin-4-yl}cyclohexane-1,4-diamine (11.1 mg) and acetaldehyde (1.1 mg) in N,N-dimethylformamide (0.3 mL)/acetic acid (0.03 mL) was added MP-Triacetoxyborohydride (Biotage Japan Ltd.) (75 mg) at room temperature, followed by stirring overnight. To the reaction mixture were added PS-Benzaldehyde (Biotage Japan Ltd.) (50 mg) and N,N-dimethylformamide (0.3 mL) at room temperature, followed by stirring for 4 hours. Purification was performed by solid extraction using BondElut (registered trademark) SCX (eluent: concentrated aqueous ammonia/methanol=1/9). The filtrate was concentrated under reduced pressure, and then the residue was purified by preparative HPLC to obtain trans-N-{6-[2-(difluoromethyl)-1H-benzimidazol-1-yl]-2-morpholin-4-ylpyrimidin-4-yl}-N'-ethylcyclohexane-1,4-diamine (0.3 mg).
The HPLC conditions used to determine RT were the same as in Example B1.

The structures of the respective Example compounds are shown in Tables 17 to 88, and the production processes and physicochemical data are shown in Tables 89 to 129. Further, the structures of the respective Example compounds prepared in the same manner as in the methods of Examples A1 to J1 are shown in Tables 130 to 165, and the physicochemical data are shown in Tables 166 to 172.

A case where two or more numerals are shown in the column of Syn in physicochemical data indicates that preparation was conducted in order as described.

**[Table 6]**

| PEx | PSyn | Str | DATA |
|---|---|---|---|
| 1 | 1 | 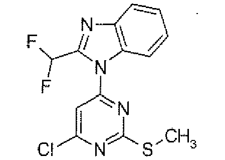 | ESI+: 327 |
| 2 | 2 | 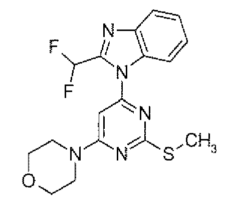 | ESI+: 378 |
| 3 | 3 | 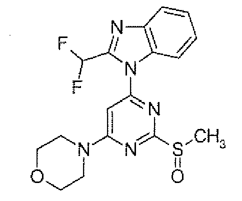 | ESI+: 394 |
| 4 | 4 | 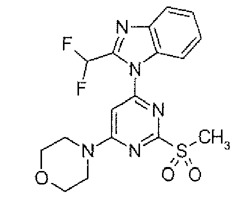 | ESI+: 410 |
| 5 | 5 | 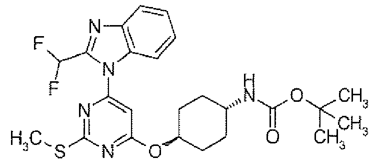 | ESI+: 528 [M+Na] |

**[Table 7]**

| PEx | PSyn | Str | DATA |
|---|---|---|---|
| 6 | 6 | 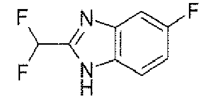 | ESI+: 187 |
| 7-1 | 7 | 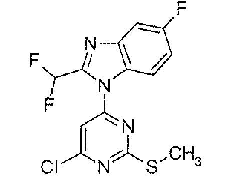 | ESI+: 345 |
| 7-2 | 7 | 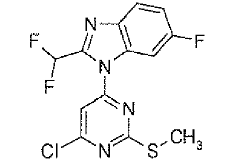 | ESI+: 345 |
| 8 | 8 | 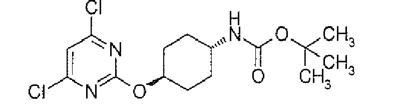 | ESI+: 384 [M+Na] |
| 9 | 1 | 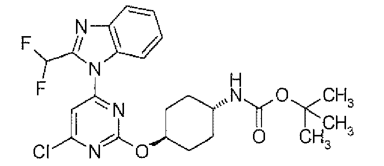 | ESI+: 516 [M+Na] |
| 10 | 2 | 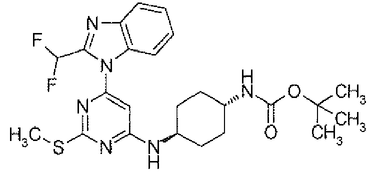 | ESI+: 527 [M+Na] |

**[Table 8]**

| PEx | PSyn | Str | DATA |
|---|---|---|---|
| 11 | 2 | 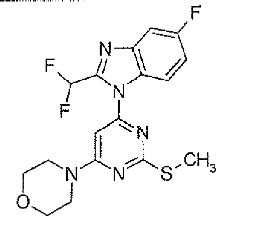 | E8I+: 396 |
| 12 | 2 | 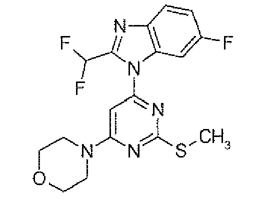 | ESI+: 396 |
| 13 | 5 | 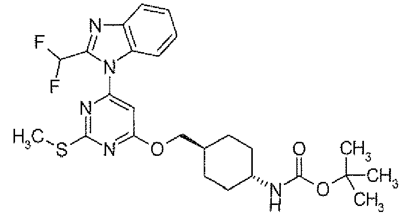 | ESI+: 542 [M+Na] |

**[Table 9]**

| PEx | PSyn | Str | DATA |
|---|---|---|---|
| 14 | 2 | 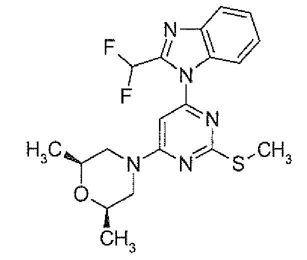 | ESI+:406 |
| 15 | 3 | 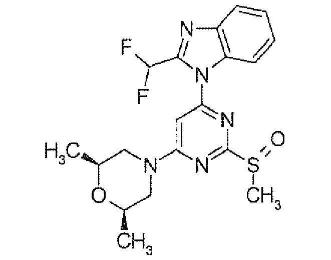 | ESI+:422 |
| 16 | 16 | 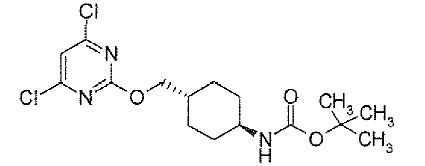 | ESI+: 398 [M+Na] |
| 17 | 1 | 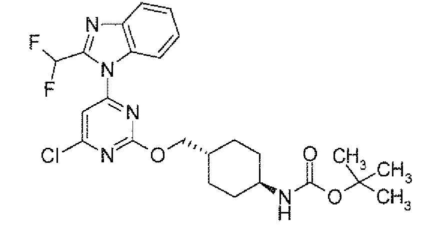 | ESI+: 530 [M+Na] |

**[Table 10]**

| PEx | PSyn | Str | |
|---|---|---|---|
| 18 | 2 | 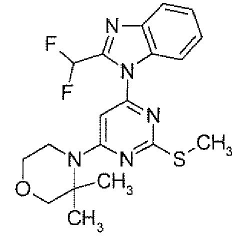 | ESI+: 406 |
| 19 | 3 | 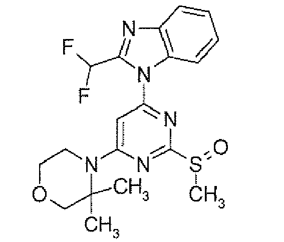 | ESI+:422 |
| 20 | 2 | 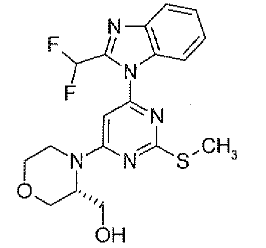 | ESI+: 408 |
| 21 | 2 | 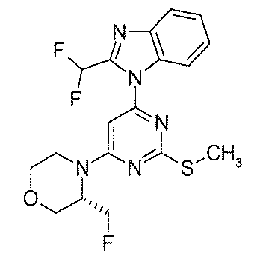 | ESI+: 410 |

**[Table 11]**

| PEx | PSyn | Str | DATA |
|---|---|---|---|
| 22 | 4 | 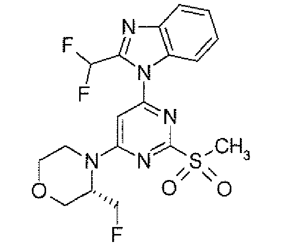 | ESI+: 442 |
| 23 | 23 | 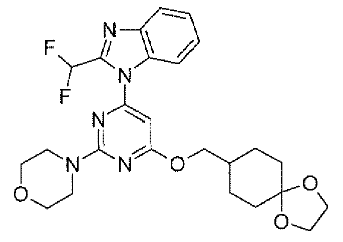 | ESI+: 502 |
| 24 | 24 | 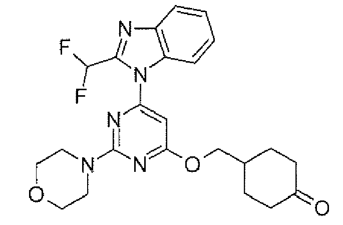 | ESI+:458 |
| 25 | 3 | 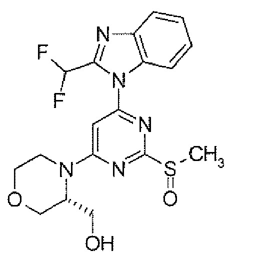 | ESI+: 424 |

**[Table 12]**

| PEx | PSyn | Str | DATA |
|---|---|---|---|
| 26 | 1 | 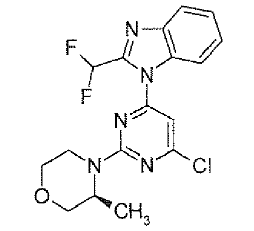 | ESI+: 380 |
| 27 | 5 | 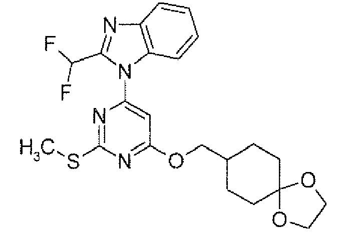 | ESI+: 463 |
| 28 | 24 | 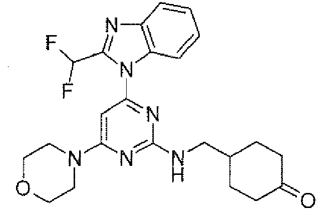 | ESI+: 479 [M+Na] |
| 29 | 1 | 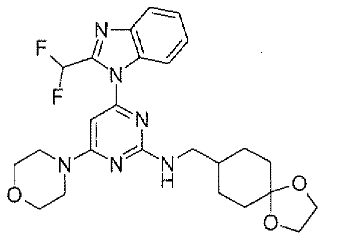 | ESI+: 523 [M+Na] |

**[Table 13]**

| PEx | PSyn | Str | DATA |
|---|---|---|---|
| 30 | 3 | 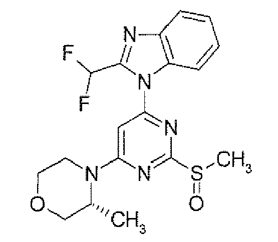 | ESI+: 408 |
| 31 | 3 | 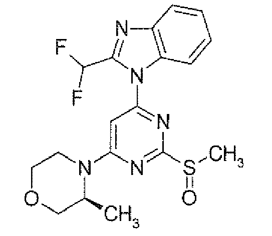 | ESI+: 408 |
| 32 | 3 | 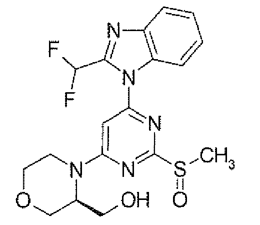 | ESI+: 424 |
| 33 | 3 | 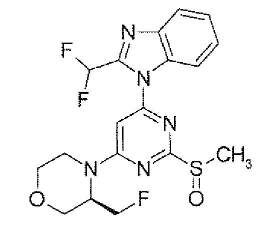 | ESI+: 426 |

**[Table 14]**

| PEx | PSyn | Str | DATA |
|---|---|---|---|
| 34 | 3 | 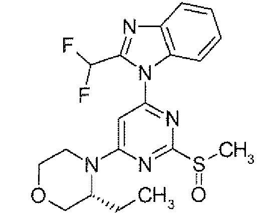 | ESI+: 422 |
| 35 | 1 | 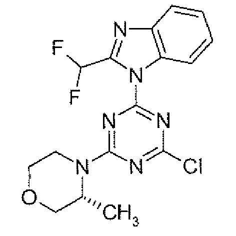 | ESI+:381 |
| 36 | 2 | 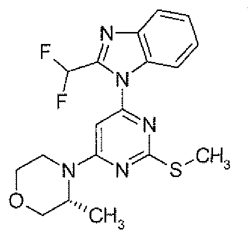 | ESI+: 392 |
| 37 | 2 | 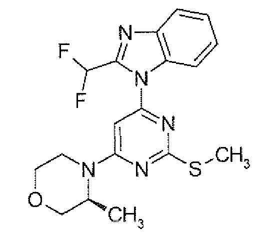 | ESI+: 392 |

**[Table 15]**

| PEx | PSyn | Str | DATA |
|---|---|---|---|
| 38 | 2 | 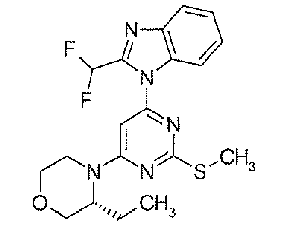 | ESI+: 406 |
| 39 | 2 | 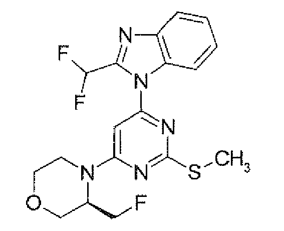 | ESI+: 410 |
| 40 | 2 | 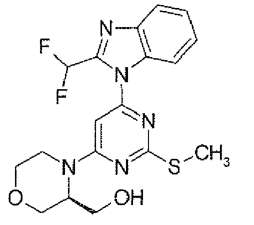 | ESI+: 408 |
| 41 | 2 | 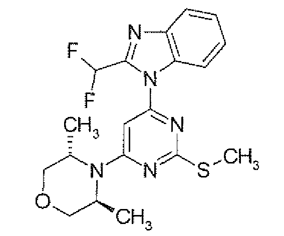 | ESI+: 406 |

**[Table 161**

| PEx | PSyn | Str | DATA |
|---|---|---|---|
| 42 | 2 | 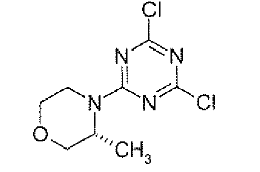 | ESI+: 249 |
| 43 | 2 | 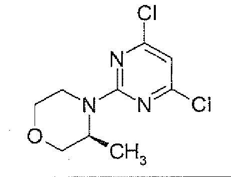 | ESI+: 248 |

**[Table 17]**

| Ex | Str | | Ex | Str |
|---|---|---|---|---|
| 1 | 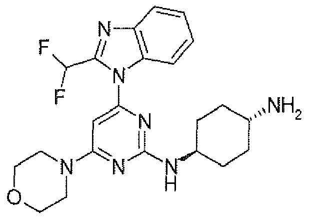 | | 5 | 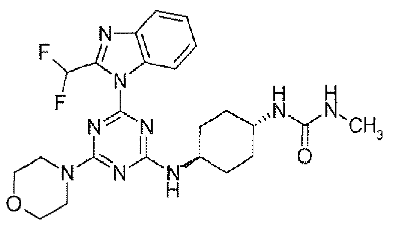 |
| 2 | 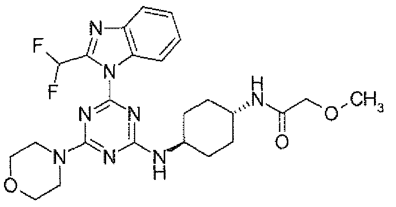 | | 6 | 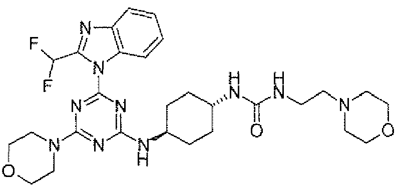 |
| 3 | 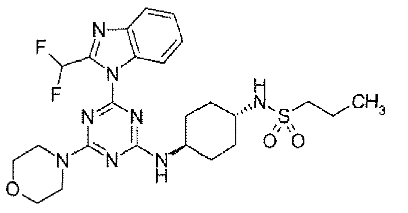 | | 7 | 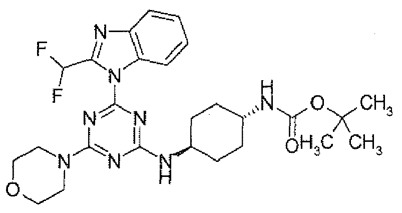 |
| 4 | 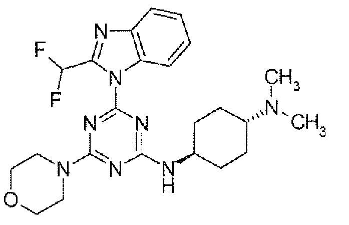 | | 8 | 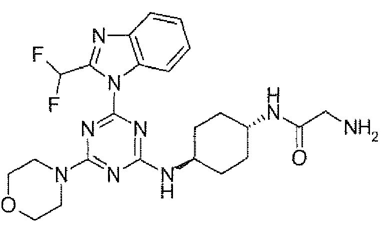 |

**[Table 18]**

| Ex | Str | | Ex | Str |
|---|---|---|---|---|
| 9 | 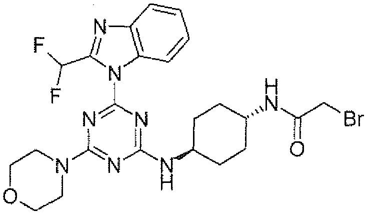 | | 13 | 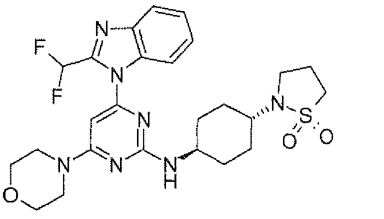 |
| 10 | 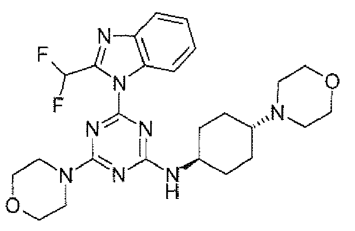 | | 14 | 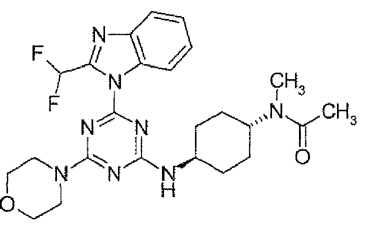 |
| 11 | 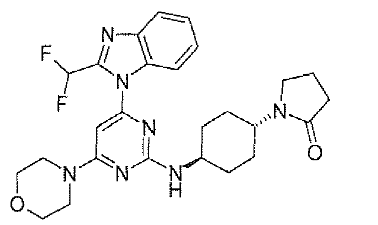 | | 15 | 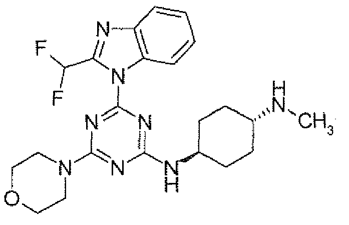 |
| 12 | 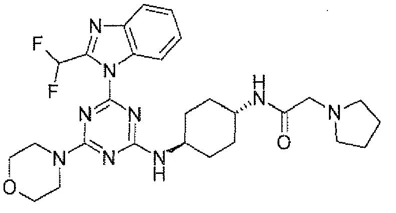 | | 16 | 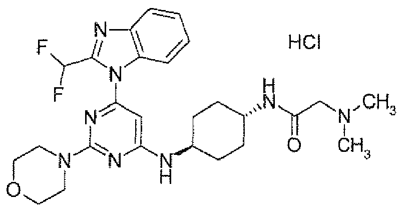 |

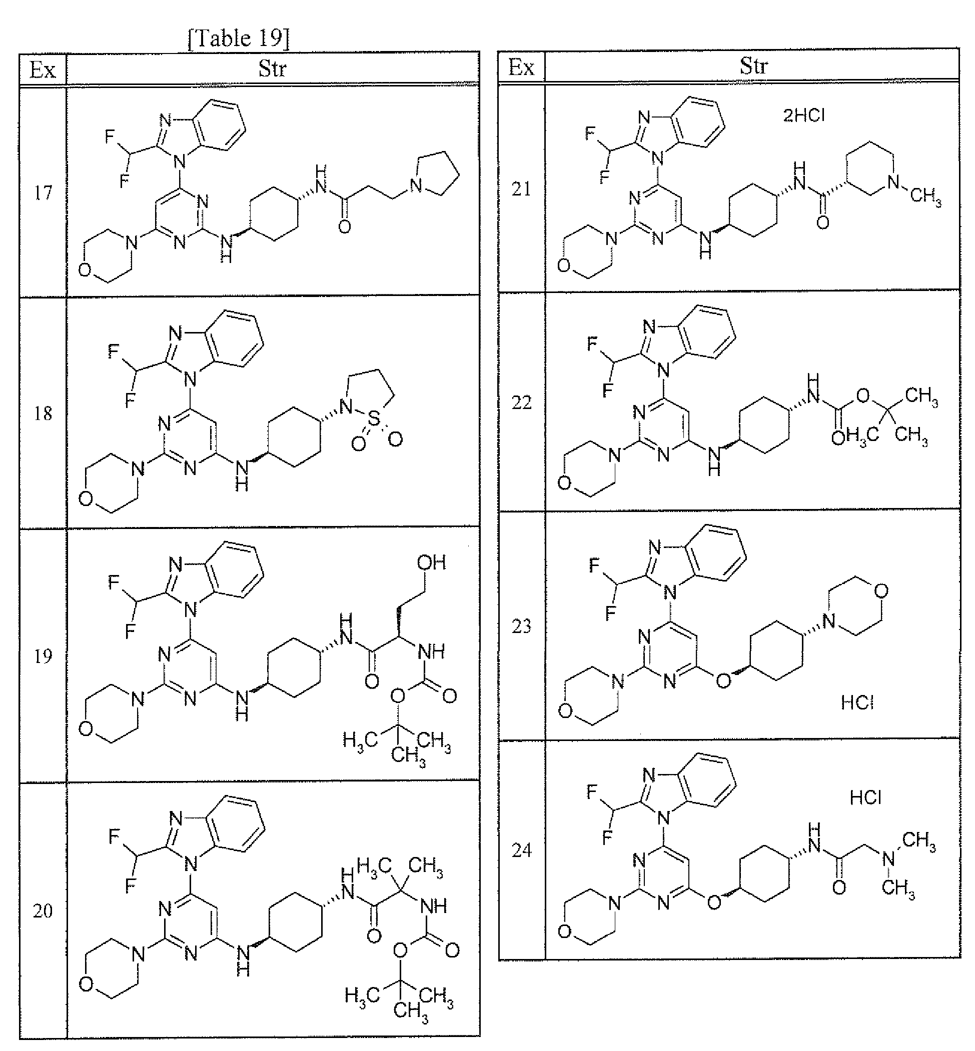

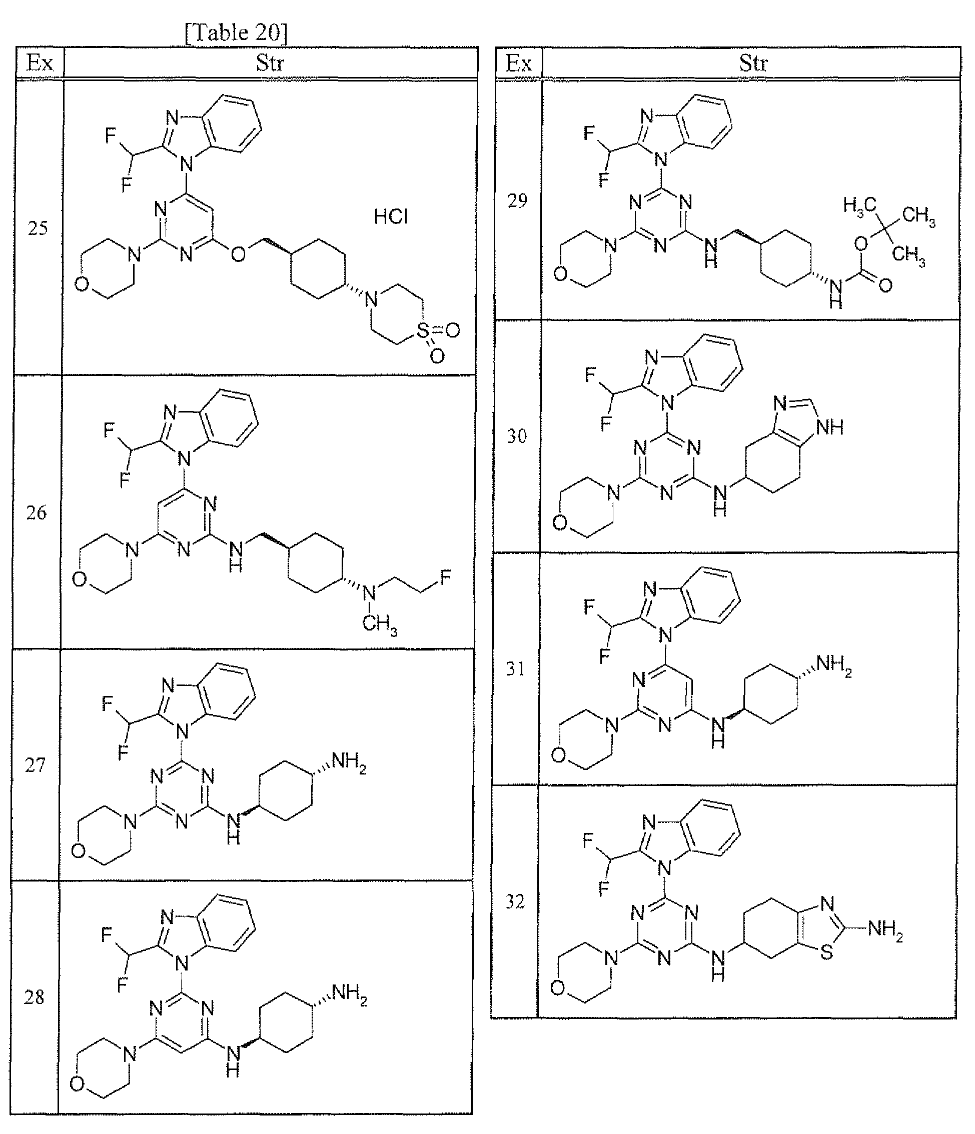

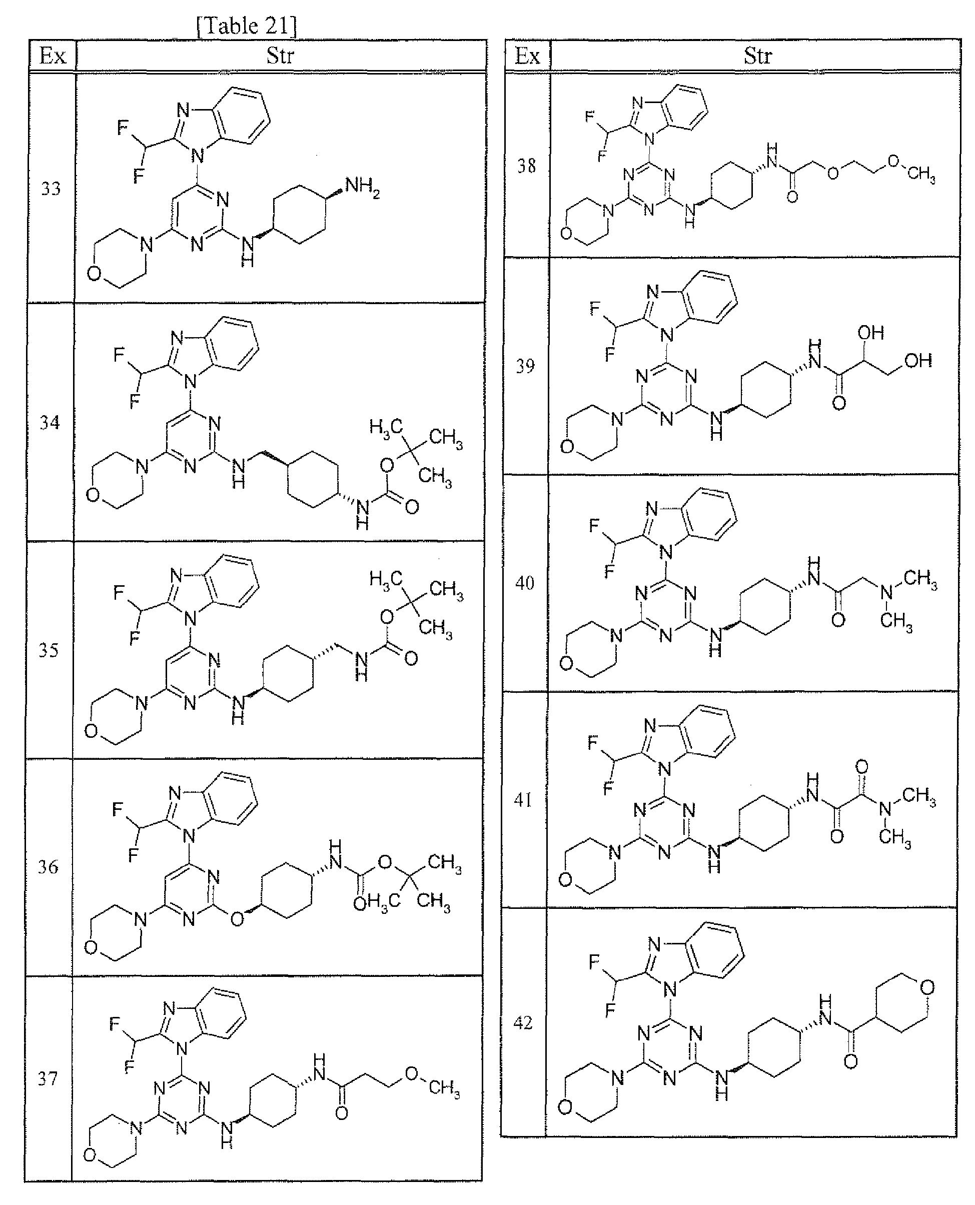

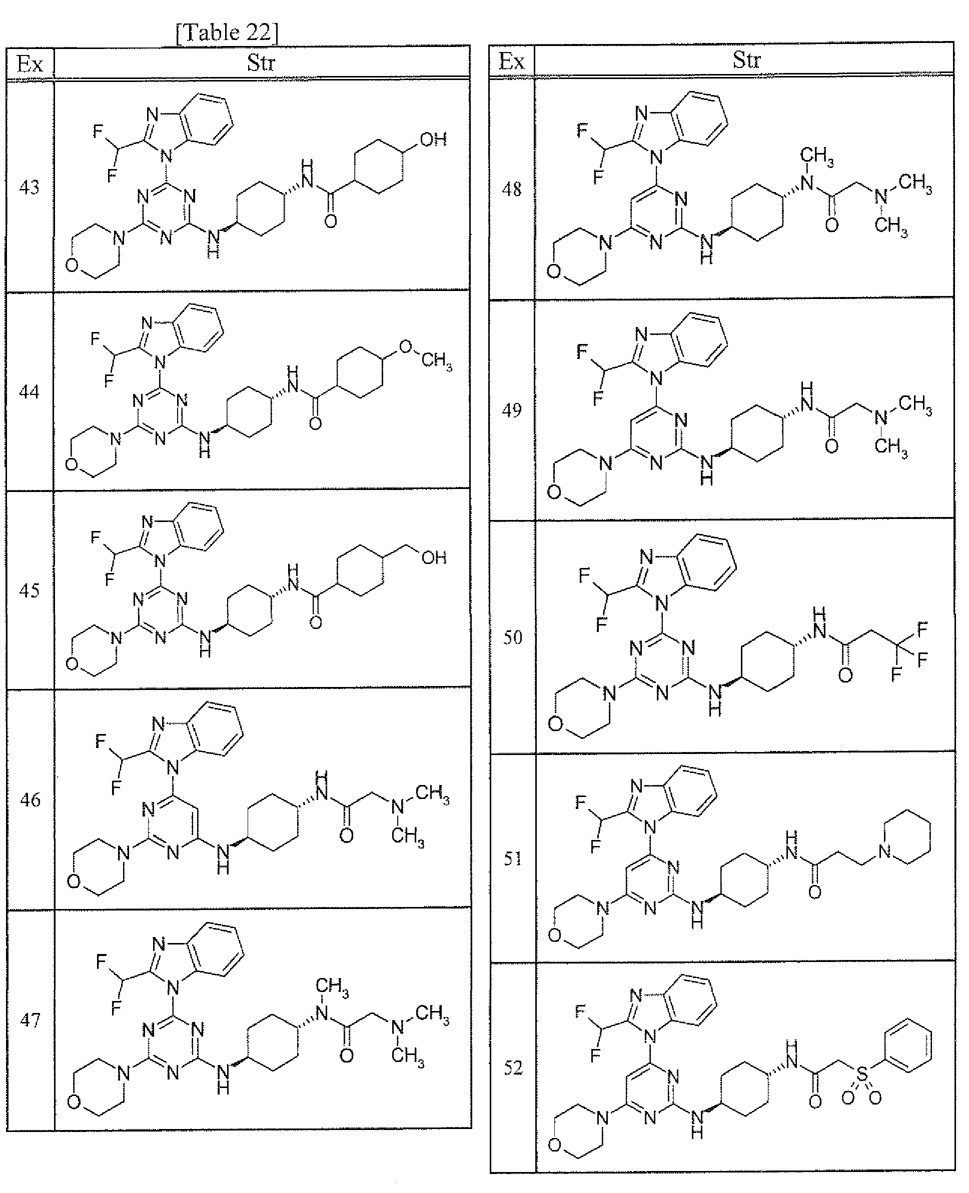

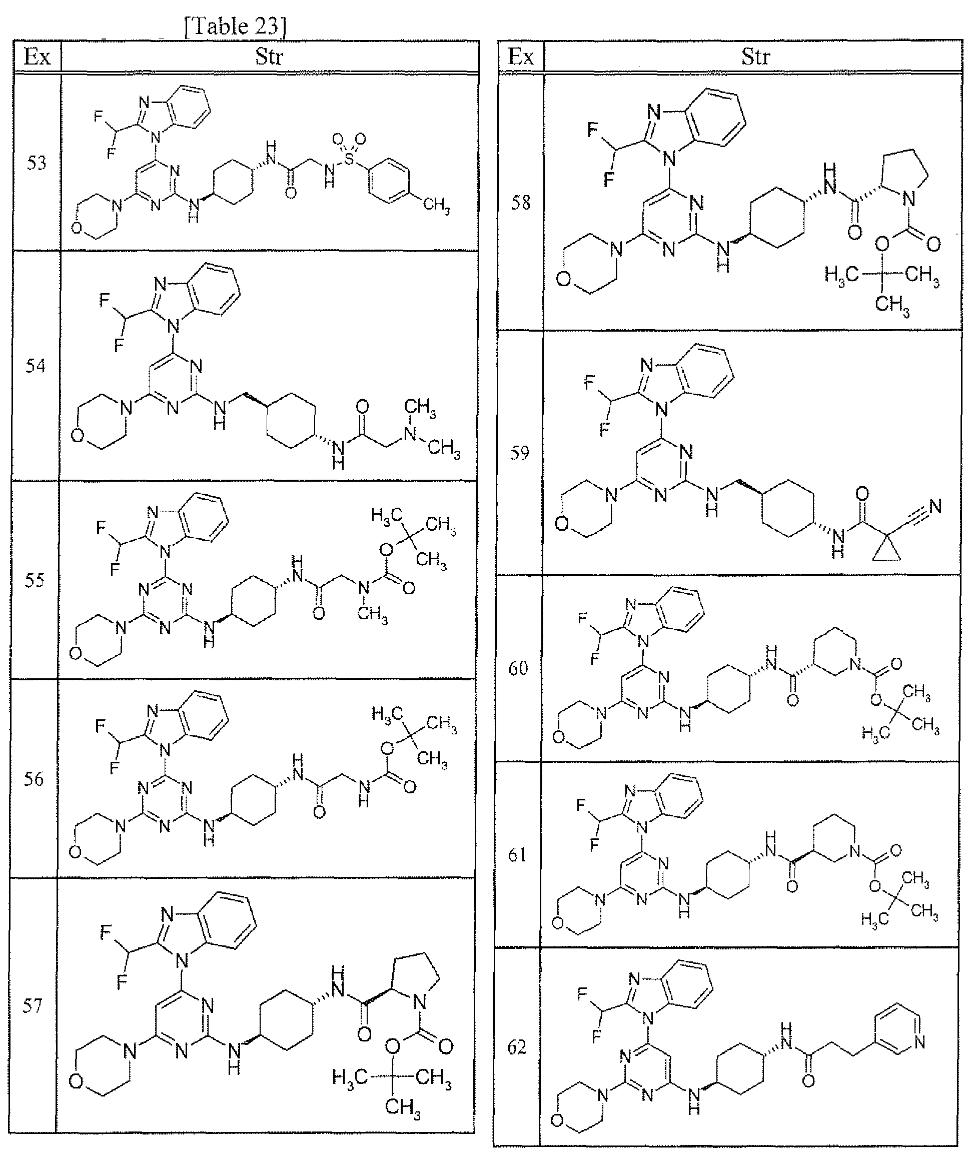

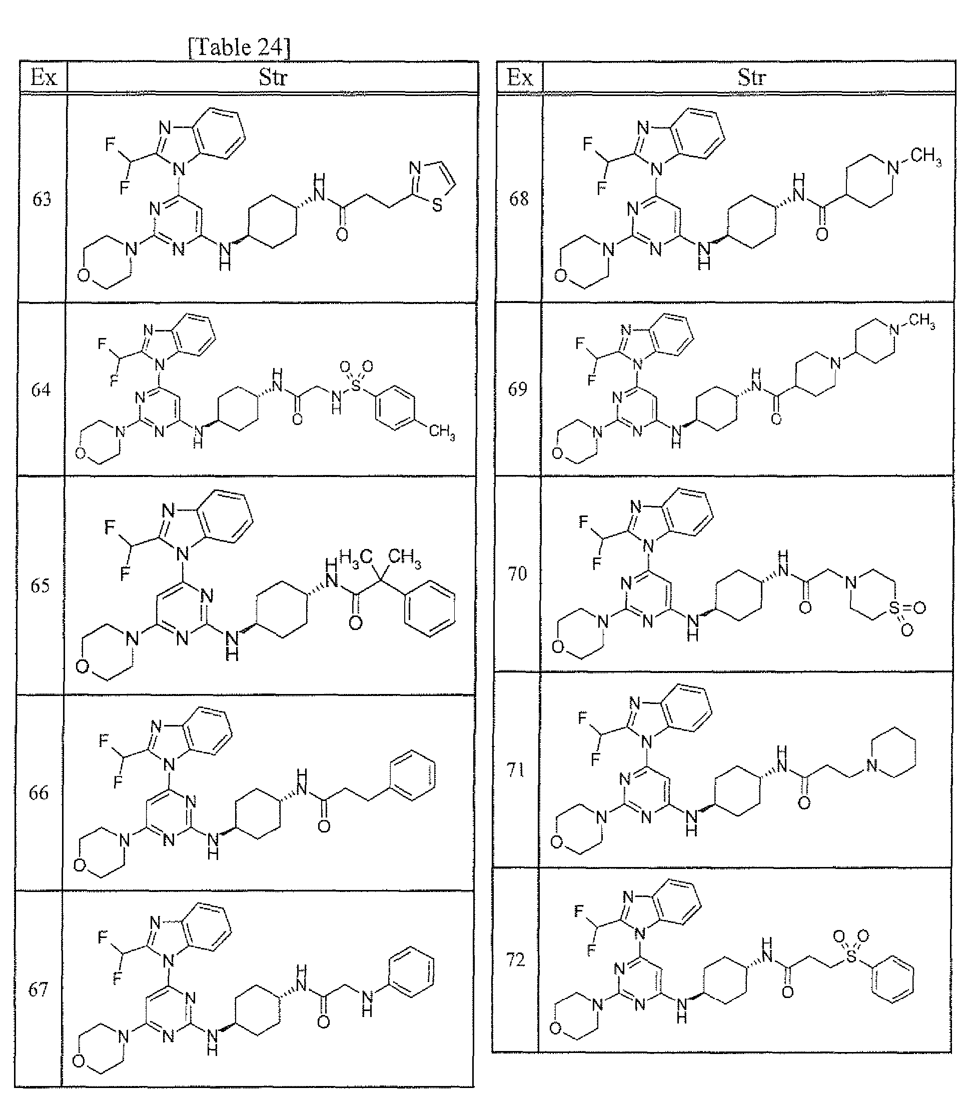

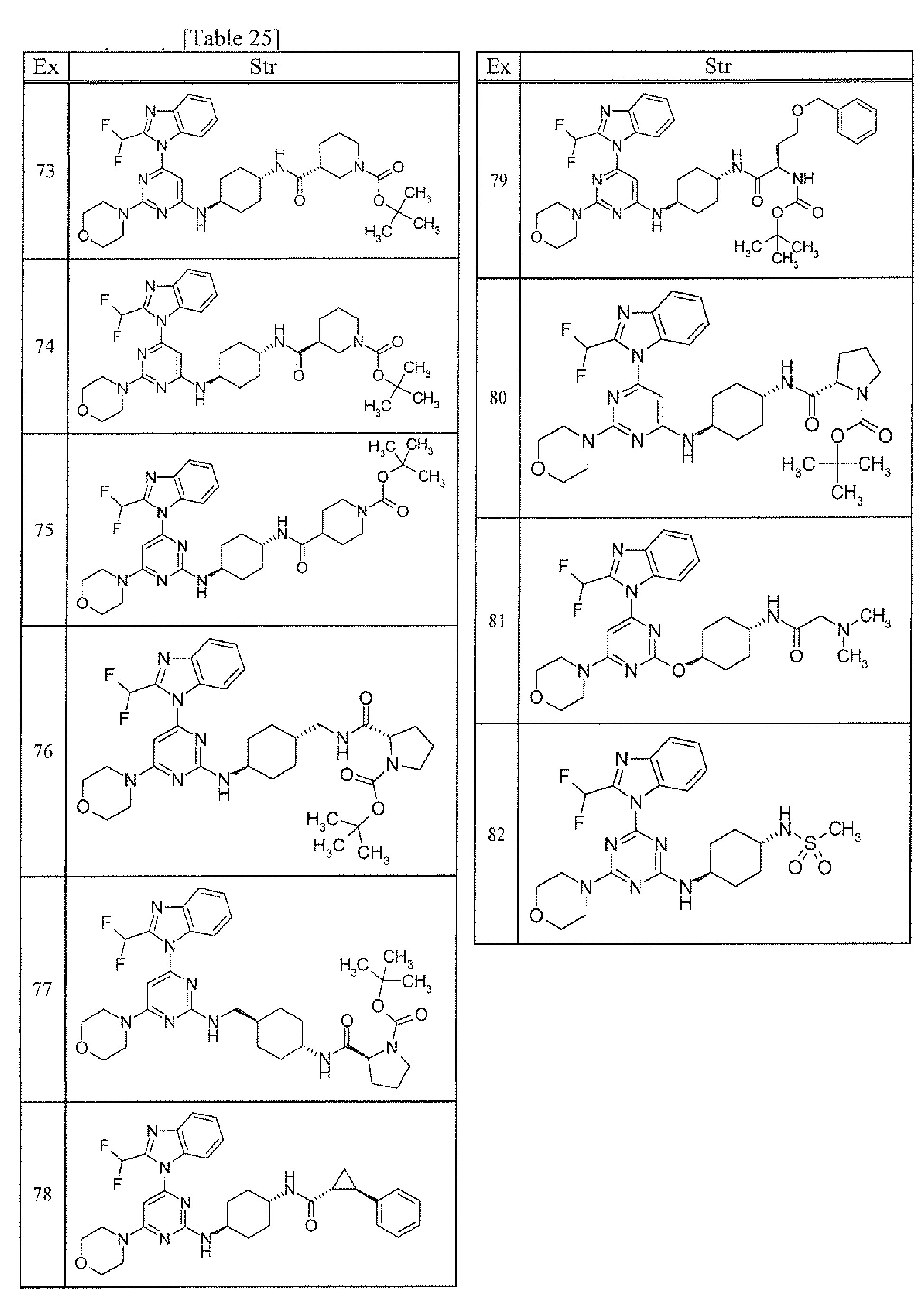

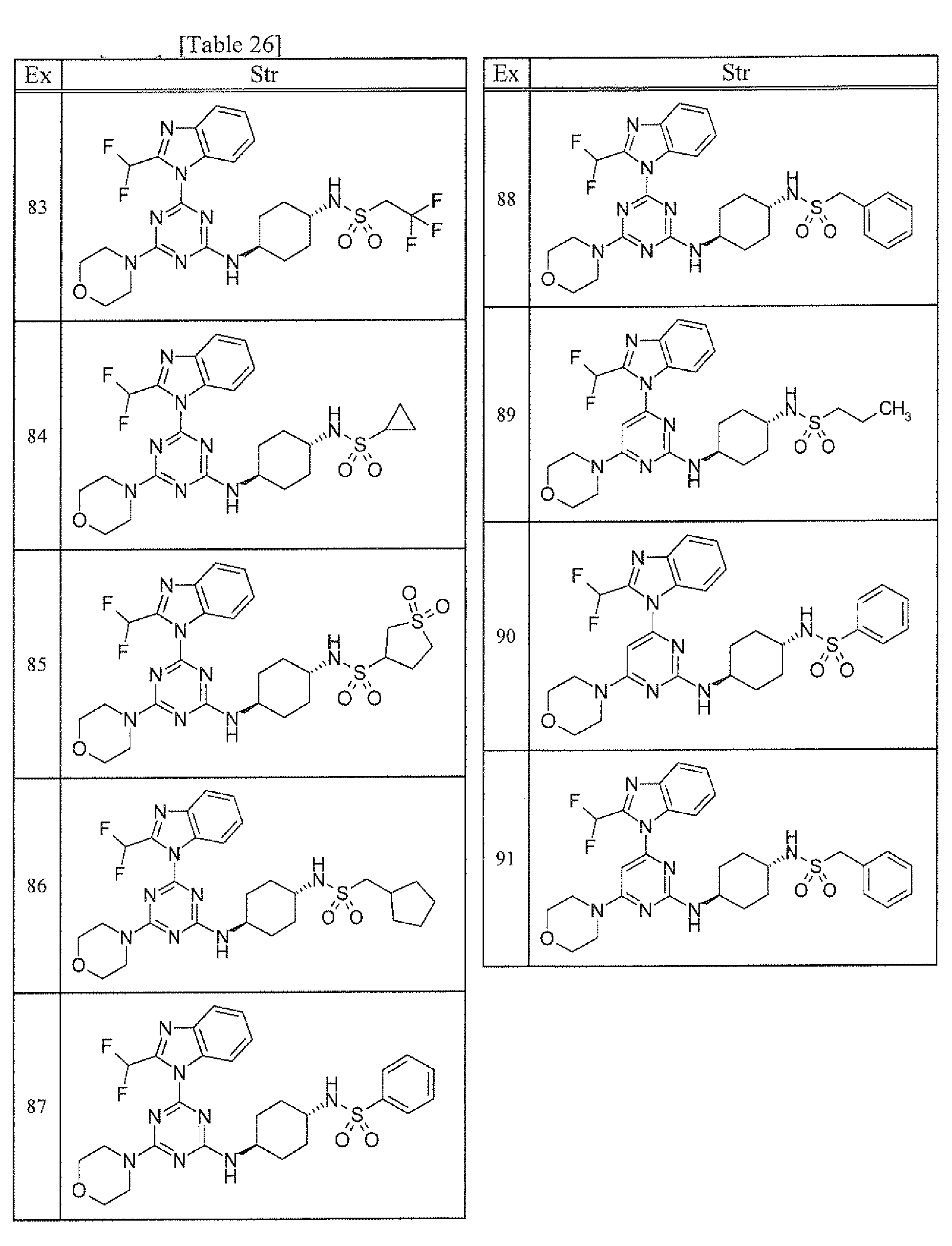

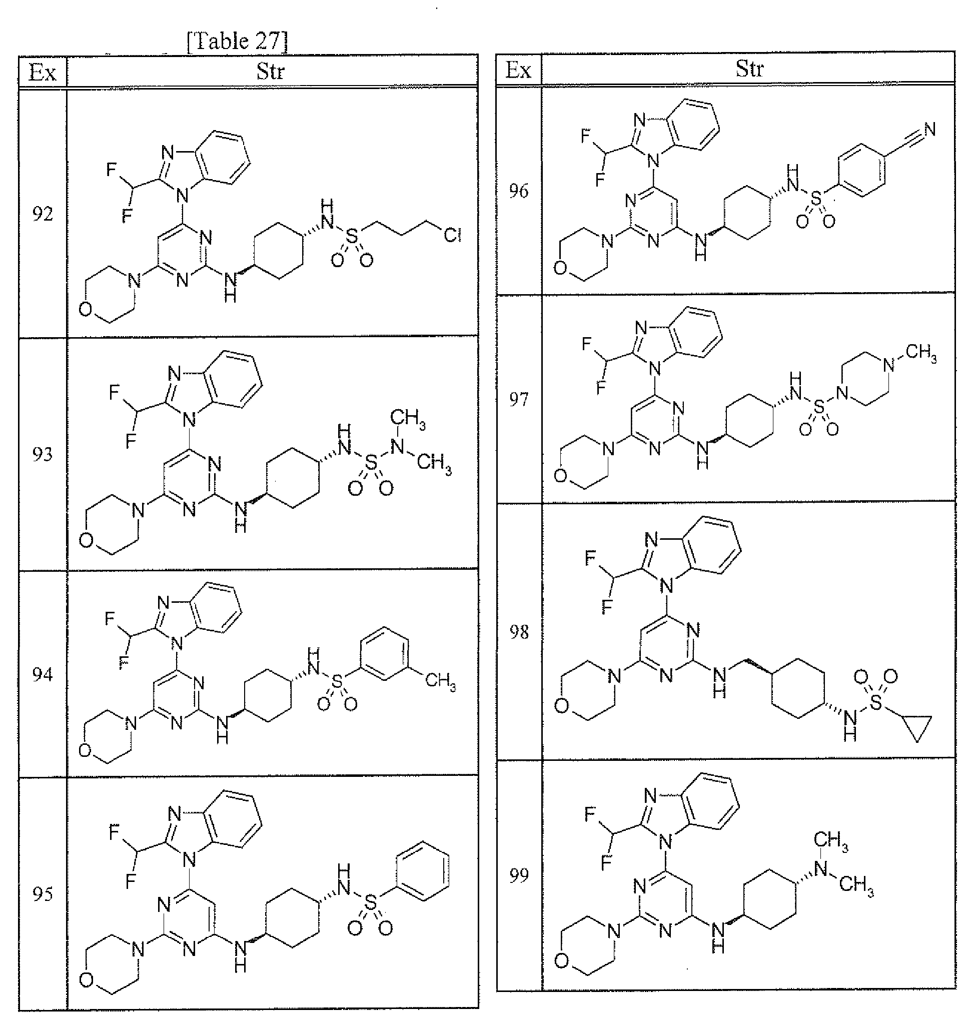

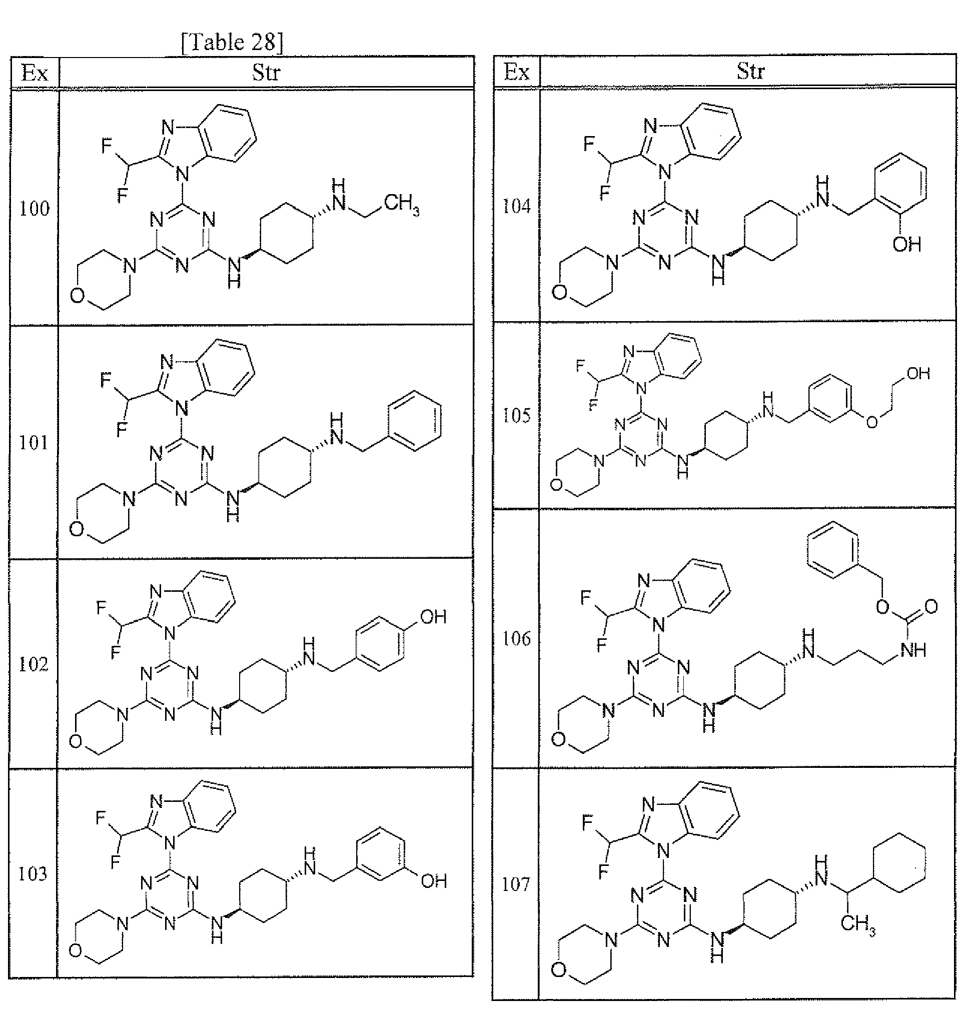

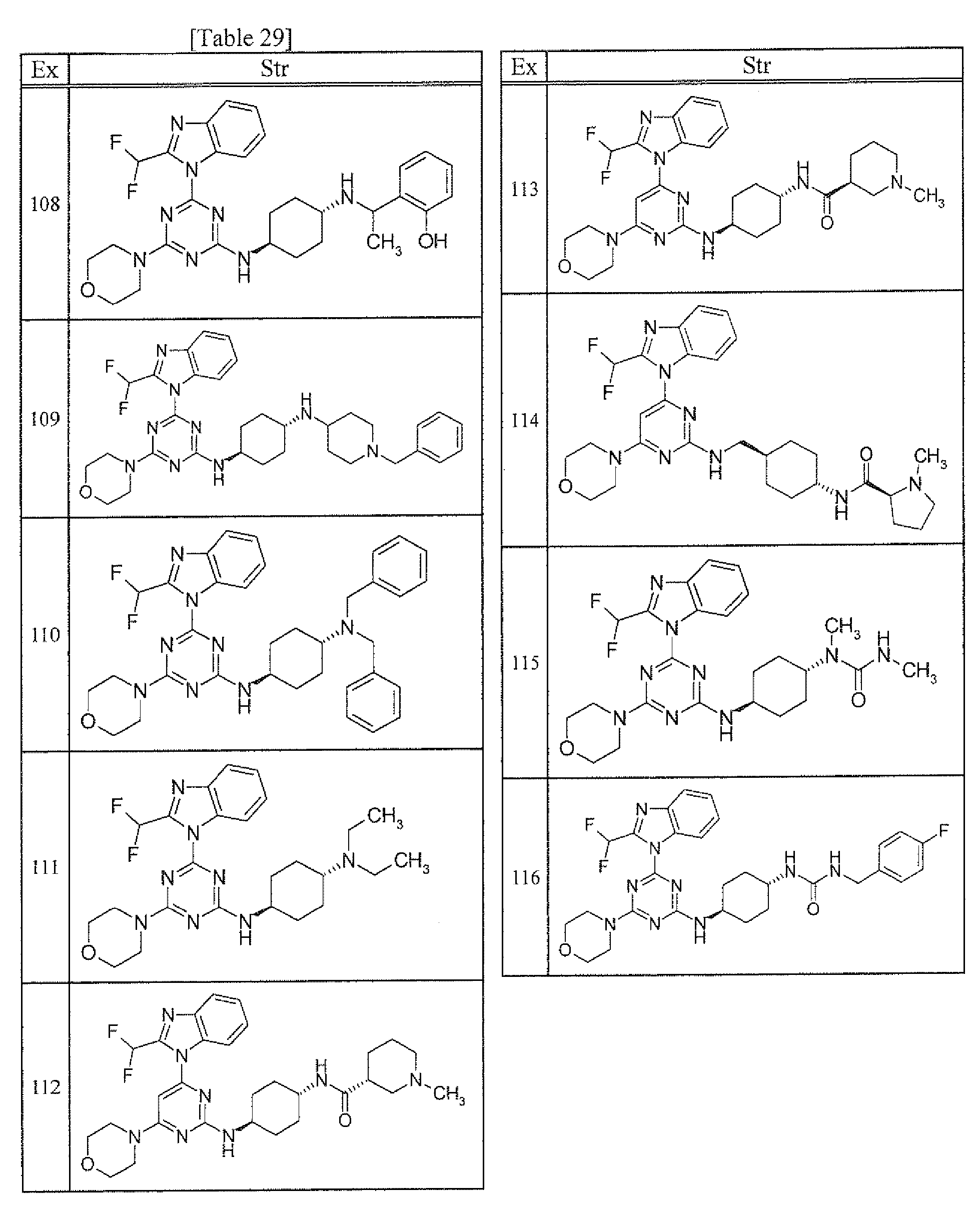

**[Table 30]**

| Ex | Str | | Ex | Str |
|---|---|---|---|---|
| 117 | 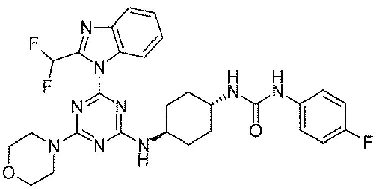 | | 122 | 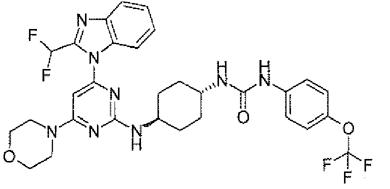 |
| 118 | 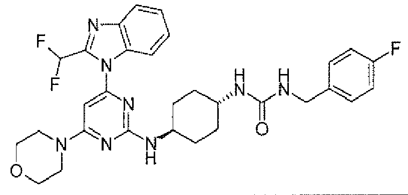 | | 123 | 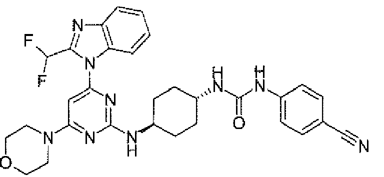 |
| 119 | 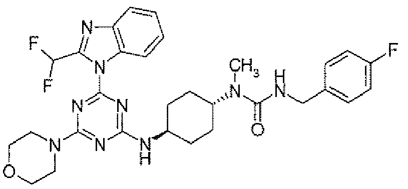 | | 124 | 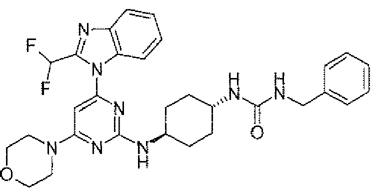 |
| 120 | 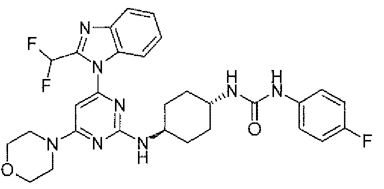 | | 125 | 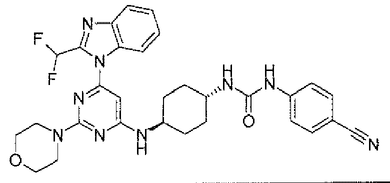 |
| 121 | 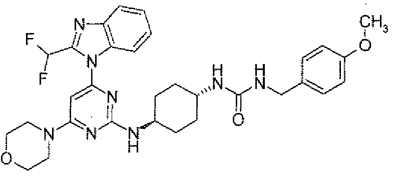 | | 126 | 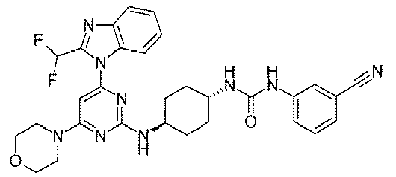 |

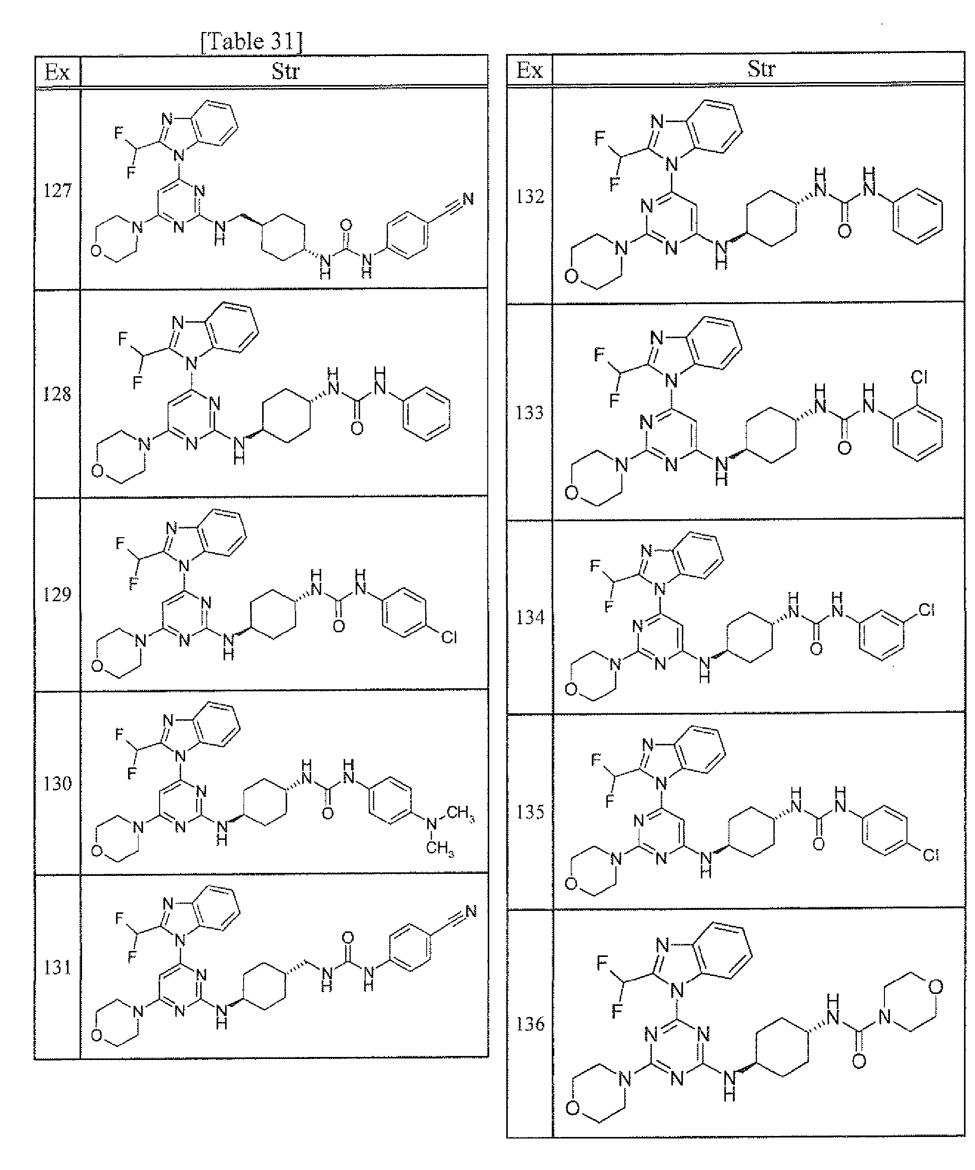

**[Table 32]**

| Ex | Str | | Ex | Str |
|---|---|---|---|---|
| 137 | 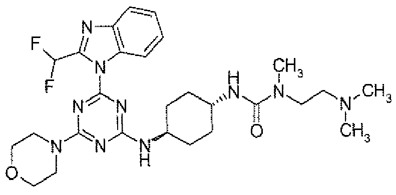 | | 142 | 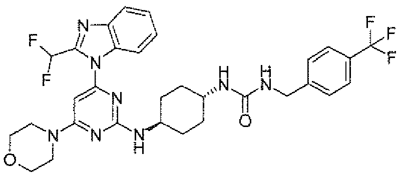 |
| 138 | 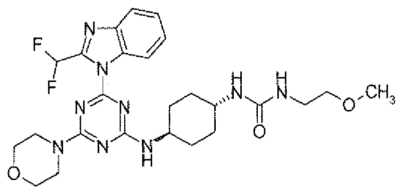 | | 143 | 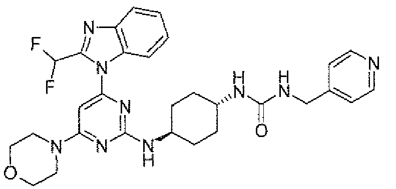 |
| 139 | 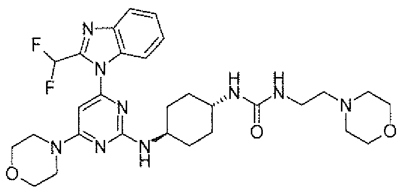 | | 144 | 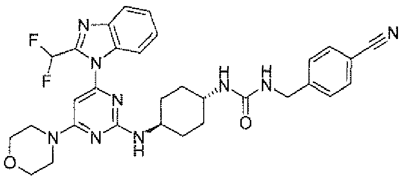 |
| 140 | 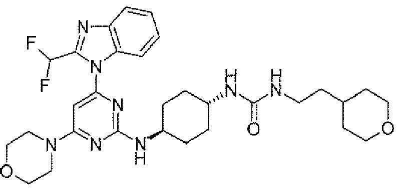 | | 145 | 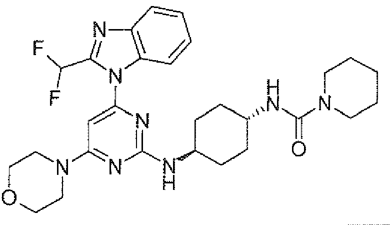 |
| 141 | 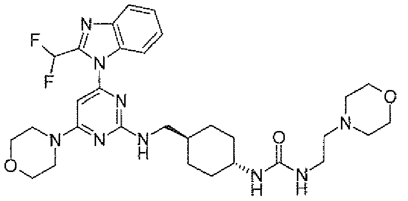 | | 146 | 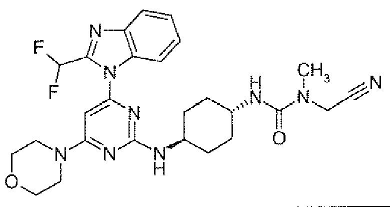 |

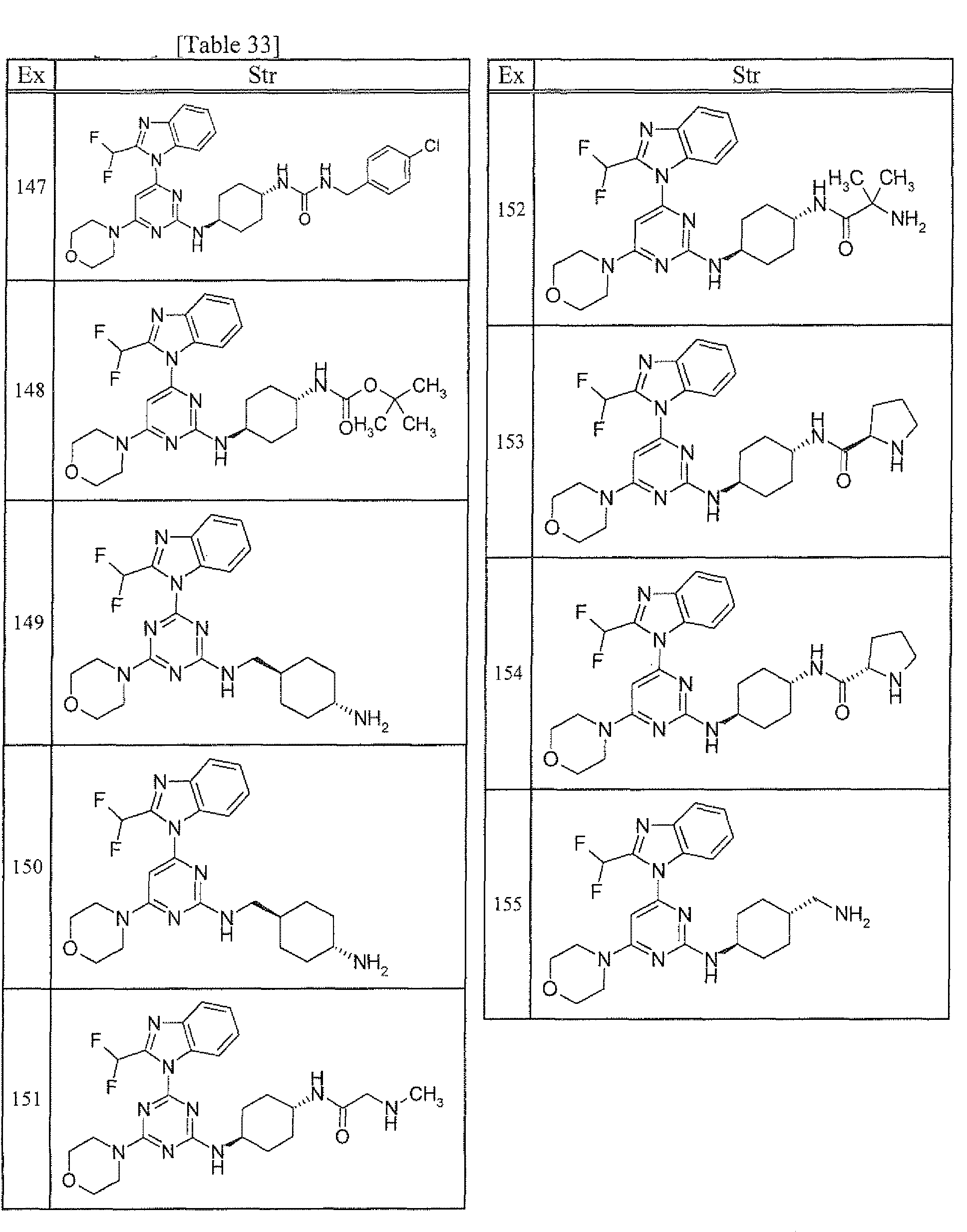

**[Table 34]**

| Ex | Str | | Ex | Str |
|---|---|---|---|---|
| 156 | 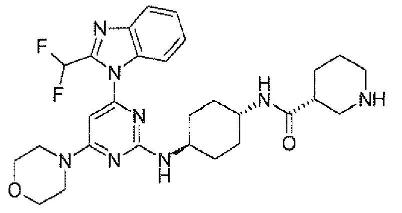 | | 161 | 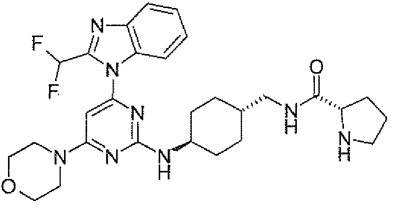 |
| 157 | 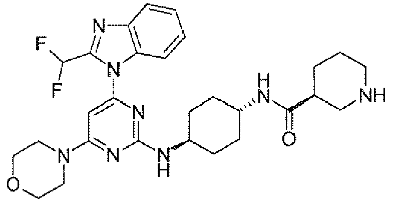 | | 162 | 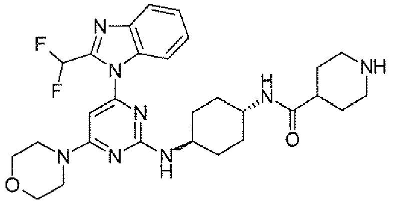 |
| 158 | 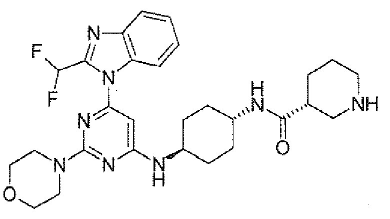 | | 163 | 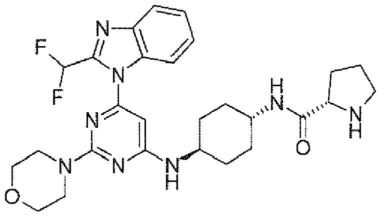 |
| 159 | 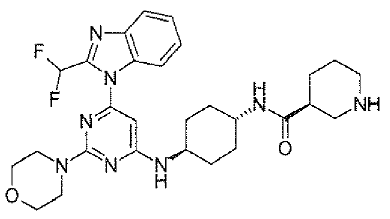 | | 164 | 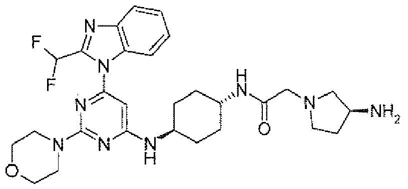 |
| 160 | 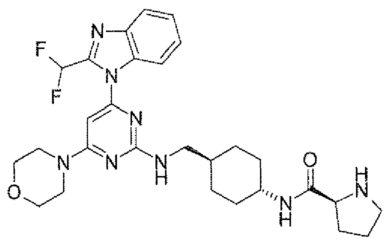 | | 165 | 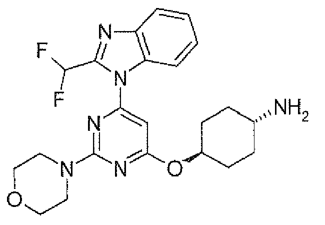 |

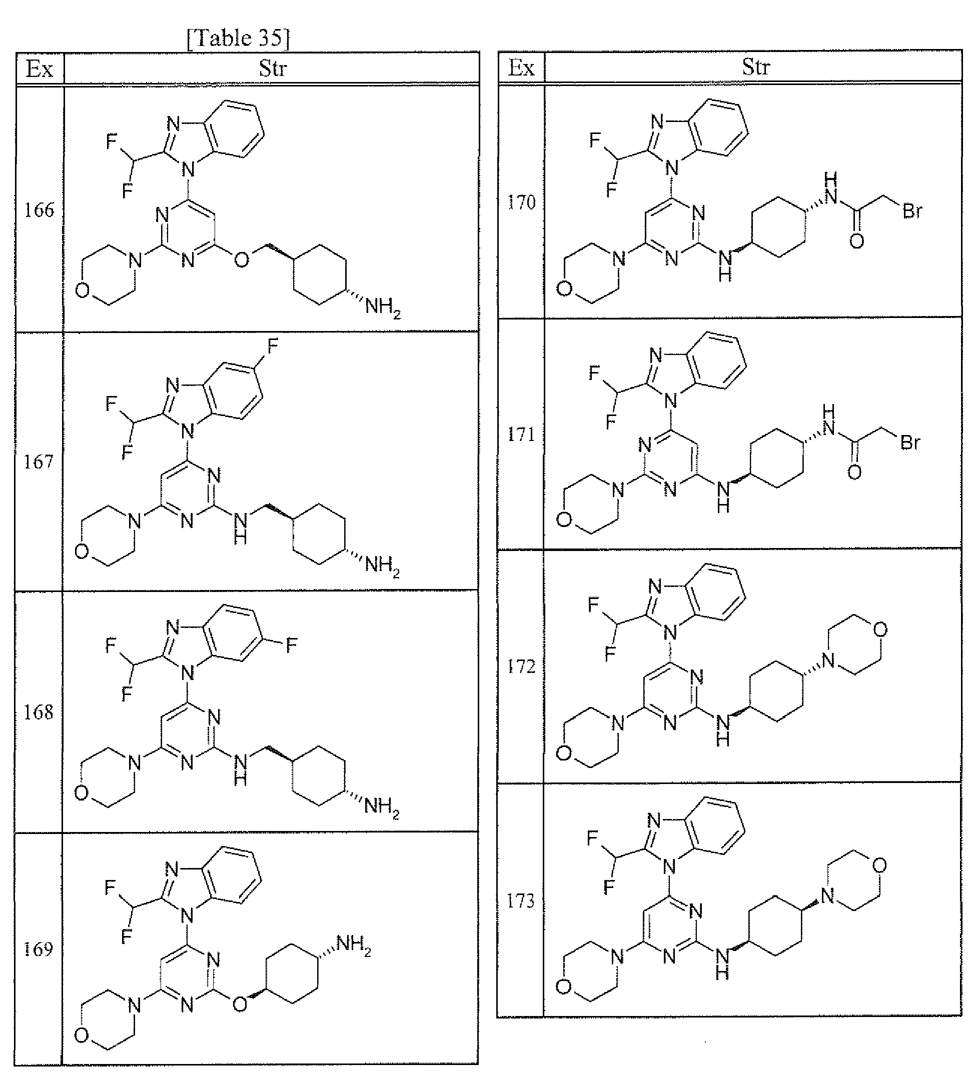

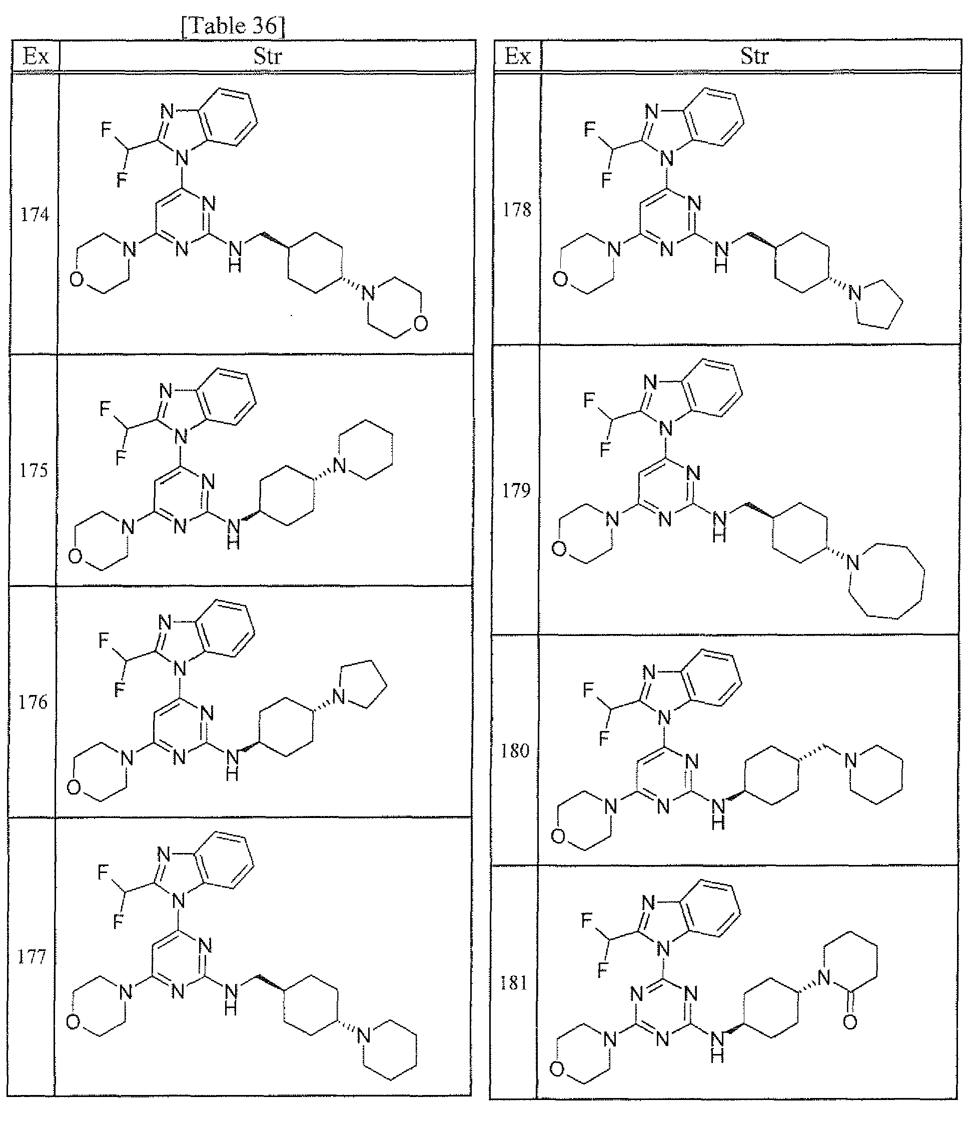

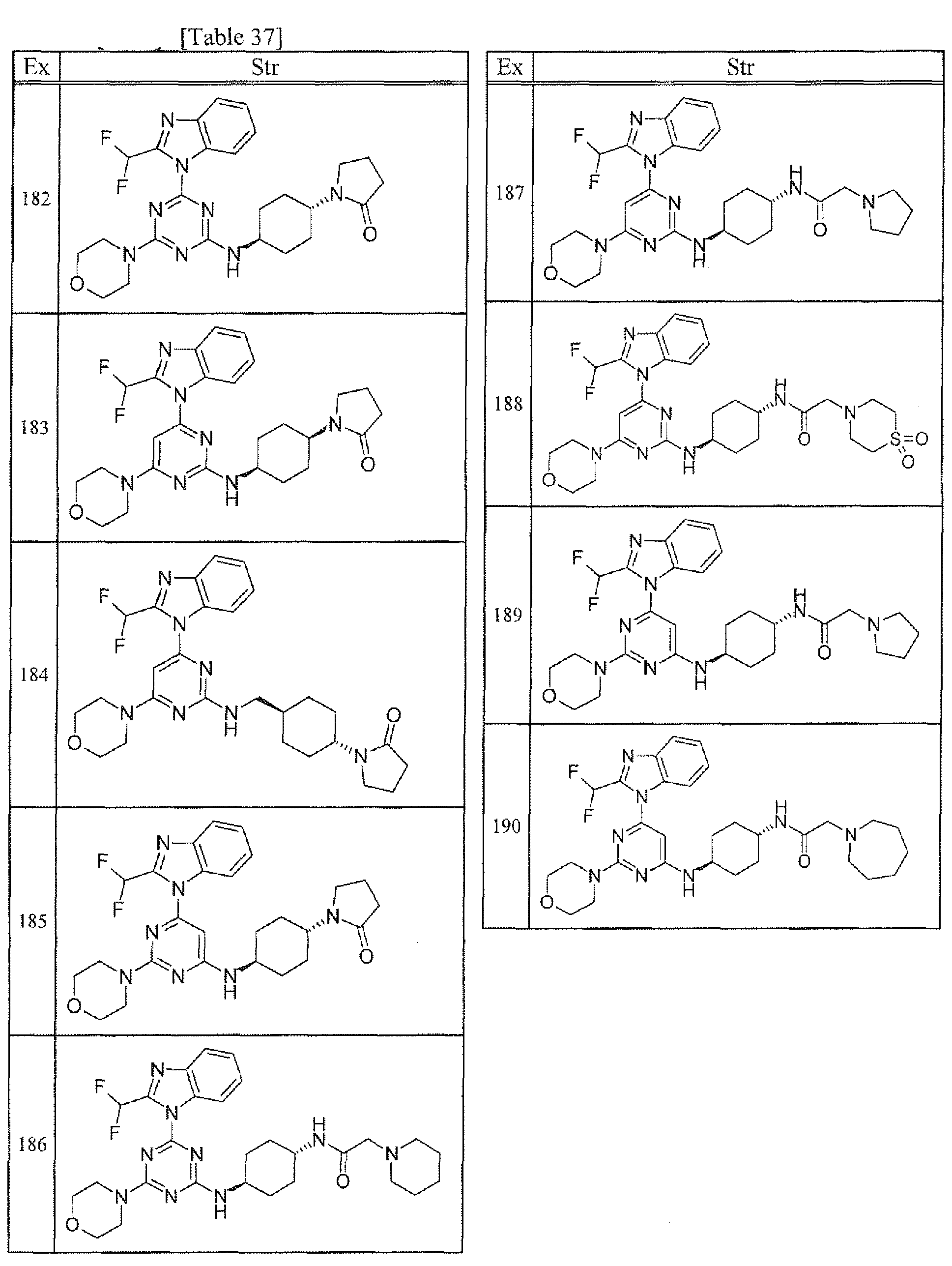

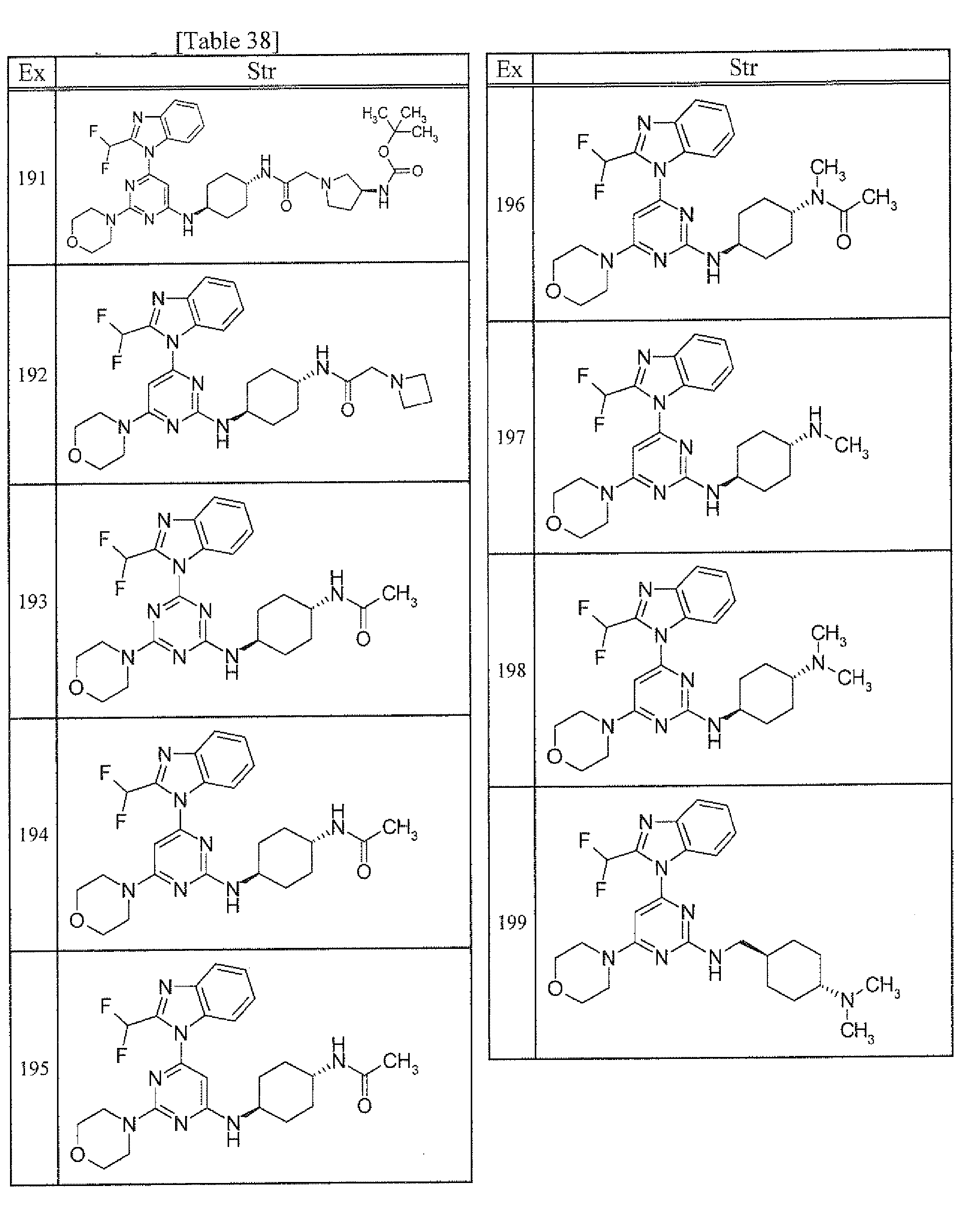

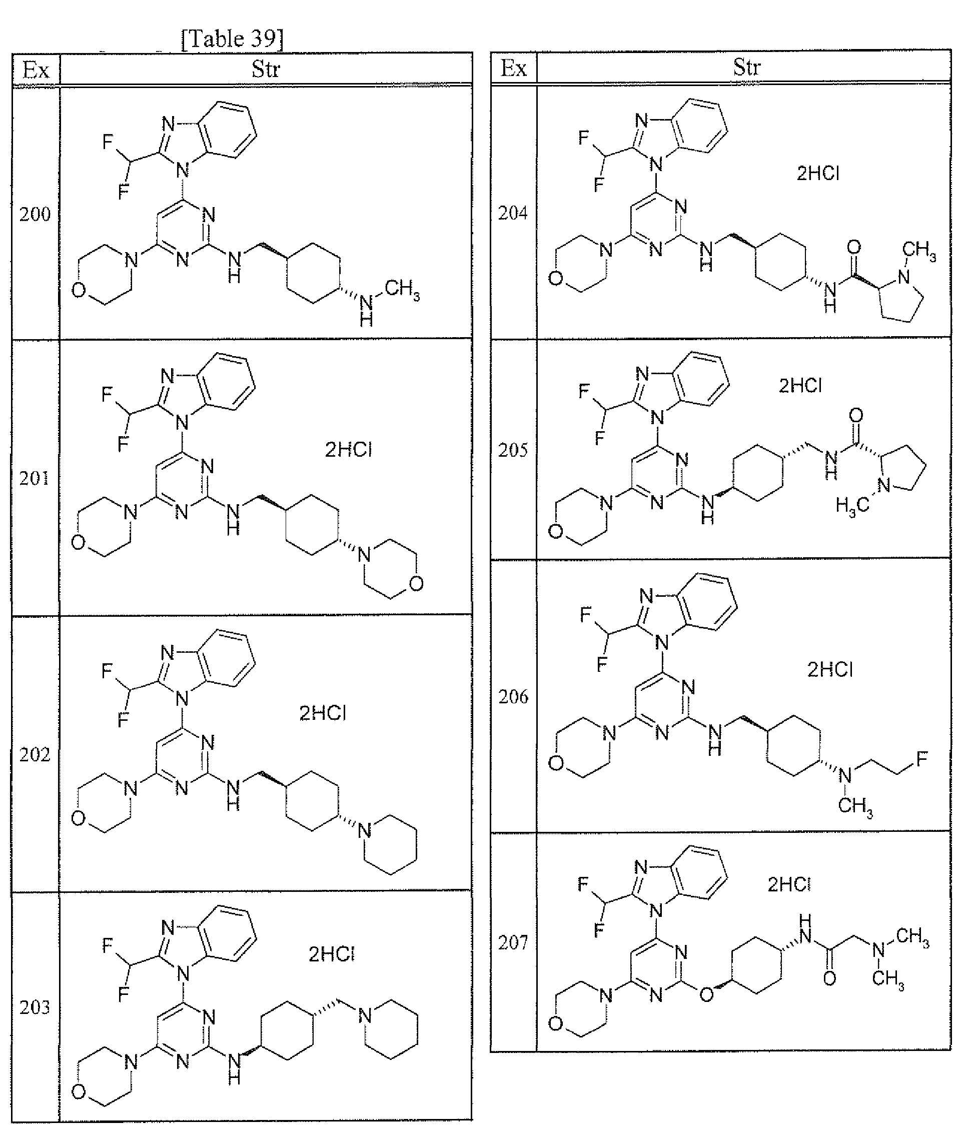

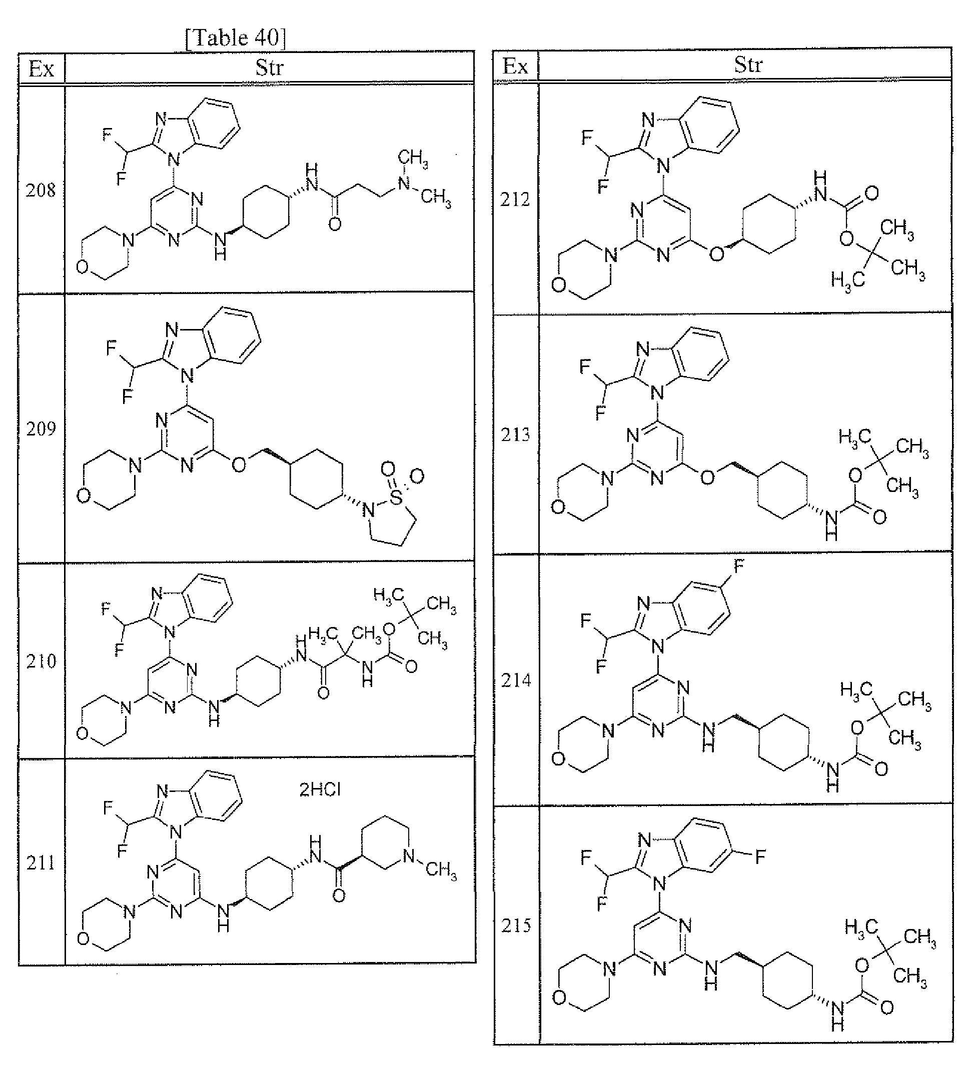

[Table 41]

| Ex | Str |
|---|---|
| 216 | 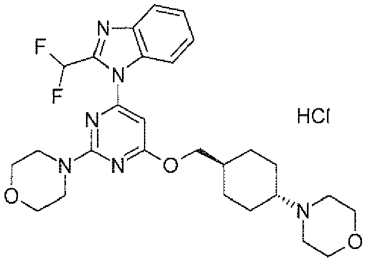 |
| 217 | 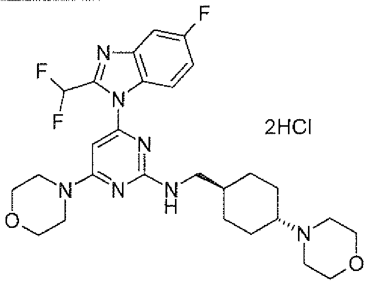 |
| 218 | 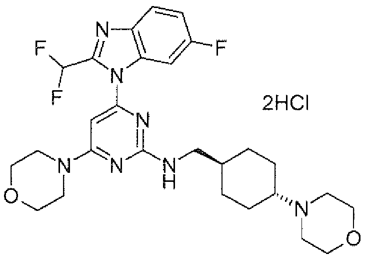 |
| 219 | 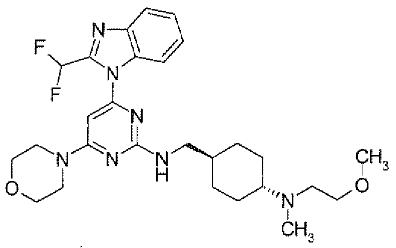 |

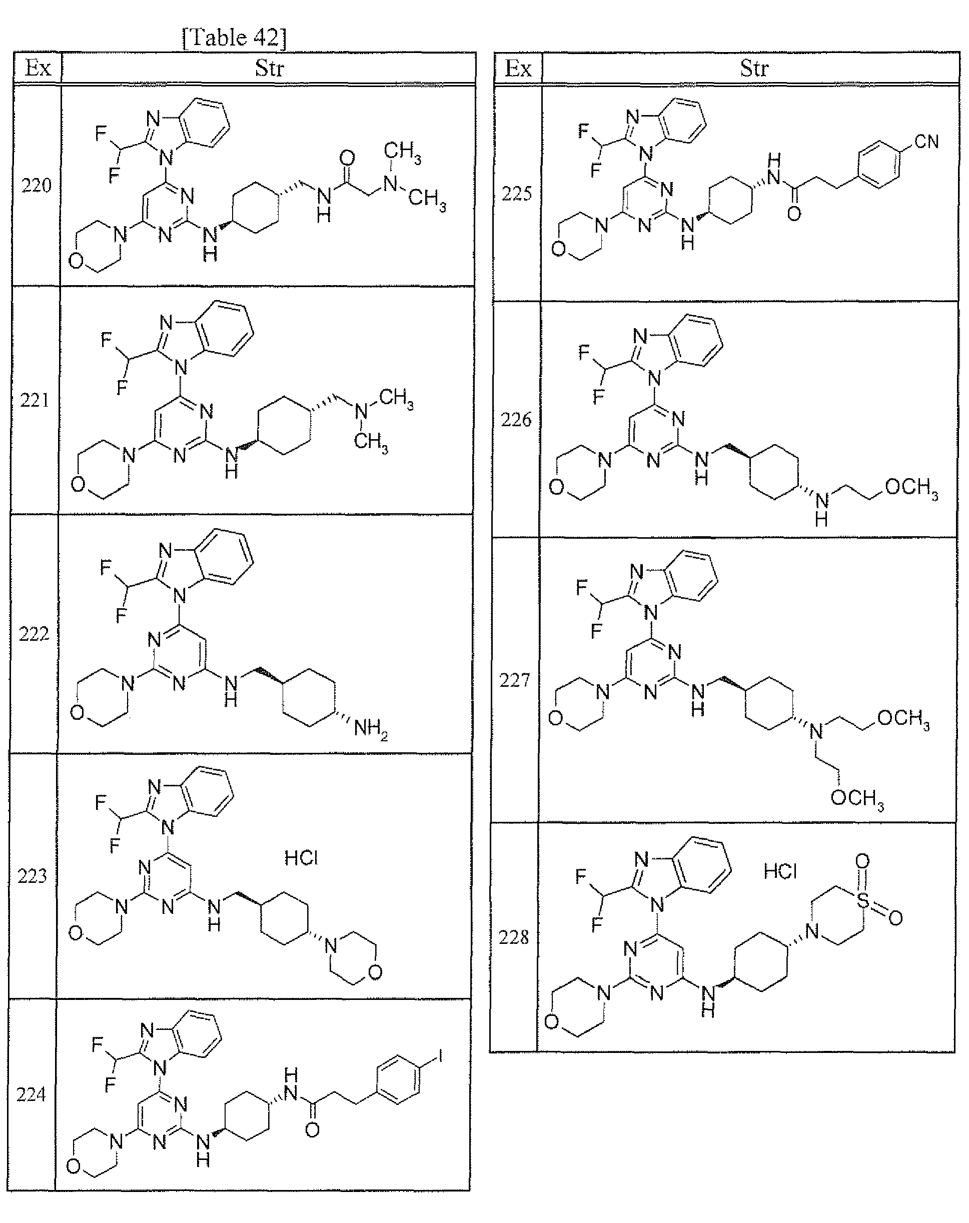

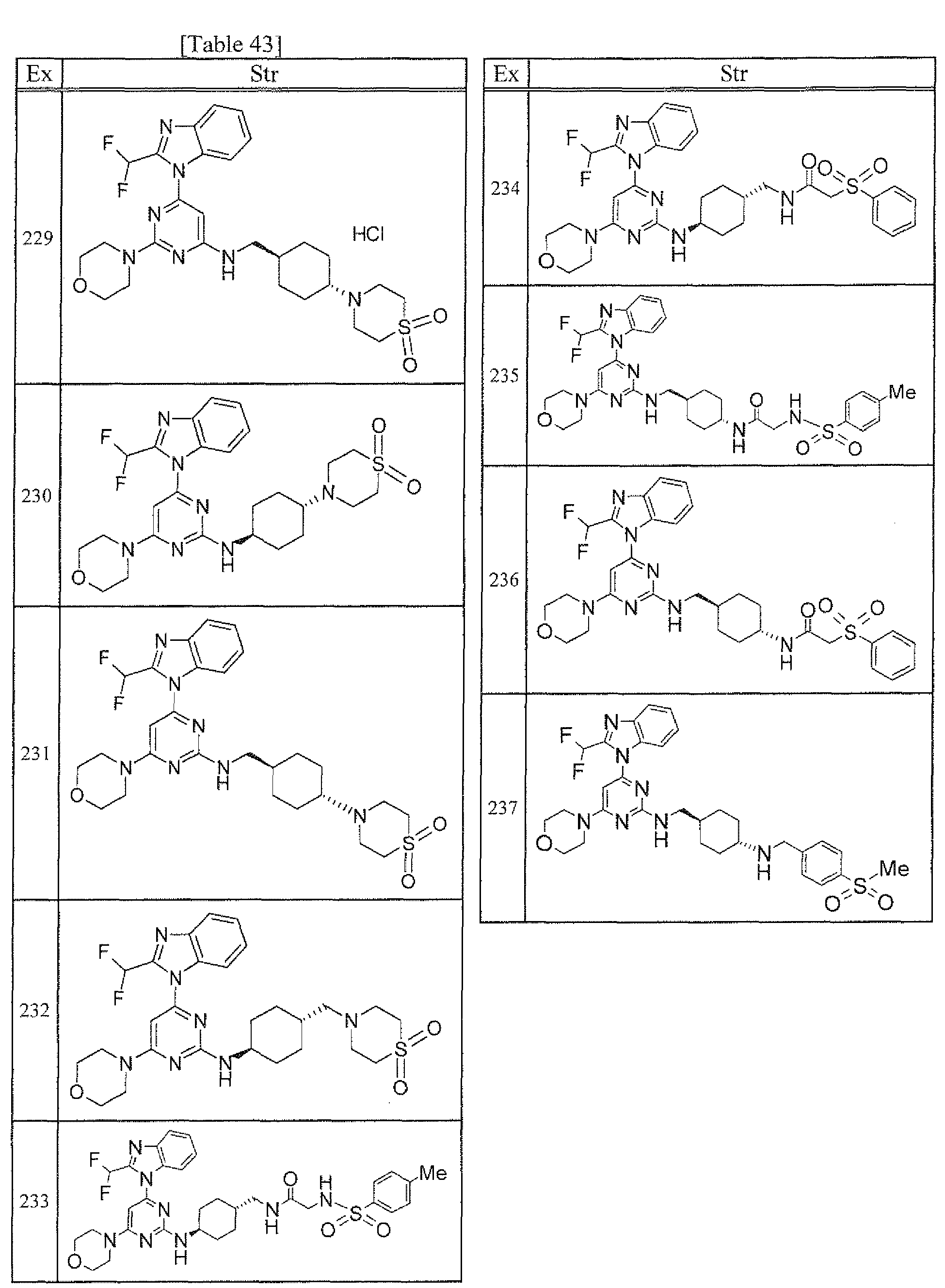

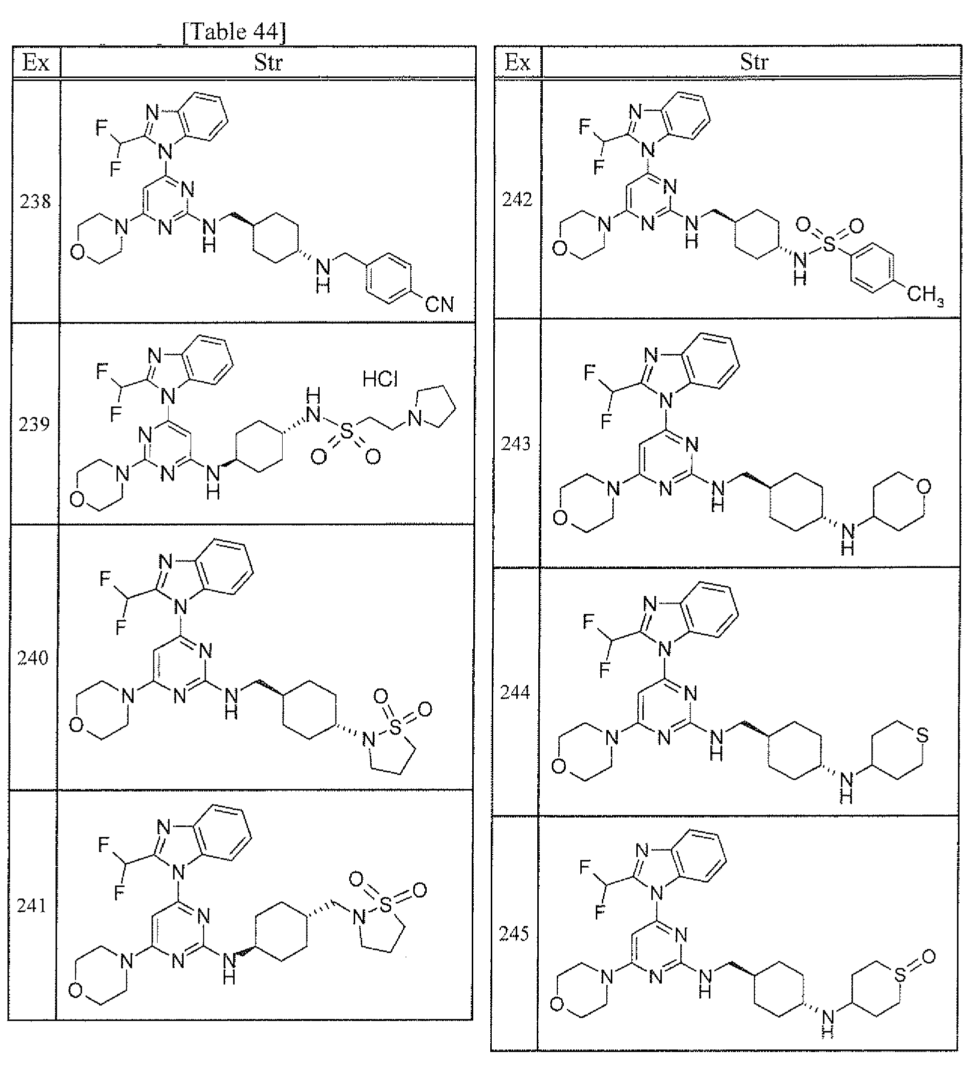

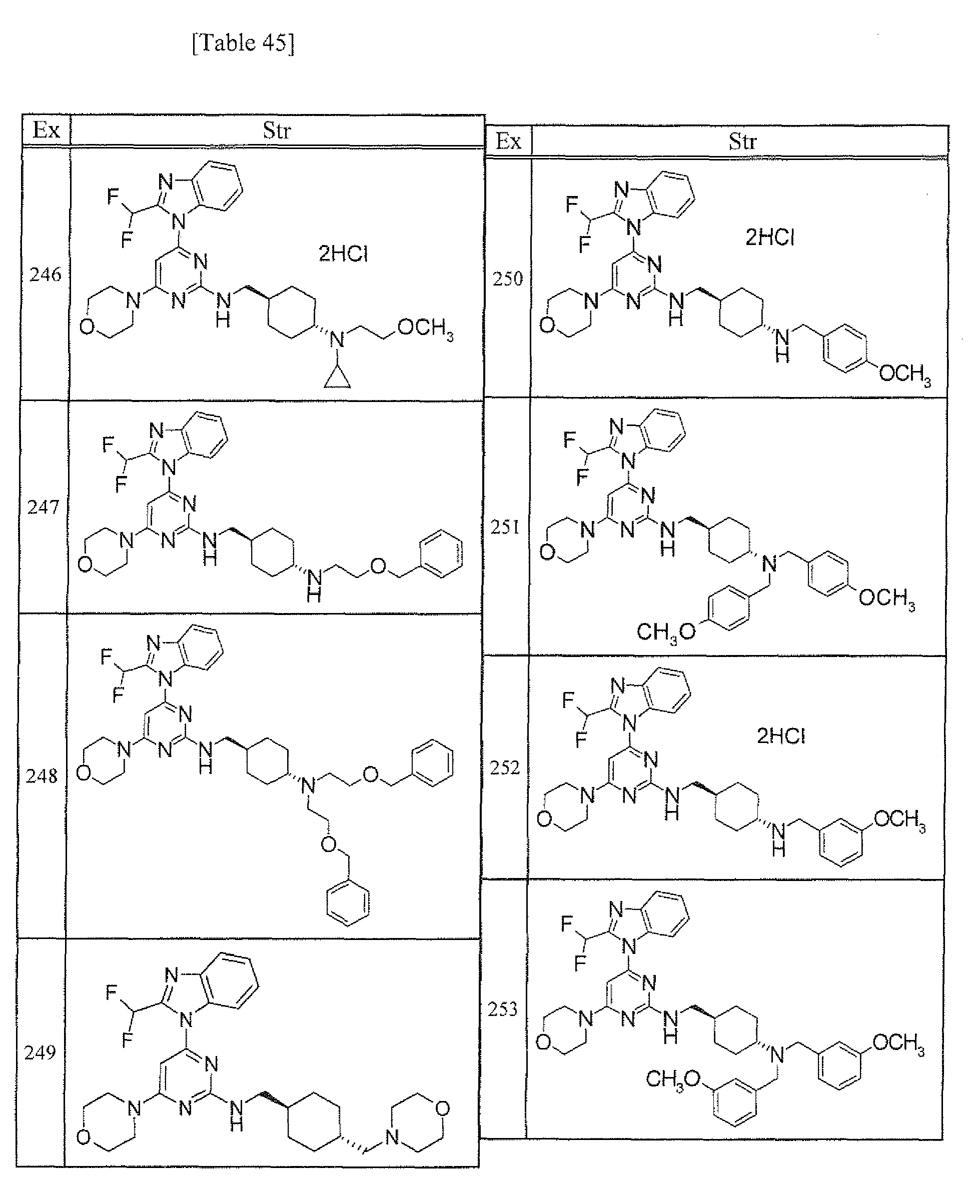

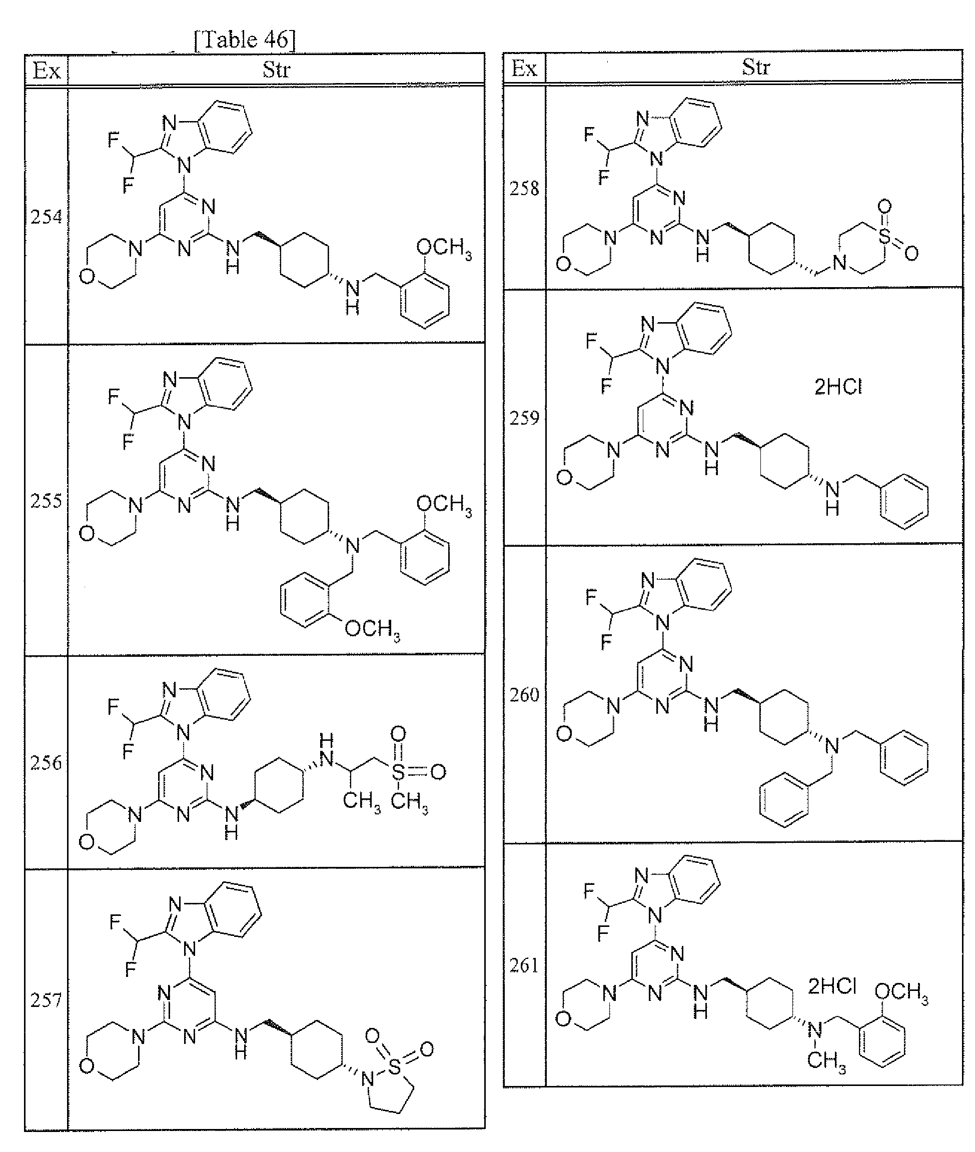

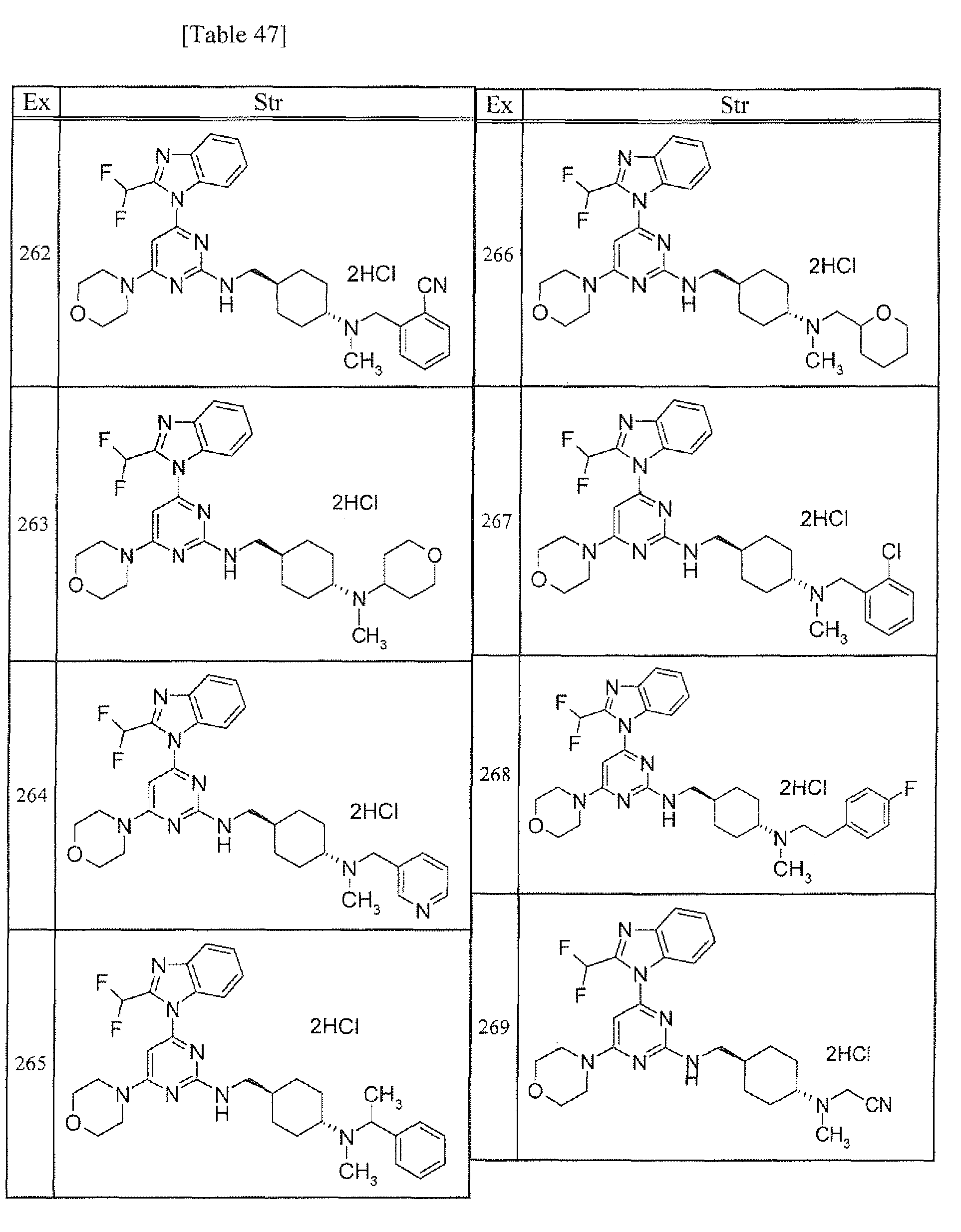

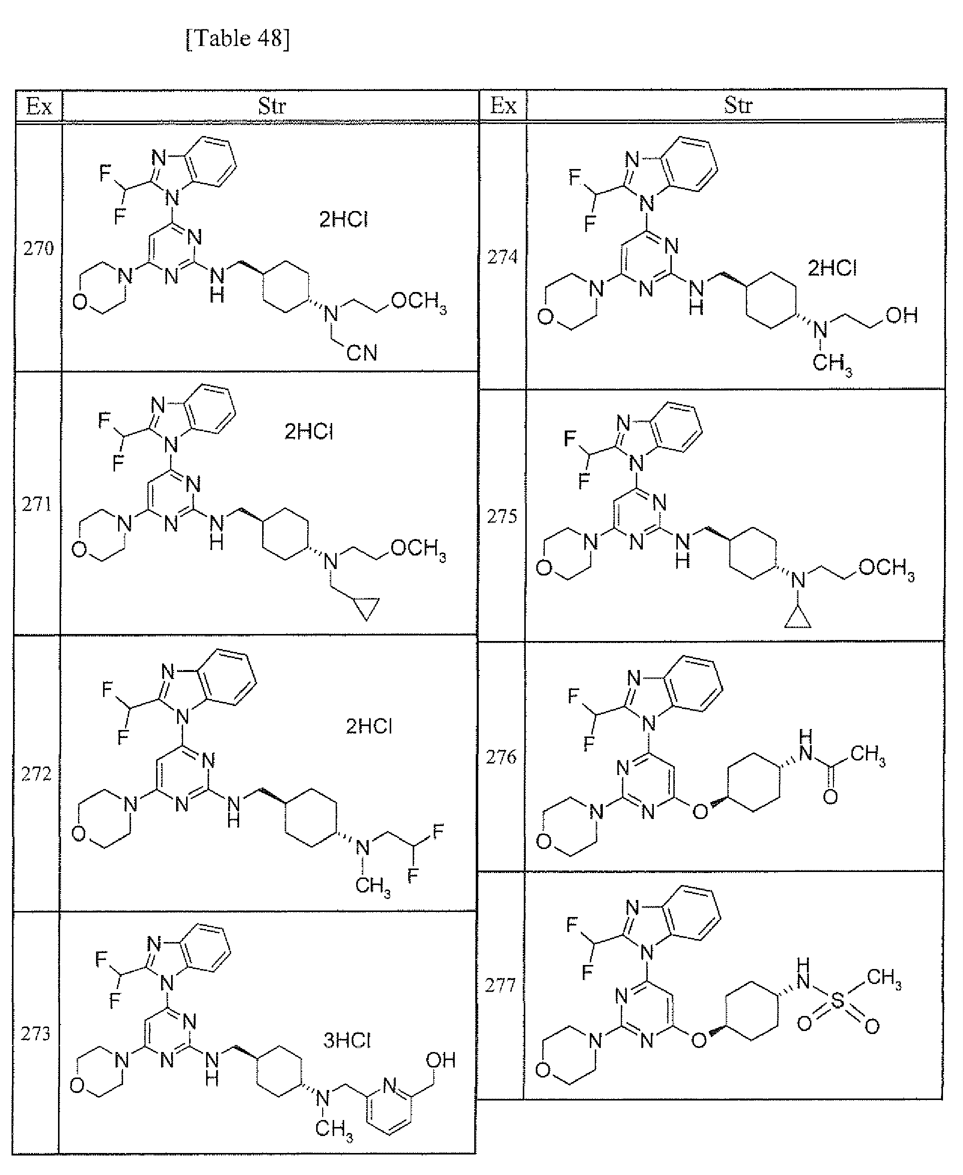

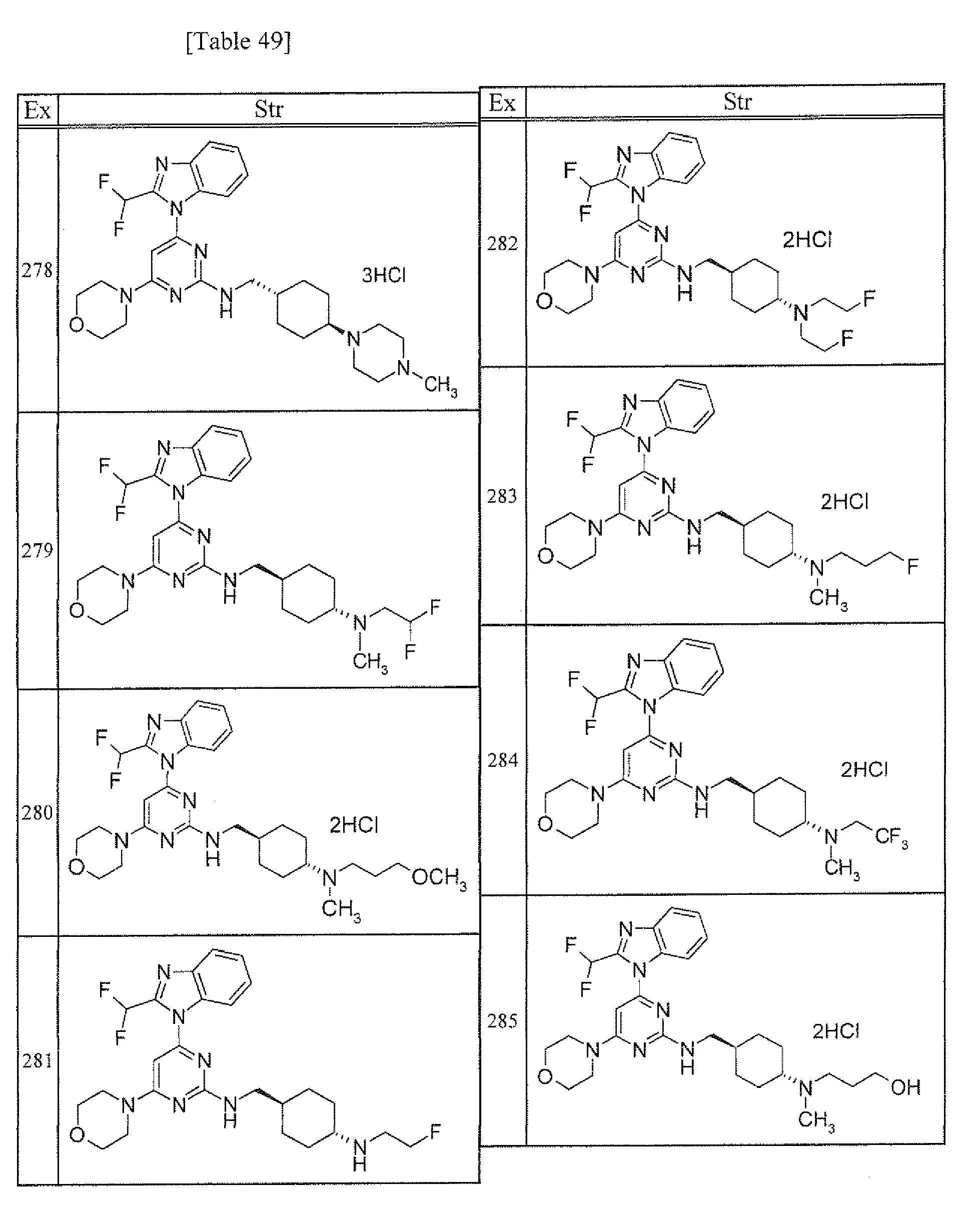

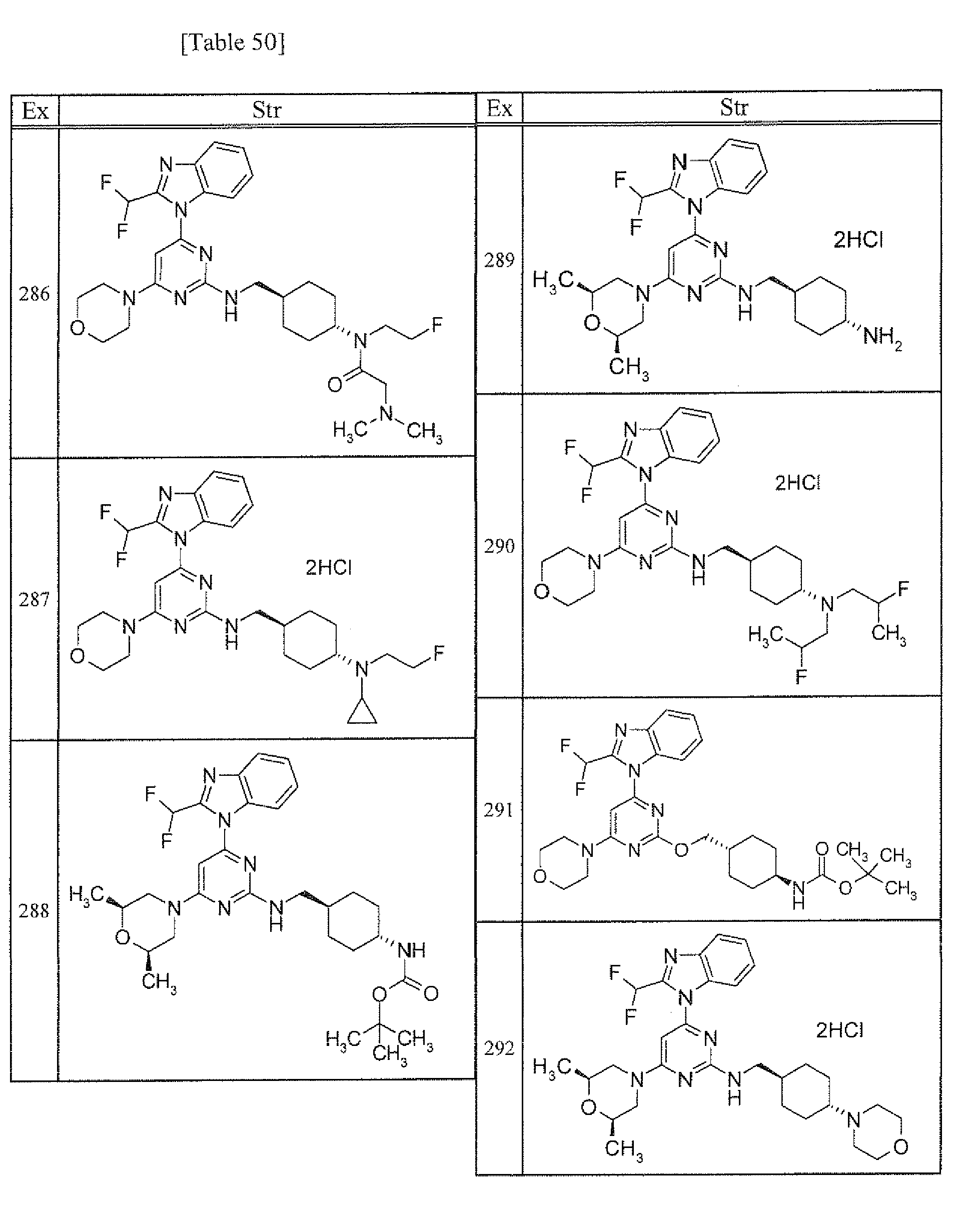

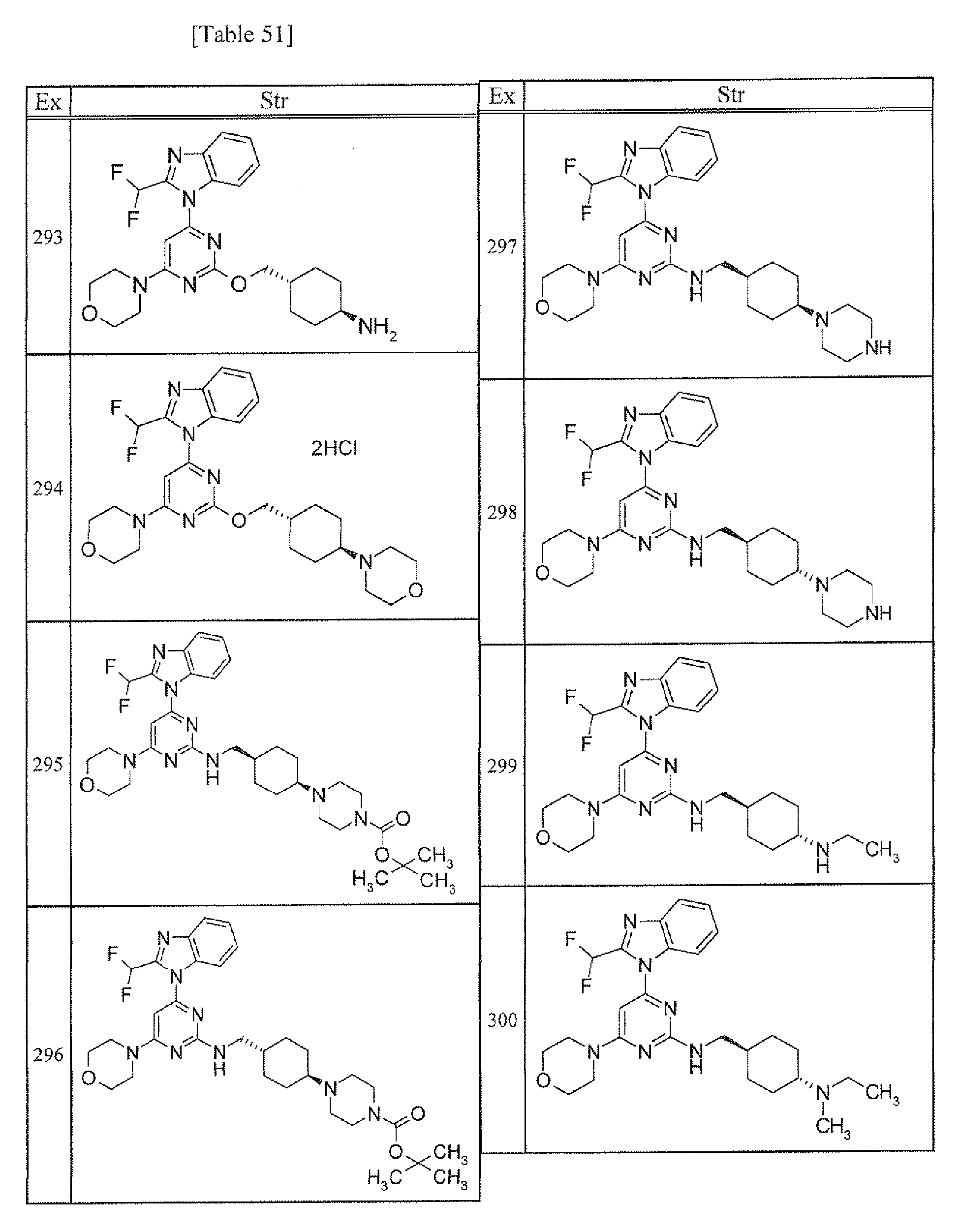

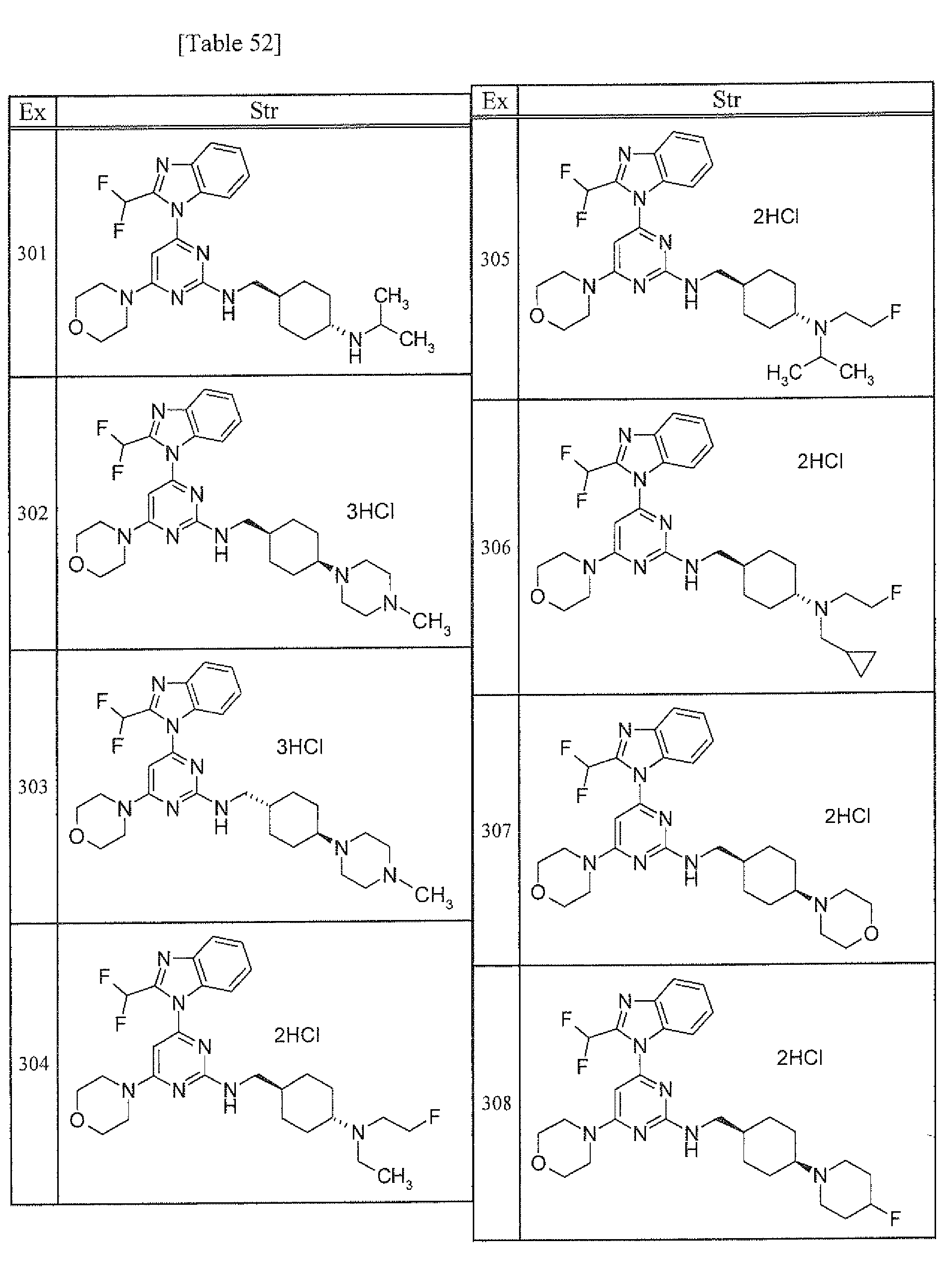

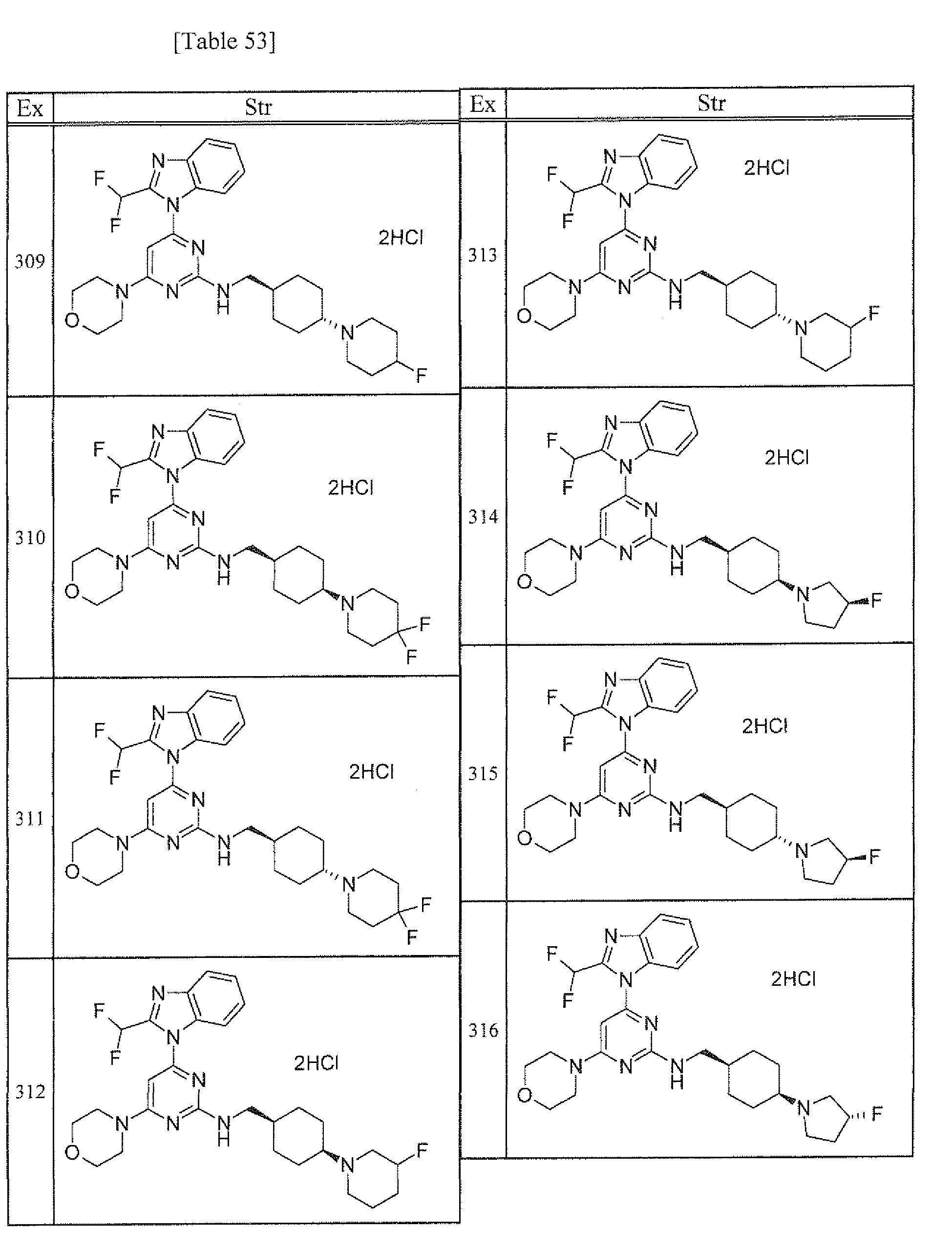

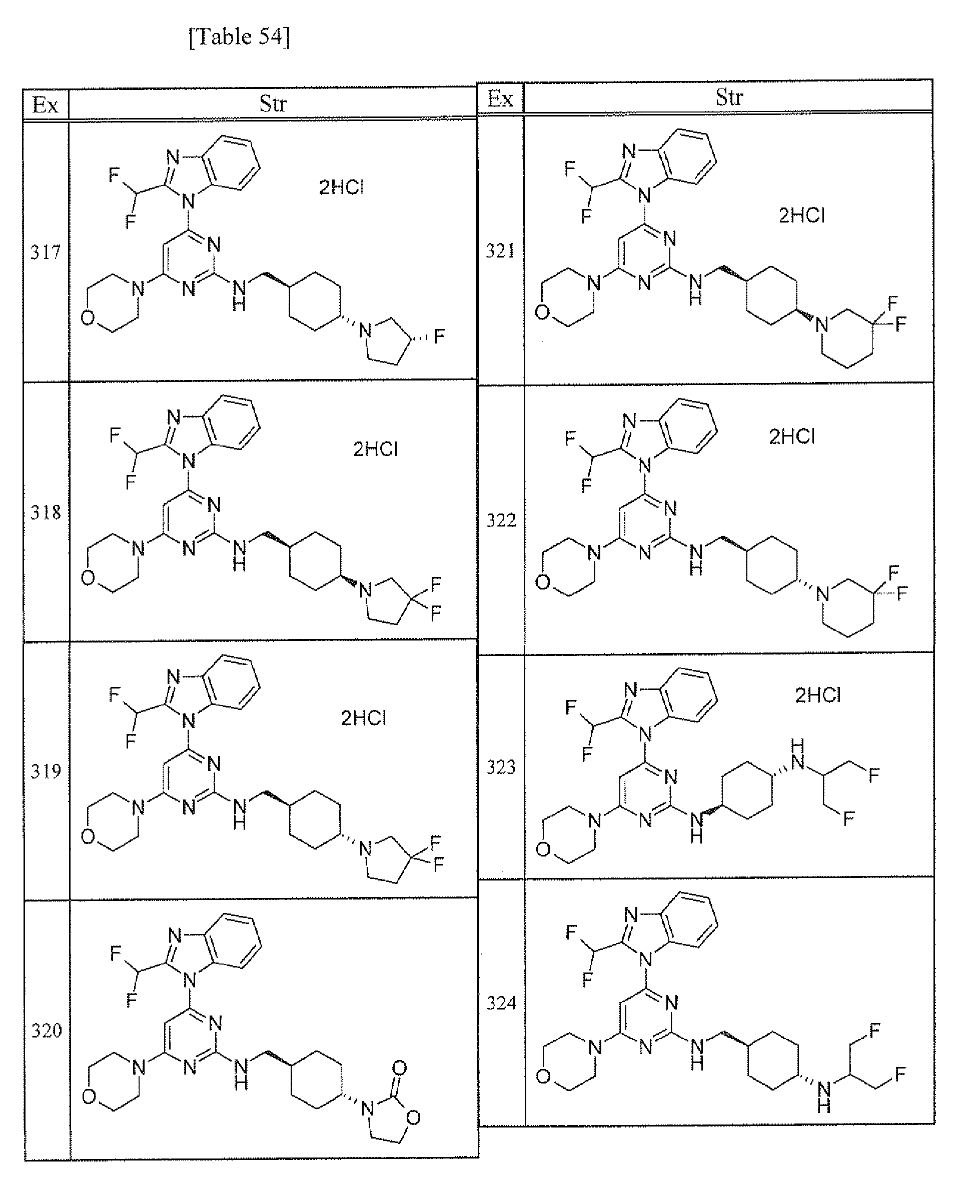

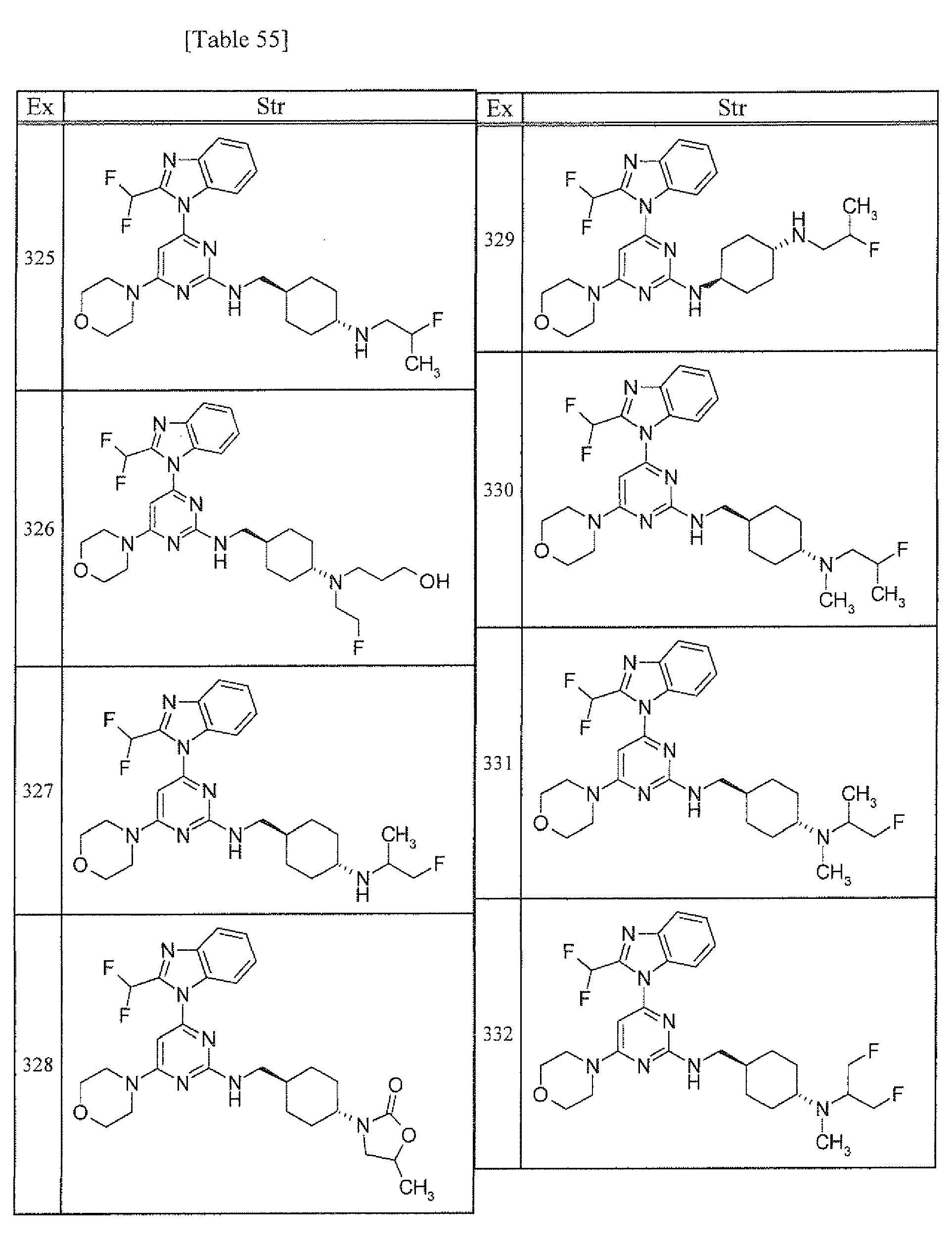

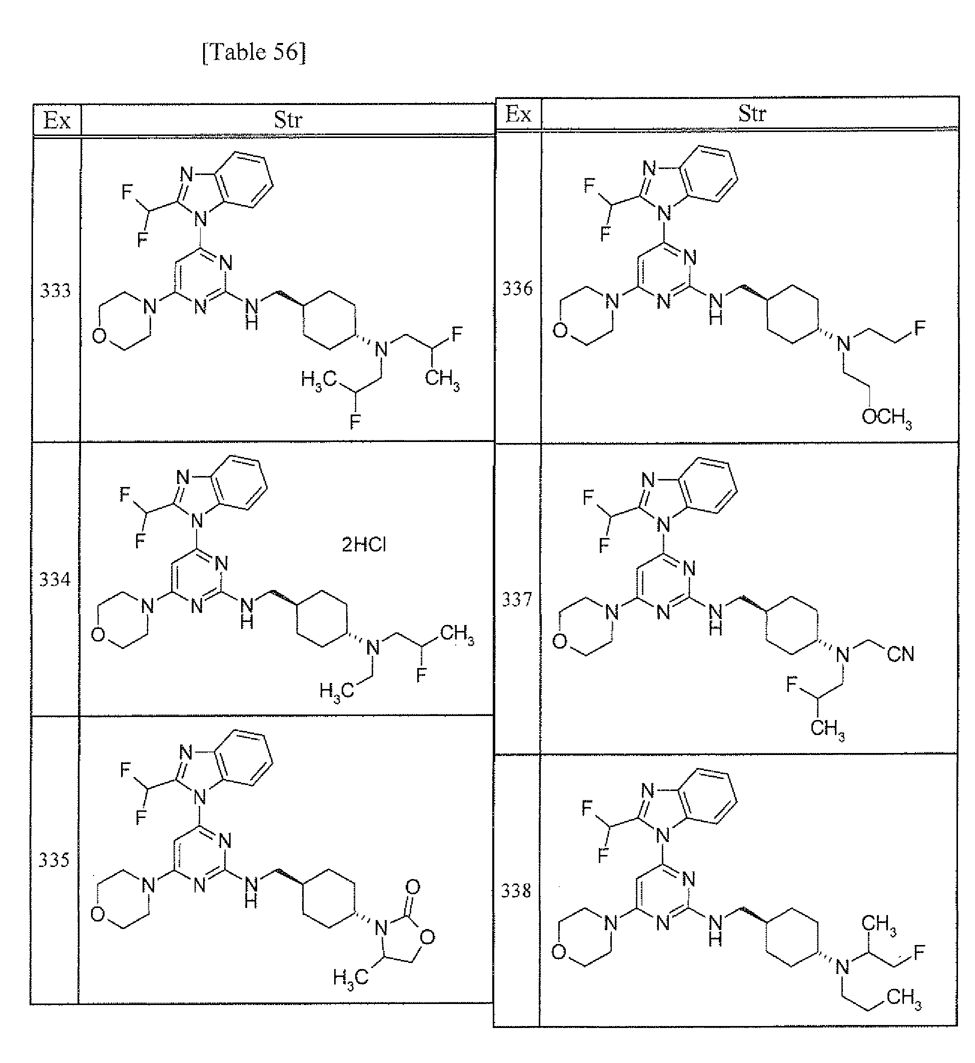

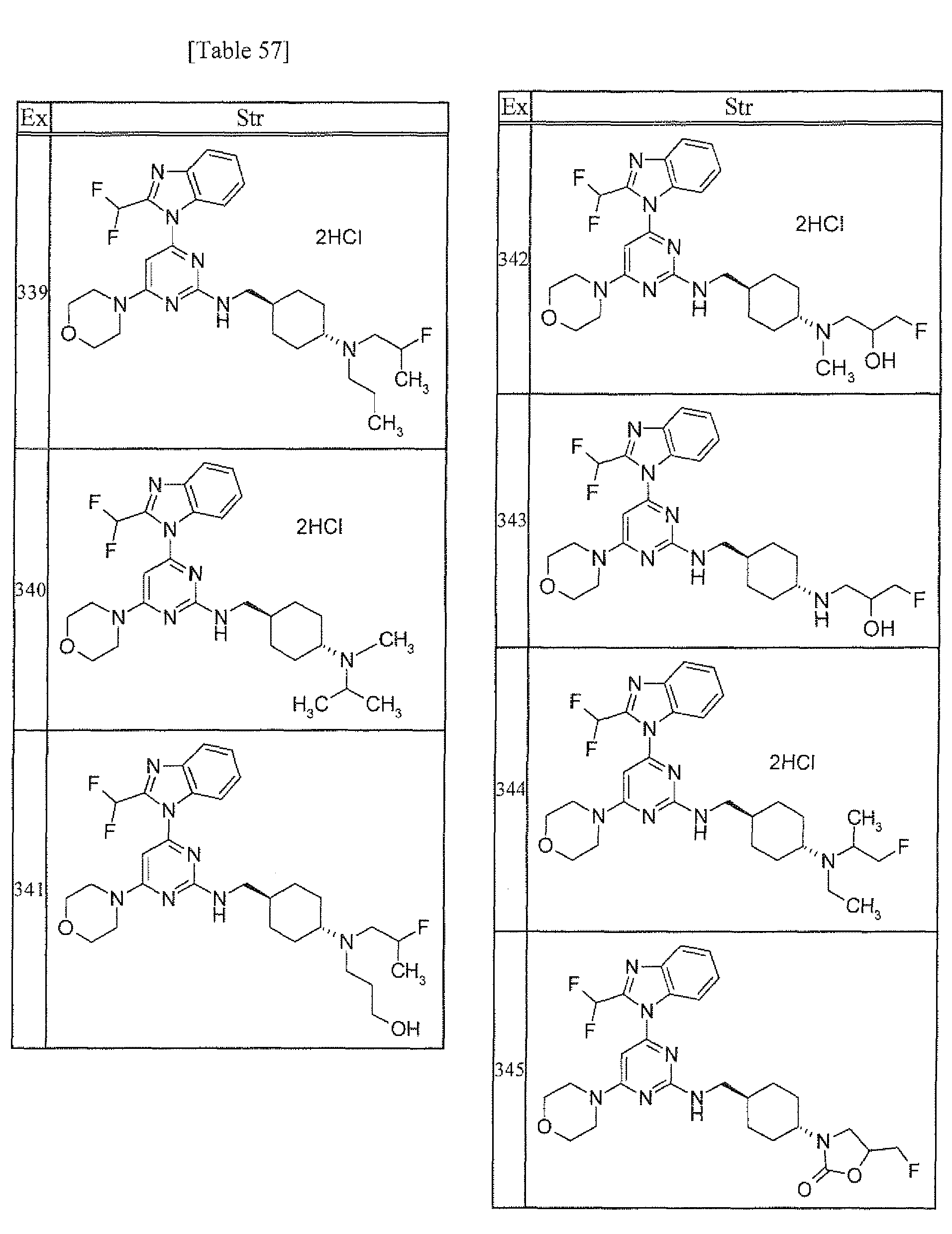

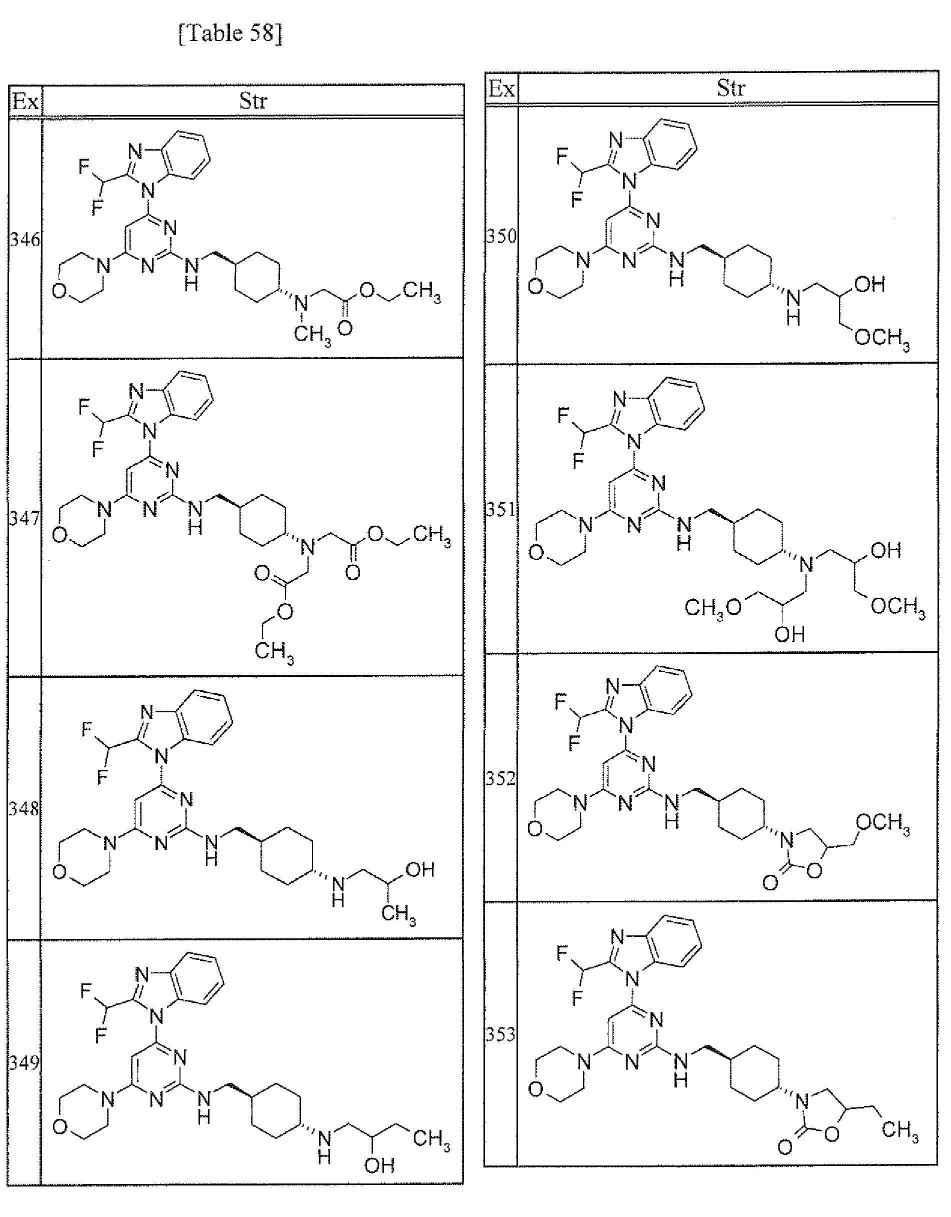

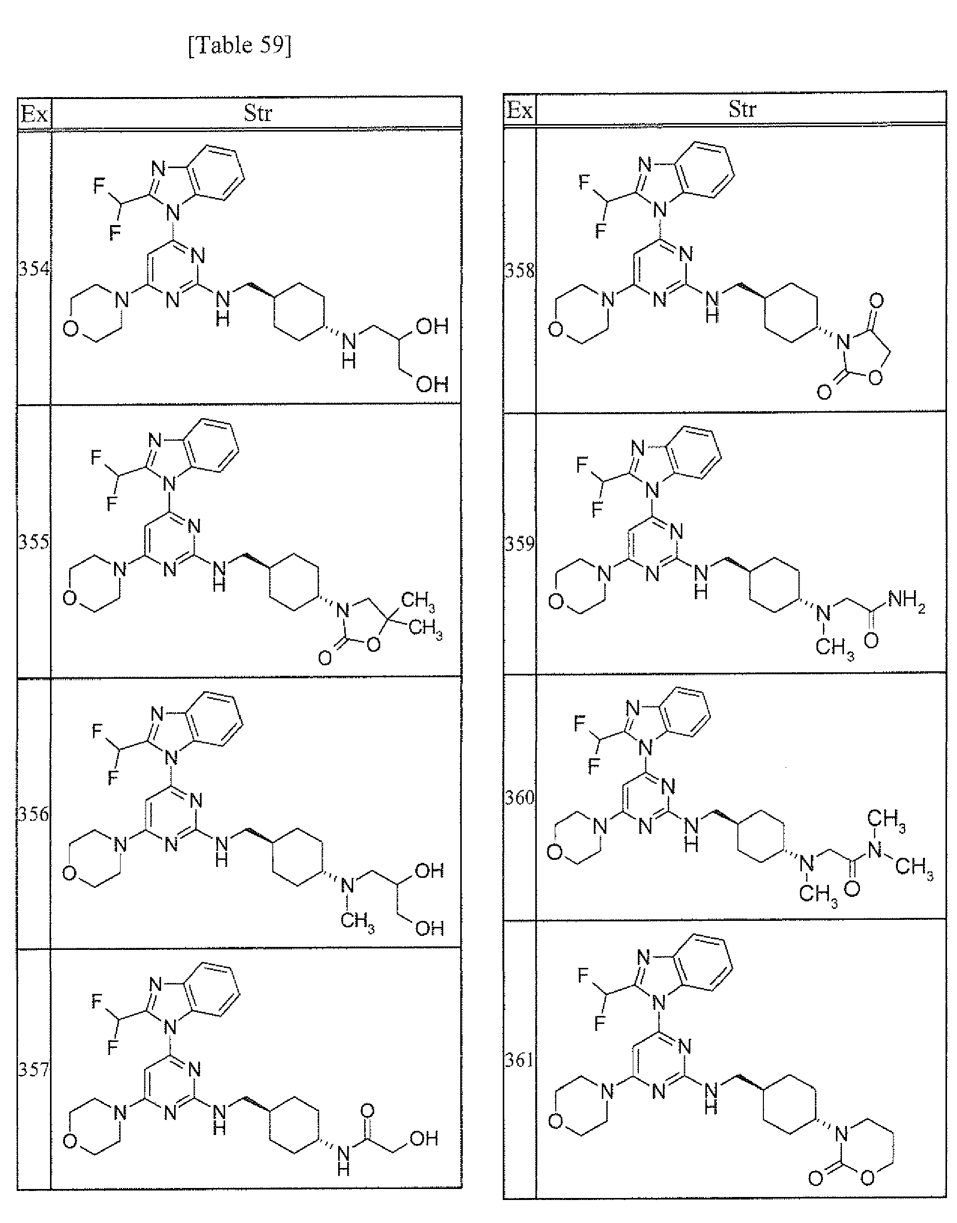

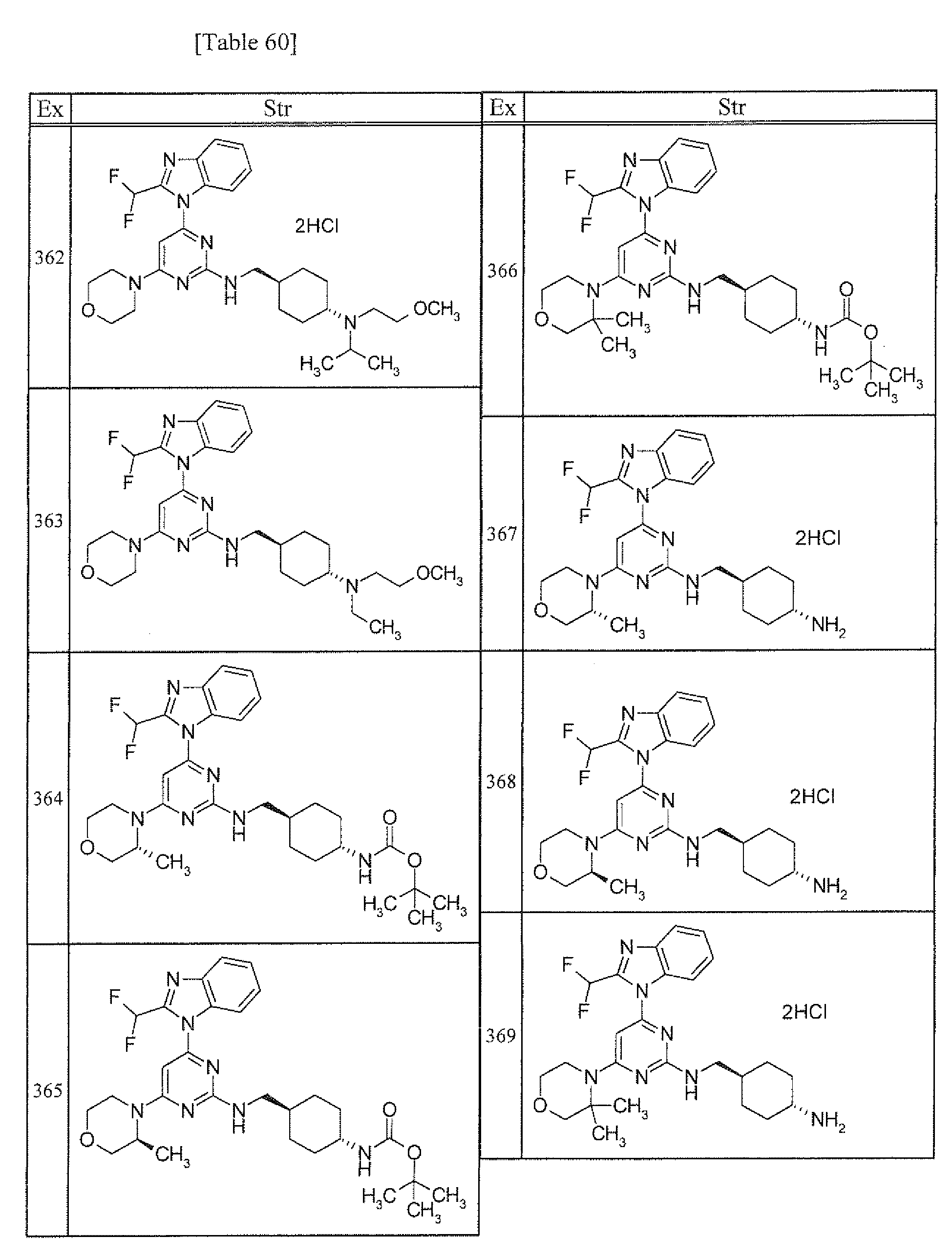

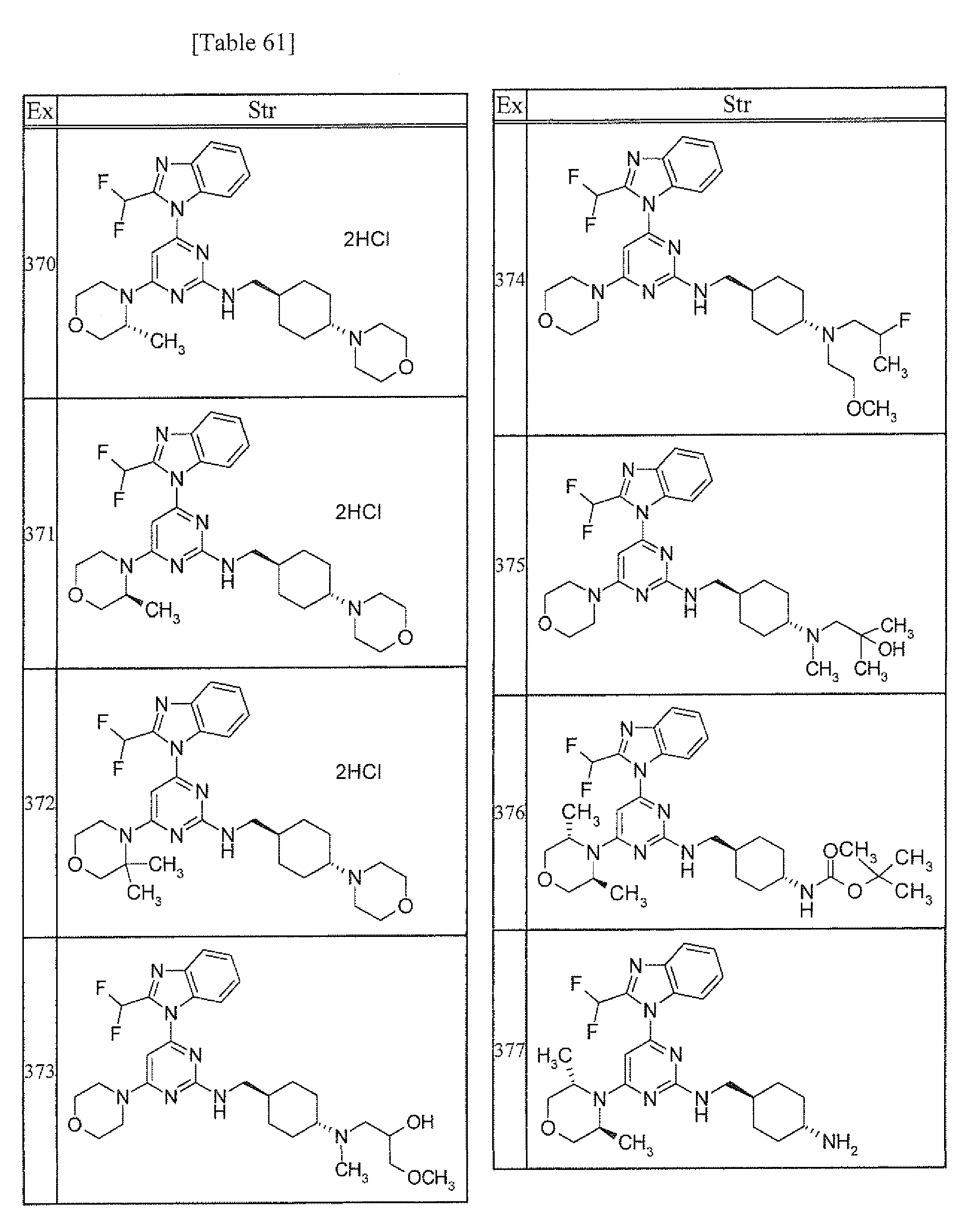

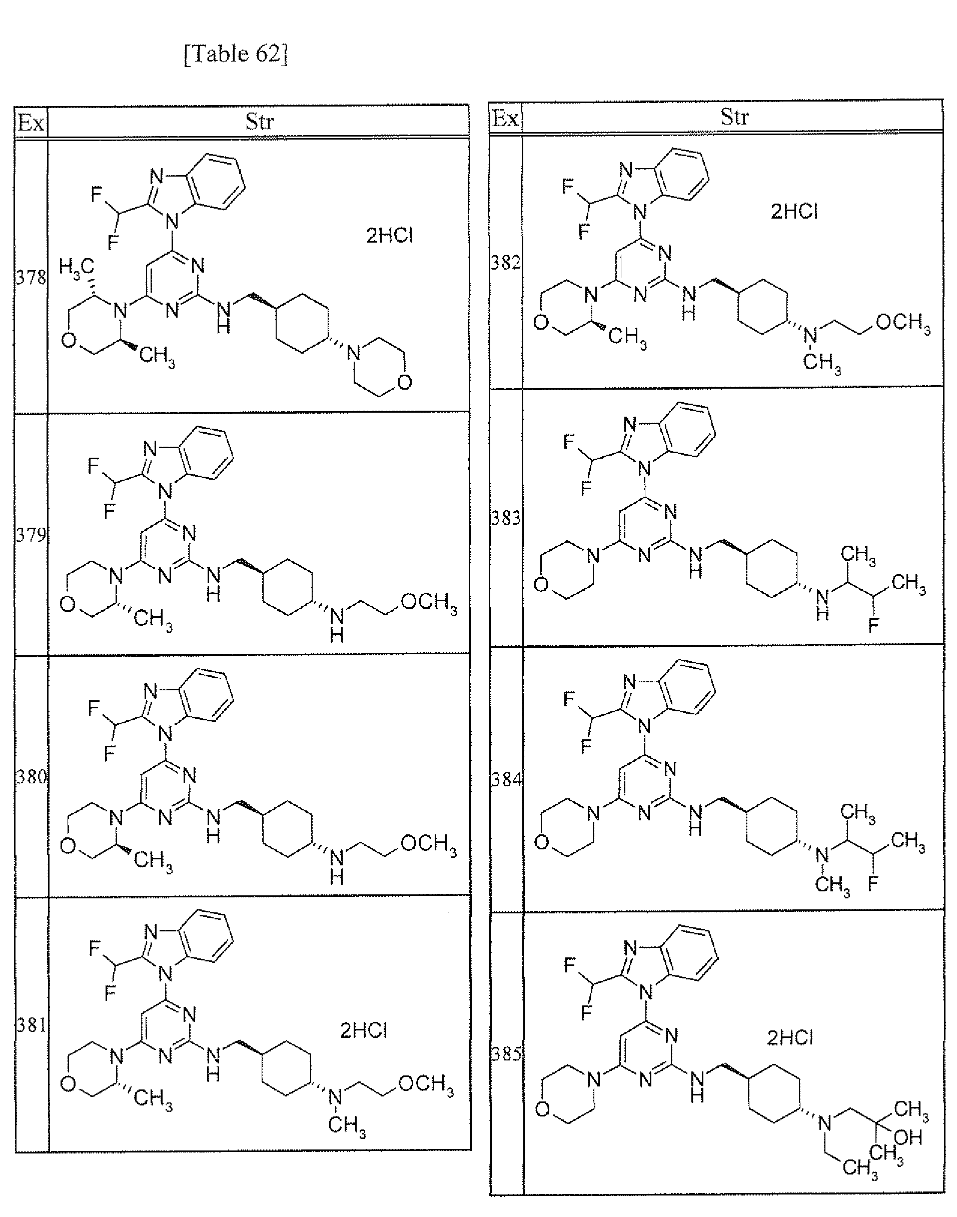

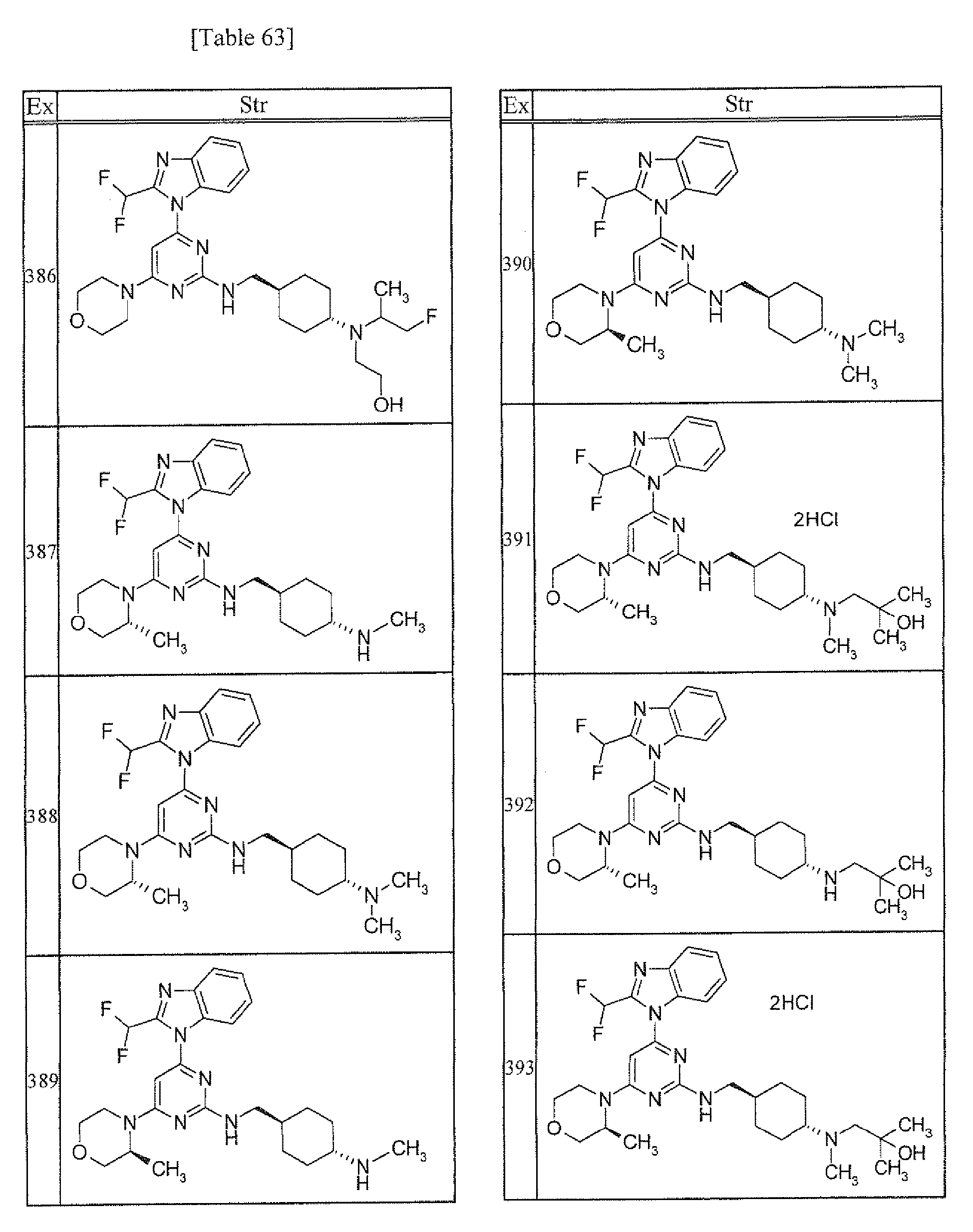

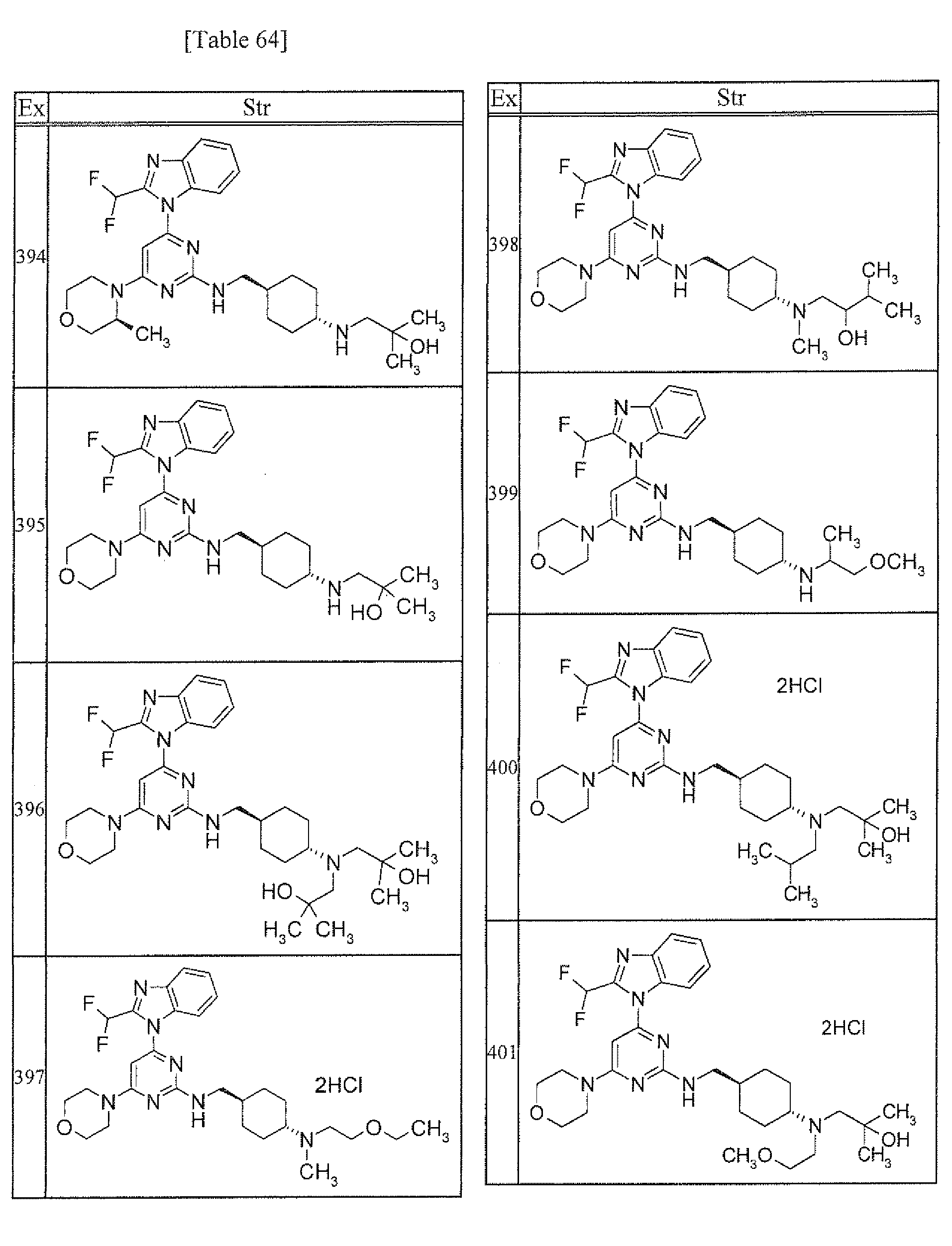

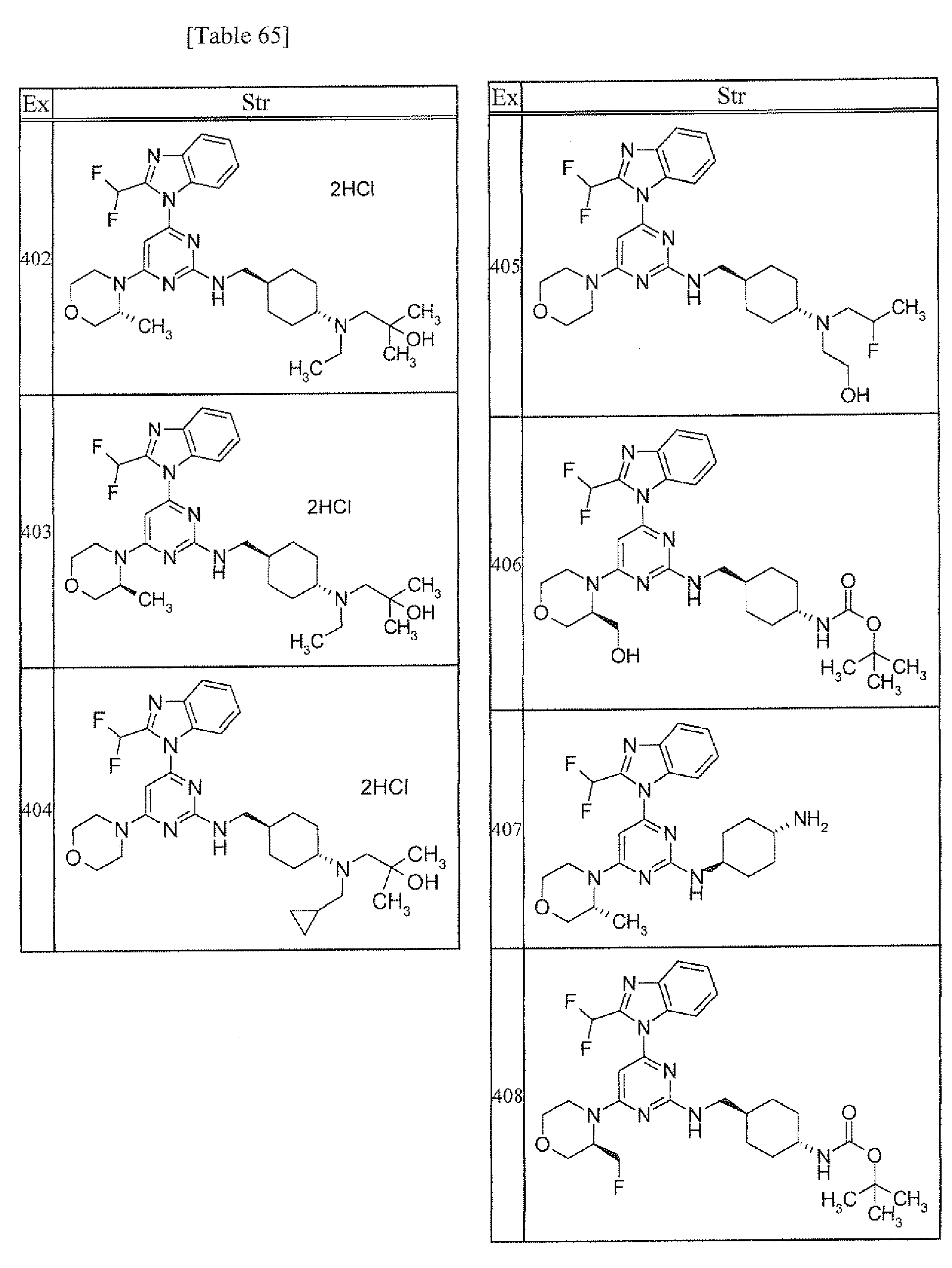

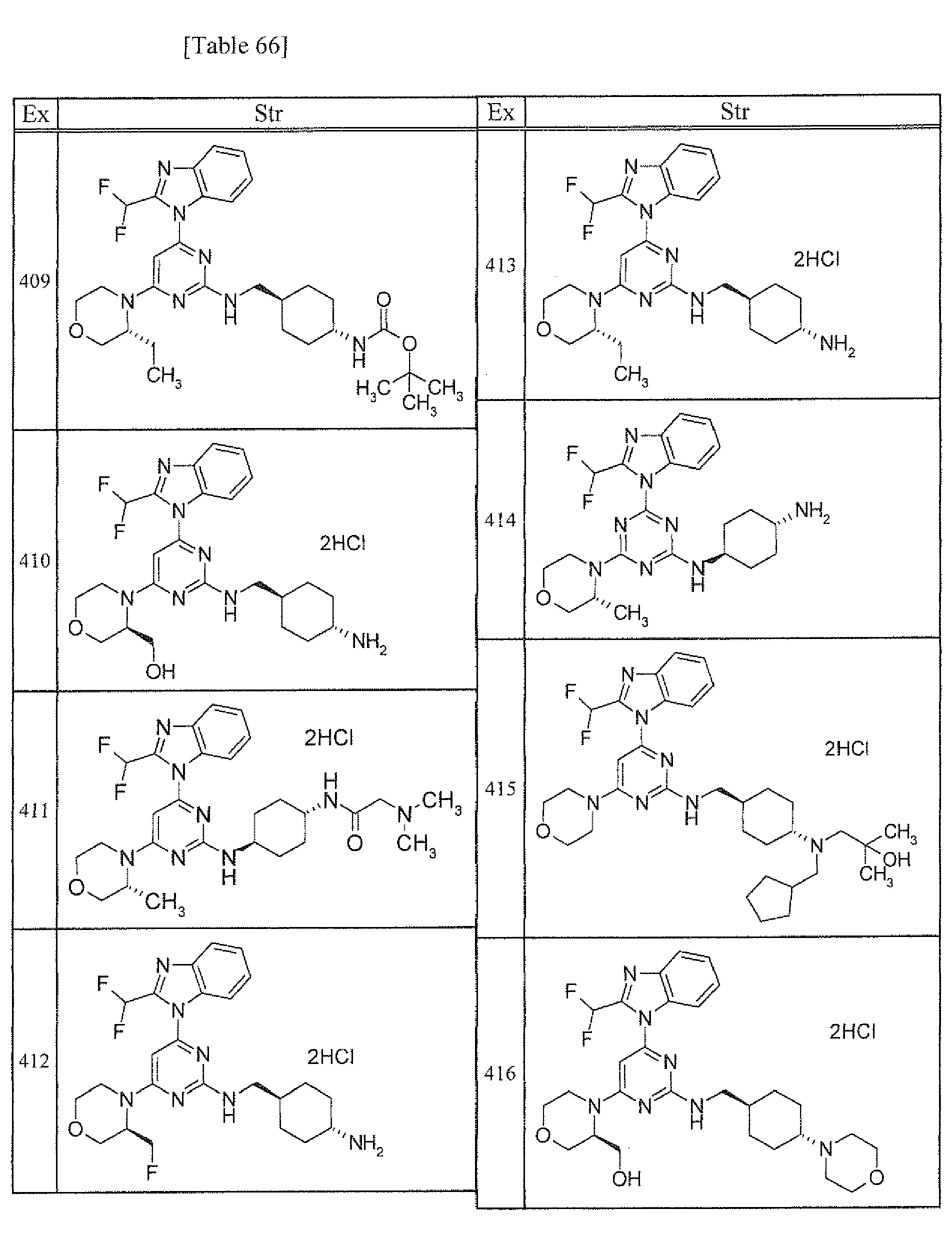

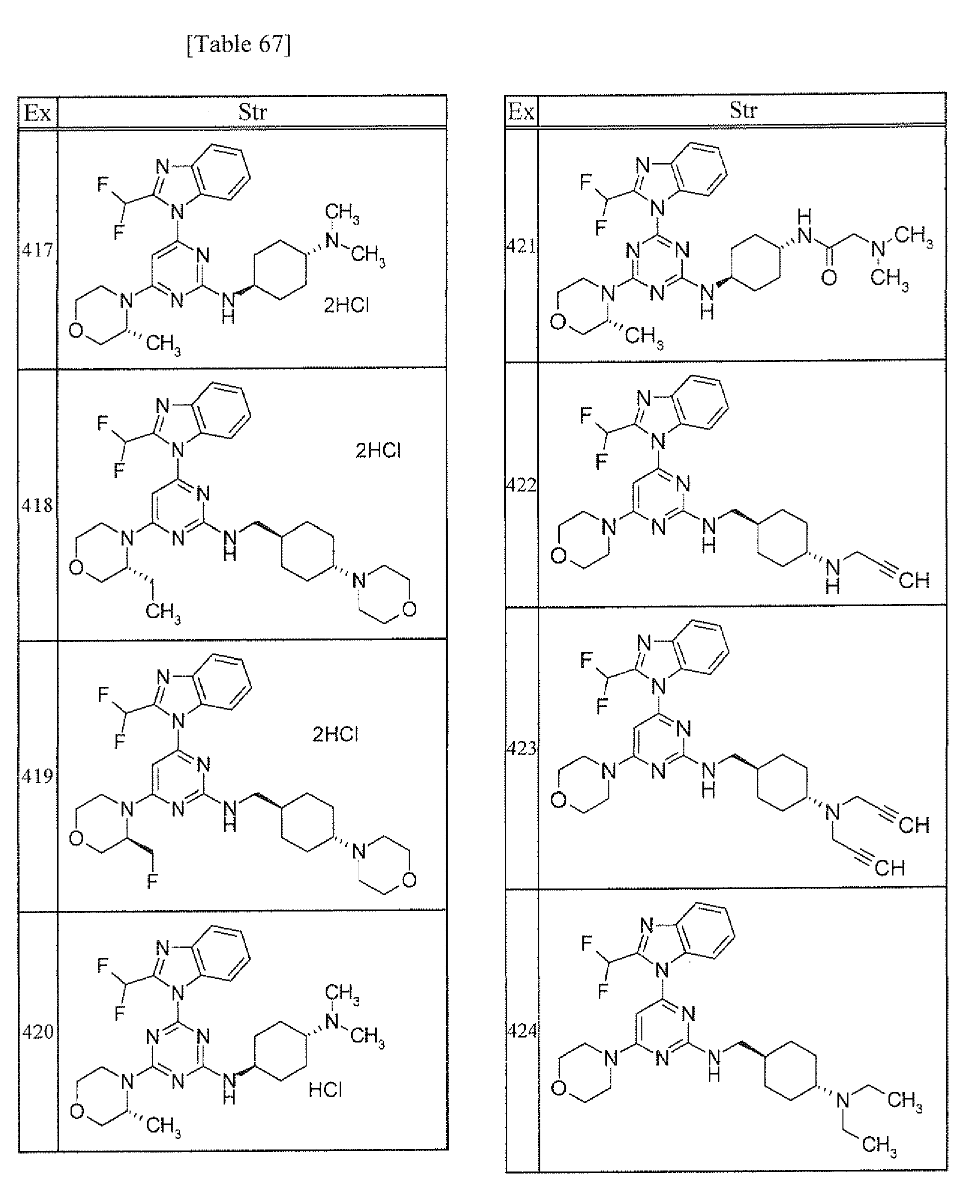

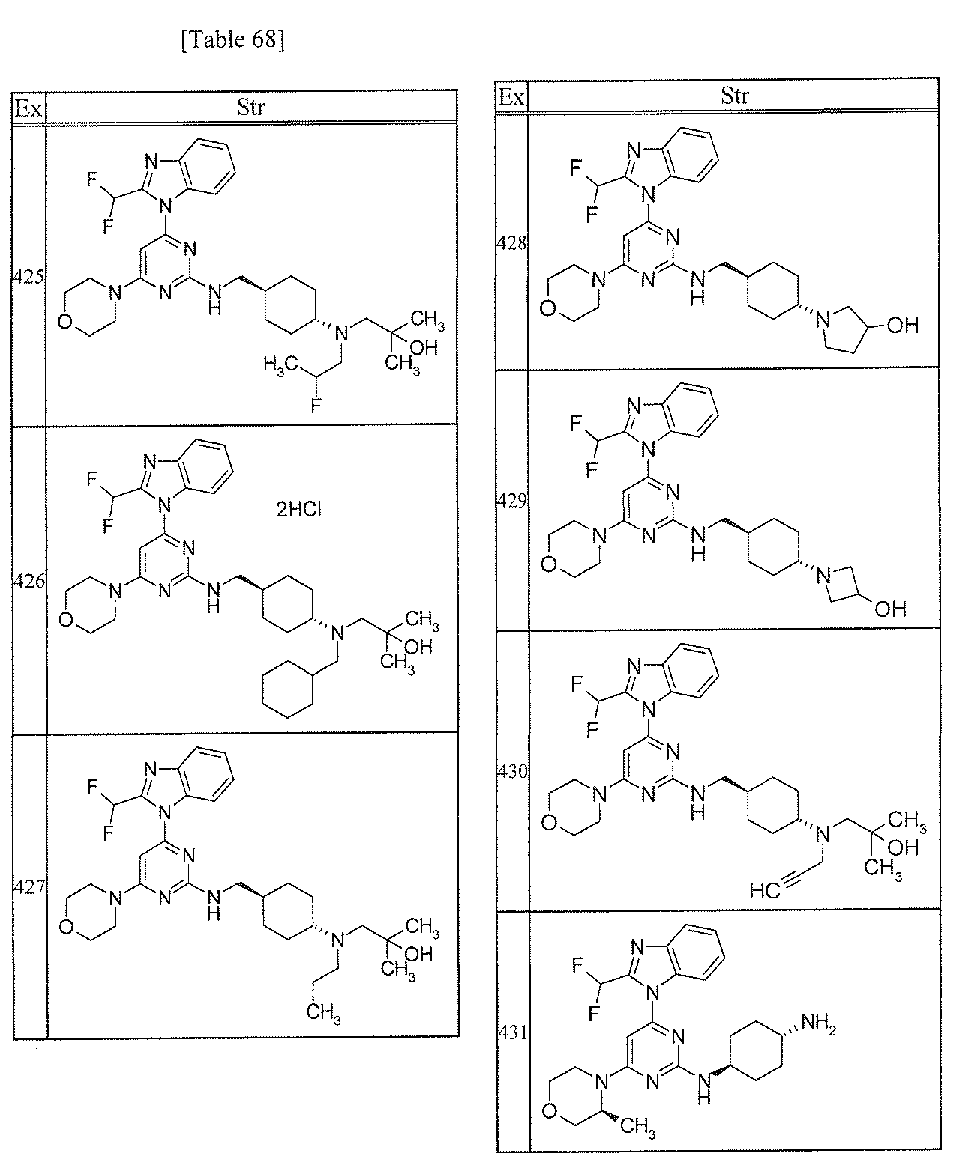

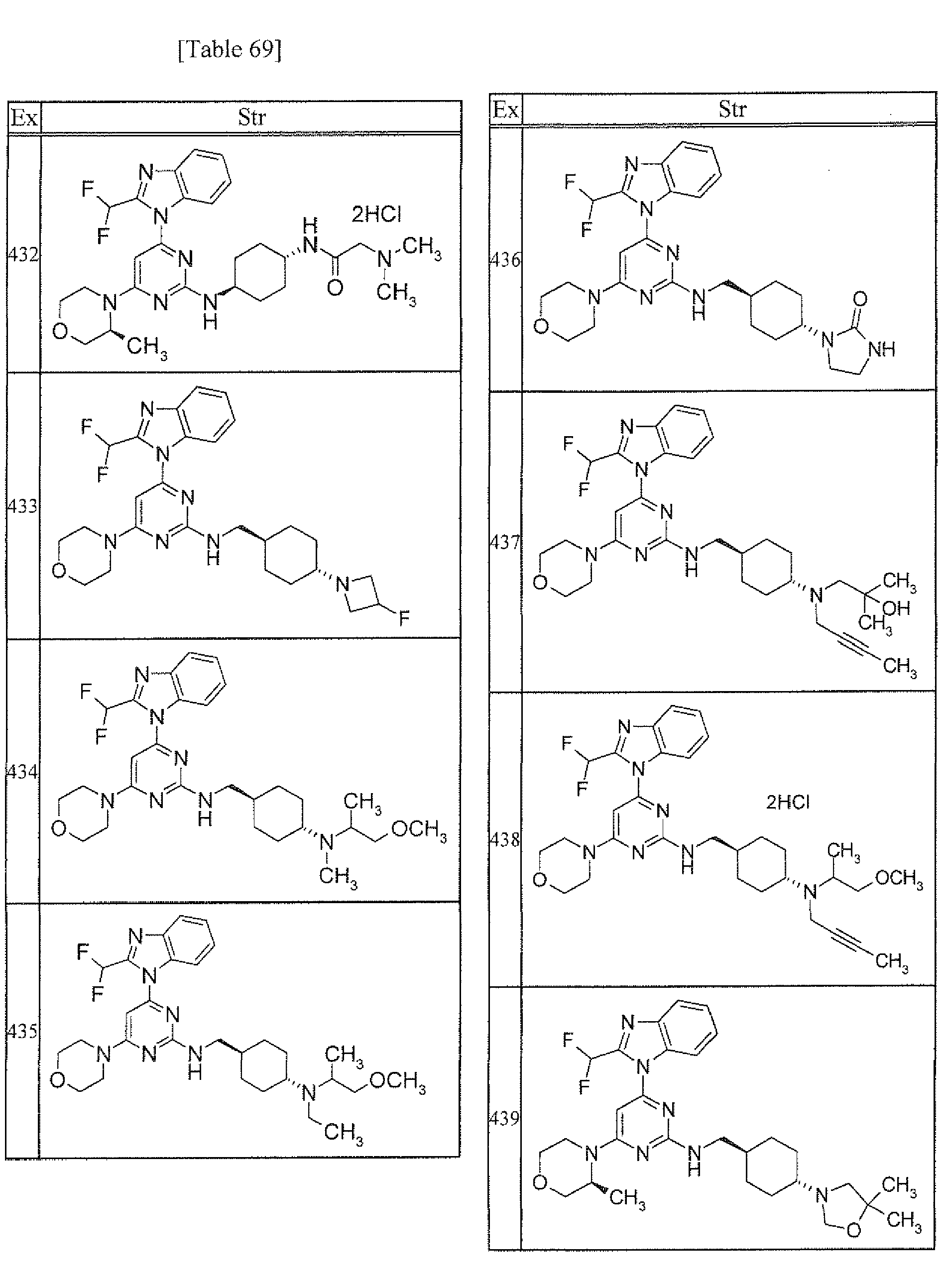

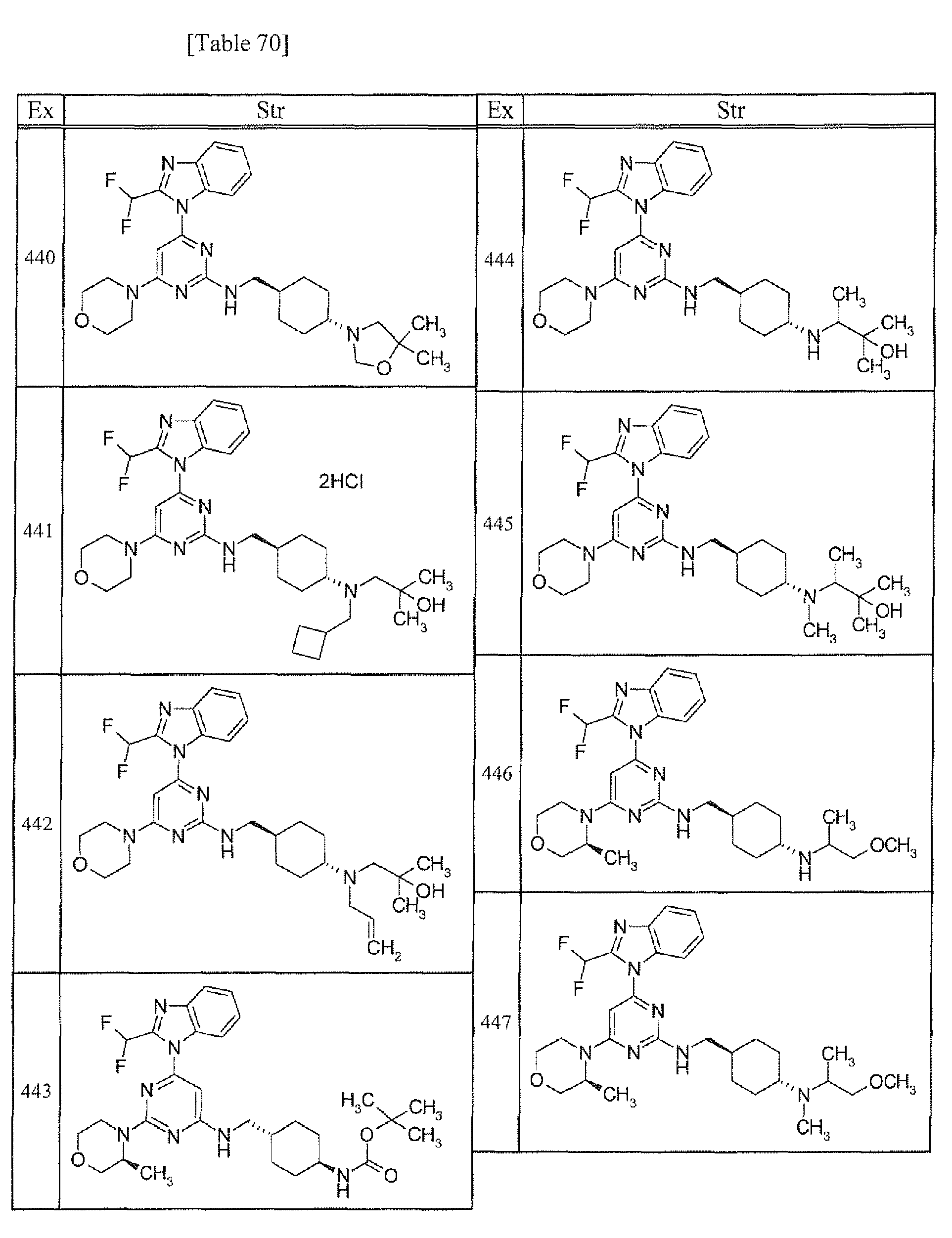

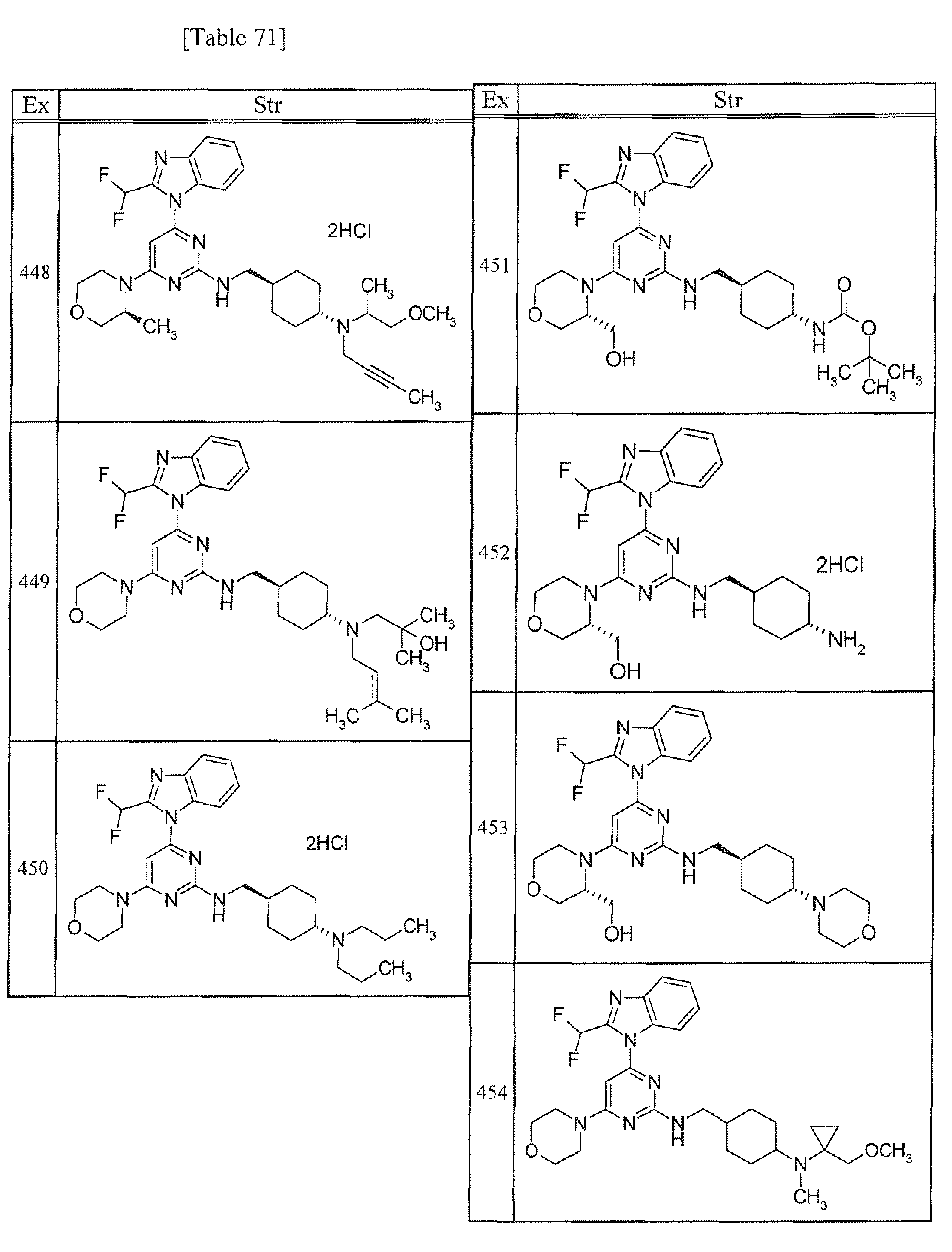

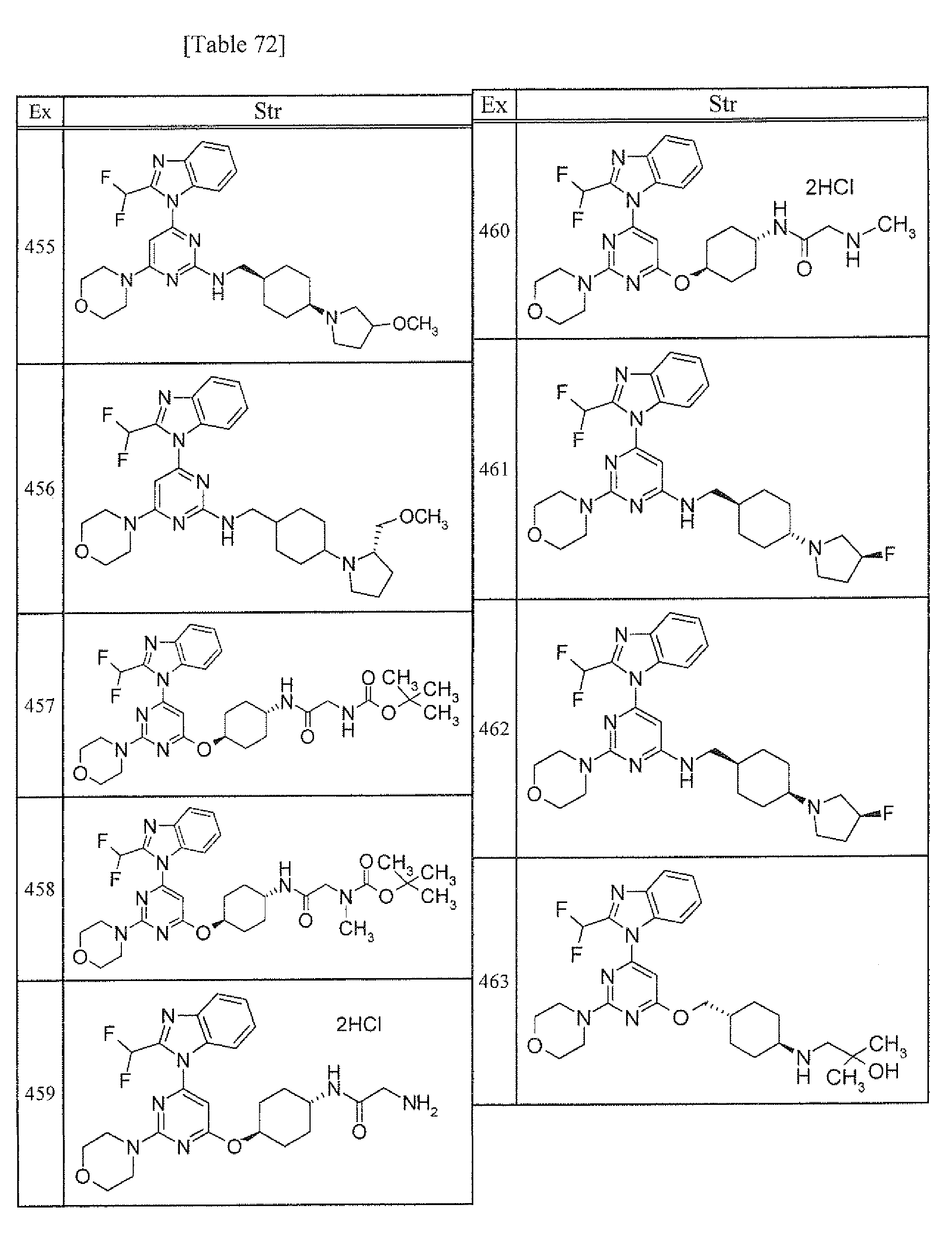

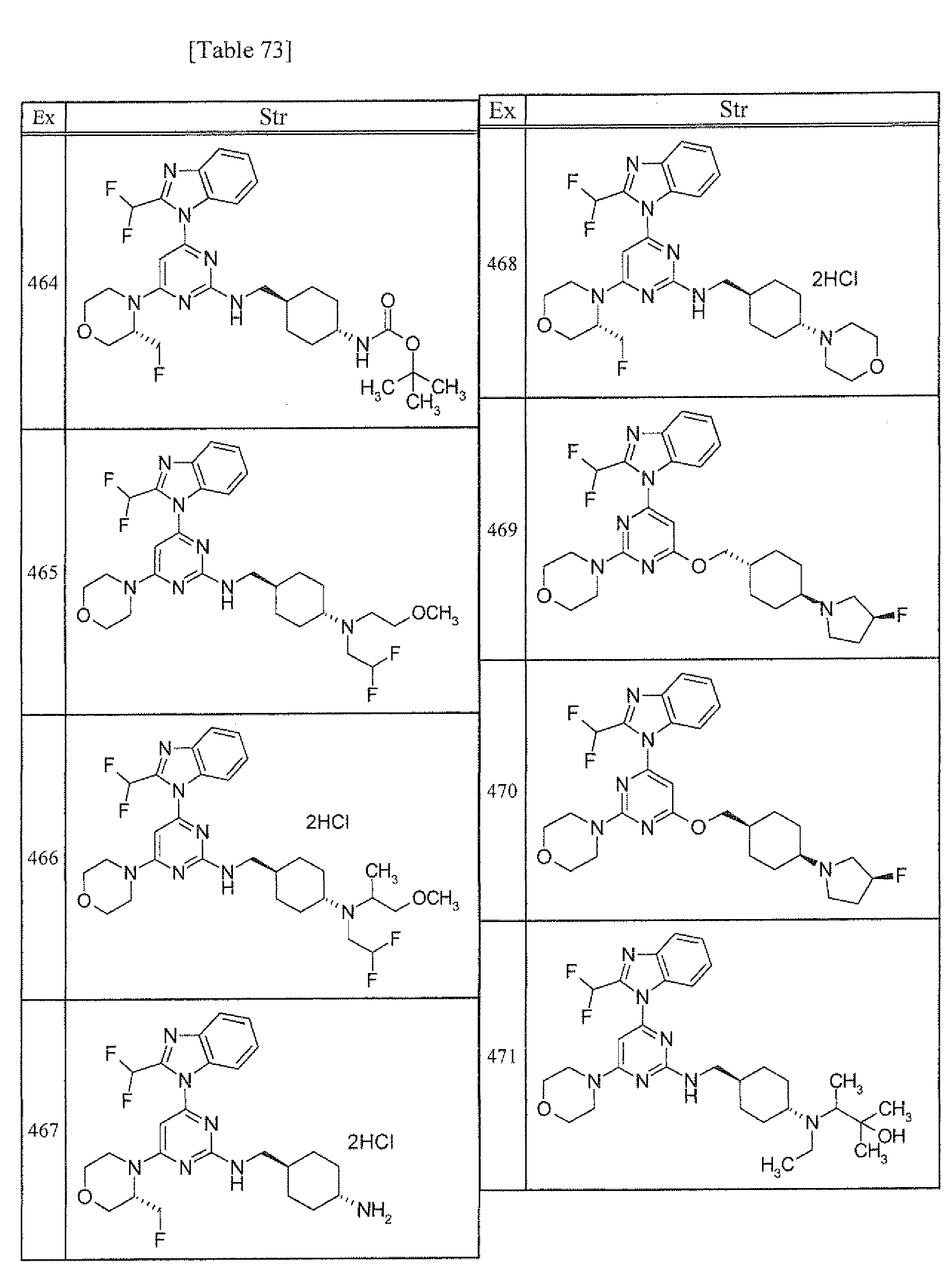

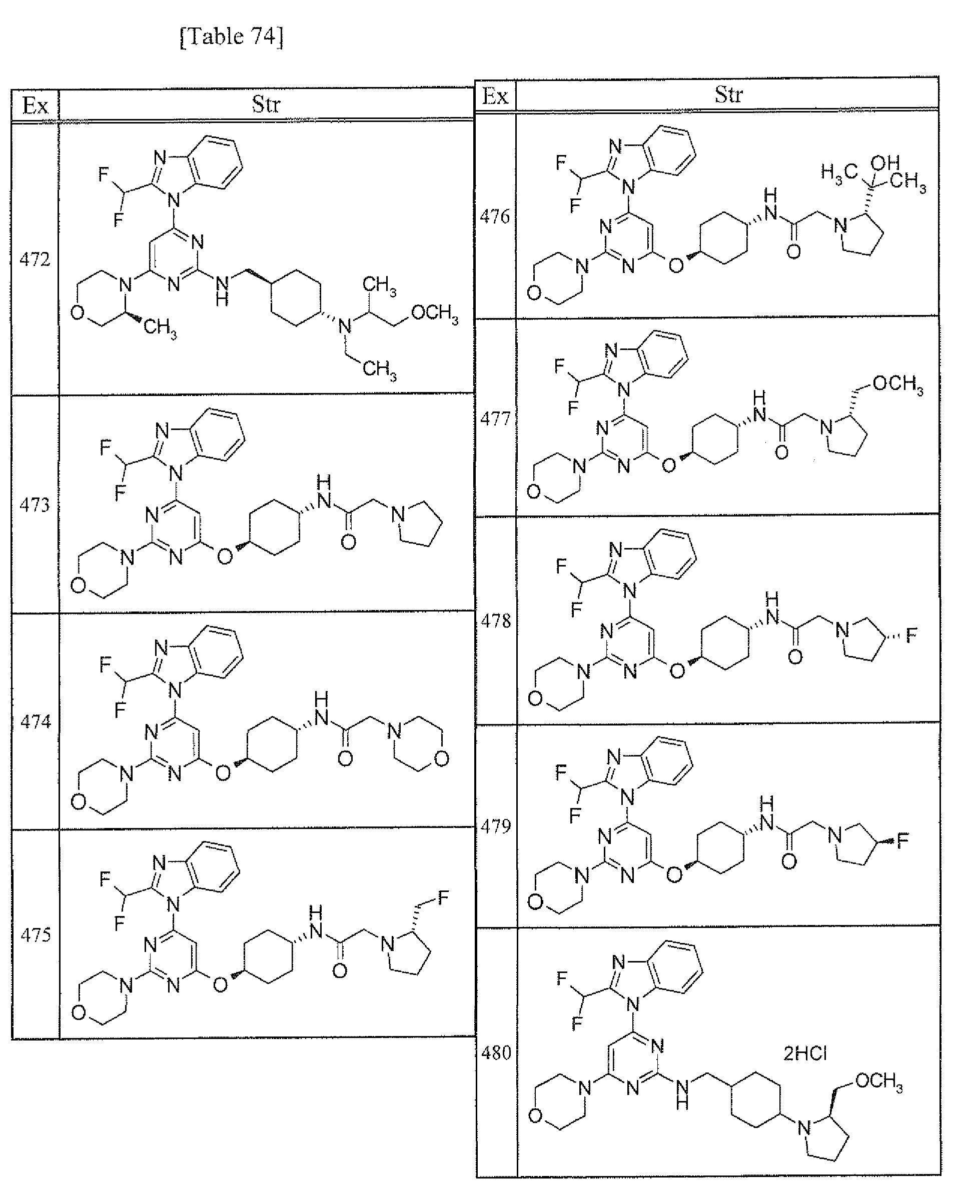

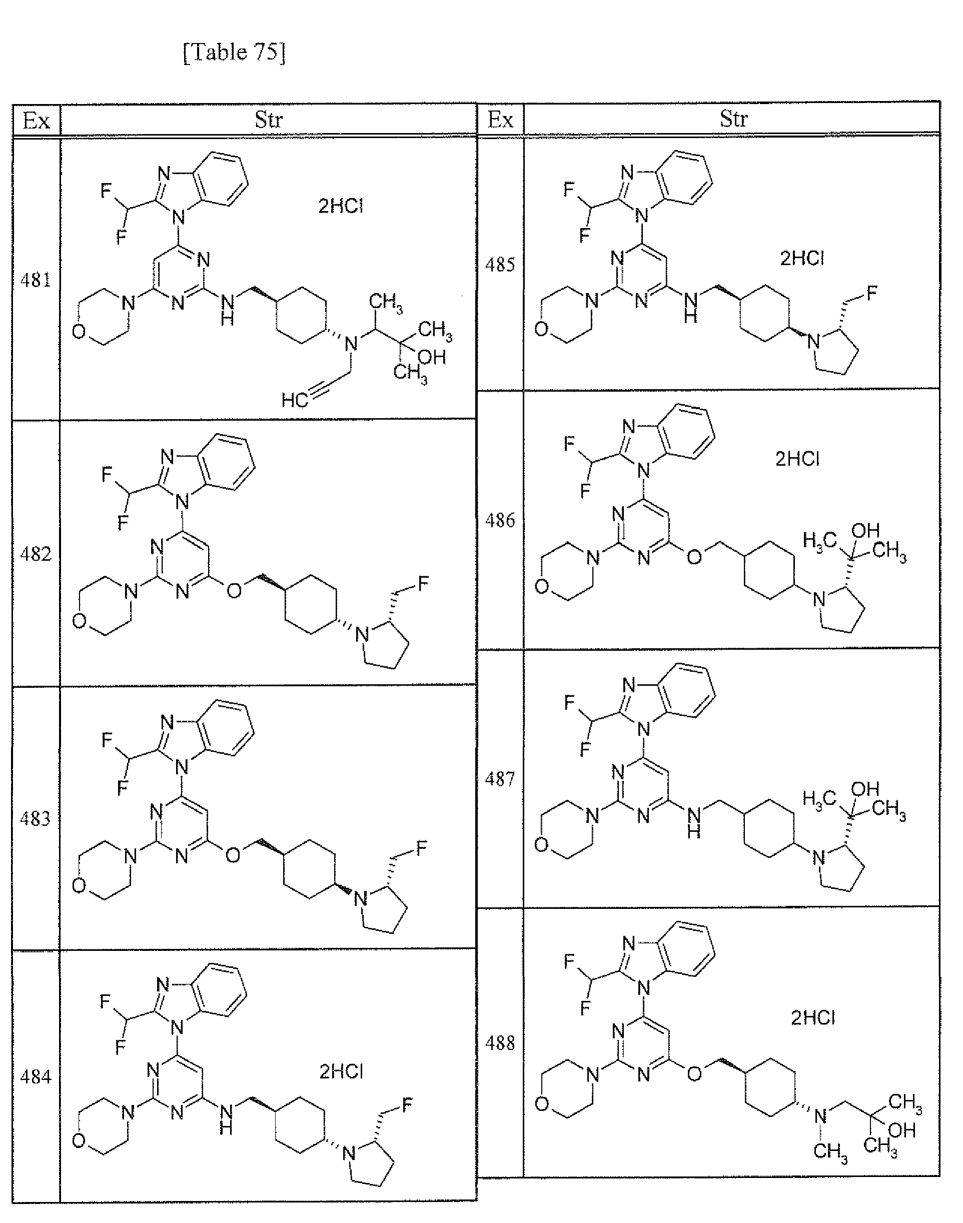

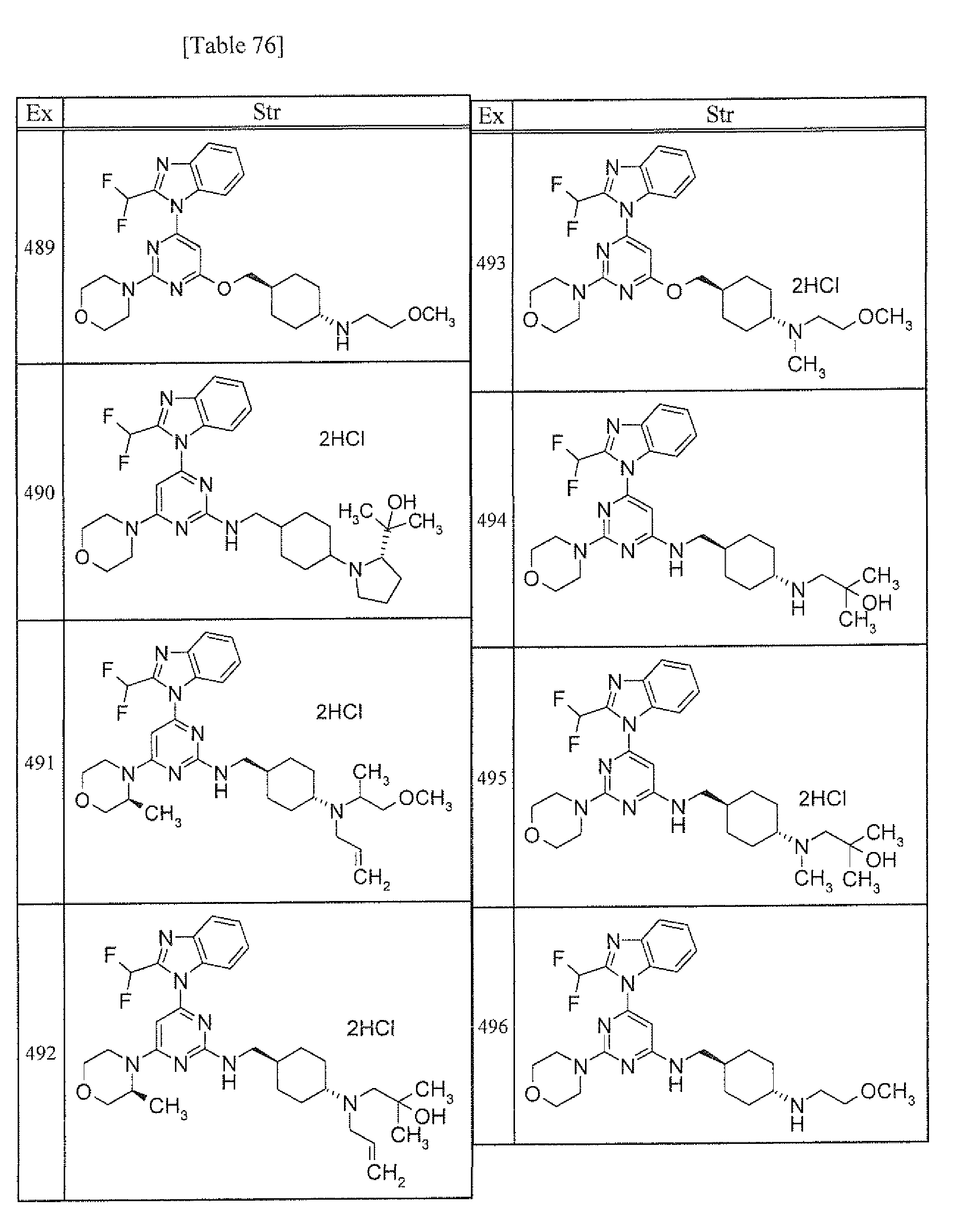

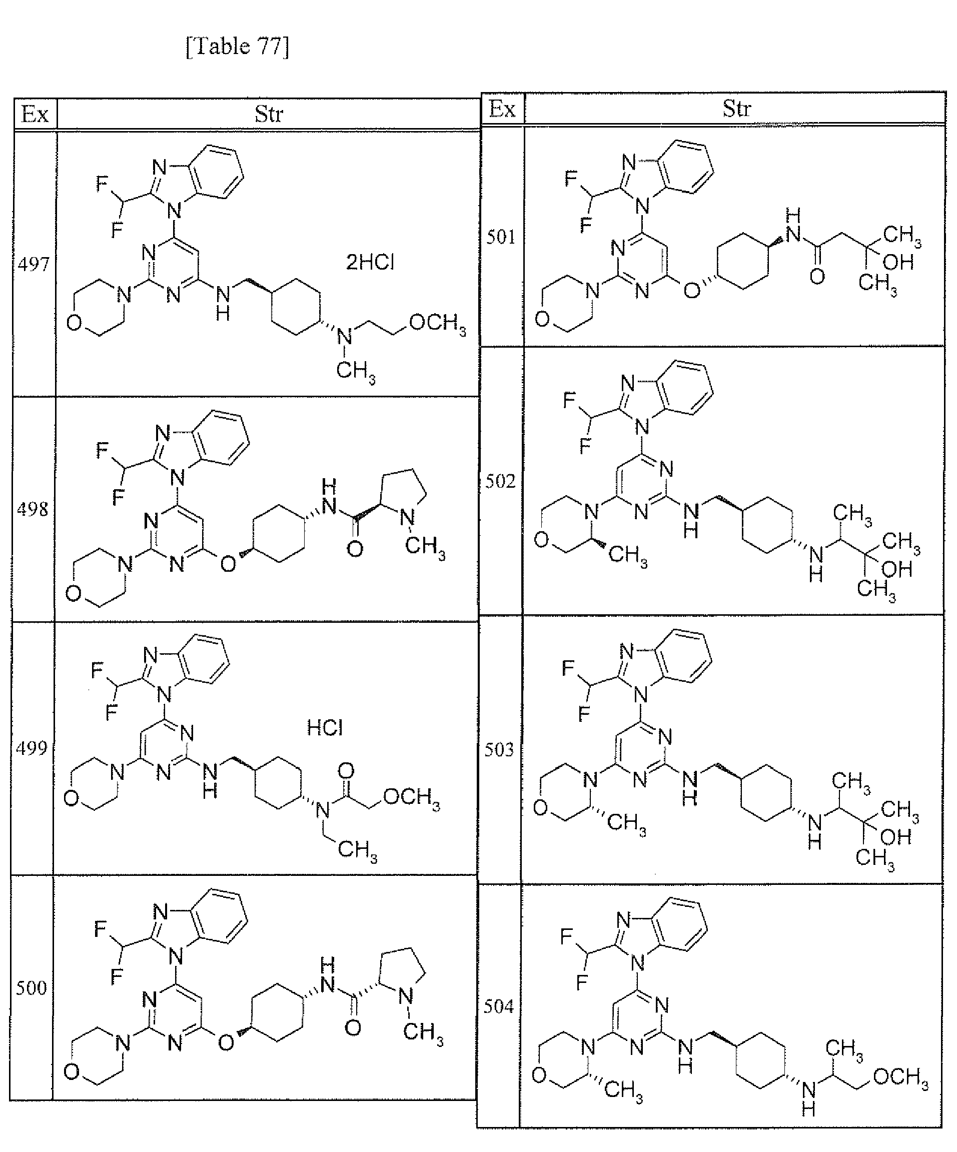

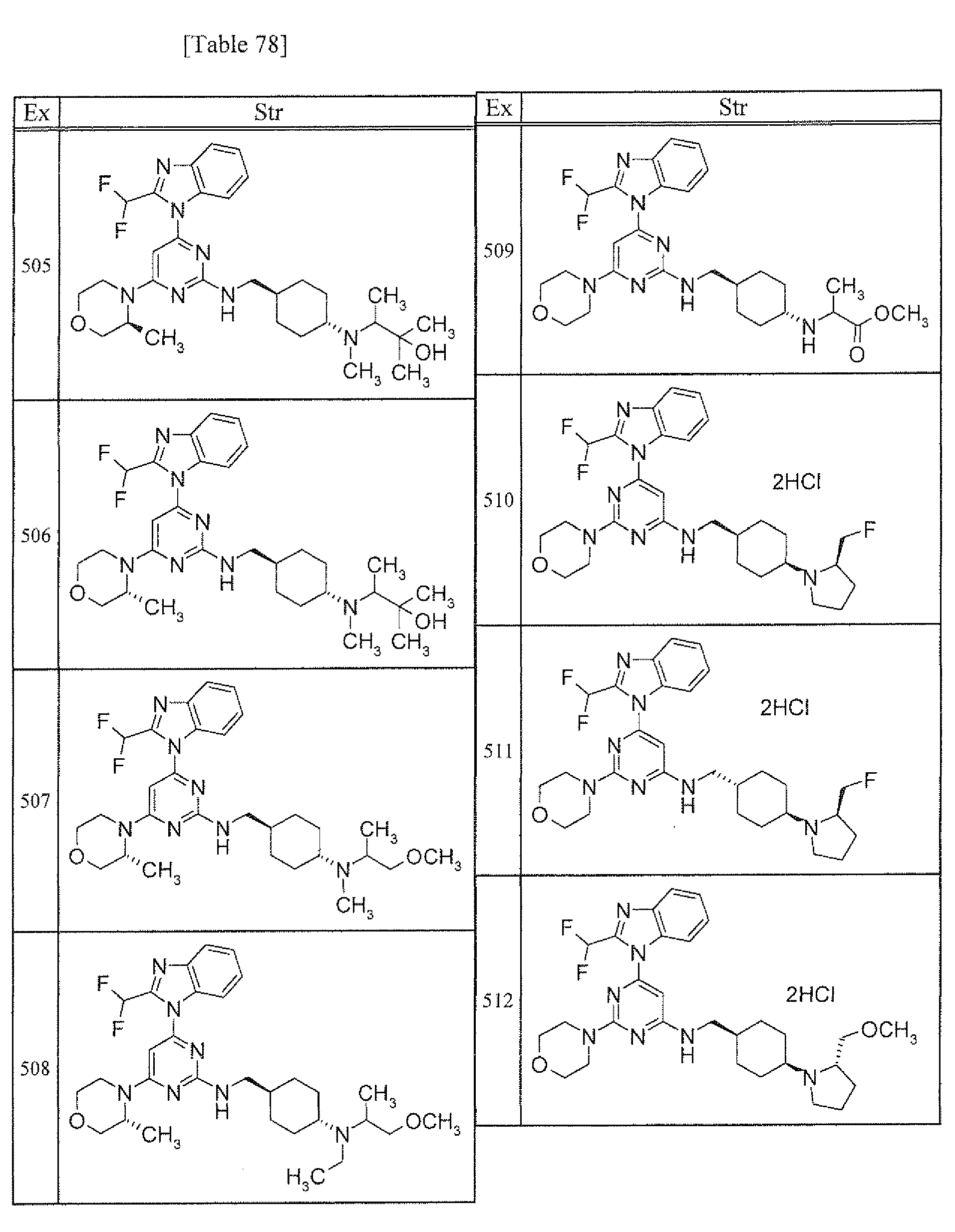

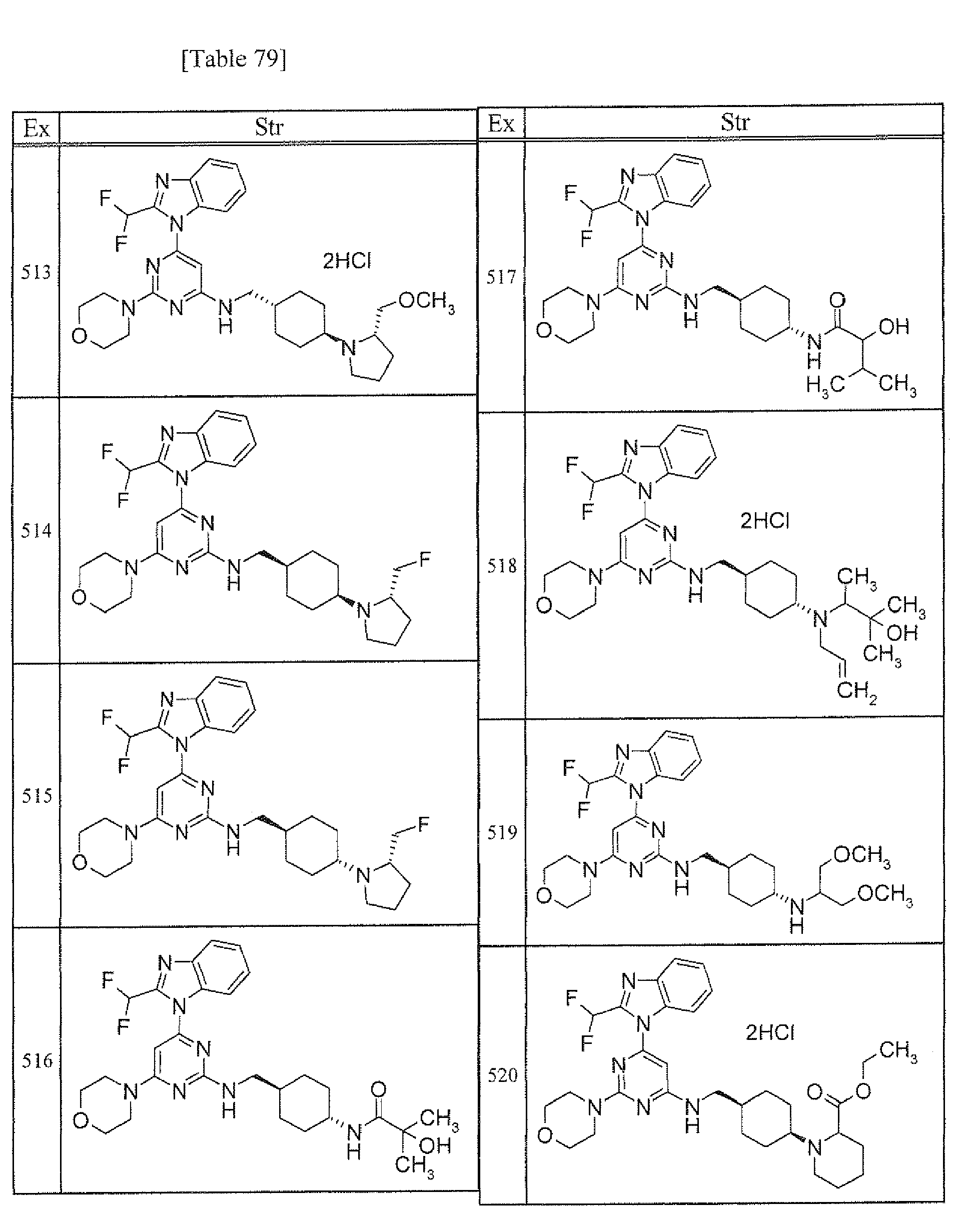

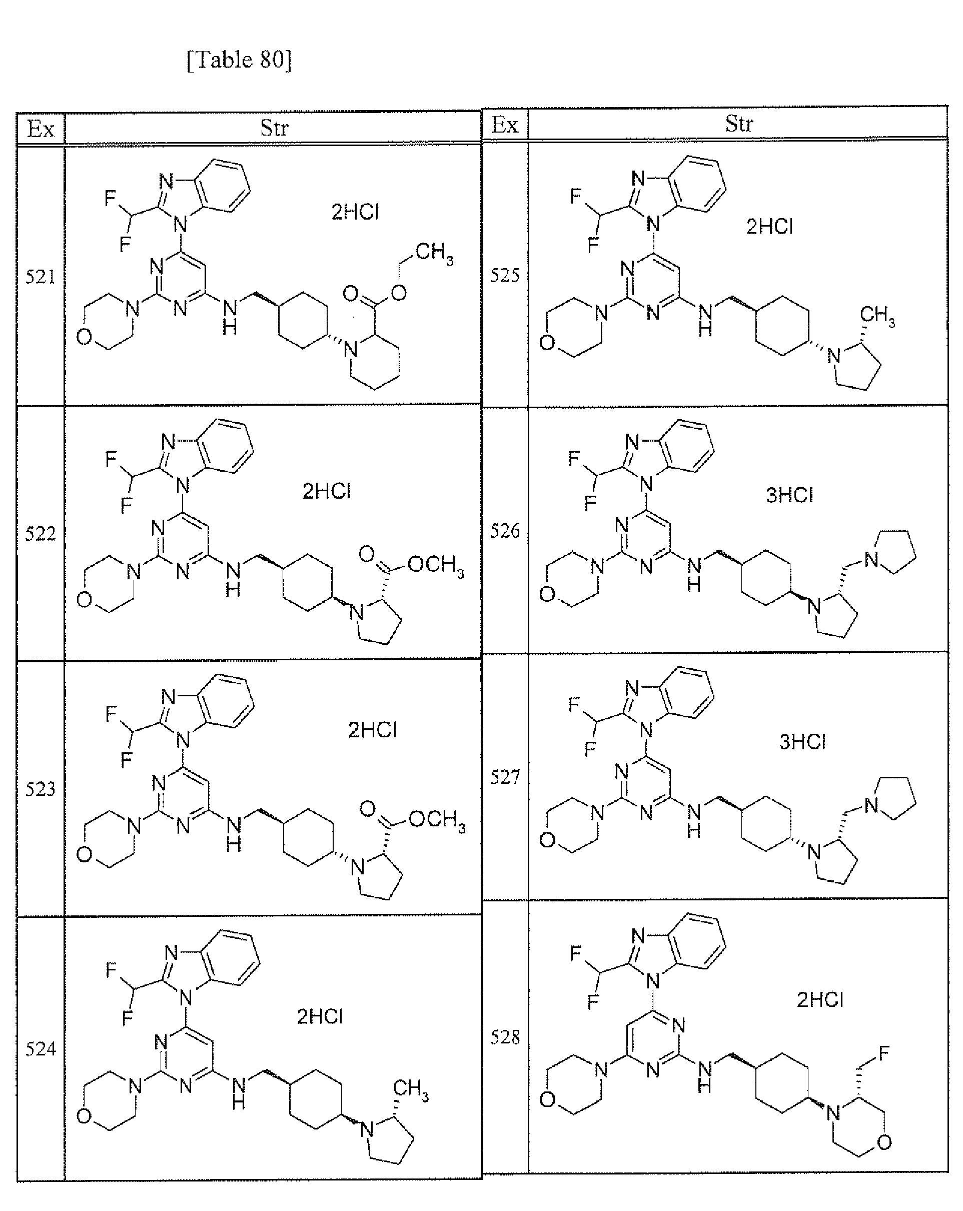

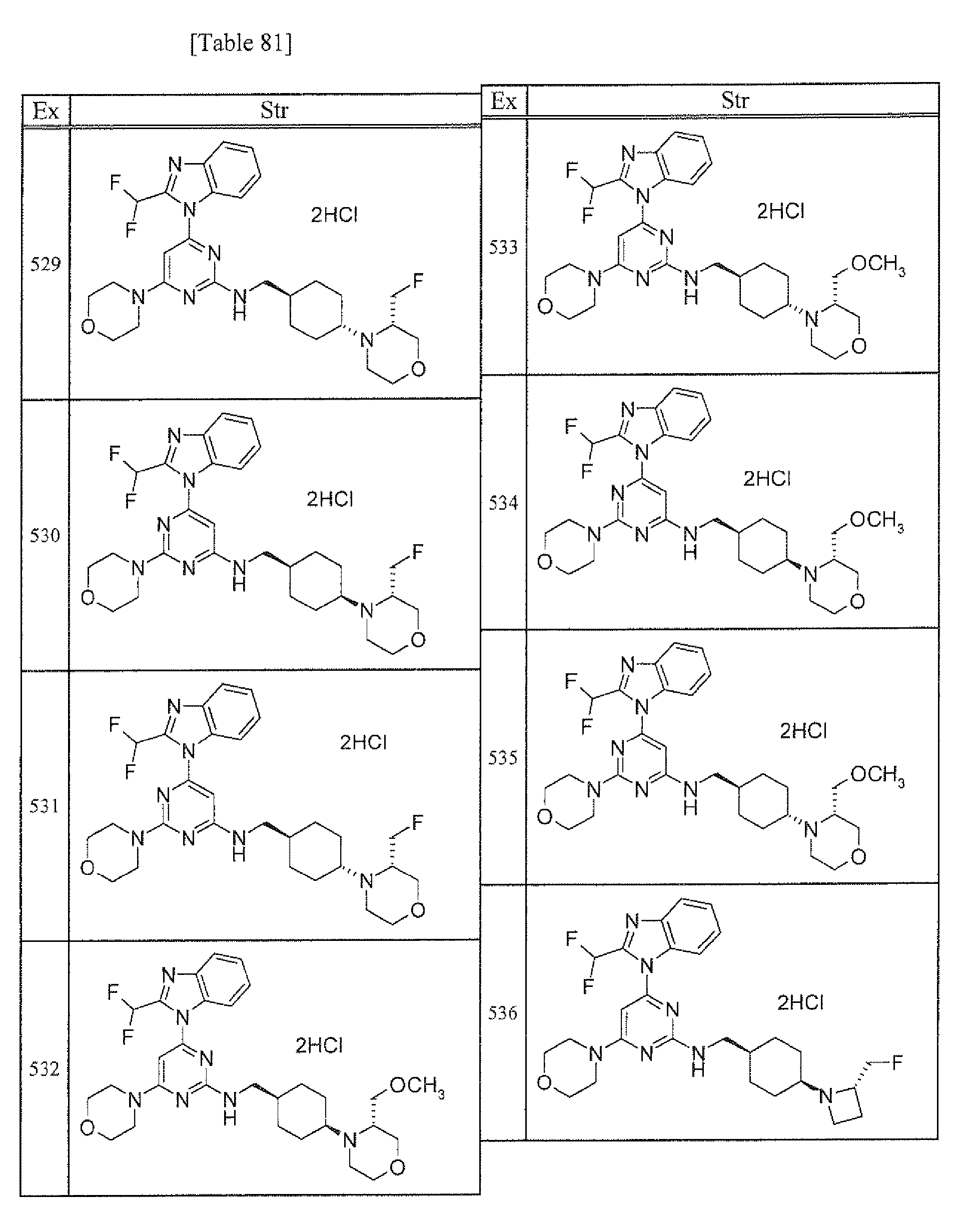

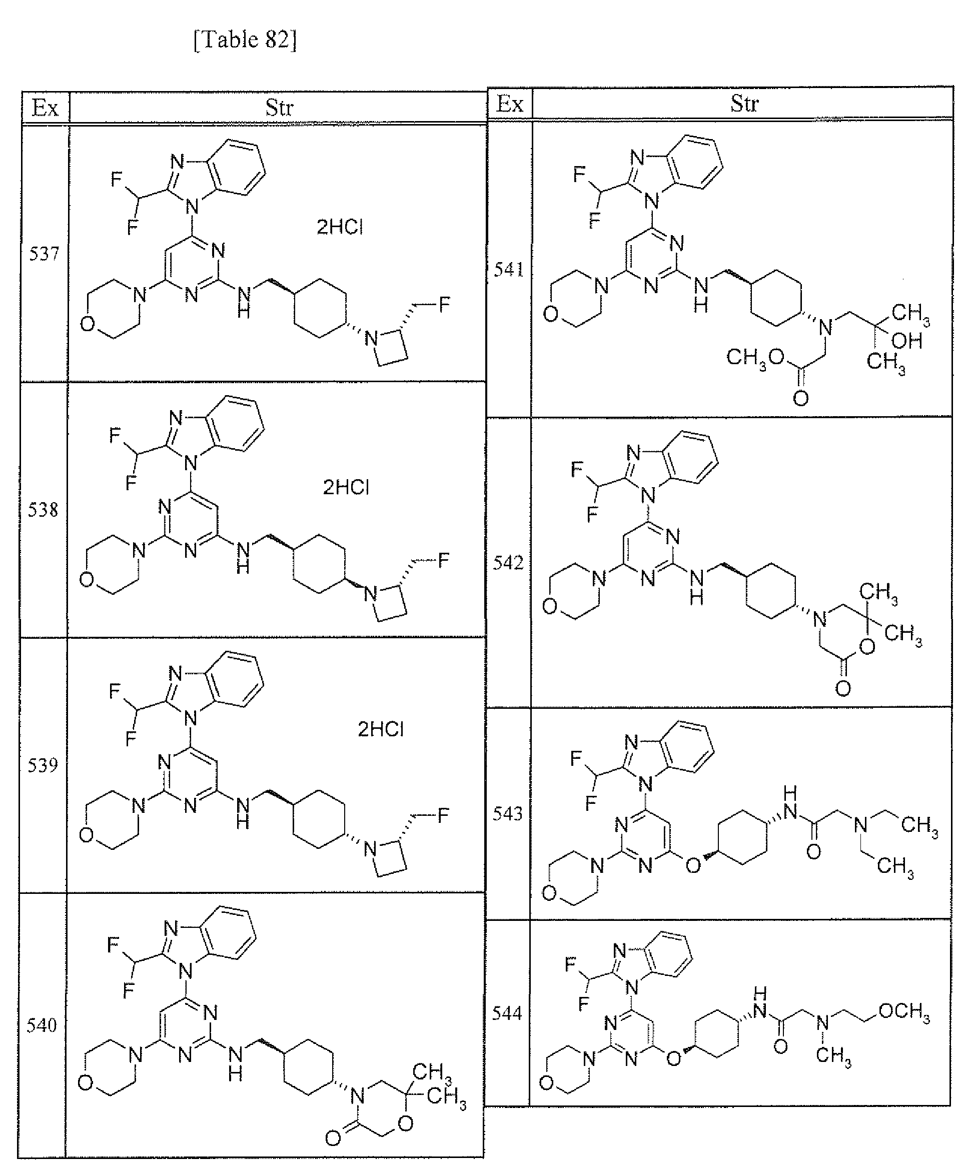

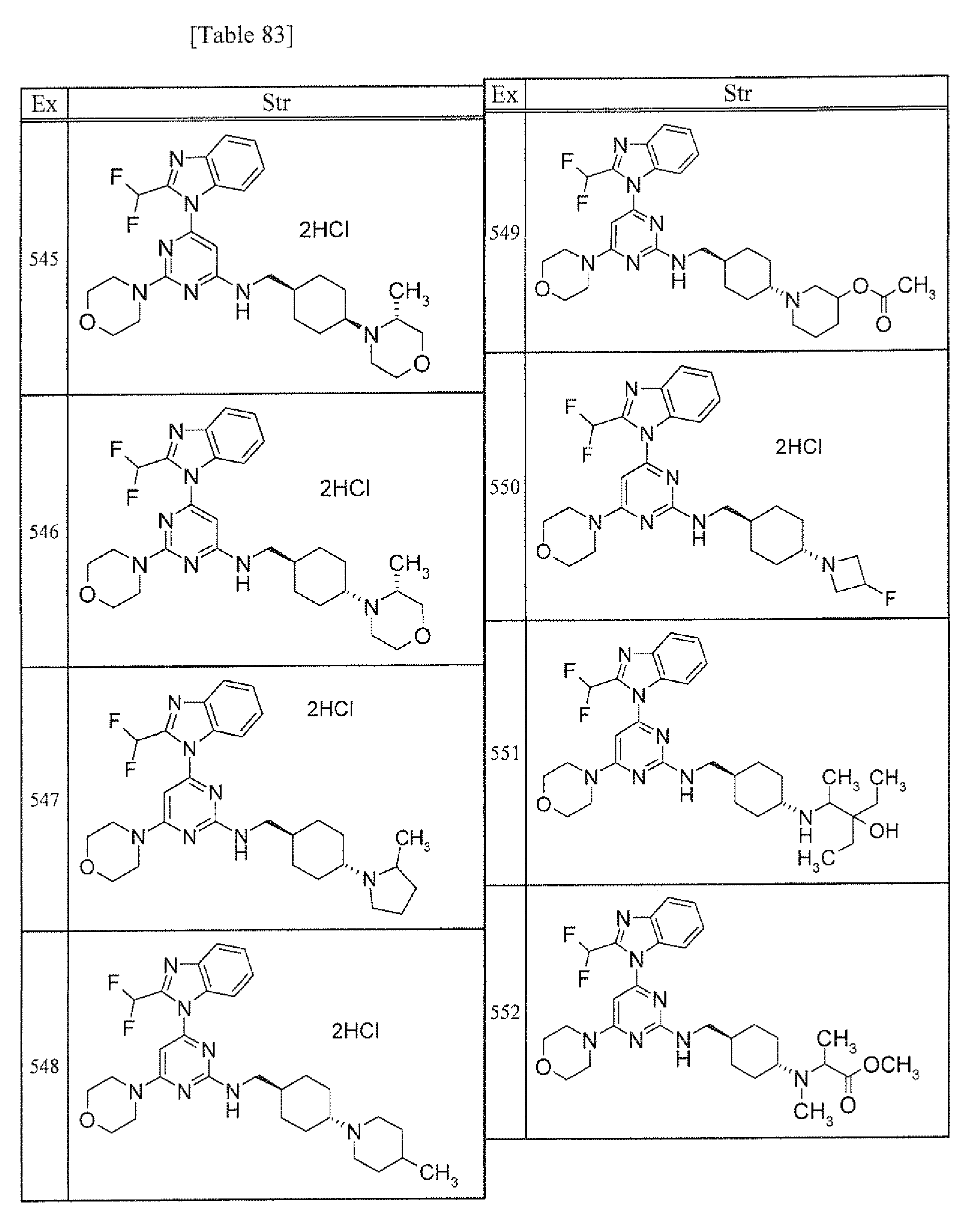

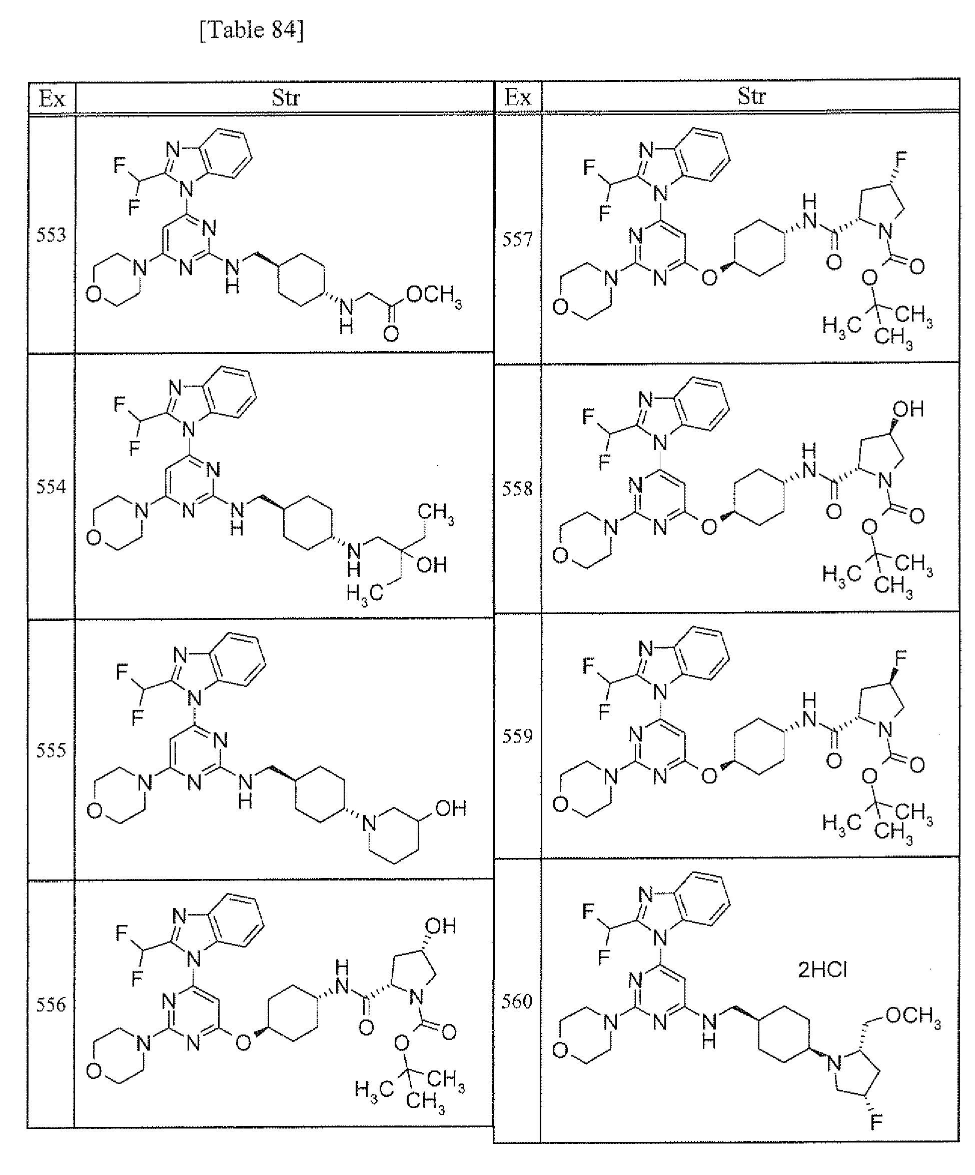

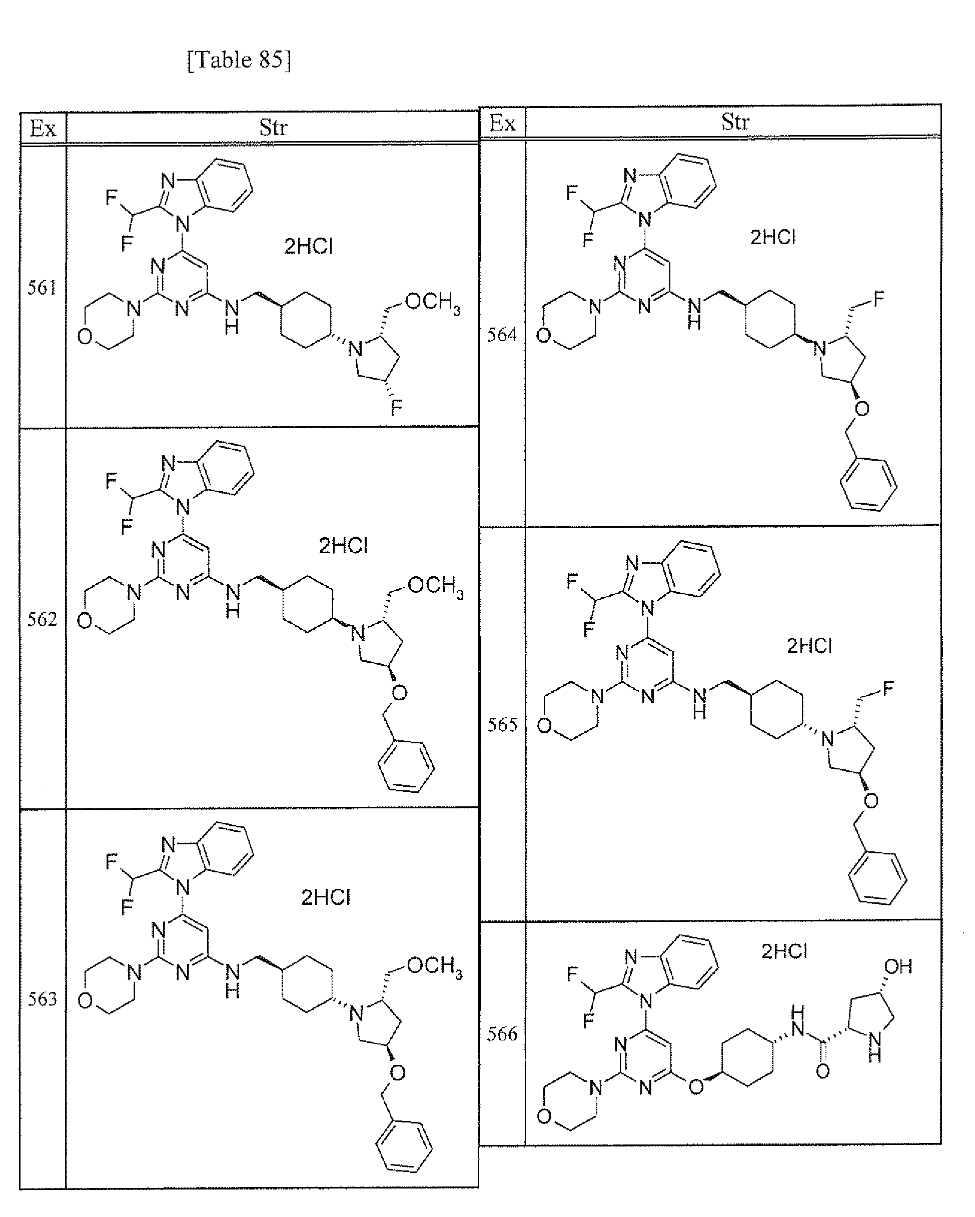

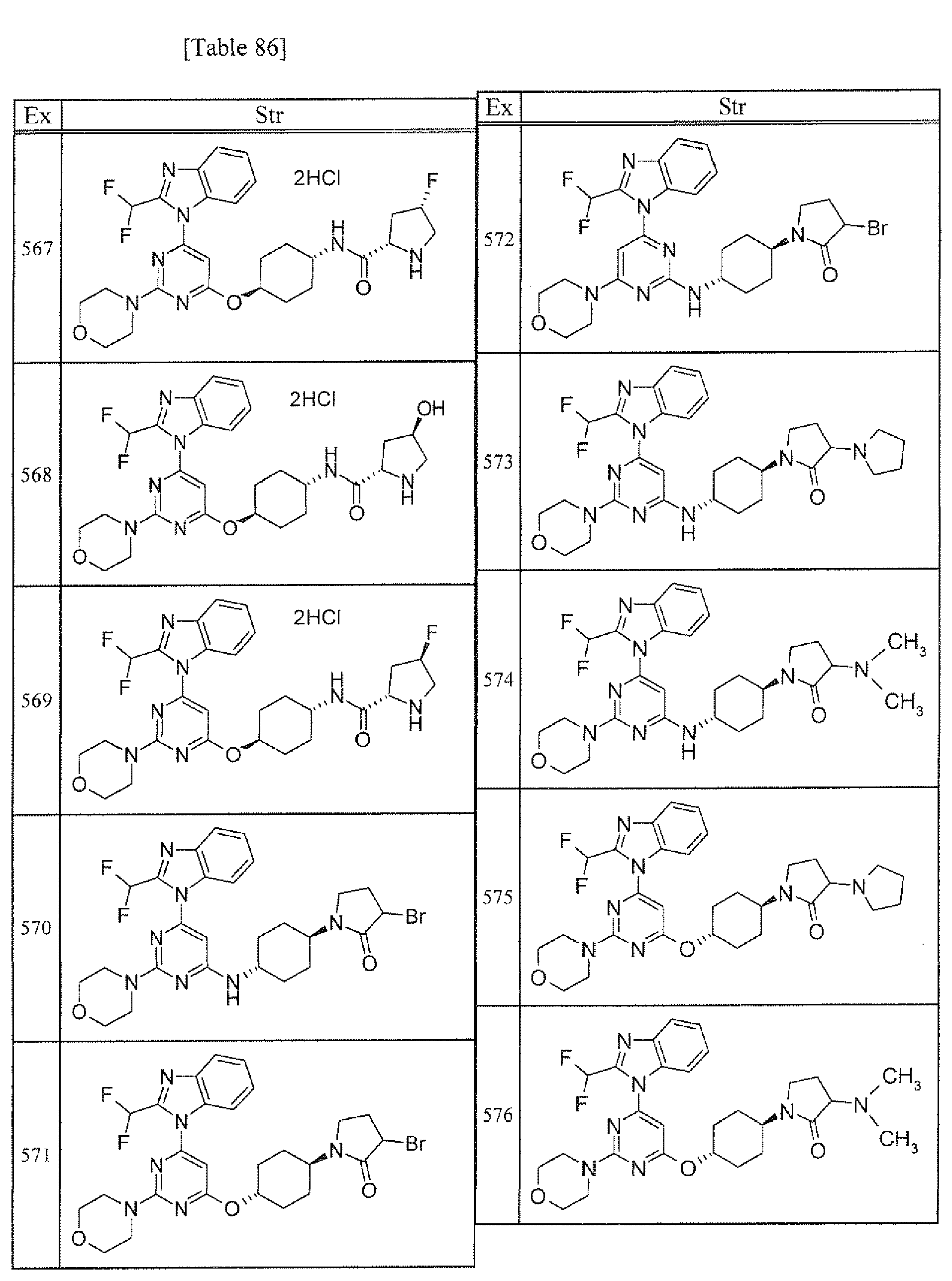

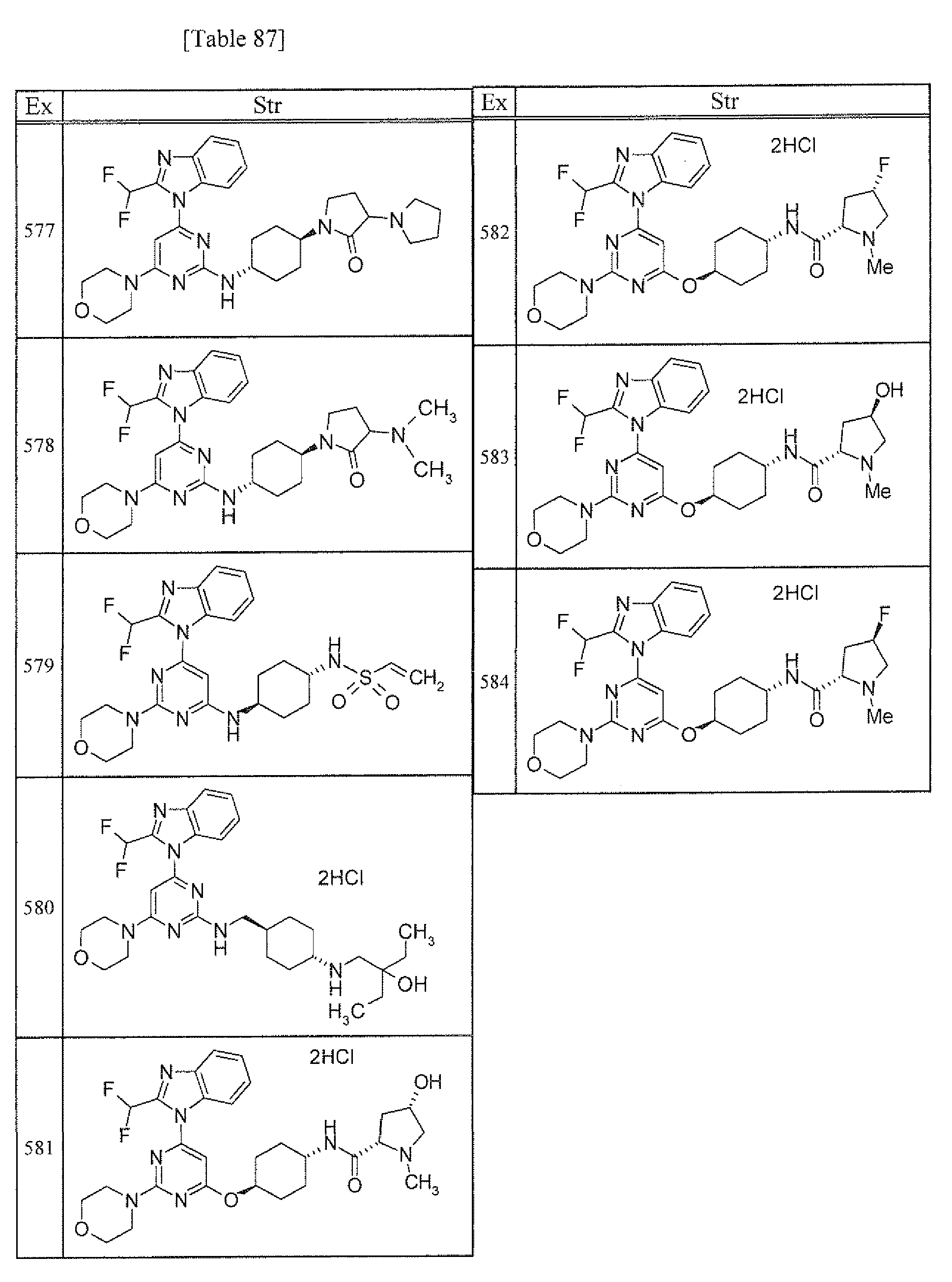

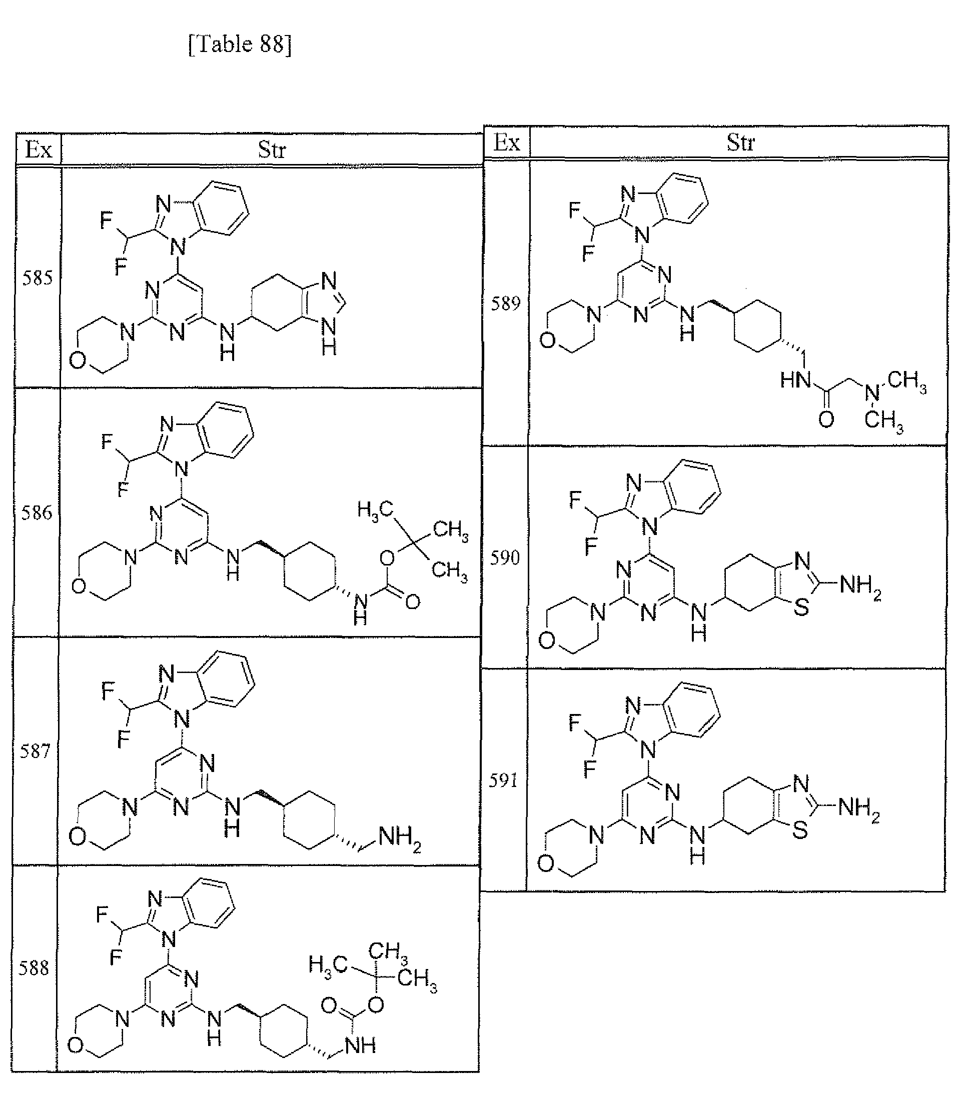

**[Table 89]**

| Ex | Syn | DATA |
|---|---|---|
| | | ESI+: 444 |
| 1 | 1 | NMR1: 0.95-2.02 (11H, m), 3.55-3.77 (9H, m), 6.23-6.39 (1H, m), 6.84-7.04 (1H, m), 7.35-7.90 (5H, m) |
| | | ESI+: 517 |
| 2 | 2 | NMR1: 1.32-1.51 (4H, m), 1.70-2.03 (4H, m), 2.29-2.30 (3H, m), 3.55-3.86 (10H, m), 3.78 (2H, s), 7.40-7.69 (3H, m), 7.78-8.05 (3H, m), 8.42-8.59 (1H, m) |
| | | ESI+: 551 |
| 3 | 3 | NMR1: 0.96-1.00 (3H, m), 1.29-1.43 (4H, m), 1.61-1.72 (2H, m), 1.87-2.01 (4H, m), 2.96-3.01 (2H, m), 3.05-3.15 (1H, m), 3.65-3.83 (9H, m), 7.02-7.06 (1H, m), 7.40-7.49 (2H, m), 7.64-8.05 (3H, m), 8.41-8.58 (1H, m) |
| | | ESI+: 473 |
| 4 | 4 | NMR: 1.22-1.40 (4H, m), 1.79-2.21 (10H, m), 3.64-3.85 (9H, m), 7.38-7.52 (2H, m), 7.63-8.07 (3H, m), 8.40-8.69 (1H, m) |
| | | ESI+: 524 [M+Na] |
| 5 | 5 | NMR1: 1.18-123 (2H, m), 1.31-1.44 (2H, m), 1.85-1.99 (4H, m), 2.53-2.54 (3H, m), 3.70-3.78 (9H, m), 5.54-5.82 (2H, m), 7.42-7.49 (2H, m), 7.64-7.92 (3H, m), 8.41-8.58 (1H, m) |
| | | ESI+: 601 |
| 6 | 6 | NMR1: 1.15-1.25 (2H, m), 1.33-1.42 (2H, m), 1.84-1.99 (4H, m), 2.29-2.37 (6H, m), 3.08-3.12 (2H, m), 3.56-3.58 (4H, m), 3.67-3.83 (8H, m), 5.58-5.66 (1H, m), 5.87-5.99 (1H, m), 7.40-7.51 (2H, m), 7.65-8.05 (3H, m), 8.41-8.58 (1H, m) |
| | | ESI+: 545 |
| 7 | 7 | NMR1: 1.22-1.42 (13H, m), 1.77-2.01 (4H, m), 3.14-3.32 (1H, m), 3.60-3.83 (9H, m), 6.75-6.79 (1H, m), 7.40-7.51 (2H, m), 7.63-8.05 (3H, m), 8.41-8.58 (1H, m) |
| | | ESI+: 502 |
| 8 | 8 | NMR1: 1.23-1.45 (4H, m), 1.72-2.01 (6H, m), 3.04 (2H, s), 3.48-3.85 (10H, m), 7.38-8.05 (6H, m), 8.42-8.60 (1H, m) |

**[Table 90]**

| Ex | Syn | DATA |
|---|---|---|
| | | ESI+: 565, 567 |
| 9 | 9 | NMR1: 1.21-1.47 (4H, m), 1.82-2.04 (4H, m), 3.45-3.85 (12H, m), 7.39-7.51 (2H, m), 7.65-8.05 (4H, m), 8.42-8.59 (1H, m) |
| | | ESI+: 515 |
| 10 | 10 | NMR1: 1.23-1.40 (4H, m), 1.81-2.06 (4H, m), 2.13-2.26 (1H, m), 2.45-2.51 (4H, m), 3.53-3.60 (4H, m), 3.65-3.87 (9H, m), 7.39-7.54 (2H, m), 7.63-8.07 (3H, m), 8.39-8.60 (1H, m) |
| 11 | 11 | ESI+: 534 [M+Na] |
| | | ESI+: 556 |
| 12 | 12 | NMR1: 1.36-1.47 (4H, m), 1.67-1.84 (6H, m), 1.92-2.01 (2H, m), 2.45-2.62 (4H, m), 3.01-3.02 (2H, m), 3.54-3.82 (10H, m), 7.40-7.64 (3H, m), 7.78-8.05 (3H, m), 8.41-8.56 (1H, m) |
| | | ESI+: 570 [M+Na] |
| 13 | 13 | NMR1: 1.27-1.43 (2H, m), 1.47-1.69 (2H, m), 1.72-2.10 (4H, m), 2.13-2.26 (2H, m), 3.09-3.28 (4H, m), 3.58-3.75 (9H, m), 6.26-6.40 (1H, m), 6.90-7.06 (1H, m), 7.36-7.96 (5H, m) |
| | | ESI+: 501 |
| 14 | 14 | NMR1: 1.33-1.75 (6H, m), 1.95-2.06 (5H, m), 2.70-2.83 (3H, m), 3.55-4.33 (10H, m), 7.40-7.53 (2H, m), 7.66-8.05 (3H, m), 8.42-8.58 (1H, m) |
| | | ESI+: 459 |
| 15 | 15 | NMR1: 1.03-1.57 (5H, m), 1.89-2.00 (4H, m), 2.17-2.45 (1H, m), 2.27 (3H, t, J = 0.8 Hz), 3.62-3.85 (9H, m), 7.40-7.51 (2H, m), 7.64-8.06 (3H, m), 8.41-8.58 (1H, m) |
| | | ESI+: 529 |
| 16 | 16 | NMR1: 1.24-1.43 (4H, m), 1.80-2.09 (4H, m), 2.79 (3H, s), 2.81 (3H, s), 3.44-3.67 (9H, m), 3.78-3.95 (3H, m), 6.12 (1H, s), 7.39-7.65 (4H, m), 7.72 (1H, d, J = 8.1 Hz), 7.86 (1H, d, J = 7.7 Hz), 8.59 (1H, d, J = 7.1 Hz), 9.81 (1H, br-s) |

**[Table 91]**

| Ex | Syn | DATA |
|---|---|---|
| | | ESI+: 569 |
| 17 | 17 | NMR1: 1.11-1.41 (4H, m), 1.59-2.03 (8H, m), 2.16-2.62 (8H, m), 3.40-3.73 (10H, m), 6.28-6.39 (1H, m), 6.90-7.06 (1H, m), 7.39-7.87 (5H, m), 8.62 (1H, s) |
| 18 | 18 | ESI+: 570 [M+Na] |
| 19 | 19 | ESI+: 667 [M+Na] |
| 20 | 20 | ESI+: 651 [M+Na] |
| 21 | 21 | ESI+: 569 |
| 22 | 22 | ESI+: 544 |
| 23 | 23 | ESI+: 515 |
| | | ESI+: 530 |
| 24 | 24 | NMR1: 1.36-1.50 (2H, m), 1.51-1.66 (2H, m), 1.88-1.98 (2H, m), 2.11-2.21 (2H, m), 2.79 (6H, s), 3.66-3.77 (9H, m), 3.86 (2H, s), 5.02-5.12 (1H, m), 6.41 (1H, s), 7.40-7.50 (2H, m), 7.54 (1H, t), 7.72-7.76 (1H, m), 7.85-7.89 (1H, m), 8.51-8.57 (1H, m), 9.75 (1H, br-s) |
| | | ESI+: 599 [M+Na] |
| 25 | 25 | NMR1: 1.10-1.28 (2H, m), 1.46-1.64 (2H, m), 1.71-1.83 (1H, m), 1.90-2.02 (2H, m), 2.09-2.25 (2H, m), 3.43-3.98 (17H, m), 4.24 (2H, d), 6.43 (1H, s), 7.40-7.50 (2H, m), 7.54 (1H, t), 7.74-7.79 (1H, m), 7.85-7.89 (1H, m), 11.63 (1H, br-s) |
| | | ESI+: 518 |
| 26 | 26 | NMR1: 0.83-1.01 (2H, m), 1.09-1.27 (2H, m), 1.38-1.54 (1H, m), 1.67-1.86 (4H, m), 2.17-2.22 (3H, m), 2.27-2.36 (1H, m), 2.60-2.72 (2H, m), 3.04-3.18 (2H, m), 3.58-3.75 (8H, m), 4.34-4.51 (2H, m), 6.25-6.37 (1H, m), 7.09-7.20 (1H, m), 7.36-7.49 (2H, m), 7.49-7.89 (3H, m) |
| 27 | 1 | ESI+: 445 |
| | | ESI+: 444 |
| 28 | 1 | NMR1: 1.12-1.39 (4H, m), 1.79-2.00 (4H, m), 2.55-2.64 (1H, m), 3.5-3.77 (9H, m), 5.63 (1H, s), 6.88 (1H, d, J = 7.9 Hz), 7.35-7.47 (2H, m), 7.63-7.94 (2H, m), 8.35-8.39 (1H, m) |
| 29 | 1 | ESI+: 581 [M+Na] |

**[Table 92]**

| Ex | Syn | DATA |
|---|---|---|
| | | ESI+: 468 |
| 30 | 1 | NMR1: 1.66-2.20 (2H, m), 2.49-3.04 (4H, m), 3.62-3.87 (8H, m), 4.21-4.35 (1H, m), 7.38-7.51 (3H, m), 7.63-8.11 (3H, m), 8.39-8.62 (1H, m), 11.60-11.69 (1H, m) |
| 31 | 1 | ESI+:444 |
| | | ESI+: 500 |
| 32 | 1 | NMR1: 1.71-2.13 (2H, m), 2.50-2.95 (4H, m), 3.64-3.86 (8H, m), 4.28 (1H, br-s), 6.69 (2H, s), 7.38-7.51 (2H, m), 7.63-8.10 (3H, m), 8.39-8.61 (1H, m) |
| | | ESI+: 444 |
| 33 | 1 | NMR1: 1.34-1.86 (8H, m), 2.81 (1H, br-s), 3.60-3.72 (8H, m), 3.80 (1H, br-s), 6.32 (1H, br-s), 6.73-6.99 (1H, m), 7.35-7.98 (5H, m) |
| 34 | 1 | ESI+: 580 [M+Na] |
| 35 | 1 | ESI+: 580 [M+Na] |
| 36 | 1 | ESI+: 567 [M+Na] |
| | | ESI+: 531 |
| 37 | 2 | NMR1: 1.21-1.44 (4H, m), 1.80-2.00 (4H, m), 2.28 (2H, t, J = 5.2 Hz), 3.20-3.21 (3H, m), 3.51 (2H, m, J = 6.4 Hz), 3.46-3.57 (1H, m), 3.65-3.84 (9H, m), 7.40-7.51 (2H, m), 7.65-8.05 (4H, m), 8.42-8.58 (1H, m) |
| | | ESI+: 561 |
| 38 | 2 | NMR1: 1.33-1.49 (4H, m), 1.75-2.02 (4H, m), 3.29-3.30 (3H, m), 3.47-3.51 (2H, m), 3.56-3.83 (12H, m), 3.86 (2H, s), 7.40-7.59 (3H, m), 7.65-8.05 (3H, m), 8.42-8.59 (1H, m) |
| | | ESI+: 533 |
| 39 | 2 | NMR1: 1.26-1.52 (4H, m), 1.74-2.03 (4H, m), 3.41-3.88 (12H, m), 4.63-4.65 (1H, m), 5.42-5.46 (1H, m), 7.40-8.05 (6H, m), 8.42-8.59 (1H, m) |
| | | ESI+: 530 |
| 40 | 2 | NMR1: 1.31-1.53 (4H, m), 1.73-2.05 (4H, m), 2.19-2.20 (6H, m), 2.83 (2H, s), 3.54-3.87 (10H, m), 7.40-7.65 (3H, m), 7.78-8.06 (3H, m), 8.42-8.59 (1H, m) |

**[Table 93]**

| Ex | Syn | DATA |
|---|---|---|
| | | ESI+: 544 |
| 41 | 2 | NMR1: 1.30-1.47 (4H, m), 1.82-2.05 (4H, m), 2.84-2.85 (3H, m), 2.95-2.96 (3H, m), 3.55-3.85 (10H, m), 7.40-7.51 (2H, m), 7.65-8.05 (3H, m), 8.41-8.60 (2H, m) |
| | | ESI+: 557 |
| 42 | 2 | NMR1: 1.20-1.44 (4H, m), 1.51-1.62 (4H, m), 1.79-1.87 (2H, m), 1.90-2.02 (2H, m), 2.25-2.35 (1H, m), 3.24-3.37 (2H, m), 3.47-3.57 (1H, m), 3.64-3.89 (11H, m), 7.40-7.51 (2H, m), 7.62-8.05 (4H, m), 8.41-8.5 (1H, m) |
| | | ESI+: 571 |
| 43 | 2 | NMR1: 1.03-1.45 (8H, m), 1.57-2.10 (9H, m), 3.43-3.56 (1H, m), 3.63-3.86 (9H, m), 4.27-4.52 (1H, m), 7.42-8.05 (6H, m), 8.41-8.58 (1H, m) |
| | | ESI+: 585 |
| 44 | 2 | NMR1: 0.99-1.43 (8H, m), 1.59-2.13 (9H, m), 3.19-3.22 (3H, m), 3.43-3.57 (1H, m), 3.63-3.88 (9H, m), 3.40-3.51 (2H, m), 7.58-8.05 (4H, m), 8.41-8.58 (1H, m) |
| | | ESI+: 585 |
| 45 | 2 | NMR1: 1.17-1.84 (15H, m), 1.91-2.00 (2H, m), 2.12-2.22 (1H, m), 3.27-3.31 (2H, m), 3.42-3.58 (1H, m), 3.63-3.87 (9H, m), 4.36 (1H, t, J = 5.3 Hz), 7.40-7.62 (3H, m), 7.64-8.05 (3H, m), 8.41-8.58 (1H, m) |
| | | ESI+: 529 |
| 46 | 2 | NMR1: 1.22-1.48 (4H, m), 1.75-1.85 (2H, m), 1.97-2.07 (2H, m), 2.19 (6H, s), 2.82 (2H, s), 3.55-3.87 (10H, m), 6.10 (1H, s), 7.37-7.54 (5H, m), 7.69-7.74 (1H, m), 7.83-7.87 (1H, m) |
| | | ESI+: 544 |
| 47 | 2 | NMR1: 1.36-1.85 (6H, m), 1.91-2.08 (2H, m), 2.15-2.21 (6H, m), 2.65-3.10 (4H, m), 3.56-3.91 (10H, m), 7.39-7.58 (2H, m), 7.66-8.05 (3H, m), 8.42-8.58 (1H, m) |

**[Table 94]**

| Ex | Syn | DATA |
|---|---|---|
| | | ESI+: 543 |
| 48 | 2 | NMR1: 1.27-1.70 (6H, m), 1.86-2.09 (2H, m), 2.17-2.18 (6H, m), 2.62-3.08 (4H, m), 3.58-4.25 (10H, m), 6.26-6.37 (1H, m), 6.92-7.06 (1H, m), 7.35-7.87 (5H, m) |
| | | ESI+: 529 |
| 49 | 2 | NMR1: 1.24-1.47 (4H, m), 1.67-2.05 (4H, m), 2.18 (6H, s), 2.81 (2H, s), 3.49-3.77 (10H, m), 6.25-6.40 (1H, m), 6.88-7.09 (1H, m), 7.35-7.91 (6H, m) |
| | | ESI+: 577 [M+Na] |
| 50 | 2 | NMR1: 1.15-1.47 (4H, m), 1.83-2.04 (4H, m), 3.15-3.28 (2H, m), 3.49-3.87 (10H, m), 6.24-6.37 (1H, m), 7.39-7.51 (2H, m), 7.65-8.01 (4H, m), 8.12-8.25 (1H, m) 8.39-8.60 (1H, m) |
| 51 | 2 | ESI+: 583 |
| 52 | 2 | ESI+: 626 |
| 53 | 2 | ESI+: 655 |
| 54 | 2 | ESI+: 543 |
| | | ESI+: 616 |
| 55 | 2 | NMR1: 1.26-1.46 (6H, m), 1.35 (9H, s), 1.80-2.02 (4H, m), 2.80 (3H, s), 3.49-3.84 (10H, m), 7.39-7.51 (2H, m), 7.65-8.05 (4H, m), 8.41-8.59 (1H, m) |
| | | ESI+: 602 |
| 56 | 2 | NMR1: 1.22-1.45 (5H, m), 1.39 (9H, s), 1.80-2.01 (4H, m), 3.42-3.84 (11H, m), 6.80-6.88 (1H, m), 7.40-8.05 (6H, m), 8.42-8.59 (1H, m) |
| 57 | 2 | ESI+: 663 [M+Na] |
| 58 | 2 | ESI+: 663 [M+Na] |
| 59 | 2 | ESI+: 573 [M+Na] |
| 60 | 2 | ESI+: 655 |
| 61 | 2 | ESI+: 655 |
| 62 | 2 | ESI+: 599 [M+Na] |
| 63 | 2 | ESI+: 605 [M+Na] |
| 64 | 2 | ESI+: 677 [M+Na] |

**[Table 95]**

| Ex | Syn | DATA |
|---|---|---|
| 65 | 2 | ESI+: 612 [M+Na] |
| 66 | 2 | ESI+: 598 [M+Na] |
| 67 | 2 | ESI+: 599 [M+Na] |
| 68 | 2 | ESI+: 569 |
| 69 | 2 | ESI+: 652 |
| 70 | 2 | ESI+: 641 [M+Na] |
| 71 | 2 | ESI+: 583 |
| 72 | 2 | ESI+: 662 [M+Na] |
| 73 | 2 | ESI+: 677 [M+Na] |
| 74 | 2 | ESI+: 677 [M+Na] |
| 75 | 2 | ESI+: 655 |
| 76 | 2 | ESI+: 677 [M+Na] |
| 77 | 2 | ESI+: 677 [M+Na] |
| 78 | 2 | ESI+: 610 [M+Na] |
| 79 | 2 | ESI+: 757 [M+Na] |
| 80 | 2 | ESI+: 663 [M+Na] |
| 81 | 2 | ESI+: 530 |
| | | ESI+: 545 [M+Na] |
| 82 | 3 | NMR1: 1.28-1.47 (4H, m), 1.90-2.03 (4H, m), 2.91-2.96 (3H, m), 3.07-3.19 (1H, m), 3.64-3.83 (9H, m), 7.00-7.08 (1H, m), 7.39-7.53 (2H, m), 7.63-8.07 (3H, m), 8.40-8.60 (1H, m) |
| | | ESI+: 591 |
| 83 | 3 | NMR1: 1.31-1.47 (3H, m), 1.88-2.04 (3H, m), 3.15-3.36 (2H, m), 3.58-3.85 (9H, m), 4.37-4.47 (2H, m), 7.40-7.51 (2H, m), 7.62-8.05 (4H, m), 8.41-8.58 (1H, m) |
| | | ESI+: 549 |
| 84 | 3 | NMR1: 0.85-0.98 (4H, m), 1.31-1.47 (4H, m), 1.90-2.06 (4H, m), 2.53-2.63 (1H, m), 3.04-3.21 (1H, m), 3.59-3.87 (9H, m), 7.03-7.12 (1H, m), 7.40-7.51 (2H, m), 7.65-8.05 (3H, m), 8.41-8.58 (1H, m) |

**[Table 96]**

| Ex | Syn | DATA |
|---|---|---|
| | | EST+: 627 |
| 85 | 3 | NMR1: 1.10-1.48 (5H, m), 1.81-2.08 (5H, m), 2.20-3.12 (4H, m), 3.62-3.86 (11H, m), 7.40-7.93 (6H, m), 8.40-8.60 (1H, m) |
| | | ESI+: 591 |
| 86 | 3 | NMR1: 1.21-1.66 (10H, m), 1.79-1.99 (6H, m), 2.13-2.23 (1H, m), 2.96-3.15 (3H, m), 3.61-3.85 (9H, m), 6.99-7.03 (1H, m), 7..40-7.51 (2H, m), 7.64-8.05 (3H, m), 8.41-8.58 (1H, m) |
| | | ESI+: 585 |
| 87 | 3 | NMR1: 1.15-1.35 (4H, m), 1.61-1.72 (2H, m), 1.80-1.91 (2H, m), 2.86-3.01 (1H, m), 3.56-3.83 (9H, m), 7.38-7.50 (2H, m), 7.57-8.03 (9H, m), 8.36-8.56 (1H, m) |
| | | ESI+: 599 |
| 88 | 3 | NMR1: 1.23-1.41 (4H, m), 1.86-1.99 (4H, m), 2.95-3.09 (1H, m), 3.61-3.85 (9H, m), 4.33-4.34 (2H, m), 7.08-7.11 (1H, m), 7.34-7.50 (7H, m), 7.64-8.05 (3H, m), 8.41-8.58 (1H, m) |
| | | ESI+: 550 |
| 89 | 3 | NMR1: 0.97 (3H, t, J = 7.4 Hz), 1.22-1.39 (4H, m), 1.59-1.72 (2H, m), 1.79-2.00 (4H, m), 2.91-3.11 (3H, m), 3.54-3.74 (9H, m), 6.26-6.39 (1H, m), 6.91-7.06 (2H, m), 7.36-7.88 (5H, m) |
| | | ESI+: 584 |
| 90 | 3 | NMR1: 1.10-1.31 (4H, m), 1.53-1.93 (4H, m), 2.81-2.97 (1H, m), 3.51-3.73 (9H, m), 6.25-6.37 (1H, m), 6.83-6.96 (1H, m), 7.33-7.87 (11H, m) |
| | | ESI+: 598 |
| 91 | 3 | NMR1: 1.13-1.36 (4H, m), 1.77-1.99 (4H, m), 2.87-3.06 (1H, m), 3.51-3.76 (9H, m), 4.31 (2H, s), 6.21-6.38 (1H, m), 6.89-7.08 (2H, m), 7.30-7.52 (8H, m), 7.73-7.88 (2H, m) |
| 92 | 3 | ESI+: 606 [M+Na] |

**[Table 97]**

| Ex | Syn | DATA |
|---|---|---|
| | | ESI+: 573 [M+Na] |
| 93 | 3 | NMR1: 1.23-1.37 (4H, m), 1.84-2.01 (4H, m), 2.59-2.68 (6H, m), 2.88-3.04 (1H, m), 3.57-3.74 (9H, m), 6.25-6.38 (1H m), 6.85-7.16 (4H, m), 7.36-7.58 (2H, m), 7.74-7.88 (2H, m) |
| 94 | 3 | ESI+: 598 |
| 95 | 3 | ESI+: 606 [M+Na] |
| 96 | 3 | ESI+: 609 |
| 97 | 3 | ESI+: 606 |
| 98 | 3 | ESI+: 584 [M+Na] |
| | | ESI+: 472 |
| 99 | 4 | NMR1: 1.13-1.37 (4H, m), 1.75-2.11 (4H, m), 2.18 (6H, s), 3.60-3.84 (9H, m), 6.09 (1H, s), 7.36-7.66 (4H, m), 7.69-7.74 (1H, m), 7.83-7.34 (1H, m) |
| | | ESI+: 473 |
| 100 | 4 | NMR1: 1.00 (3H, t, J = 7.0 Hz), 0.92-1.39 (4H, m), 1.78-2.01 (4H, m), 2.32-2.53 (2H, m), 2.56 (2H, q, J = 7.0 Hz), 3.55-3.85 (9H, m), 7.40-7.51 (2H, m), 7.64-8.05 (3H, m), 8.42-8.58 (1H, m) |
| | | ESI+: 535 |
| 101 | 4 | NMR1: 1.09-1.36 (4H, m), 1.88-2.03 (5H, m), 2.29-2.42 (1H, m), 3.62-3.83 (11H, m), 7.19-7.51 (7H, m), 7.64-8.05 (3H, m), 8.42-8.57 (1H, m) |
| | | ESI+: 551 |
| 102 | 4 | NMR1: 1.28-1.60 (4H, m), 1.99-2.26 (4H, m), 2.94-3.07 (1H, m), 3.62-3.85 (9H, m), 4.01-4.08 (2H, m), 6.82 (2H, d), 7.34-7.53 (4H, m), 7.65-8.05 (3H, m), 8.42-8.57 (1H, m), 8.90 (1H, br-s), 9.70 (1H, s) |
| | | ESI+: 551 |
| 103 | 4 | NMR1: 1.29-1.63 (4H, m), 1.99-2.27 (4H, m), 2.97-3.11 (1H, m), 3.66-3.85 (9H, m), 4.04-4.13 (2H, m), 6.81-6.98 (3H, m), 7.22-7.54 (3H, m), 7.65-8.05 (3H, m), 8.42-8.58 (1H, m), 8.93-9.11 (1H, m), 9.69 (1H, s) |
| | | ESI+: 551 |
| 104 | 4 | NMR1: 1.15-1.32 (4H, m), 1.89-2.04 (4H, m), 2.31-2.55 (3H, m), 3.62-3.83 (9H, m), 3.89 (2H, s), 6.66-6.72 (2H, m), 7.04-7.08 (2H, m), 7.40-7.50 (2H, m), 7.64-8.05 (3H, m), 8.41-8.57 (1H, m) |

**[Table 98]**

| Ex | Syn | DATA |
|---|---|---|
| | | ESI+: 595 |
| 105 | 4 | NMR1: 1.10-1.36 (4H, m), 1.89-2.01 (4H, m), 2.29-2.42 (1H, m), 3.65-3.83 (14H, m), 3.97 (2H, t, J = 4.9 Hz), 4.82-4.88 (1H, m), 6.77-6.93 (3H, m), 7.18-7.51 (3H, m), 7.64-8.04 (3H, m), 8.41-8.57 (1H, m) |
| | | ESI+: 636 |
| 106 | 4 | NMR1: 1.02-1.56 (7H, m), 1.76-2.01 (4H, m), 2.24-2.37 (1H, m), 3.01-3.10 (2H, m), 3.63-3.85 (9H, m), 5.01 (2H, s), 7.22-7.51 (8H, m), 7.64-8.05 (4H, m), 8.41-8.58 (1H, m) |
| | | ESI+: 555 |
| 107 | 4 | NMR1: 0.73-1.39 (11H, m), 0.91 (3H, t, J = 6.4 Hz), 1.56-1.99 (9H, m), 2.33-2.57 (2H, m), 3.64-3.85 (8H, m), 7.40-7.51 (2H, m), 7.64-8.05 (3H, m), 8.41-8.58 (1H, m), 8.62 (1H, br-s) |
| | | ESI+: 565 |
| 108 | 4 | NMR1: 1.13-1.37 (8H, m), 1.78-2.38 (6H, m), 3.62-4.17 (10H, m), 6.64-6.74 (2H, m), 7.03-7.07 (2H, m), 7.39-7.50 (2H, m), 7.64-8.03 (3H, m), 8.41-8.56 (1H, m) |
| | | ESI+: 618 |
| 109 | 4 | NMR1: 1.30-1.58 (4H, m), 1.98-2.28 (7H, m), 2.87-2.47 (7H, m), 3.63-3.83 (9H, m), 4.20-4.29 (2H, m), 7.38-7.66 (7H, m), 7.77-8.05 (2H, m), 8.41-8.57 (1H, m), 9.09-9.28 (1H, m), 10.94 (1H, br-s) |
| | | ESI+: 625 |
| 110 | 4 | NMR1: 1.10-1.26 (2H, m), 1.48-1.61 (2H, m), 1.83-2.05 (4H, m), 2.37-2.58 (1H, m), 3.54-3.84 (13H, m), 7.19-7.49 (12H, m), 7.64-8.02 (3H, m), 8.40-8.54 (1H, m) |
| | | ESI+: 501 |
| 111 | 4 | NMR1: 0.96 (6H, t, J = 7.1 Hz), 1.22-1.40 (4H, m), 1.68-2.05 (4H, m), 2.43-2.49 (4H, m), 3.65-3.82 (9H, m), 7.40-7.51 (2H, m), 7.78-8.05 (3H, m), 8.42-8.58 (1H, m) |
| 112 | 4 | ESI+: 569 |
| 113 | 4 | ESI+: 569 |
| 114 | 4 | ESI+: 569 |

**[Table 99]**

| Ex | Syn | DATA |
|---|---|---|
| | | ESI+ 538 [M+Na] |
| 115 | 5 | NMR1: 1.37-1.62 (6H, m), 1.96-2.04 (2H, m), 2.56-2.65 (6H, m), 3.70-3.98 (9H, m), 6.15 (1H, m), 7.42-7.51 (2H, m), 7.65-8.05 (3H, m), 8.42-8.58 (1H, m) |
| | | ESI+: 618 [M+Na] |
| 116 | 5 | NMR1: 1.18-1.29 (2H, m), 1.33-1.44 (2H, m), 1.87-2.00 (4H, m), 3.70-3.78 (9H, m), 4.17-4.12 (2H, m), 5.80-5.94 (1H, m), 6.17-6.28 (1H, m), 7.11-7.16 (2H, m), 7.25-7.30 (2H, m), 7.40-7.51 (2H, m), 7.65-8.05 (3H, m), 8.41-8.58 (1H, m) |
| | | ESI+: 604 [M+Na] |
| 117 | 5 | NMR1: 1.22-1.48 (4H, m), 1.91-2.03 (4H, m), 3.41-3.52 (1H, m), 3.67-3.85 (9H, m), 6.07 (1H, dd, J = 8.4, 4.2 Hz), 7.02-7.13 (2H, m), 7.36-7.52 (4H, m), 7.66-7.06 (3H, m), 8.23-8.78 (2H, m) |
| | | ESI+: 617 [M+Na] |
| 118 | 5 | NMR1: 1.17-1.38 (4H, m), 1.78-2.01 (4H, m), 3.59-3.72 (9H, m), 4.17 (2H, br-s), 5.82 (1H, br-s), 6.13-6.37 (2H, m), 6.89-7.05 (1H, m), 7.09-7.12 (2H, m), 7.23-7.30 (2H, m), 7.37-7.52 (2H, m), 7.75-7.88 (2H, m) |
| | | ESI+: 632[M+Na] |
| 119 | 5 | NMR1: 1.36-1.52 (2H, m), 1.52-1.66 (4H, m), 1.96-2.05 (2H, m), 2.71 (3H, S), 3.66-3.84 (8H, m), 3.92-4.07 (2H, m), 4.22 (2H, d, J = 6 Hz), 6.84-6.90 (1H, m), 7.09-7.15 (2H, m), 7.26-7.31 (2H, m), 7.40-7.54 (2H, m), 7.66-8.05 (3H, m), 8.42-8.58 (1H, m) |
| | | ESI+: 581 |
| 120 | 5 | NMR1: 1.02-1.42 (4H, m), 169-2.03 (4H, m), 3.60-3.74 (8H, m), 6.01-6.07 (1H, m), 6.26-6.38 (1H, m), 6.92-7.08 (2H, m), 7.33-7.88 (6H, m), 8.28-8.43 (1H, m) |
| | | ESI+: 607 |
| 121 | 5 | NMR1: 1.08-1.39 (4H, m), 1.79-2.01 (4H, m), 3.59-3.74 (12H, m), 4.08-4.11 (2H, m), 5.71-5.79 (1H, m), 6.01-6.16 (1H, m), 6.25-6.37 (1H, m), 6.84-7.04 (3H, m), 7.05-7.19 (2H, m), 7.37-7.55 (2H, m), 7.75-7.96 (2H, m) |

**[Table 100]**

| Ex | Syn | DATA |
|---|---|---|
| | | ESI+: 669 [M+Na] |
| 122 | 5 | NMR1; 1.16-1.42 (4H, m), 1.84-2.06 (4H, m), 3.36-3.48 (1H, m), 3.59-3.75 (9H, m), 6.10-6.14 (1H, m), 6.28-6.38 (1H, m), 6.93-7.06 (1H, m), 7.18-7.31 (2H, m), 7.38-7.87 (8H, m), 8.46-8.62 (1H, m) |
| | | ESI+: 610 [M+Na] |
| 123 | 5 | NMR1: 1.19-1.44 (4H, m), 1.86-2.05 (4H, m), 3.36-3.51 (1H, m), 3.58-3.75 (9H, m), 6.26-6.38 (1H, m), 6.91-7.97 (1H, m), 7.38-7.87 (8H, m), 8.86-8.96 (1H, m) |
| | | ESI+: 599 [M+Na] |
| 124 | 5 | NMR1: 1.12-1.38 (4H, m), 1.79-2.02 (4H, m), 3.60-3.74 (9H, m), 4.19 (2H, d, J = 6 Hz), 5.77-5.87 (1H, m), 6.10-6.38 (2H, m), 6.90-7.05 (1H, m), 7.18-7.36 (5H, m), 7.36-7.59 (2H, m), 7.63-7.88 (2H, m) |
| 125 | 5 | ESI+: 610 [M+Na] |
| 126 | 5 | ESI+: 610 [M+Na] |
| 127 | 5 | ESI+: 624 [M+Na] |
| 128 | 5 | ESI+: 585 [M+Na] |
| 129 | 5 | ESI+: 619 [M+Na] |
| 130 | 5 | ESI+: 628 [M+Na] |
| 131 | 5 | ESI+: 624 [M+Na] |
| 132 | 5 | ESI+: 585 [M+Na] |
| 133 | 5 | ESI+: 619 [M+Na] |
| 134 | 5 | ESI+: 619 [M+Na] |
| 135 | 5 | ESI+: 619 [M+Na] |
| | | ESI+: 580 [M+Na] |
| 136 | 6 | NMR1: 1.63-2.50 (2H, m), 3.35-3.79 (2H, m), 1.82-2.00 (4H, m), 3.51-3.82 (16H, m), 5.52-5.74 (1H, m), 7.41-7.50 (2H, m), 7.65-8.05 (3H, m), 8.41-8.59 (1H, m) |
| | | ESI+: 573 |
| 137 | 6 | NMR1: 1.30-1.42 (4H, m), 1.81-1.87 (2H, m), 1.91-1.99 (2H, m), 2.16 (6H, s), 2.28-2.23 (2H, m), 2.77 (3H, s), 3.22-3.46 (3H, m), 3.67-3.81 (9H, m), 6.14-6.33 (1H, m), 7.40-7.51 (2H, m), 7.64-8.05 (3H, m), 8.42-8.58 (1H, m) |

**[Table 101]**

| Ex | Syn | DATA |
|---|---|---|
| | | ESI+: 568 [M+Na] |
| 138 | 6 | NMR1 : 1.17-1.47 (4H, m), 1.84-1.91 (4H, m), 3.11-3.17 (1H, m), 3.67-3.83 (11H, m), 5.50-5.94 (2H, m), 7.41-7.50 (2H, m), 7.66-8.04 (3H, m), 8.41-8.59 (1H, m) |
| | | ESI+: 600 |
| 139 | 6 | NMR1: 1.06-1.38 (4H, m), 1.77-2.02 (4H, m), 2.26-3.14 (4H, m), 3.55-3.74 (10H, m), 5.50-5.65 (1H, m), 6.27-6.37 (1H, m), 6.90-7.09 (1H, m), 7.37-7.96 (6H, m) |
| | | ESI+: 599 |
| 140 | 6 | NMR1: 1.00-2.20 (15H, m), 2.89-3.14 (1H, m), 2.29-2.37 (6H, m), 3.08-3.12 (2H, m), 3.57-3.84 (9H, m), 5.48-5.65 (1H, m), 6.25-6.37 (1H, m), 6.88-7.05 (1H, m), 7.35-8.01 (5H, m) |
| 141 | 6 | ESI+: 614 |
| | | ESI+: 645 |
| 142 | 6 | NMR1: 1.09-1.34 (4H, m), 1.79-2.02 (4H, m), 3.56-3.81 (10H, m), 4.27 (2H, d, J = 6 Hz), 5.87-5.95 (1H, m), 6.24-6.41 (2H, m), 6.87-7.04 (1H, m), 7.37-7.60 (4H, m), 7.63-7.72 (2H, m), 7.75-7.91 (2H, m) |
| | | ESI+: 600 [M+Na] |
| 143 | 6 | NMR1: 1.12-1.40 (4H, m), 1.81-2.01 (4H, m), 3.59-3.76 (9H, m), 4.21 (2H, d, J = 6 Hz), 5.91-5.99 (1H, m), 6.23-6.41 (2H, m), 6.87-7.04 (1H, m), 7.19-7.24 (2H, m), 7.37-7.61 (2H, m), 7.64-7.90 (2H, m), 8.31-8.50 (1H, m) |
| 144 | 6 | ESI+: 624 [M+Na] |
| 145 | 6 | ESI+: 577 [M+Na] |
| 146 | 6 | ESI+: 562 [M+Na] |
| 147 | 6 | ESI+: 611 |
| 148 | 7 | ESI+: 544 |
| 149 | 8 | ESI+: 459 |
| | | ESI+: 458 |
| 150 | 8 | NMR1: 0.82-1.03 (4H, m), 1.35-2.60 (2H, m), 1.65.-1.82 (4H, m), 2.99-3.03 (2H, m), 3.56-3.75 (8H, m), 6.23-6.37 (1H, m), 7.09-7.17 (1H m), 7.37-7.48 (2H, m), 7.75-7.79 (1H, m), 7.82-7.88 (1H, m) |

**[Table 102]**

| Ex | Syn | DATA |
|---|---|---|
| | | ESI+: 516 |
| 151 | 8 | NMR1: 1.28-1.45 (4H, m), 1.77-2.03 (4H, m), 2.23 (3H, s), 2.99 (2H, s), 3.52-3.84 (10H, m), 7.40-8.05 (6H, m), 8.41-8.60 (1H, m) |
| 152 | 8 | ESI+: 529 |
| 153 | 8 | ESI+: 541 |
| 154 | 8 | ESI+: 541 |
| 155 | 8 | ESI+: 458 |
| 156 | 8 | ESI+: 555 |
| 157 | 8 | ESI+: 555 |
| 158 | 8 | ESI+: 555 |
| 159 | 8 | ESI+: 555 |
| 160 | 8 | ESI+: 555 |
| 161 | 8 | ESI+: 555 |
| 162 | 8 | ESI+: 555 |
| 163 | 8 | ESI+: 541 |
| 164 | 8 | ESI+: 570 |
| 165 | 8 | ESI+: 445 |
| | | NMR1: 1.13-1.28 (2H, m), 1.41-1.73 (3H, m), 1.78-1.87 (2H, m), 2.05-2.15 (2H, m), 2.57-2.69 (1H, m), 3.66-3.76 (8H, m), 4.97-5.07 (1H, m), 6.38 (1H, s), 7.39-7.49 (2H, m), 7.52 (1H, t), 7.72-7.77 (1H, m), 7.84-7.88 (1H, m) |
| 166 | 8 | ESI+: 459 |
| | | NMR1: 0.98-1.15 (4H, m), 1.62-1.86 (5H, m), 3.64-3.78 (8H, m), 4.20 (2H, d), 6.43 (1H, s), 7.39-7.50 (2H, m), 7.54 (1H, t), 7.74-7.79 (1H, m), 7.84-7.89 (1H, m) |
| 167 | 8 | ESI+: 476 |
| 168 | 8 | ESI+: 476 |
| 169 | 8 | ESI+: 445 |
| 170 | 9 | ESI+: 564, 566 |
| 171 | 9 | E81+: 586 [M+Na] |

**[Table 103]**

| Ex | Syn | DATA |
|---|---|---|
| | | ESI+: 514 |
| 172 | 10 | NMR1: 1.19-1.36 (4H, m), 1.79-2.22 (4H, m), 3.41-3.50 (2H, m), 3.43-3.80 (16H, m), 6.24-6.37 (1H, m), 6.89-7.03 (1H, m), 7.37-7.55 (2H, m), 7.64- 6.96 (3H, m) |
| | | ESI+: 514 |
| 173 | 10 | NMR1: 1.39-1.89 (8H, m), 2.09-2.18 (1H, m), 2.38-2.47 (4H, m), 3.51-3.77 (12H, m), 3.91 (1R, br-s), 6.32 (1H, br-s), 6.98 (1H, br-s), 7.34-8.04 (5H, m) |
| | | ESI+: 528 |
| 174 | 10 | NMR1: 0.75-1.58 (8H, m), 1.75-1.88 (4H, m), 2.06-1.20 (1H, m), 2.99-3.20 (1H, m), 3.46-3.74 (9H, m), 6.18-6.40 (1H, m), 7.09-7.18 (1H, m), 7.38-7.50 (2H, m), 7.50-7.89 (3H, m) |
| 175 | 10 | ESI+: 512 |
| 176 | 10 | ESI+: 498 |
| 177 | 10 | ESI+: 526 |
| 178 | 10 | ESI+: 512 |
| 179 | 10 | ESI+: 554 |
| 180 | 10 | ESI+: 526 |
| | | ESI+: 549 [M+Na] |
| 181 | 11 | NMR1: 1.34-1.50 (2H, m), 1.51-1.75 (8H, m), 1.96-2.07 (2H, m), 2.18-2.26 (2H, m), 3.15-3.22 (2H, m), 3.64-3.86 (9H, m), 4.24-4.36 (1H, m), 7.38-7.55 (2H, m), 7.64-8.06 (3H, m), 8.40-8.60 (1H, m) |
| 182 | 11 | ESI+: 535 [M+Na] |
| | | ESI+: 512 |
| 183 | 11 | NMR1: 1.32-1.43 (2H, m), 1.52-1.69 (2H, m), 1.75-2.03 (6H, m), 2.15-2.25 (2H, m), 3.59-3.88 (9H, m), 4.03-4.13 (1H, m), 6.35 (1H, br-s), 7.07-7.15 (1H, m), 7.35-7.98 (5H, m) |
| 184 | 11 | ESI+: 548 [M+Na] |
| 185 | 11 | ESI+: 534 [M+Na] |

**[Table 104]**

| Ex | Syn | DATA |
|---|---|---|
| | | ESI+: 570 |
| 186 | 12 | NMR1: 1.32-1.57 (10H, m), 1.74-2.01 (4H, m), 2.29-2.40 (4H, m), 2.82-2.83 (2H, m), 3.53-3.84 (10H, m), 7.39-7.52 (3H, m), 7.64-8.05 (3H, m), 8.41-8.58 (1H, m) |
| 187 | 12 | ESI+: 555 |
| 188 | 12 | ESI+: 619 |
| 189 | 12 | ESI+: 555 |
| 190 | 12 | ESI+: 583 |
| 191 | 12 | ESI+: 670 |
| 192 | 12 | ESI+: 541 |
| | | ESI+: 509 [M+Na] |
| 193 | 14 | NMR1: 1.20-1.45 (4H, m), 1.76-2.02 (7H, m), 3.45-3.56 (1H, m), 3.65-3.85 (9H, m), 7.49-7.54 (2H, m), 7.63-8.07 (4H, m), 8.40-8.61 (1H, m) |
| 194 | 14 | ESI+: 508 [M+Na] |
| | | ESI+: 508 [M+Na] |
| 195 | 14 | NMR1: 1.19-1.38 (4H m), 1.78 (3H, s), 1.79-2.08 (4H, m), 3.53 (1H, br-s), 3.66 (8H, br-s), 3.80 (1H, br-s), 6.10 (1H, s), 7.37-7.66 (4H, m), 7.68-7.75 (2H, m), 7.84 (1H, d, J = 7.6 Hz) |
| | | ESI+: 500 |
| 196 | 14 | NMR1: 1.33-1.70 (5H, m), 1.89-2.06 (5H, m), 2.47-2.50 (3H, m), 2.64-2.84 (2H, m), 3.58-3.74 (9H, m), 6.25 (1H, s), 6.63 (1H, d, J = 7.5 Hz), 7.36-7.66 (3H, m), 7.72 (1H, d, J = 7.8 Hz), 7.82 (1H, d, J = 7.6 Hz) |
| | | ESI+: 458 |
| 197 | 15 | NMR1: 0.94-1.33 (3H, m), 1.04 (3H, d, J = 6 Hz), 1.71-2.34 (7H, m), 3.59-3.71 (9H, m), 6.24-6.38 (1H., m), 6.85-7.01 (1H, m), 7.76-7.86 (2H, m) |
| | | ESI+: 472 |
| 198 | 15 | NMR1: 1.14-1.32 (4H, m), 1.71-2.26 (11H, m), 3.56-3.73 (9H, m), 6.22-6.37 (1H, m), 6.84-7.04 (1H, m), 7.33-7.97 (4H, m) |

**[Table 105]**

| Ex | Syn | DATA |
|---|---|---|
| | | ESI+: 486 |
| 199 | 15 | NMR1: 0.81-1.01 (2H, m), 1.04-1.27 (2H, m), 1.39-1.53 (1H, m), 1.73-1.88 (4H, m), 2.01-2.10 (1H, m), 2.10-2.38 (4H, m), 3.03-3.18 (2H, m), 3.58-3.73 (8H, m), 6.24-6.38 (1H, m), 7.10-7.18 (1H, m), 7.37-7.47 (2H, m), 7.50-7.88 (2H, m) |
| | | ESI+: 472 |
| 200 | 15 | NMR1: 0.82-1.01 (4H, m), 1.38-1.61 (2H, m), 1.68-1.92 (4H, m), 2.12-2.26 (3H, m), 3.03-3.18 (2H, m), 3.58-3.73 (8H, m), 6.25-6.37 (1H, m), 7.10-7.17 (1H, m), 7.37-7.48 (2H, m), 7.49-7.89 (3H, m) |
| | | ESI+: 528 |
| 201 | 16 | NMR1: 0.89-1.13 (2H, m), 1.37-1.60 (3H, m), 1.80-2.00 (6H, m), 2.10-2.24 (2H, m), 2.96-3.14 (2H, m), 3.30-3.41 (2H, m), 3.45-3.78 (9H, m), 3.84-3.98 (4H, m), 6.36-6.47 (1H, m), 7.30-7.71 (4H, m), 7.71-8.00 (3H, m), 11.04-11.32 (1H, m) |
| 202 | 16 | ESI+: 526 |
| 203 | 16 | ESI+: 526 |
| 204 | 16 | ESI+: 569 |
| 205 | 21 | ESI+: 569 |
| | | ESI+: 518 |
| 206 | 16 | NMR1: 0.93-1.11 (2H, m), 1.35-1.62 (3H, m), 1.83-2.13 (5H, m), 2.66-2.78 (3H, m), 3.06-3.27 (2H, m), 3.30-3.74 (10H, m), 4.71-5.02 (2H, m), 6.28-6.43 (1H, m), 7.18-7.33 (1H, m), 7.38-7.50 (2H, m), 7.50-7.88 (3H, m), 10.21-10.46 (1H, m) |
| 207 | 16 | ESI+: 530 |
| 208 | 17 | ESI+: 543 |
| | | ESI+: 585 [M+Na] |
| 209 | 18 | NMR1: 1.11-1.28 (2H, m), 1.42-1.60 (2H, m), 1.67-1.94 (5H, m), 2.14-2.26 (2H, m), 3.14 (2H, t), 3.22 (2H, t), 3.61-3.81 (8H, m), 4.21 (2H, d), 6.44 (1H, s), 7.38-7.50 (2H, m), 7.54 (1H, t), 7.77 (1H, d), 7.87 (1H, d) |
| 210 | 20 | ESI+: 651 [M+Na] |
| 211 | 21 | ESI+: 569 |
| 212 | 22 | ESI+: 567 [M+Na] |

**[Table 106]**

| Ex | Syn | DATA |
|---|---|---|
| 213 | 22 | ESI+: 581 [M+Na] |
| 214 | 22 | ESI+: 598 [M+Na] |
| 215 | 22 | ESI+: 598 [M+Na] |
| | | ESI+: 529 |
| 216 | 23 | NMR1: 1.10-1.26 (2H, m), 1.42-1.56 (2H, m), 1.71-1.84 (1H, m), 1.93-2.03 (2H, m), 2.13-2.22 (2H, m), 3.03-3.83 (15H, m), 3.94-4.01 (2H, m), 4.24 (2H, d), 6.43 (1H, s), 7.39-7.50 (2H, m), 7.54 (1H, t), 7.74-7.78 (1H, m), 7.85-7.89 (1H, m), 10.32 (1H, br-s) |
| 217 | 23 | ESI+: 546 |
| 218 | 23 | ESI+: 546 |
| | | ESI+: 530 |
| 219 | 26 | NMR1: 0.82-1.01 (2H, m), 1.08-1.23 (2H, m), 1.37-1.52 (1H, m), 1.66-1.86 (4H, m), 1.96 (3H, s), 2.16 (3H, s), 2.23-2.34 (1H, m), 3.01-3.16 (2H, m), 3.19-3.24 (4H, m), 3.58-3.73 (8H, m), 6.25-6.37 (1H, m), 7.08-7.18 (1H, m), 7.37-7.48 (2H, m), 7.50-7.88 (3H, m) |

**[Table 107]**

| Ex | Syn | DATA |
|---|---|---|
| 220 | 2 | ESI+: 543 |
| 221 | 4 | ESI+: 486 |
| 222 | 8 | ESI+: 458 |
| 223 | 23 | ESI+: 528 |
| 224 | 2 | ESI+: 724 [M+Na] |
| 225 | 225 | ESI+: 601 |
| 226 | 10 | ESI+: 516 |
| 227 | 10 | ESI+: 574 |
| 228 | 25 | ESI+: 562 |
| | | ESI+: 598 [M+Na] |
| 229 | 25 | NMR1: 0.99-1.14 (2H, m), 1.44-1.61 (3H, m), 1.87-1.98 (2H, m), 2.12-2.22 (2H, m), 2.99-4.56 (19H, m), 6.15 (1H, s), 7.37-7.68 (4H, m), 7.70-7.75 (1H, m), 7.83-7.88 (1H, m) |
| 230 | 258 | ESI+: 584 [M+Na] |
| | | ESI+: 598 [M+Na] |
| 231 | 258 | NMR1: 0.81-1.04 (2H, m), 1.12-1.31 (2H, m), 0.81-1.31 (1H, m), 1.39-1.52 (1H, m), 1.66-1.88 (4H, m), 2.85-3.04 (8H, m), 3.04-3.18 (2H, m), 3.57-3.73 (8H, m), 6.25-6.37 (1H, m), 7.11-7.19 (1H, t, J = 5.6 Hz), 7.35-7.88 (5H, m) |
| 232 | 258 | ESI+: 598 [M+Na] |
| 233 | 2 | ESI+: 691 [M+Na] |
| 234 | 2 | ESI+: 662 [M+Na] |
| 235 | 2 | ESI+: 691 [M+Na] |
| 236 | 2 | ESI+: 662 [M+Na] |
| 237 | 10 | ESI+: 626 |
| 238 | 10 | ESI+: 573 |
| 239 | 239 | ESI+: 605 |
| 240 | 18 | ESI+: 584 [M+Na] |
| 241 | 18 | ESI+: 584 [M+Na] |
| 242 | 3 | ESI+: 634 [M+Na] |
| 243 | 4 | ESI+: 542 |

**[Table 108]**

| Ex | Syn | DATA |
|---|---|---|
| 244 | 4 | ESI+: 558 |
| 245 | 245 | ESI+: 574 |
| | | ESI+: 556 |
| 246 | 275,16 | NMR1: 0.79-1.29 (8H, m), 1.42-1.65 (3H, m), 1.80-1.94 (2H, m), 2.01-2.19 (2H, m), 2.7 5-2.87 (1H, m), 3.06-3.22 (2H, m), 3.39-3.85 (14H, m), 6.27-6.41 (1H, m), 7.17-7.28 (1H, m), 7.37-7.89 (5H, m), 8.94-9.14 (1H, m) |
| 247 | 10 | ESI+: 592 |
| 248 | 10 | ESI+: 726 |
| 249 | 14 | ESI+: 542 |
| 250 | 26,16 | ESI+: 578 |
| 251 | 26 | ESI+: 698 |
| 252 | 26,16 | ESI+: 578 |
| 253 | 26 | ESI+: 698 |
| 254 | 26 | ESI+: 578 |
| 255 | 26 | ESI+: 698 |
| 256 | 4 | ESI+: 564 |
| 257 | 18 | ESI+: 584 [M+Na] |
| 258 | 258 | ESI+: 612 [M+Na] |
| 259 | 26,16 | ESI+: 548 |
| 260 | 26 | ESI+: 638 |
| 261 | 26.16 | ESI+: 592 |
| 262 | 26,16 | ESI+: 587 |
| 263 | 4,16 | ESI+: 556 |
| 264 | 26,16 | ESI+: 563 |
| 265 | 26,16 | ESI+: 576 |
| 266 | 26,16 | ESI+: 570 |
| 267 | 26,16 | ESI+: 596 |
| 268 | 26,16 | ESI+: 594 |
| 269 | 26,16 | ESI+: 533 [M+Na] |
| 270 | 26,16 | ESI+: 555 |

**[Table 109]**

| Ex | Syn | DATA |
|---|---|---|
| | | ESI+: 570 |
| 271 | 26,16 | NMR1: 0.28-0.42 (2H, m), 0.58-0.68 (2H, m), 0.92-1.26 (4H, m), 1.36-1.64 (3H, m), 1.80-2.06 (5H, m), 2.97-3.22 (4H, m), 3.04-3.18 (2H, m), 3.53-3.77 (10H, m), 2.27-6.41 (1H, m), 7.15-7.26 (1H, m), 7.35-7.88 (5H, m), 8.93-8.09 (1H, m) |
| 272 | 279,16 | ESI+: 536 |
| 273 | 26,16 | ESI+: 593 |
| | | ESI+: 516 |
| 274 | 386,16 | NMR1: 0.78-1.01 (3H, m), 1.07-1.31 (3H, m), 1.39-1.55 (1H, m), 1.66-1.88 (4H, m), 2.12-1.19 (3H, br s), 2.22-2.34 (1H, m), 3.03-3.18 (2H, m), 3.35-3.44 (1H, m), 3.59-3.78 (8H, m), 4.20 (1H, t, J = 4.8 Hz), 6.25-6.37 (1H, m), 7.09-7.17 (1H, m), 7.38-7.91 (5H, m) |
| 275 | 275 | ESI+: 556 |
| 276 | 14 | ESI+: 509 [M+Na] |
| 277 | 3 | ESI+: 545 [M+Na] |
| 278 | 1,16 | ESI+: 541 |
| 279 | 279 | ESI+: 536 |
| | | ESI+: 544 |
| 280 | 26,16 | NMR1: 0.82-1.12 (2H, m), 1.12-1.29 (1H, m), 1.33-1.61 (3H, m), 1.82-2.04 (6H, m), 2.66 (3H, s), 2.93-3.05 (1H, m), 3.05-3.24 (3H, m), 3.24 (3H, s), 3.35-3.42 (2H, m), 3.59-3.75 (8H, m), 6.28-6.42 (1H, m), 7.17-7.25 (1H, m), 7.38-7.90 (5H, m), 9.46-9.65 (1H, m) |
| 281 | 26 | ESI+: 504 |
| 282 | 26,16 | ESI+: 550 |
| 283 | 26,16 | ESI+: 532 |
| 284 | 279,16 | ESI+: 554 |
| 285 | 26,16 | ESI+: 530 |
| 286 | 2 | ESI+: 589 |
| 287 | 275,16 | ESI+: 544 |
| 288 | 1 | ESI+: 586 |
| 289 | 289 | ESI+: 486 |

**[Table 110]**

| Ex | Syn | DATA |
|---|---|---|
| 290 | 333,16 | ESI+: 578 [M+Na] |
| 291 | 1 | ESI+: 559 |
| 292 | 23 | ESI+: 556 |
| 293 | 8 | ESI+: 459 |
| 294 | 23 | ESI+: 529 |
| 295 | 295 | ESI+: 627 |
| 296 | 295 | ESI+: 627 |
| 297 | 8 | ESI+: 527 |
| 298 | 8 | ESI+: 527 |
| 299 | 4 | ESI+: 486 |
| 300 | 26 | ESI+: 500 |
| 301 | 26 | ESI+: 500 |
| 302 | 4,16 | ESI+: 541 |
| 303 | 4,16 | ESI+: 541 |
| 304 | 26,16 | ESI+: 532 |
| 305 | 26,16 | ESI+: 546 |
| 306 | 26,16 | ESI+: 558 |
| 307 | 295,16 | ESI+: 528 |
| 308 | 295,16 | ESI+: 544 |
| 309 | 295,16 | ESI+: 544 |
| 310 | 295,16 | ESI+: 562 |
| 311 | 295,16 | ESI+: 562 |
| 312 | 295,16 | ESI+: 544 |
| 313 | 295,16 | ESI+: 544 |
| 314 | 295,16 | ESI+: 530 |
| 315 | 295,16 | ESI+: 530 |
| 316 | 295,16 | ESI+: 530 |
| 317 | 295,16 | ESI+: 530 |
| 318 | 295,16 | ESI+: 548 |
| 319 | 295,16 | ESI+: 548 |
| 320 | 335 | ESI+: 550 [M+Na] |

**[Table 111]**

| Ex | Syn | DATA |
|---|---|---|
| 321 | 295,16 | ESI+: 562 |
| 322 | 295,16 | ESI+: 562 |
| 323 | 4,16 | ESI+: 522 |
| 324 | 4 | ESI+: 536 |
| 325 | 325 | ESI+: 518 |
| 326 | 326 | ESI+: 562 |
| 327 | 4 | ESI+: 518 |
| 328 | 328 | ESI+: 564 [M+Na] |
| 329 | 325 | ESI+: 504 |
| | | ESI+: 532 |
| 330 | 15 | NMR1: 0.77-1.04 (3H, m), 1.07-1.31 (6H, m), 1.37-1.54 (1H, m), 1.65-1.87 (4H, m), 2.21 (3H, s), 2.25-2.35 (1H, m), 3.01-3.17 (2H, m), 3.56-3.75 (8H, m), 4.57-4.80 (1H, m), 6.25-6.38 (1H, m), 7.10-7.19 (1H, m), 7.37-7.89 (5H, m) |
| 331 | 15 | ESI+: 532 |
| 332 | 15 | ESI+: 550 |
| 333 | 333 | ESI+: 578 |
| 334 | 326,16 | ESI+: 546 |
| 335 | 335 | ESI+: 542 |
| 336 | 326 | ESI+: 562 |
| 337 | 326 | ESI+: 579 [M+Na] |
| | | ESI+: 560 |
| 338 | 4 | NMR1: 0.73-1.05 (8H, m), 1.08-1.35 (4H, m), 1.38-1.51 (1H, m), 1.51-1.62 (1H, m), 2.36-2.50 (3H, m), 3.00-3.18 (3H, m), 3.56-3.76 (8H, m), 4.03-4.34 (2H, m), 6.24-6.40 (1H, m), 7.07-7.19 (1H, m), 7.37-7.90 (5H, m) |
| 339 | 4,16 | ESI+: 560 |
| 340 | 26,16 | ESI+: 514 |
| 341 | 326 | NMR1: 0.78-0.86 (3H, m), 0.86-1.01 (2H, m), 1.09-1.23 (4H, m), 1.26 (2H, d, J = 6.4 Hz), 1.29-1.39 (2H, m), 1.40-1.53 (1H, m), 1.65-1.86 (4H, m), 2.35-2.45 (3H, m), 3.04-3.19 (1H m), 3.6-3.74 (8H, m), 4.49-4.70 (1H, m), 6.26-6.37 (1H, m), 7.05-7.14 (1H, m), 7.37-7.48 (5H, m) |

**[Table 112]**

| Ex | Syn | DATA |
|---|---|---|
| 342 | 343,16 | ESI+: 548 |
| 343 | 343 | FAB+: 534 |
| | | ESI+: 546 |
| 344 | 4,16 | NMR1: 0.98-0.38 (8H, m), 1.43-1.65 (3H, m), 1.79-1.94 (2H, m), 1.95-2.17 (4H, m), 3.05-3.39 (5H, m), 3.59-3.72 (8H, m), 3.82-3.98 (1H, m), 4.59-4.93 (2H, m), 6.25-6.43 (1H, m), 7.17-7.29 (1H, m), 7.37-7.88 (5H, m), 9.33-9.61 (1H, m) |
| 345 | 345 | ESI+: 560 |
| 346 | 26 | ESI+: 558 |
| 347 | 26 | ESI+: 630 |
| 348 | 343 | ESI+: 516 |
| 349 | 343 | ESI+: 530 |
| 350 | 343 | ESI+: 546 |
| 351 | 343 | ESI+: 634 |
| 352 | 353 | ESI+: 572 |
| 353 | 353 | ESI+: 556 |
| 354 | 343 | ESI+: 532 |
| 355 | 353 | ESI+: 556 |
| 356 | 343 | ESI+: 546 |
| 357 | 2 | ESI+: 516 |
| 358 | 353 | ESI+: 542 |
| 359 | 2 | ESI+: 529 |
| 360 | 2 | ESI+: 557 |
| 361 | 353 | ESI+: 542 |
| 362 | 26,16 | ESI+: 558 |
| | | ESI+: 544 |
| 363 | 4 | NMR1: 0.81-1.01 (5H, m), 1.07-1.28 (2H, m), 1.37-1.56 (1H, m), 1.65-1.86 (4H, m), 2.34-2.44 (1H, m), 3.02-3.17 (2H, m), 3.21 (3H, s), 3.25-3.31 (3H, m), 3.50-3.75 (8H, m), 4.47-4.70 (1H, m), 6.25-6.37 (1H, m), 7.01-7.16 (1H, m), 7.37-7.88 (5H, m) |
| 364 | 1 | ESI+: 572 |

**[Table 113]**

| Ex | Syn | DATA |
|---|---|---|
| 365 | 1 | ESI+: 572 |
| 366 | 1 | ESI+: 586 |
| 367 | 289 | ESI+: 472 |
| 368 | 289 | ESI+: 472 |
| 369 | 289 | ESI+: 486 |
| | | ESI+: 542 |
| 370 | 23 | NMR1: 0.93-1.10 (2H, m), 1.25 (3H, d, J = 6.7 Hz), 1.39-1.59 (3H, m), 1.86-1.94 (2H, m), 2.13-2.21 (2H, m), 2.98-3.27 (5H, m), 3.32-3.39 (2H, m), 3.43-3.76 (4H, m), 3.86-3.97 (5H, m), 4.07-4.15 (1H, m), 4.38-4.48 (1H, m), 6.36 (1H, s), 7.40-7.72 (4H, m), 7.77 (1H, d, J = 8.0 Hz), 7.86 (1H, d, J = 8.0 Hz) |
| | | ESI+: 542 |
| 371 | 23 | NMR1: 0.93-1.07 (2H, m), 1.25 (3H, d, J = 6.8 Hz), 1.39-1.59 (3H, m), 1.86- 1.94 (2H, m), 2.12-2.21 (2H, m), 2.98-3.28 (5H, m), 3.31-3.39 (2H, m), 3.43-3.65 (3H, m), 3.71-3.77 (1H, m), 3.87-3.97 (5H, m), 4.07-4.16 (1H, m), 4.38-4.49 (1H, m), 6.38 (1H, s), 7.40-7.69 (4H, m), 7.77 (1H, d, J = 8.0 Hz), 7.86 (1H, d, J = 8.0 Hz) |
| 372 | 23 | ESI+: 556 |
| 373 | 343 | ESI+: 560 |
| | | ESI+: 576 |
| 374 | 326 | NMR1: 7.94-1.03 (2H, m), 1.08-1.30 (5H, m), 1.38-1.53 (1H, m), 1.64-1.87 (4H, m), 2.54-2.66 (3H, m), 3.02-3.16 (2H, m), 3.21 (3H, s), 3.29 (3H, s), 3.59-3.76 (8H, m), 4.47-4.70 (1H, m), 6.25-6.37 (1H, m), 7.08-7.18 (1H, m), 7.37-7.89 (5H, m) |
| | | ESI+: 544 |
| 375 | 15 | NMR1: 0.80-0.98 (2H, m), 1.03 (6H, s), 1.10-1.31 (2H, m), 1.39-1.62 (1H, m), 1.67-1.87 (4H, m), 2.20-2.33 (6H, m), 3.01-3.19 (2H, m), 3.58-3.75 (8H, m), 3.89 (1H, s), 6.25-6.37 (1H, m), 7.07-7.15 (1H, m), 7.38-7.87 (5H, m) |
| 376 | 22 | ESI+: 586 |
| 377 | 8 | ESI+: 486 |
| 378 | 23 | ESI+: 556 |

**[Table 114]**

| Ex | Syn | DATA |
|---|---|---|
| 379 | 26 | ESI+: 530 |
| 380 | 26 | ESI+: 530 |
| 381 | 417 | ESI+: 544 |
| 382 | 417 | ESI+: 544 |
| 383 | 4 | ESI+: 532 |
| | | ESI+: 546 |
| 384 | 15 | NMR1: 0.77-1.05 (5H, m), 1.05-1.34 (5H, m), 1.38-1.55 (1H, m), 1.65-1.91 (4H, m), 2.12-2.24 (3H, m), 2.28-2.40 (1H, m), 2.64-2.85 (1H, m), 3.01-3.18 (2H, m), 3.58-3.76 (8H, m), 4.37-4.62 (1H, m), 6.24-6.39 (1H, m), 7.09-7.17 (1H, m), 7.36-7.90 (5H, m) |
| | | ESI+: 558 |
| 385 | 4,16 | NMR1: 0.87-0.89 (1H, m), 0.95-1.15 (2H, m), 1.15-1.31 (7H, m), 1.31-1.44 (2H, m), 1.44-1.63 (3H, m), 1.79-2.18 (5H, m), 2.78-2.87 (1H, m), 3.05-3.38 (4H, m), 3.53-3.75 (9H, m), 5.00-5.09 (1H, m), 6.26-6.40 (1H, m), 7.16-7.26 (1H, m), 7.37-7.89 (5H, m), 8.07-8.20 (1H, m) |
| | | ES1+: 562 |
| 386 | 386 | NMR1: 0.82-1.08 (5H, m), 1.08-1.34 (2H, m), 1.38-1.53 (1H, m), 1.58-1.65 (1H, m), 1.68-1.88 (3H, m), 3.02-3.18 (3H, m), 3.24-3.37 (3H, m), 3.60-3.81 (8H, m), 4.01-4.36 (3H, m), 6.25-6.39 (1H, m), 7.07-7.19 (1H, m), 7.38-7.93 (5H, m) |
| 387 | 15 | ESI+: 486 |
| 388 | 15 | ESI+: 500 |
| 389 | 15 | ESI+: 486 |
| 390 | 15 | ESI+: 500 |
| 391 | 343,16 | ESI+: 558 |
| 392 | 343 | ESI+: 544 |
| | | ESI+: 558 |
| 393 | 343, 16 | NMR1: 0.81-1.11 (2H, m), 1.12-1.31 (9H, m), 1.31-1.62 (2H, m), 1.80-2.01 (3H, m), 2.04-2.14 (1H m), 2.75-2.88 (4H, m), 3.04-3.32 (4H, m), 3.32-4.14 (8H, m), 4.41 (1H, s), 6.21-6.37 (1H, m), 7.14-7.26 (1H, m), 7.37-7.88 (5H, m), 8.41-8.54 (1H, m) |

**[Table 115]**

| Ex | Syn | DATA |
|---|---|---|
| 394 | 343 | ESI+: 544 |
| 395 | 343 | ESI+: 530 |
| | | ESI+: 602 |
| 396 | 343 | NMR1: 0.98-0.97 (3H, m), 1.04 (12H, s), 1.05-1.21 (1H, m), 1.21-1.33 (1H, m), 1.37-1.62 (1H, m), 1.67-1.86 (4H, m), 2.29-2.45 (4H, m), 3.01-3.16 (2H, m), 3.57-3.75 (8H, m), 5.15 (2H, s), 6.23-6.38 (1H, m), 7.07-7.16 (1H, m), 7.37-7.90 (5H, m) |
| | | ESI+: 544 |
| 397 | 26,16 | NMR1: 0.79-0.90 (1H, m), 0.91-1.20 (2H, m), 1.13 (3H, t, J = 7.6 Hz), 1.22-1.32 (1H, m), 1.35-1.62 (3H, s), 1.79-1.93 (2H, m), 1.95-2.11 (2H, m), 2.64-2.75 (3H, m), 3.05-3.24 (4H, m), 3.28-3.40 (1H, m), 3.44-3.53 (3H, m), 3.59-3.76 (8H, m), 6.25-6.41 (1H, m), 7.15-7.78 (1H, m), 7.38-7.90 (5H, m), 9.54-9.75 (1H, m) |
| | | ESI+: 558 |
| 398 | 343 | NMR1: 0.81-1.11 (8H, m), 1.11-1.35 (2H, m), 1.40-1.63 (2H, m), 1.67-1.96 (4H, m), 2.14-2.42 (6H, m), 3.03-3.19 (2H, m), 3.19-3.28 (1H, m), 3.58-3.87 (8H, m), 3.87-4.08 (1H, m), 6.23-6.41 (1H, m), 7.06-7.19 (1H, m), 7.34-7.91 (5H, m), 8.31 (1H, s) |
| | | ESI+: 530 |
| 399 | 4 | NMR1: 0.77-1.04 (8H, m), 1.08-1.33 (1H, m), 1.39-1.61 (1H, m), 1.67-1.96 (4H, m), 2.84-2.97 (1H, m), 3.04-3.20 (4H, m), 3.22 (3H, s), 3.59-3.80 (8H, m), 6.24-6.36 (1H, m), 7.12 (1H, t, J = 5.4 Hz), 7.37-7.90 (5H, m) |
| 400 | 26,16 | ESI+: 586 |
| | | ESI+: 588 |
| 401 | 26,16 | NMR1: 0.79-1.12 (2H, m), 1.16-1.28 (7H, m), 1.33-1.61 (4H, m), 1.81-1.92 (2H, m), 1.92-1.97 (3H, s), 2.00-2.12 (2H, m), 3.06-3.19 (2H, m), 3.28 (6H, s), 3.40-3.50 (2H, m), 3.61-3 .75 (11H, m), 3.77-3.89 (1H, m), 4.24-4.64 (1H, m), 6.29-6.43 (1H, m), 7.19-7.30 (1H, m), 7.38-7.94 (5H, m), 8.29-8.46 (1H, m) |

**[Table 116]**

| Ex | Syn | DATA |
|---|---|---|
| | | ESI+: 572 |
| 402 | 4,16 | NMR1: 0.80-1.14 (2H, m), 1.14-1.30 (11H, m), 1.30-1.46 (1H, m), 1.46-1.65 (2H, m), 1.79-1.93 (2H, m), 1.93-2.13 (3H, m), 2.77-2.88 (1H, m), 3.03-3.33 (7H, m), 3.41-3.98 (5H, m), 4.05-4.34 (2H, m), 4.34-4.78 (1H, m), 6.23-6.36 (1H, m), 7.16-7.29 (1H, m), 7.38-7.92 (5H, m), 8.22-8.41 (1H, m) |
| | | ESI+: 572 |
| 403 | 4,16 | NMR1: 0.96-1.12 (2H, m), 1.12-1.31 (12H, m), 1.31-1.47 (1H, m), 1.47-1.63 (2H, m), 1.79-1.93 (2H, m), 1.94-2.22 (2H, m), 2.75-2.89 (1H, m), 3.05-3.34 (4H, m), 3.39-3.98 (5H, m), 4.04-4.60 (5H, m), 6.23-6.37 (1H, m), 7.19-7.32 (1H, m), 7.38-7.93 (5H, m), 8.29-8.47 (1H, m) |
| 404 | 26,16 | ESI+: 584 |
| 405 | 386 | ESI+: 562 |
| 406 | 1 | ESI+: 588 |
| 407 | 1 | ESI+: 458 |
| 408 | 1 | ESI+: 590 |
| 409 | 1 | ESI+: 586 |
| 410 | 289 | ESI+: 488 |
| 411 | 2 | ESI+: 543 |
| 412 | 289 | ESI+: 490 |
| 413 | 289 | ESI+: 486 |
| 414 | 1 | ESI+: 459 |
| 415 | 4,16 | ESI+: 612 |
| 416 | 23 | ESI+: 558 |
| | | ESI+: 486 |
| 417 | 417 | NMR1: 1.15-1.36 (3H, m), 1.24 (3H, d, J = 6.7 Hz), 1.46-1.60 (2H, m), 1.95-2.16 (4H, m), 2.68 (3H, s), 2.69 (3H, s), 3.04-3.25 (2H, m), 3.40-4.16 (5H, m), 4.35-4.45 (1H, m), 6.31 (1H, s), 6.98-7.98 (6H, m) |
| 418 | 23 | ESI+: 556 |
| 419 | 23 | ESI+: 560 |
| 420 | 417 | ESI+: 487 |

**[Table 117]**

| Ex | Syn | DATA |
|---|---|---|
| 421 | 2 | ESI+: 544 |
| | | ESI+: 496 |
| 422 | 26 | NMR1: 0.78-1.05 (4H, m), 1.05-1.30 (1H, m), 1.40-1.62 (1H, m), 1.68-1.95 (4H, m), 2.97-3.48 (4H, m), 3.55-3.73 (8H, m), 6.24-6.38 (1H, m), 7.12 (1H, t, J = 4 Hz), 7.37-7.88 (5H, m) |
| | | ESI+: 534 |
| 423 | 26 | NMR1: 0.82-1.01 (2H, m), 1.07-1.30 (2H, m), 1.39-1.57 (1H, m), 1.72-1.96 (4H, m), 2.35-2.46 (1H, m), 3.03-3.50 (8H, m), 3.55-3.74 (8H, m), 6.24-6.37 (1H, m), 7.1-7.17 (1H, m), 7.37-7.89 (5H, m) |
| | | ESI+: 514 |
| 424 | 4 | NMR1: 0.83-1.01 (8H, m), 1.08-1.23 (2H, m), 1.38-1.53 (1H, m), 1.65-1.86 (4H, m), 3.03-3.18 (2H, m), 3.59-3.73 (8H, m), 6.23-6.38 (1H, m), 7.09-7.15 (1H, m), 7.36-7.88 (5H, m) |
| 425 | 343 | ESI+: 590 |
| 426 | 4,16 | ESI+: 626 |
| 427 | 4 | ESI+: 572 |
| 428 | 26 | ESI+: 528 |
| 429 | 26 | ESI+: 514 |
| | | ESI+: 568 |
| 430 | 26 | NMR1: 0.79-0.99 (2H, m), 0.99-1.09 (6H, m), 1.09-1.29 (1H, m), 1.39-1.60 (1H, m), 1.65-1.90 (4H, m), 2.32-2.41 (2H, m), 2.96 (1H, s), 3.02-1.89 (2H, m), 3.49 (2H, s), 3.59-3.75 (8H, m), 4.01 (1H, s), 6.25-6.38 (1H, m), 7.08-7.16 (1H, m), 7.38-7.89 (5H, m) |
| 431 | 1 | ESI+: 458 |
| 432 | 2 | ESI+: 543 |
| 433 | 433 | ESI+: 516 |
| | | ESI+: 544 |
| 434 | 15 | NMR1: 0.83-1.02 (6H, m), 1.10-1.28 (2H, m), 1.38-1.53 (1H, m), 1.67-1.83 (4H, m), 2.13 (3H, s), 2.87-2.98 (1H, m), 3.03-3.16 (3H, m), 3.21 (3H, s), 3.59-3.76 (8H, m), 6.25-6.37 (1H, m), 7.08-7.16 (1H, m), 7.37-7.89 (5H, m) |

**[Table 118]**

| Ex | Syn | DATA |
|---|---|---|
| | | ESI+: 558 |
| 435 | 4 | NMR1: 0.83-1.05 (8H, m), 1.11-1.31 (2H, m), 1.40-1.52 (1H, m), 1.55-1.84 (3H, m), 2.92-3.16 (3H, m), 3.19-3.26 (3H, m), 3.28-3.29 (1H, m), 3.59-3.73 (8H, m), 6.25-6.37 (1H, m), 7.08-7.14 (1H, m), 7.37-7.89 (5H, m) |
| 436 | 436 | ESI+: 527 |
| 437 | 26 | ESI+: 582 |
| | | ESI+: 582 |
| 438 | 26,16 | NMR1: 0.96-1.12 (2H, m), 1.25-1.35 (3H, m), 1.45-1.69 (3H, m), 1.81-1.92 (5H, m), 1.94-2.01 (1H, m), 2.01-2.16 (1H, m), 3.06-3.21 (2H, m), 3.28-3.40 (3H, m), 3.44-3.51 (1H, m), 3.57 (2H, s), 3.60-3.75 (8H, m), 3.81-3.94 (1H, m), 3.99-4.12 (2H, m), 6.31-6.42 (1H, m), 7.2-7.35 (1H, m), 7.38-7.91 (5H, m), 9.61-9.99 (1H, m) |
| 439 | 439 | ESI+: 556 |
| 440 | 439 | ESI+: 542 |
| | | ESI+: 598 |
| 441 | 26,16 | NMR1: 0.91-1.12 (2H, m), 1.18-1.29 (6H, m), 1.32-1.63 (3H, m), 1.68-1.93 (5H, m), 1.96 (2H, s), 2.00-2.17 (4H, m), 2.64-2.74 (1H, m), 3.04-3.20 (3H, m), 3.22-3.35 (3H, m), 3.61-3.77 (8H, m), 6.32-6.47 (1H, m), 7.25-7.94 (6H, m), 8.61-8.81 (1H, m) |
| | | ESI+: 570 |
| 442 | 26 | NMR1: 0.80-0.97 (2H, m), 1.03 (6H, s), 1.09-1.30 (2H, m), 1.40-1.60 (1H, m), 1.68-1.87 (4H, m), 2.25-2.30 (2H, m), 3.02-3.15 (1H, m), 3.15-3.22 (1H, ), 3.60-3.73 (8H, m), 3.87 (1H, s), 4.95 (1H, d, J = 9,6 Hz), 5.12 (1H, d, J = 17 Hz), 5.73-5.84 (1H, m), 6.24-6.38 (1H, m), 7.07-7.14 (1H, m), 7.38-7.88 (5H, m) |
| 443 | 1 | ESI+: 572 |
| 444 | 4 | ESI+: 544 |
| 445 | 15 | ESI+: 558 |

**[Table 119]**

| Ex | Syn | DATA |
|---|---|---|
| | | ESI+: 544 |
| 446 | 4 | NMR1: 0.79-1.00 (4H, m), 0.91 (3H, d, J = 10.4 Hz), 1.07-1.17 (1H, m), 1.23 (3H, d, J = 7.6 Hz), 1.40-1.57 (1H, m), 1.68-1.93 (4H, m), 2.35-2.44 (1H, m), 2.86-2.94 (1H, m), 3.02-3.20 (3H, m), 3.22 (3H, s), 3.40-3.52 (2H, m), 3.58-3.75 (2H, m), 3.89-4.12 (2H, m), 4.34-4.47 (1H, m), 6.20-6.31 (1H, m), 7.07-7.17 (1H, m), 7.37-7.89 (5H, m) |
| | | ESI+: 558 |
| 447 | 15 | NMR1: 0.82-1.03 (3H, m), 0.93 (3H, d, J = 7.2 Hz), 1.10-1.29 (2H, m), 1.23 (3H, d, J = 8 Hz), 1.39-1.55 (1H, m), 1.67-1.89 (4H, m), 2.14 (3H, s), 2.33-2.42 (1H, m), 2.87-2.98 (1H, m), 3.02-3.19 (3H, m), 3.21 (3H, s), 3.41.3.53 (2H, m), 3.58-3.75 (2H, m), 3.89-4.14 (2H, m), 4.34-4.49 (1H, m), 6.20-6.31 (1H, m), 7.07-7.17 (1H, m), 7.37-7.90 (5H, m) |
| 448 | 26,16 | ESI+: 596 |
| | | ESI+: 598 |
| 449 | 26 | NMR1: 0.76-0.97 (2H, m), 1.02 (6H, s), 1.07-1.31 (2H, m), 1.36-1.53 (1H, m), 1.58 (3H, s), 1.66 (3H, s), 1.66-1.86 (4H, m), 2.24 (2H, s), 2.37-2.50 (1H, m), 3.01-3.17 (4H, m), 3.58-3.75 (8H, m), 3.85 (1H, s), 5.13-5.20 (1H, m), 6.24-6.38 (1H, m), 7.07-7.16 (1H, m), 7.37-7.90 (5H, m) |
| | | ESI+: 542 |
| 450 | 4,16 | NMR1: 0.77-1.11 (2H, m), 0.81 (6H, t, J = 7.2 Hz), 1.07-1.23 (1H, m), 1.23-1.40 (3H, m), 1.40-1.54 (1H, m), 1.62-1.74 (2H, m), 1.74-1.88 (2H, m), 2.27-2.43 (4H, m), 3.01-3.18 (2H, m), 3.58-3.75 (8H, m), 6.24-6.37 (1H, m), 7.07-7.15 (1H, m), 7.36-7.89 (5H, m) |
| 451 | 1 | ESI+: 588 |
| 452 | 289 | ESI+: 488 |
| 453 | 10 | ESI+: 558 |
| 454 | 295 | ESI+: 556 |
| 455 | 295 | ESI+: 542 |
| | | ESI+: 556 |
| 456 | 295 | NMR1: 0.82-1.03 (1H, m), 1.07-1.30 (1H, m), 1.36-1.72 (9H, m), 1.72-1.94 (2H, m), 2.37-2.46 (1H, m), 2.64-3.03 (5H, m), 3.12-3.27 (4H, m), 3.59-3.77 (8H, m), 6.24-6.38 (1H, m), 7.06-7.19 (1H, m), 7.37-7.89 (5H, m) |

**[Table 120]**

| Ex | Syn | DATA |
|---|---|---|
| 457 | 2 | ESI+: 602 |
| 458 | 2 | ESI+: 616 |
| 459 | 289 | ESI+: 502 |
| 460 | 289 | ESI+: 516 |
| | | ESI+: 530 |
| 461 | 295 | NMR1: 0.80-2.18 (12H, m), 2.29-2.39 (1H, m), 2.54-2.70 (1H, m), 2.75-2.92 (2H, m), 3.18-3.29 (2H, m), 3.59-3.73 (8H, m), 5.05-5.26 (1H, m), 6.14 (1H, s), 7.36-7.66 (4H, m), 7.70-7.75 (1H, m), 7.83-7.88 (1H, m) |
| 462 | 295 | ESI+: 530 |
| 463 | 343 | ESI+: 531 |
| 464 | 1 | ESI+: 590 |
| 465 | 26 | ESI+: 580 |
| 466 | 26,16 | ESI+: 594 |
| 467 | 289 | ESI+: 490 |
| 468 | 23 | ESI+: 560 |
| 469 | 295 | ESI+: 531 |
| 470 | 295 | ESI+: 531 |
| | | ESI+: 572 |
| 471 | 4 | NMR1: 0.78-1.17 (17H, m), 1.17-1.40 (2H, m), 1.40-1.61 (2H, m), 1.68-1.90 (4H, m), 3.01-3.19 (2H, m), 3.58-3.75 (8H, m), 4.00-4.40 (1H, m), 6.23-6.38 (1H, m), 7.05-7.17 (1H, m), 7.37-7.88 (5H, m) |
| 472 | 4 | ESI+: 572 |
| | | ESI+: 556 |
| 473 | 12 | NMR-CDCl3: 1.35-1.49 (2H, m), 1.60-1.74 (2H, m), 1.77-1.86 (4H, m), 2.07-2.26 (4H, m), 2.56-2.65 (4H, m), 3.14 (2H, s), 3.75-3.83 (8H, m), 3.83-4.00 (1H, m), 5.01-5.12 (1H, m), 6.22 (1H, s), 7.02-7.09 (1H, m), 7.25 (1H, t, J = 53 Hz), 7.36-7.45 (2H, m), 7.65-7.72 (1H, m), 7.85-7.93 (1H, m) |
| 474 | 12 | ESI+: 572 |
| 475 | 12 | ESI+: 588 |
| 476 | 12 | ESI+: 614 |
| 477 | 12 | ESI+: 600 |

**[Table 121]**

| Ex | Syn | DATA |
|---|---|---|
| 478 | 12 | ESI+: 574 |
| 479 | 12 | ESI+: 574 |
| 480 | 295 | ESI+: 556 |
| | | ESI+: 582 |
| 481 | 13,16 | NMR1: 0.80-1.34 (14H, m), 1.48-1.64 (1H, m), 1.67-1.85 (2H, m), 1.85-2.12 (3H, m), 2.69 (1H, s), 2.93-3.21 (3H, m), 3.25-3.25 (1H, m), 3.60-3.81 (8H, m), 4.29-4.42 (2H, m), 5.14-5.36 (1H, m), 6.46 (1H, s), 7.32-7.89 (6H, m), 7.96-8.14 (1H, m) |
| | | ESI+: 545 |
| 482 | 295 | NMR1: 0.99-1.93 (13H, m), 2.39-2.56 (2H, m), 2.80-2.88 (1H, m), 3.03-3.14 (1H, m), 3.63-3.80 (8H, m), 3.94-4.28 (4H, m), 6.42 (1H, s), 7.39-7.69 (3H, m), 7.73-7.89 (1H, m), 7.83-7.90 (1H, m) |
| | | ESI+: 545 |
| 483 | 295 | NMR1: 1.43-1.81 (12H, m), 1.92-2.04 (1H, m), 2.39-2.60 (2H, m), 2.81-2.90 (1H, m), 2.98-3.10 (1H, m), 3.63-3.81 (8H, m), 3.96-4.38 (4H, m), 6.43 (1H, s), 7.39-7.68 (3H, m), 7.74-7.79 (1H, m), 7.84-7.89 (1H, m) |
| | | ESI+: 544 |
| 484 | 295,16 | NMR1: 0.98-1.15 (2H, m), 1.38-1.69 (3H, m), 1.71-2.25 (8H, m), 2.97-3.90 (13H, m), 4.00-4.18 (1H, m), 4.61-4.90 (2H, m), 6.16 (1H, s), 7.37-7.76 (5H, m), 7.83-7.88 (1H, m) |
| 485 | 295,16 | ESI+: 544 |
| | | NMR1: 1.45-2.17 (13H, m), 3.18-3.33 (2H, m), 3.35-4.50 (12H, m), 4.61-4.92 (2H, m), 6.17 (1H, s), 7.36-7.76 (5H, m), 7.83-7.88 (1H, m) |
| 486 | 295,16 | ESI+: 571 |
| 487 | 295 | ESI+: 570 |
| 488 | 26, 16 | ESI+: 545 |
| | | NMR1: 0.80-3.34 (21H, m), 3.58-3.86 (8H, m), 4.194.28 (2H, m), 6.43 (1H, s), 7.38-7.69 (3H, m), 7.72-7.78 (1H, m), 7.84-7.90 (1H, m) |

**[Table 122]**

| Ex | Syn | DATA |
|---|---|---|
| 489 | 10 | ESI+: 517 |
| | | ESI+: 570 |
| 490 | 295,16 | NMR1: 0.85-1.08 (1H, m), 1.08-1.32 (6H, m), 1.37-2.08 (10H, m), 2.12-2.28 (1H, m), 3.01-3.29 (2H, m), 3.55-3.80 (8H, m), 5.06-5.39 (1H, m), 6.27-6.40 (1H, m), 7.13-7.23 (1H, m), 7.37-7.97 (5H, m), 8.41-8.66 (1H, m) |
| | | ESI+: 584 |
| 491 | 26,16 | NMR1: 0.84-1.11 (2H, m), 1.15-1.28 (10H, m), 1.32-1.47 (1H, m), 1.47-1.63 (2H, m), 1.79-1.93 (2H, m), 1.96 (3H, s), 1.96-2.12 (2H, m), 3.02-3.25 (3H, m), 3.25-3.34 (1H, m), 3.41-3.51 (1H, m), 3.55-3.64 (3H, m), 3.69-3.99 (3H, m), 4.34-4.49 (1H, m), 5.47 (1H, d, J = 10.4 Hz), 5.54 (1H, d, J = 8.0 Hz), 6.02-6.17 (1H, m), 6.24-6.35 (1H, m), 7.18-7.27 (1H, m), 7.38-7.90 (5H, m), 8.65-8.81 (1H, m) |
| | | ESI+: 584 |
| 492 | 26,16 | NMR1: 0.88-1.15 (1H, m), 1.15-1.31 (7H, m), 1.44-1.68 (3H, m), 1.79-1.93 (2H, m), 1.96 (3H, s), 1.96-2.12 (2H, m), 3.03-3.27 (2H, m), 3.31 (3H, s), 3.55-4.14 (8H, m), 4.35-4.48 (1H, m), 5.38-5.59 (2H, m), 5.92-6.08 (1H, m), 6.23-6.34 (1H, m), 7.13-7.24 (1H, m), 7.37-7.91 (5H, m), 8.70-8.94 (1H, m) |
| 493 | 23 | ESI+: 531 |
| 494 | 343 | ESI+: 530 |
| | | ESI+: 544 |
| 495 | 23 | NMR1: 0.98-1.15 (2H, m), 1.25 (6H, s), 1.35-1.64 (3H, m), 1.85-1.96 (2H, m), 1.98-2.19 (2H, m), 2.76-2.88 (4H, m), 3.07-3.33 (4H, m), 3.58-3.77 (8H, m), 6.18 (1H, s), 7.37-7.90 (6H, m) |
| | | ESI+: 516 |
| 496 | 10 | NMR1: 0.89-1.06 (4H, m), 1.42-1.57 (1H, m), 1.71-1.95 (4H, m), 2.25-2.36 (1H, m), 2.64-2.70 (2H, m), 3.19-3.38 (7H, m), 3.61-3.72 (8H, m), 6.14 (1H, s), 7.37-7.67 (4H, m), 7.70-7.74 (1H, m), 7.83-7.88 (1H, m) |

**[Table 123]**

| Ex | Syn | DATA |
|---|---|---|
| | | ESI+: 530 |
| 497 | 23 | NMR1: 0.98-1.15 (2H, m), 1.38-1.62 (3H, m), 1.84-1.96 (2H, m), 1.98-2.14 (2H, m), 2.65-2.73 (3H, m), 3.09-3.42 (8H, m), 3.61-3.75 (10H, m), 6.17 (1H, s), 7.37-7.76 (5H, m), 7.83-7.88 (1H, m) |
| 498 | 2 | ESI+: 556 |
| | | ESI+: 558 |
| 499 | 9,16 | NMR1: 0.92-1.27 (5H, m), 1.43-1.62 (3H, m), 1.62-1.71 (1H, m), 1.75-1.88 (2H, m), 3.05-3.22 (4H, m), 3.27 (3H, s), 4.40-3.52 (1H, m), 3.60-3.71 (8H, m), 3.98-4.09 (2H, m), 6.26-6.39 (1H, m), 7.12-7.25 (1H, m), 7.37-7.87 (5H, m) |
| | | ESI+: 556 |
| 500 | 2 | NMR1: 1.41-1.74 (7H, m), 1.79-1.89 (2H, m), 1.98-2.18 (3H, m), 2.20-2.29 (4H, m), 2.63-2.71 (1H, m), 2.97-3.05 (1H, m), 3.58-3.80 (9H, m), 4.99-5.11 (1H, m), 6.39 (1H, s), 7.38-7.69 (4H, m), 7.72-7.79 (1H, m), 7.83-7.89 (1H, m) |
| 501 | 2 | ESI+: 545 |
| 502 | 4 | ESI+: 558 |
| 503 | 4 | ESI+: 558 |
| 504 | 4 | ESI+: 544 |
| | | ESI+: 572 |
| 505 | 15 | NMR1: 0.80-1.02 (10H, m), 1.02 (3H, s), 1.14 (3H, s), 1.14-1.33 (1H, m), 1.23 (3H, d, J = 6.4 Hz), 1.39-1.70 (1H, m), 1.73-1.87 (3H, m), 2.19 (2H, s), 3.02-3.23 (2H, m), 3.38-3.51 (1H, m), 3.58-3.77 (2H, m), 3.89-3.96 (1H, m), 3.99-4.45 (2H, m), 6.20-6.32 (1H, m), 7.06-7.19 (1H, m), 7.37-7.90 (5H, m) |
| | | ESI+: 572 |
| 506 | 15 | NMR1: 0.81-1.02 (10H, m), 1.02 (3H, s), 1.14 (3H, s), 1.14-1.33 (1H, m), 1.23 (3H, d, J = 6.4 Hz), 1.39-1.70 (1H, m), 1.73-1.87 (3H, m), 2.20 (2H, s), 3.01-3.23 (2H, m), 3.38-3.51 (1H, m), 3.58-3.77 (2H, m), 3.89-3.96 (1H, m), 3.99-4.45 (2H, m), 6.21-6.31 (1H, m), 7.06-7.17 (1H, m), 7.37-7.87 (5H, m) |

**[Table 124]**

| Ex | Syn | DATA |
|---|---|---|
| | | ESI+: 558 |
| 507 | 15 | NMR1: 0.82-1.03 (3H, m), 0.93 (3H, d, J = 6 Hz), 1.09-1.27 (2H, m), 1.23 (3H, d, J = 5.6 Hz), 1.39-1.53 (1H, m), 1.66-1.85 (4H, m), 2.14 (3H, s), 2.33-2.41 (1H, m), 2.87-2.98 (1H, m), 3.01-3.21 (3H, m), 3.21 (3H, s), 3.40-3.52 (1H, m), 3.58-3.65 (1H, m), 3.65-3.77 (1H, m), 3.89-3.97 (1H, m), 4.01-4.13 (1H, m), 4.35-4.47 (1H, m), 6.19-6.32 (1H, m), 7.06-7.18 (1H, m), 7.37-7.89 (5H, m) |
| | | ESI+: 572 |
| 508 | 4 | NMR1: 0.82-1.05 (9H, m), 1.23-1.33 (2H, m), 1.23 (3H, d, J = 6.4 Hz), 1.39-1.53 (1H, m), 1.56-1.65 (1H, m), 1.66-1.84 (3H, m), 1.92-2.01 (1H, m), 2.01-2.12 (2H, m), 3.12-3.28 (2H, m), 3.21 (3H, s), 3.40-3.51 (1H, m), 3.58-3.65 (1H, m), 3.65-3.77 (1H, m), 3.89-3.97 (1H, m), 4.01-4.13 (1H, m), 4.34-4.47 (1H, m), 6.20-6.32 (1H, m), 7.06-7.18 (1H, m), 7.37-7.89 (5H, m) |
| 509 | 4 | ESI+: 544 |
| | | ESI+: 544 |
| 510 | 295,16 | NMR1: 1.45-1.59 (2H, m), 1.66-2.02 (10H, m), 2.04-2.17 (1H, m), 3.19-3.32 (2H, m), 3.35-3.55 (3H, m), 3.60-376 (8H, m), 4.02-4.21 (2H, m), 4.59-4.95 (1H, m), 6.18 (1H, s), 7.37-7.76 (5H, m), 7.89-7.88 (1H, m) |
| | | ESI+: 544 |
| 511 | 295,16 | NMR1: 0.97-1.16 (2H, m), 1.40-1.65 (3H, m), 1.69-2.00 (5H, m), 2.02-2.15 (2H, m), 2.17-2.28 (1H, m), 3.02-4.17 (15H, m), 4.61-4.96 (1H, m), 6.18 (1H, s), 7.36-7.89 (6H, m) |
| | | ESI+: 556 |
| 512 | 295,16 | NMR1: 1.44-1.58 (2H, m), 1.64-2.11 (11H, m), 3.05-3.98 (19H, m), 6.19 (1H, s), 7.37-7.90 (6H, m) |
| | | ESI+: 556 |
| 513 | 295,16 | NMR1: 0.97-1.16 (2H, m), 1.37-1.63 (3H, m), 1.66-1.78 (1H, m), 1.80-1.96 (4H, m), 1.97-2.11 (2H, m), 2.13-2.30 (1H, m), 3.00-4.00 (19H, m), 6.16 (1H, s), 7.37-7.76 (5H, m), 7.83-7.89 (1H, m) |

**[Table 125]**

| Ex | Syn | DATA |
|---|---|---|
| | | ESI+: 544 |
| 514 | 295 | NMR1: 0.80-1.22 (1H, m), 1.22-1.90 (14H, m), 2.70-2.92 (1H, m), 2.92-3.10 (1H, m), 3.16-3.29 (1H, m), 3.57-3.81 (8H, m), 3.91-4.31 (2H, m), 6.24-6.38 (1H, m), 7.06-7.19 (1H, m), 7.37-7.90 (5H, m) |
| | | ESI+: 544 |
| 515 | 295 | NMR1: 0.81-1.29 (5H, m), 1.38-1.99 (10H, m), 2.78-2.86 (1H, m), 3.00-3.19 (2H, m), 3.58-3.76 (8H, m), 3.92-4.27 (2H, m), 6.25-6.39 (1H, m), 7.08-7.19 (1H, m), 7.37-7.91 (5H, m) |
| 516 | 2 | ESI+: 544 |
| | | ESI+: 558 |
| 517 | 2 | NMR1: 0.74 (3H, d, J = 7.2 Hz), 0.87 (3H, d, J = 6.4 Hz), 0.92-1.08 (1H, m), 1.12-1.32 (4H, m), 1.41-1.58 (1H, m), 1.67-1.83 (4H, m), 1.89-1.98 (1H, m), 3.03-3.20 (2H, m), 3.26-3.47 (1H, m), 3.47-3.58 (1H, m), 3.58-3.77 (8H, m), 6.26-6.39 (1H, m), 7.12-7.21 (1H, m), 7.35-7.95 (5H, m) |
| | | ESI+: 584 |
| 518 | 13,16 | NMR1: 0.79-1.37 (11H, m), 1.44-1.65 (1H, m), 1.65-1.85 (2H, m), 1.85-1.97 (1H, m), 1.97-2.21 (3H, m), 2.91-3.15 (2H, m), 3.31-3.53 (4H, m), 3.60-3.77 (8H, m), 4.14-4.26 (1H, m), 5.03-5.33 (2H, m), 5.84-6.00 (1H, m), 6.36-6.47 (1H, m), 7.24-8.03 (6H, m), 8.12-8.42 (1H, m) |
| 519 | 4 | ESI+: 560 |
| 520 | 295,16 | ESI+: 598 |
| 521 | 295,16 | ESI+: 598 |
| 522 | 295,16 | ESI+: 570 |
| 523 | 295,16 | ESI+: 570 |
| 524 | 295,16 | ESI+: 526 |
| | | ESI+: 526 |
| 525 | 295,16 | NMR1: 1.00-2.19 (16H, m), 2.87-4.42 (14H, m), 6.15 (1H, m), 7.37-7.76 (5H, m), 7.83-7.89 (1H, m) |
| 526 | 295,16 | ESI+: 595 |

**[Table 126]**

| Ex | Syn | DATA |
|---|---|---|
| | | ESI+: 595 |
| 527 | 295,16 | NMR1: 0.99-1.30 (2H, m), 1.41-1.66 (3H, m), 1.80-2.38 (12H, m), 2.95-3.12 (2H, m), 3.17-4.15 (18H, m), 6.16 (1H, s), 7.37-7.76 (5H, m), 7.83-7.88 (1H, m) |
| 528 | 295,16 | ESI+: 560 |
| 529 | 295,16 | ESI+: 560 |
| 530 | 295,16 | ESI+: 560 |
| | | ESI+: 560 |
| 531 | 295,16 | NMR1: 0.92-1.29 (2H, m), 1.36-1.71 (3H, m), 1.78-2.08 (3H, m), 2.14-2.29 (1H, m), 3.02-4.47 (18H, m), 4.68-5.28 (2H, m), 6.16 (1H, m), 7.37-7.77 (5H, m), 7.83-7.89 (1H, m) |
| 532 | 295,16 | ESI+: 572 |
| 533 | 295,16 | ESI+: 572 |
| 534 | 295,16 | ESI+: 572 |
| | | ESI+: 572 |
| 535 | 295,16 | NMR1: 0.97-1.27 (2H, m), 1.36-1.69 (3H, m), 1.84-2.14 (4H, m), 3.00-4.22 (23H, m), 6.16 (1H, s), 7.36-7.78 (5H, m), 7.82-7.91 (1H, m) |
| 536 | 295,16 | ESI+: 530 |
| 537 | 295,16 | ESI+: 530 |
| 538 | 295,16 | ESI+: 530 |
| | | ESI+: 530 |
| 539 | 295,16 | NMR1: 0.89-1.09 (2H, m), 1.13-1.35 (2H, m), 1.45-1.58 (1H, m), 1.81-1.99 (3H, m), 2.01-2.13 (1H, m), 2.16-2.39 (2H, m), 3.00-5.05 (16H, m), 6.15 (1H, s), 7.37-7.76 (5H, m), 7.83-7.89 (1H, m) |
| 540 | 540 | ESI+: 570 |
| 541 | 10 | ESI+: 602 |
| 542 | 542 | ESI+: 570 |
| 543 | 12 | ESI+: 558 |
| 544 | 12 | ESI+: 574 |
| 545 | 295,16 | ESI+: 542 |
| 546 | 295,16 | ESI+: 542 |

**[Table 127]**

| Ex | Syn | DATA |
|---|---|---|
| 547 | 26,16 | ESI+: 526 |
| 548 | 26,16 | ESI+: 540 |
| 549 | 26 | ESI+: 584 |
| 550 | 433,16 | ESI+: 516 |
| 551 | 554 | ESI+: 572 |
| 552 | 26 | ESI+: 558 |
| 553 | 10 | ESI+: 530 |
| 554 | 554 | ESI+: 558 |
| 555 | 555 | ESI+: 542 |
| 556 | 2 | ESI+: 658 |
| 557 | 2 | ESI+: 660 |
| 558 | 2 | ESI+: 658 |
| 559 | 2 | ESI+: 660 |
| 560 | 295,16 | ESI+: 574 |
| 561 | 295,16 | ESI+: 574 |
| 562 | 295,16 | ESI+: 662 |
| 563 | 295,16 | ESI+: 662 |
| 564 | 295,16 | ESI+: 650 |
| 565 | 295,16 | ESI+: 650 |
| 566 | 289 | ESI+: 558 |
| 567 | 289 | ESI+: 560 |
| 568 | 289 | ESI+: 558 |
| 569 | 289 | ESI+: 560 |
| 570 | 570 | ESI+: 590 |
| 571 | 570 | ESI+: 591 |
| 572 | 570 | ESI+: 590 |
| | | ESI+: 581 |
| 573 | 12 | NMR1: 1.26-1.40 (3H, m), 1.55-1.87 (10H, m), 2.04-2.15 (3H, m), 2.74-2.81 (2H, m), 3.08-3.42 (3H, m), 3.62-3.85 (10H, m), 6.10 (1H, s), 7.38-7.95 (6H, m) |

**[Table 128]**

| Ex | Syn | DATA |
|---|---|---|
| | | ESI+: 555 |
| 574 | 12 | NMR1: 0.82-1.41 (3H, m), 1.53-1.85 (4H, m), 1.95-2.14 (3H, m), 2.23 (6H, s), 3.11-3.38 (3H, m), 3.60-3.87 (10H, m), 6.10 (1H, s), 7.38-7.64 (4H, m), 7.70-7.73 (1H, m), 7.83-7.87 (1H, m) |
| | | ESI+: 582 |
| 575 | 12 | NMR1: 1.48-1.89 (1.2H, m), 2.05-2.25 (3H, m), 2.74-2.83 (2H, m), 3.11-3.39 (3H, m), 3.65-3.88 (10H, m), 5.01-5.07 (1H, m), 6.40 (1H, s), 7.40-7.67 (3H, m), 7.73-7.76 (1H, m), 7.85-7.88 (1H, m) |
| | | ESI+: 556 |
| 576 | 12 | NMR1: 1.45-2.23 (8H, m), 2.24 (6H, s), 3.08-3.44 (4H, m), 3.62-3.90 (10H, m), 4.99-5.08 (1H, m), 6.40 (1H, s), 7.40-7.67 (3H, m), 7.73-7.76 (1H, m), 7.85-7.88 (1H, m) |
| | | ESI+: 581 |
| 577 | 12 | NMR1: 1.27-1.68 (10H, m), 1.74-2.15 (4H, m), 2.43-2.85 (4H, m), 3.08-3.39 (3H, m), 3.59-3.78 (10H, m), 3.27-3.37 (1H, m), 6.92-7.04 (1H, m), 7.36-7.91 (5H, m) |
| | | ESI+: 555 |
| 578 | 12 | NMR1: 1.23-2.10 (10H, m), 2.22 (6H, s), 3.07-3.42 (2H, m), 3.56-3.83 (10H, m), 3.26-3.38 (1H, m), 6.92-7.05 (1H, m), 7.36-7.88 (6H, m) |
| 579 | 3 | ESI+: 556 [M+Na] |
| 580 | 554,16 | ESI+: 558 |
| 581 | 21 | ESI+: 572 |
| 582 | 21 | ESI+: 574 |
| 583 | 21 | ESI+: 572 |
| | | ESI+: 574 |
| 584 | 21 | NMR1: 1.37-1.71 (4H, m), 1.86-2.02 (2H, m), 2.05-2.26 (2H, m), 2.66-2.97 (4H, m), 3.36-3.84 (11H, m), 3.94-4.13 (1H, m), 4.30-4.44 (1H, m), 5.01-5.13 (1H, m), 5.33-5.56 (1H, m), 6.40 (1H, s), 7.38-7.69 (3H, m), 7.71-7.77 (1H, m), 7.83-7.89 (1H, m), 8.86-8.98 (1H, m) |

**[Table 129]**

| Ex | Syn | DATA |
|---|---|---|
| 585 | 22 | ESI+: 467 |
| 586 | 22 | ESI+: 580 [M+Na] |
| 587 | 8 | ESI+: 472 |
| 588 | 1 | ESI+: 594 [M+Na] |
| 589 | 2 | ESI+: 557 |
| 590 | 22 | ESI+: 499 |
| 591 | 1 | ESI+: 499 |

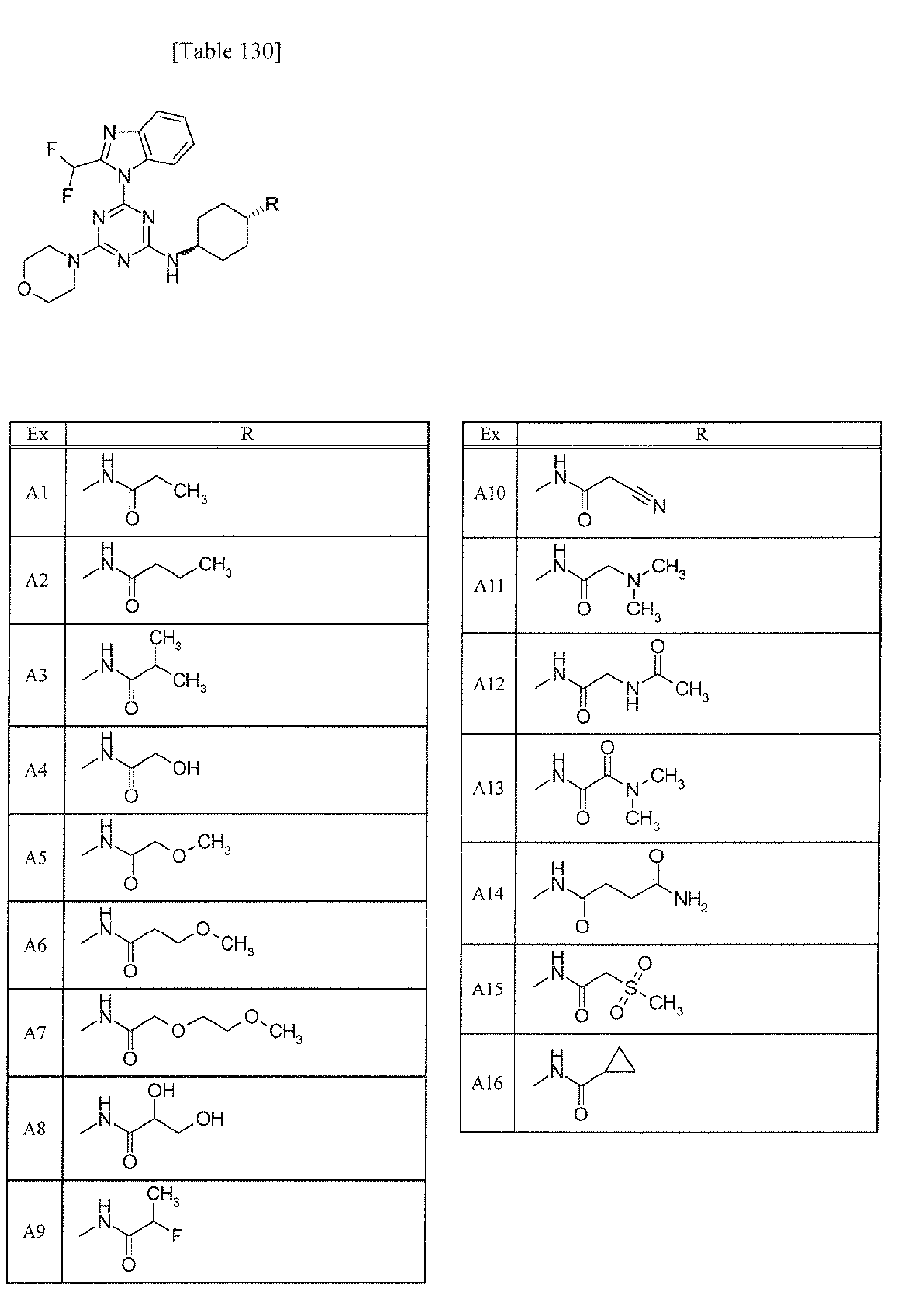

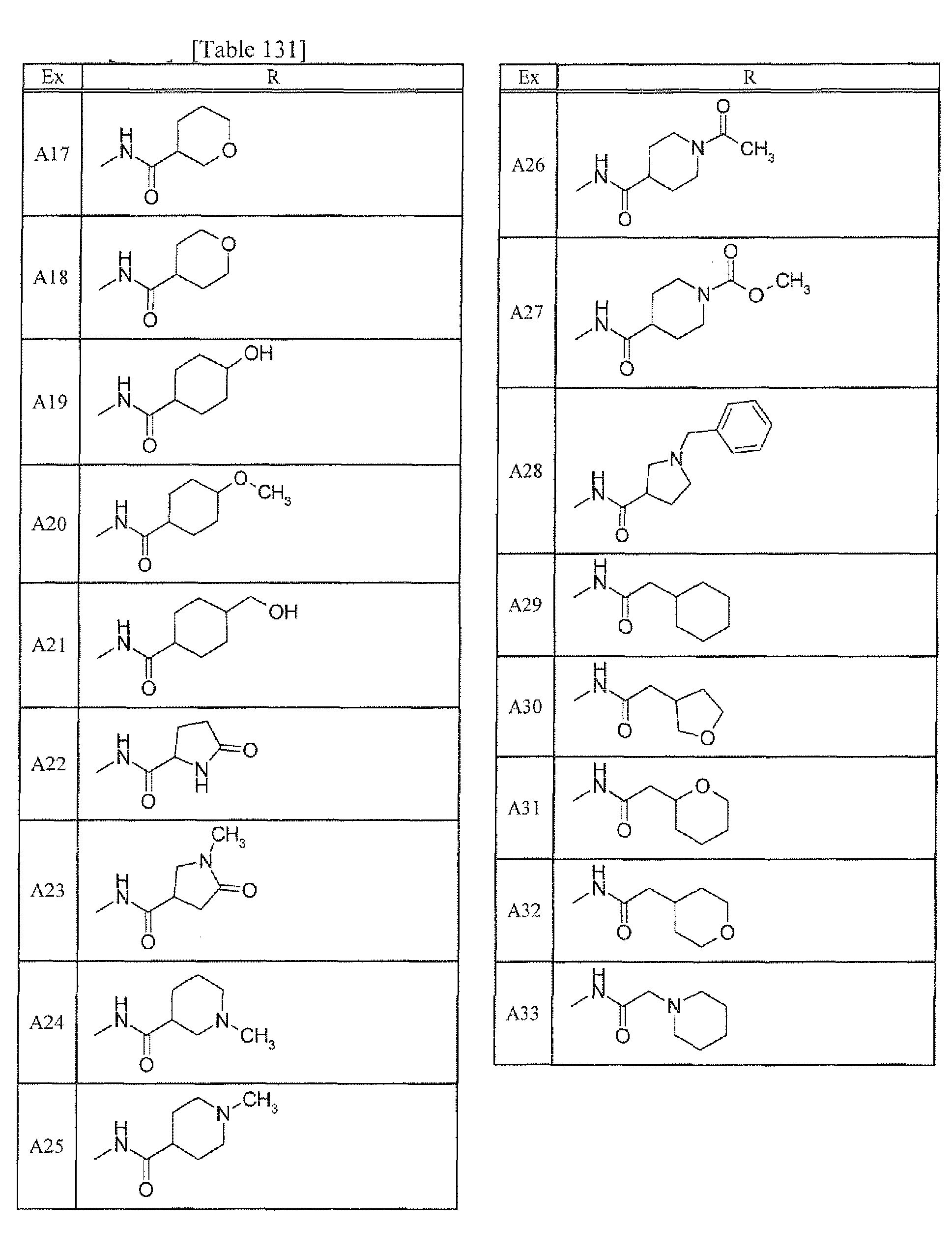

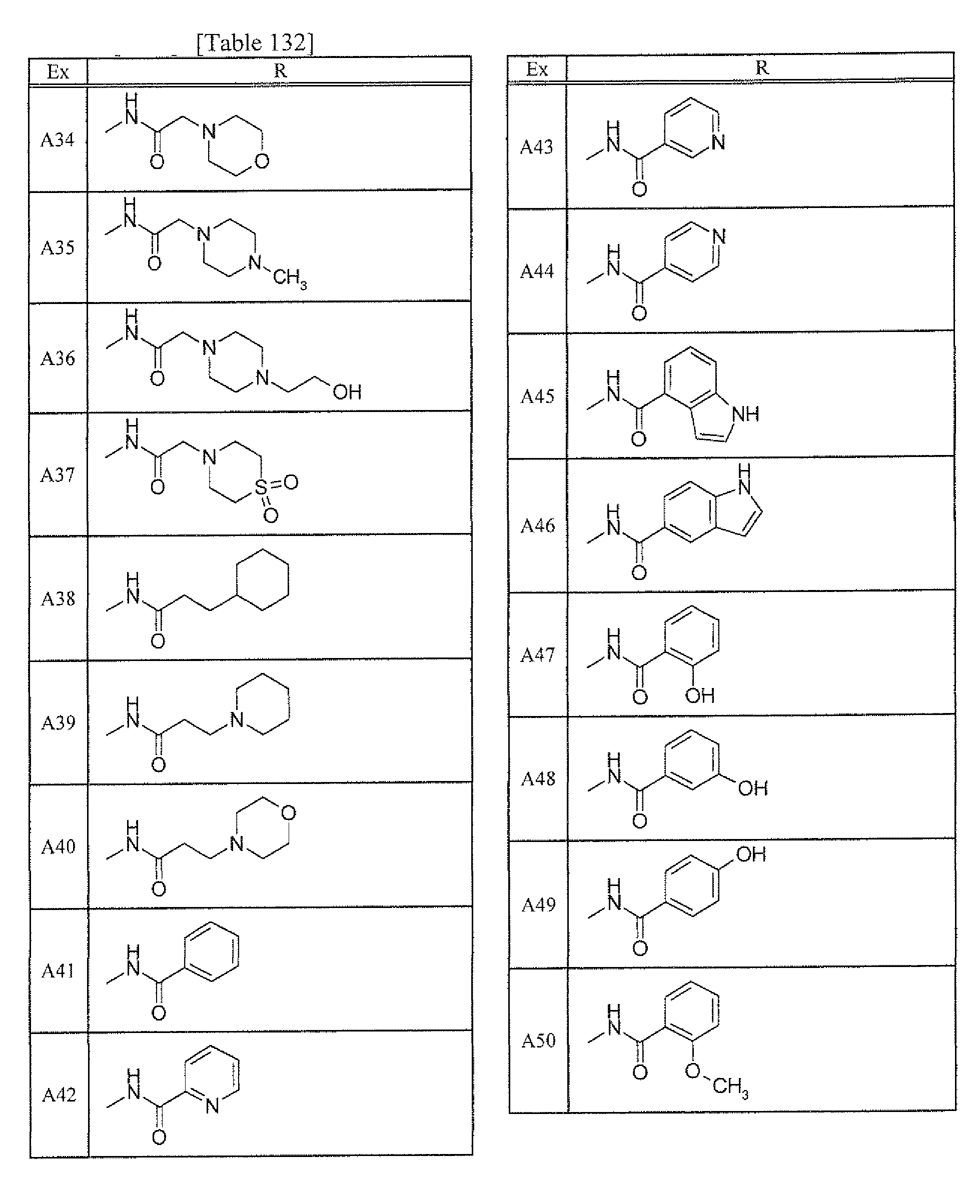

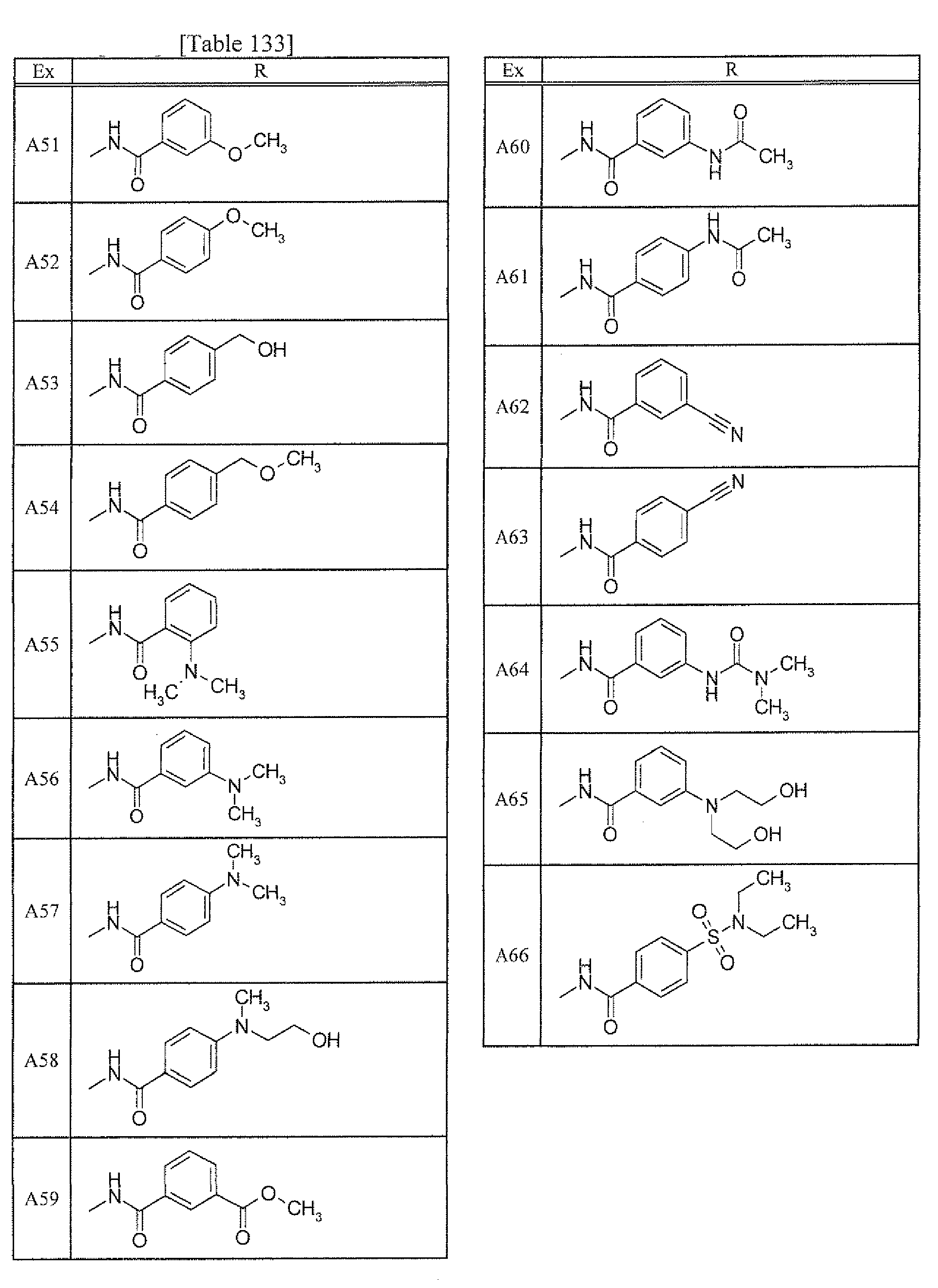

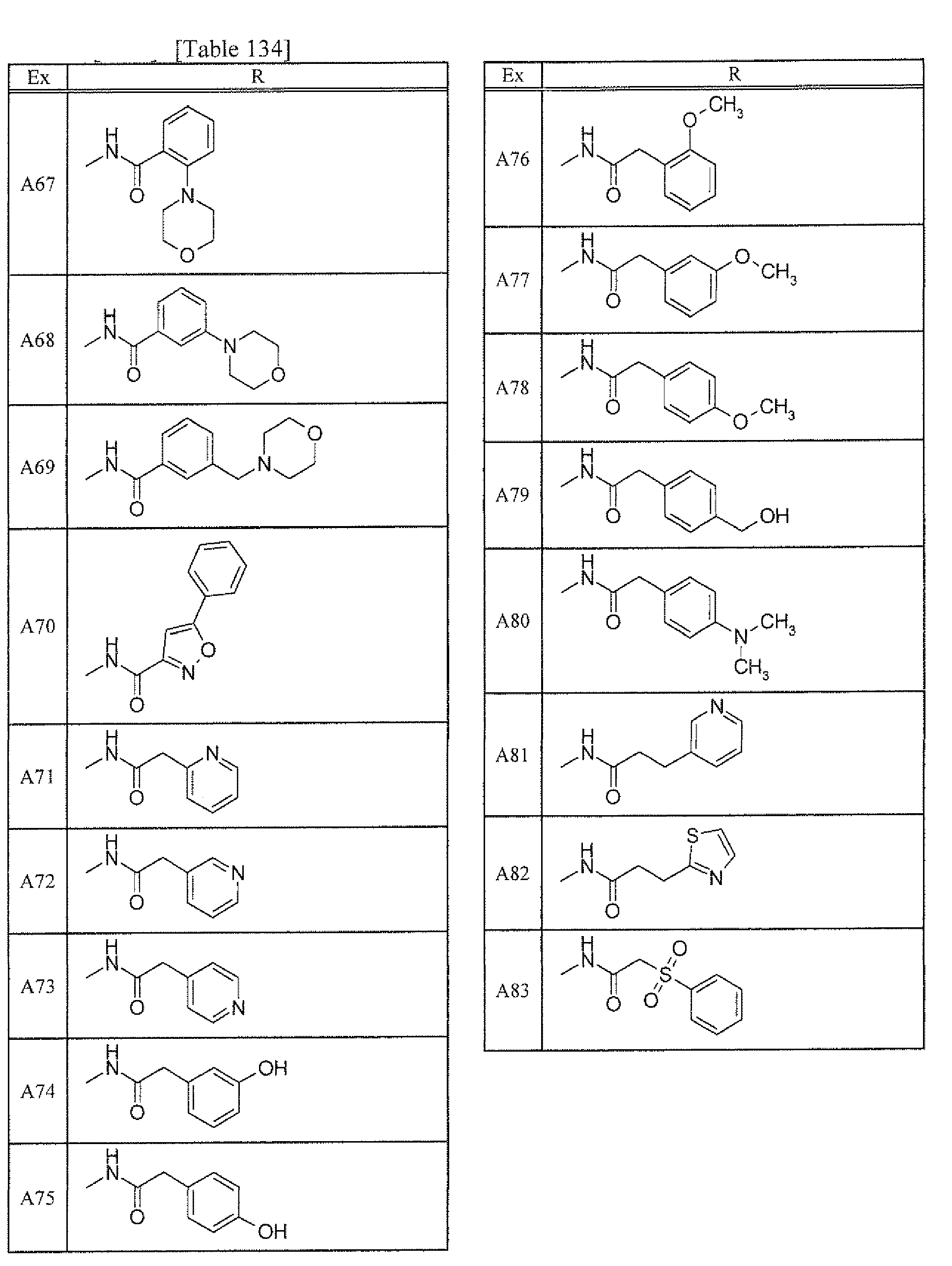

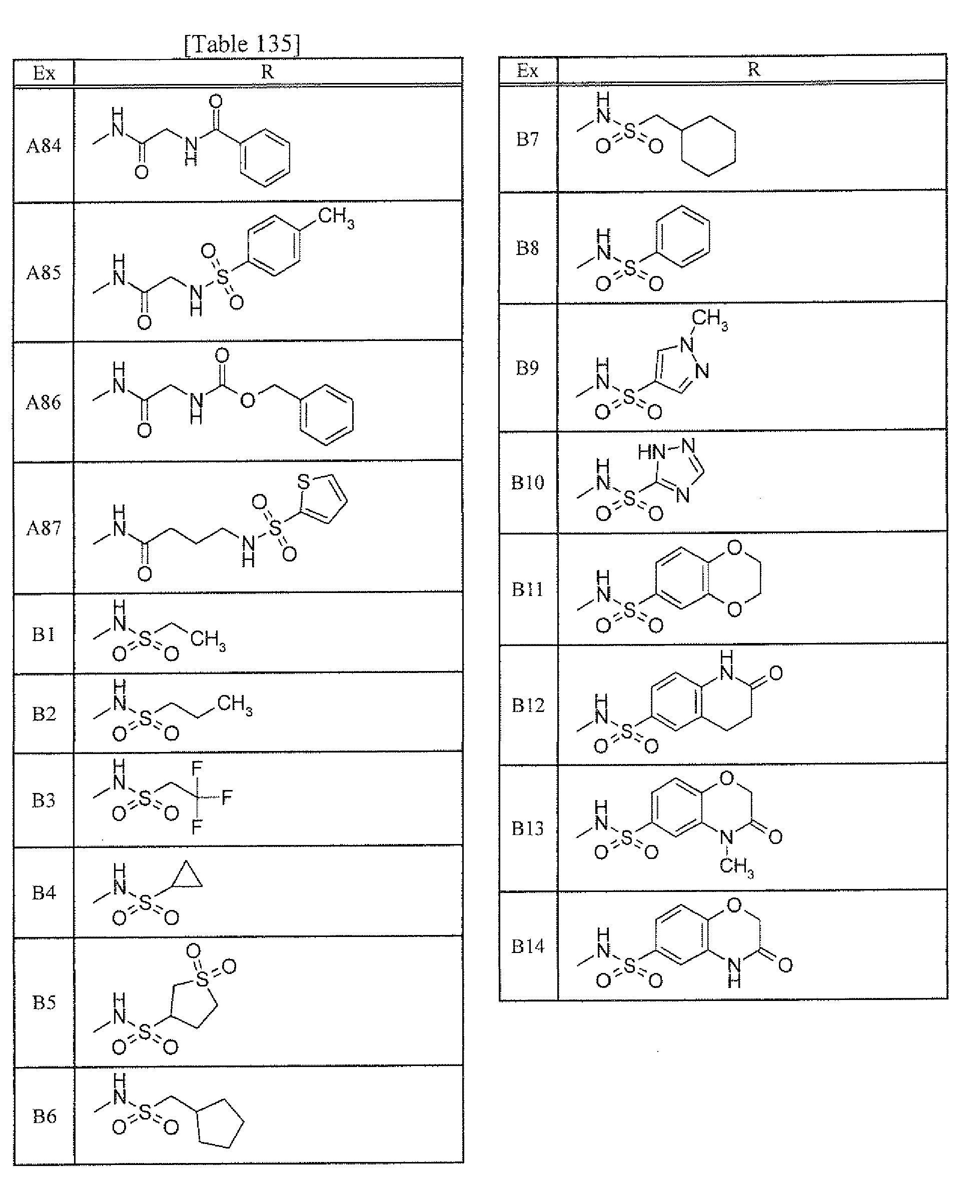

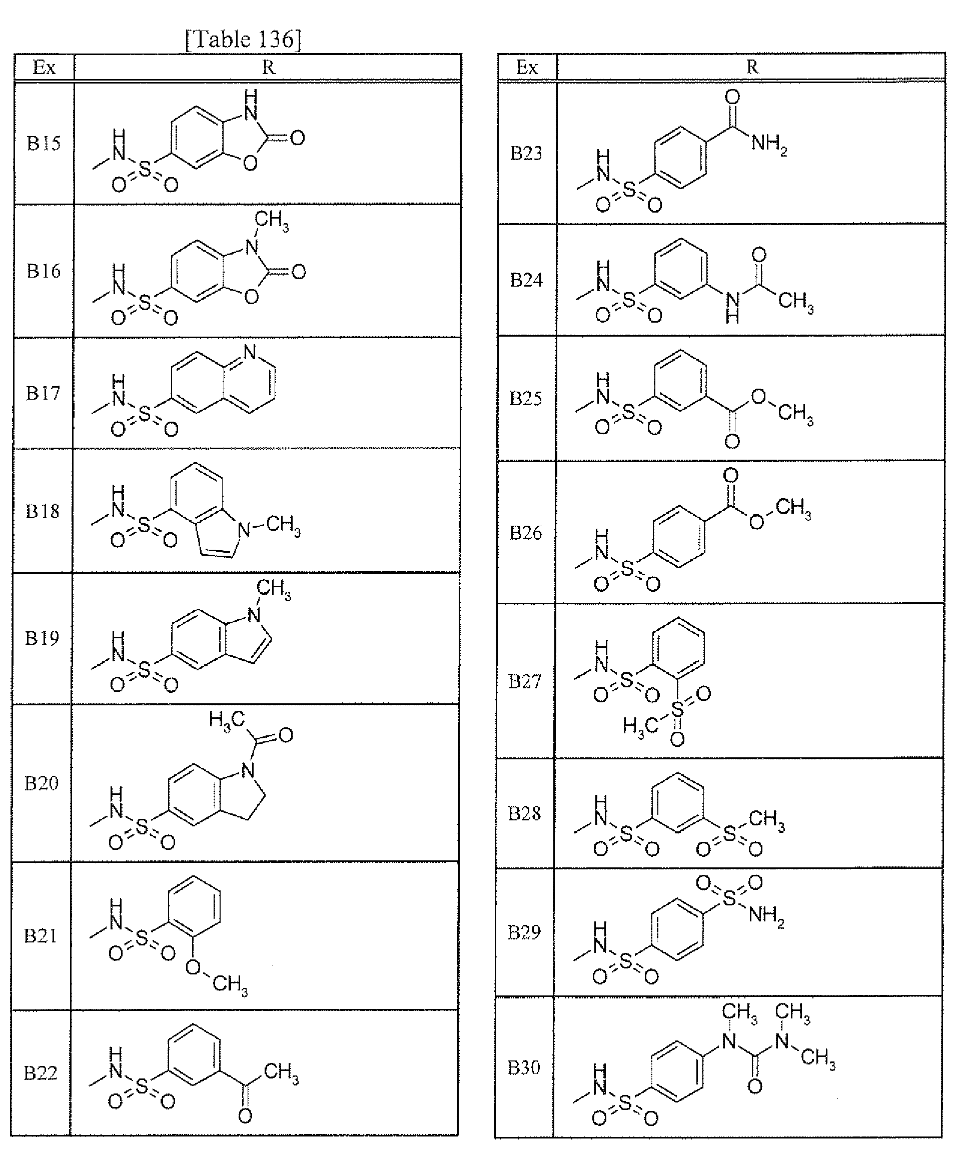

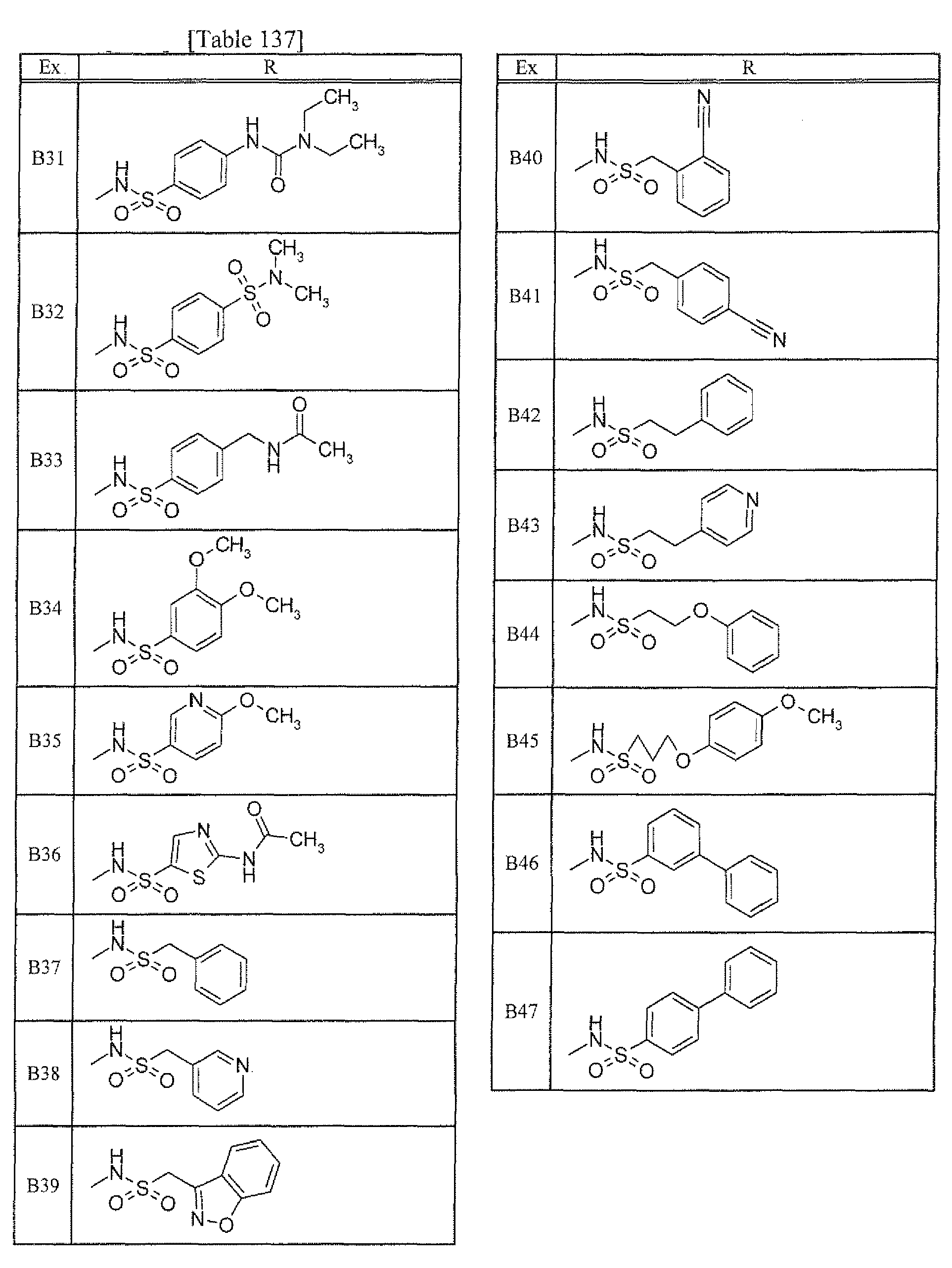

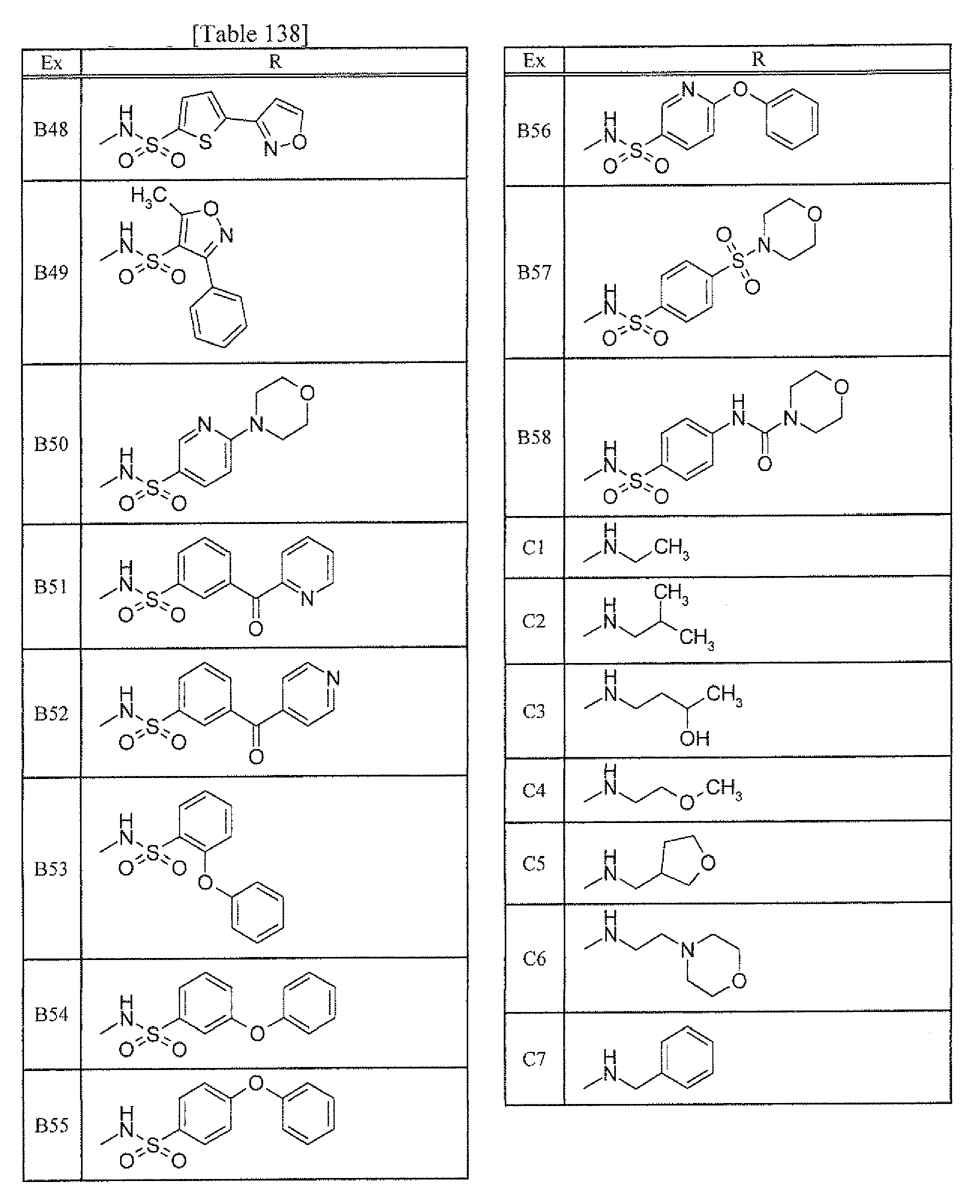

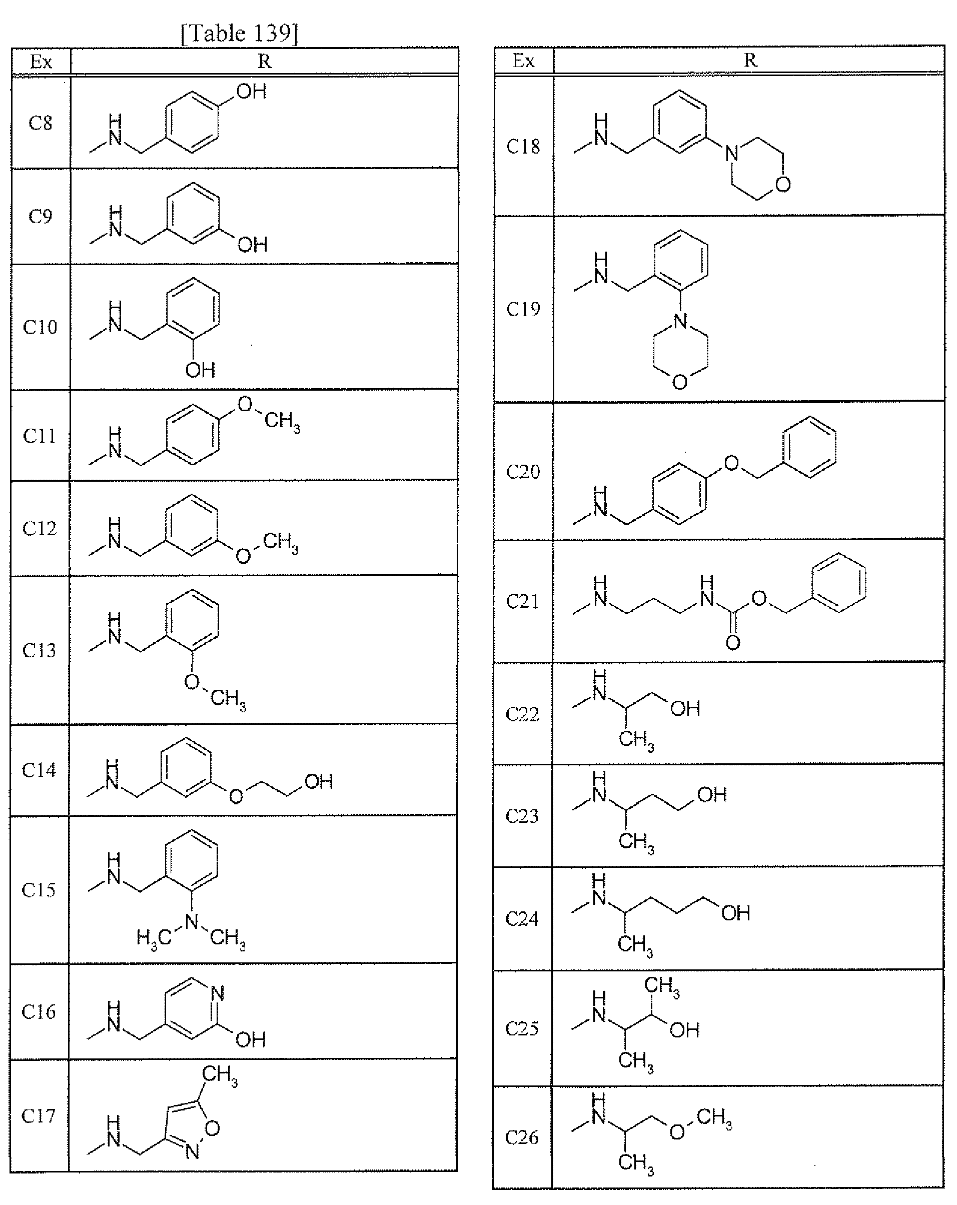

**[Table 140]**

| Ex | R |
|---|---|
| C27 | 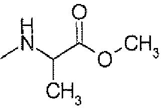 |
| C28 | 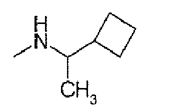 |
| C29 | 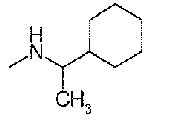 |
| C30 | 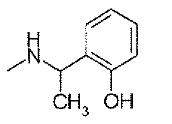 |
| C31 | 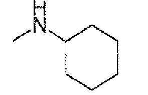 |
| C32 | 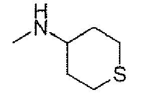 |
| C33 | 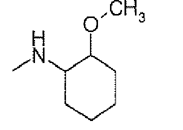 |
| C34 | 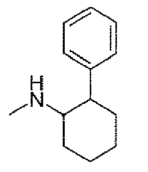 |
| C35 | 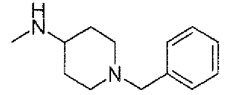 |

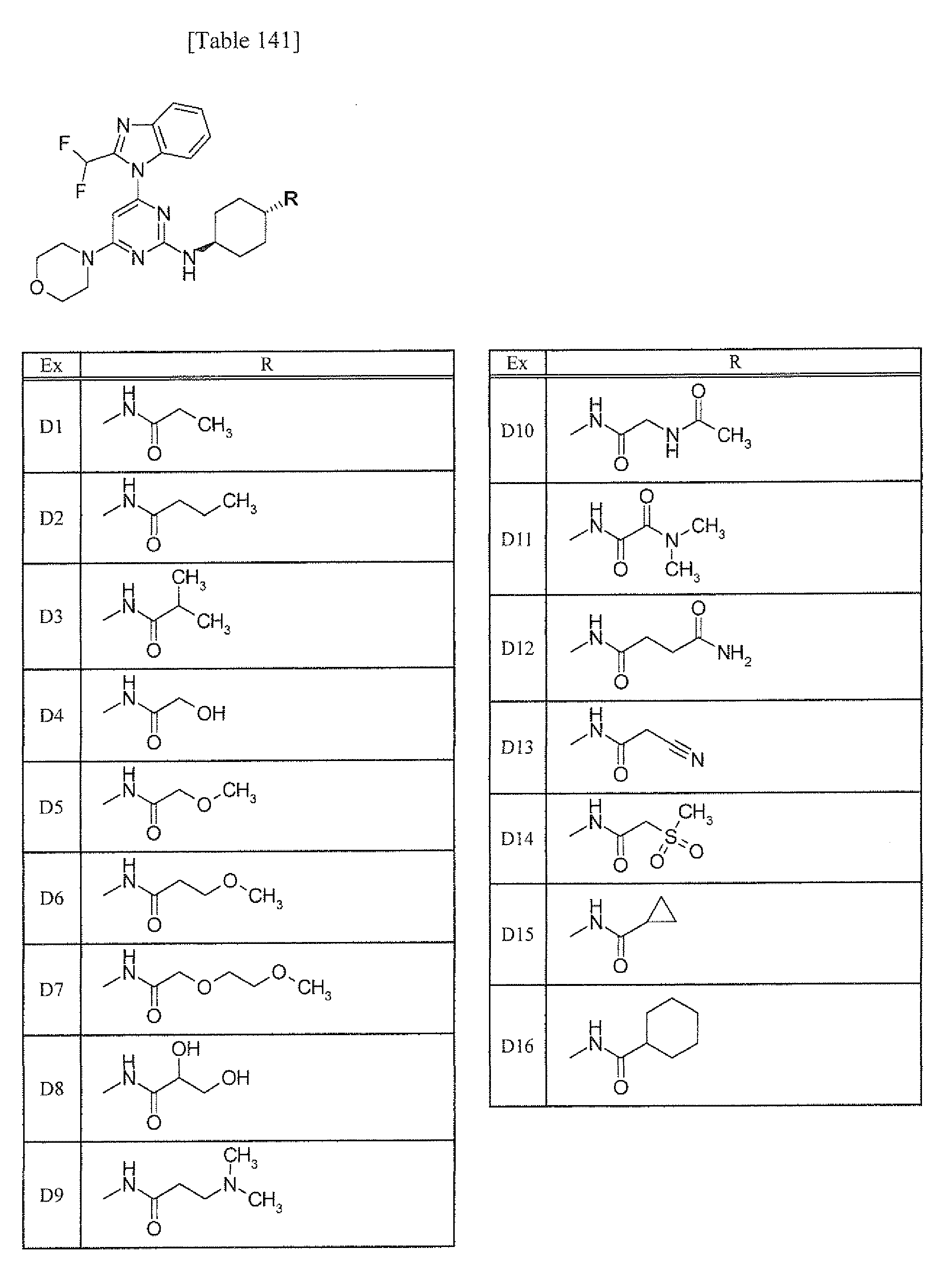

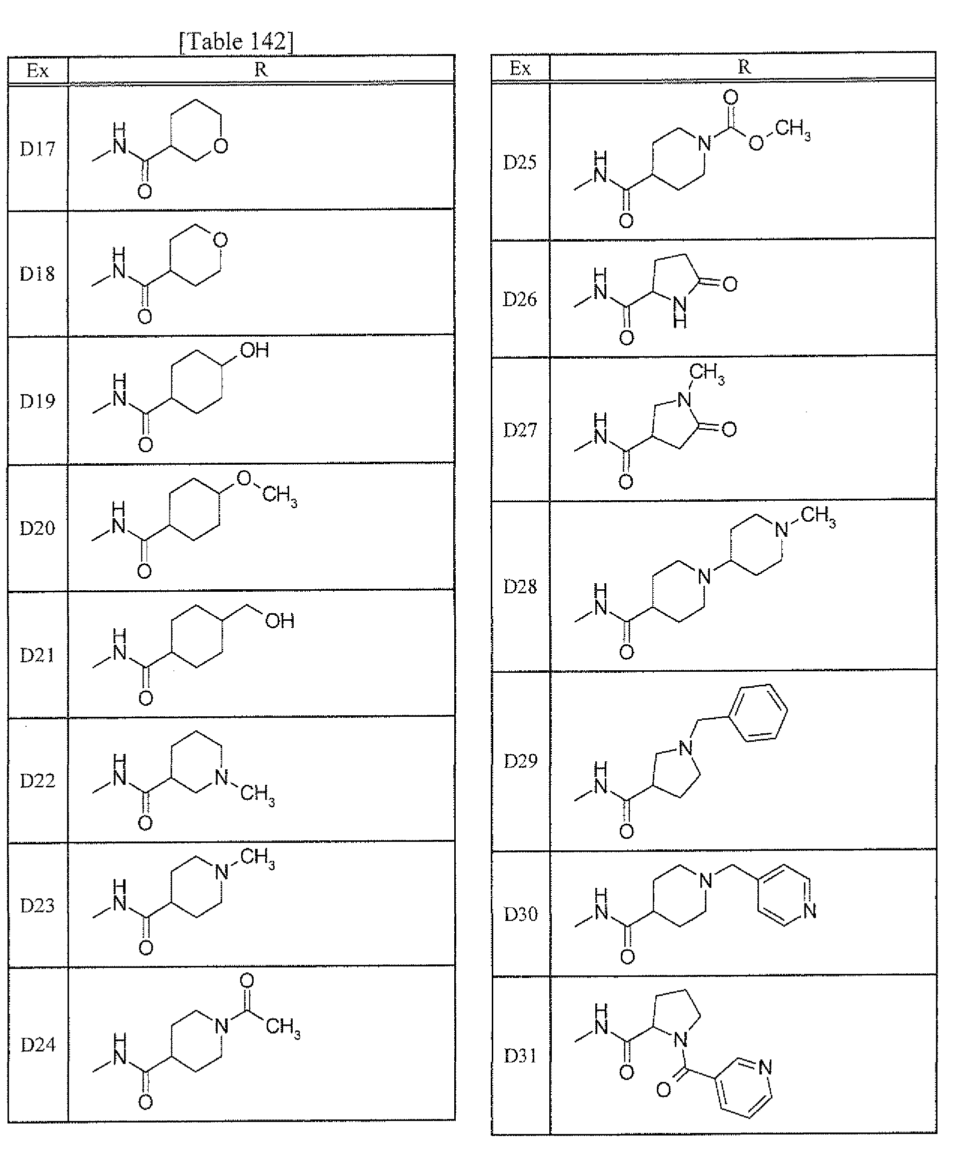

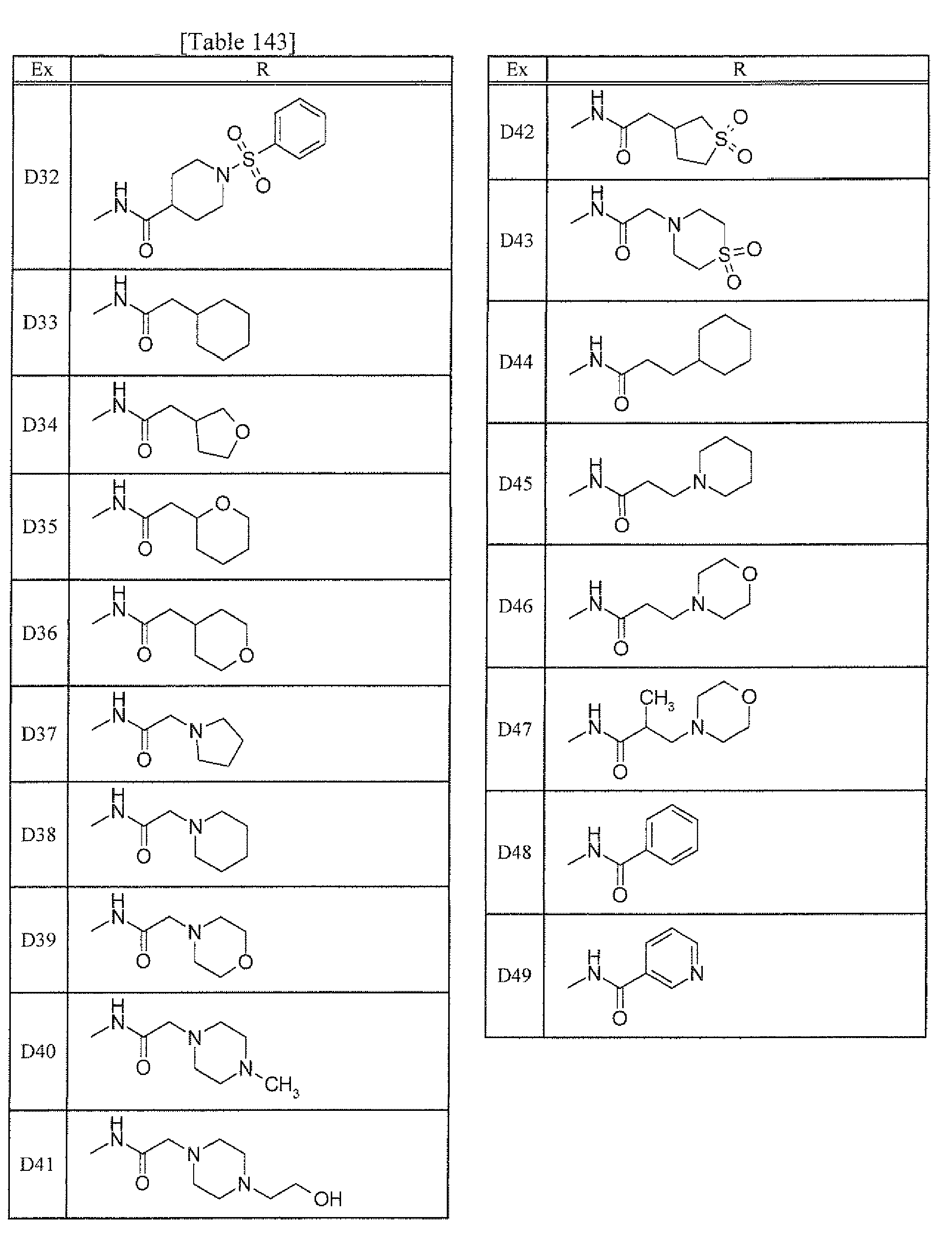

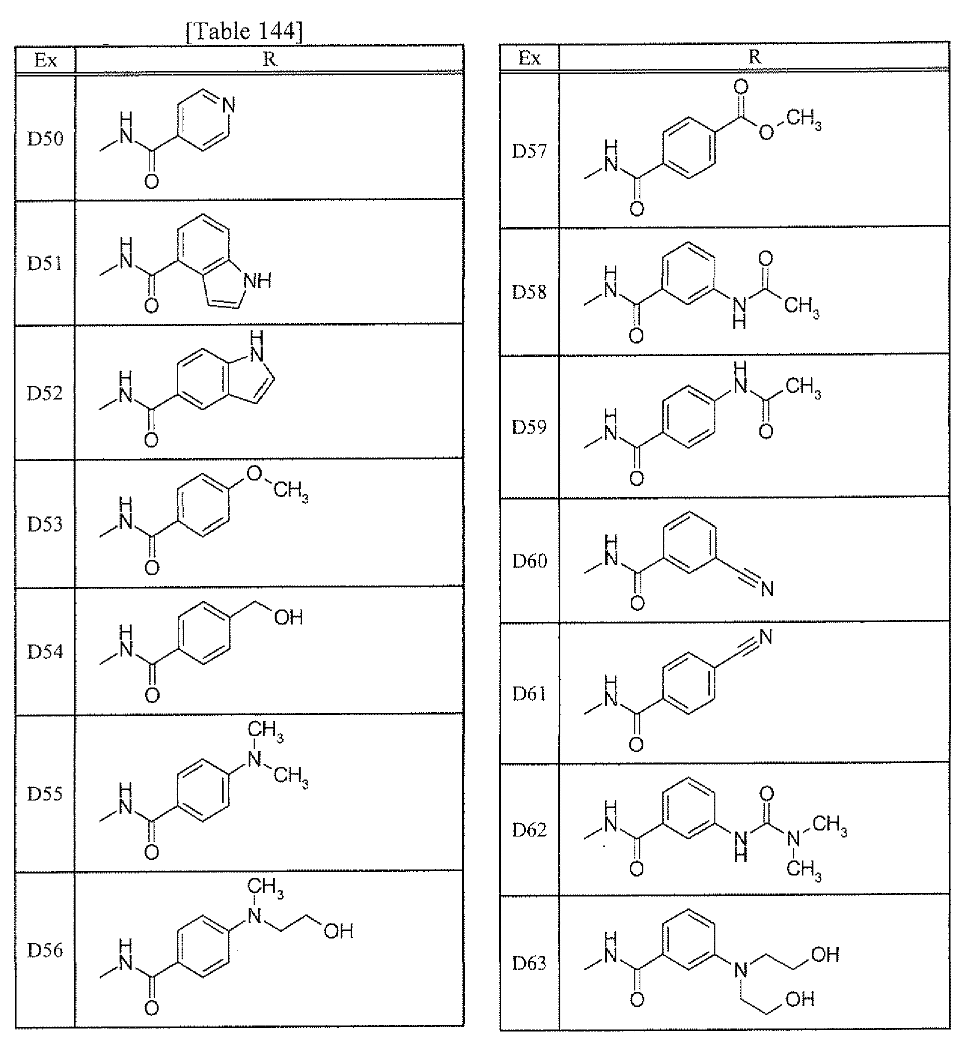

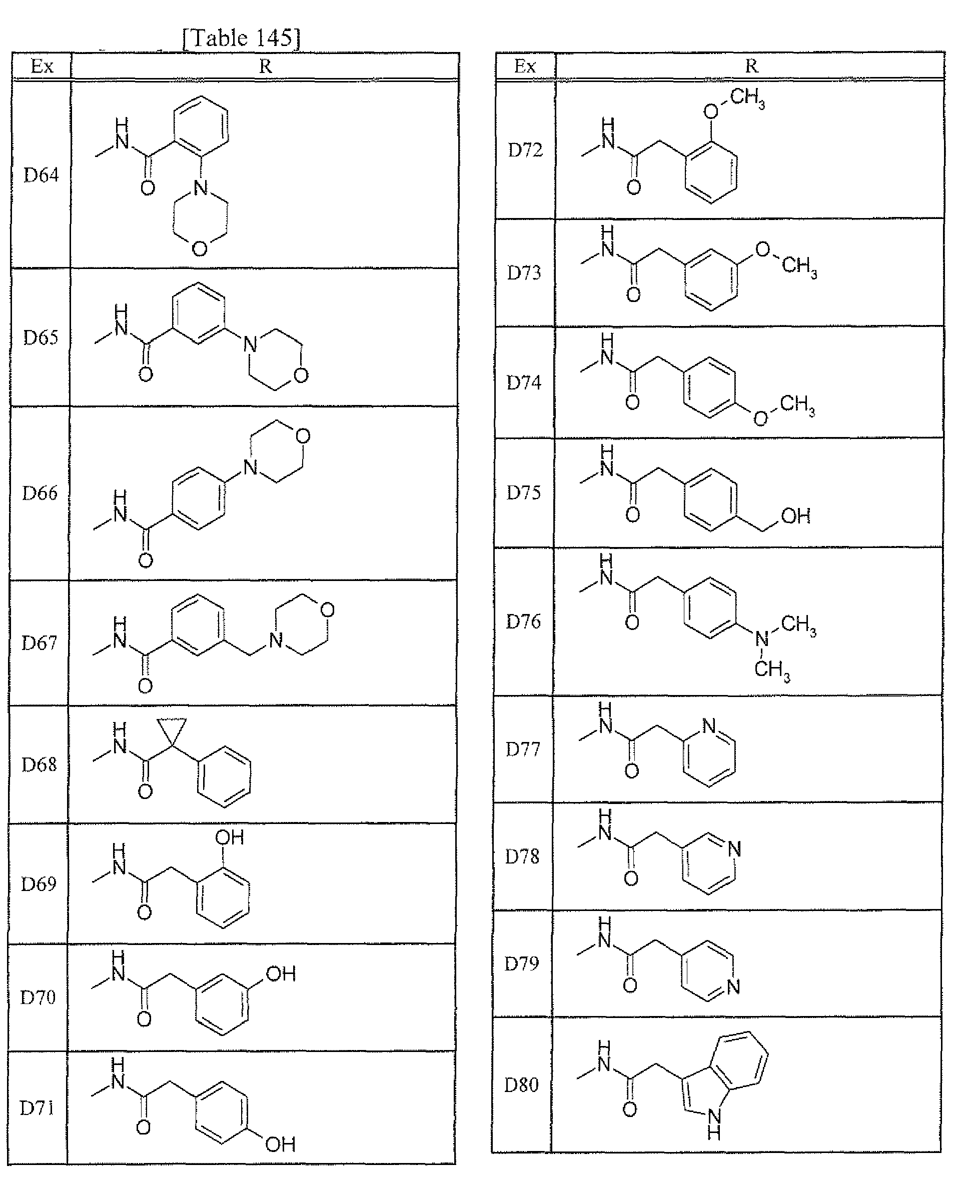

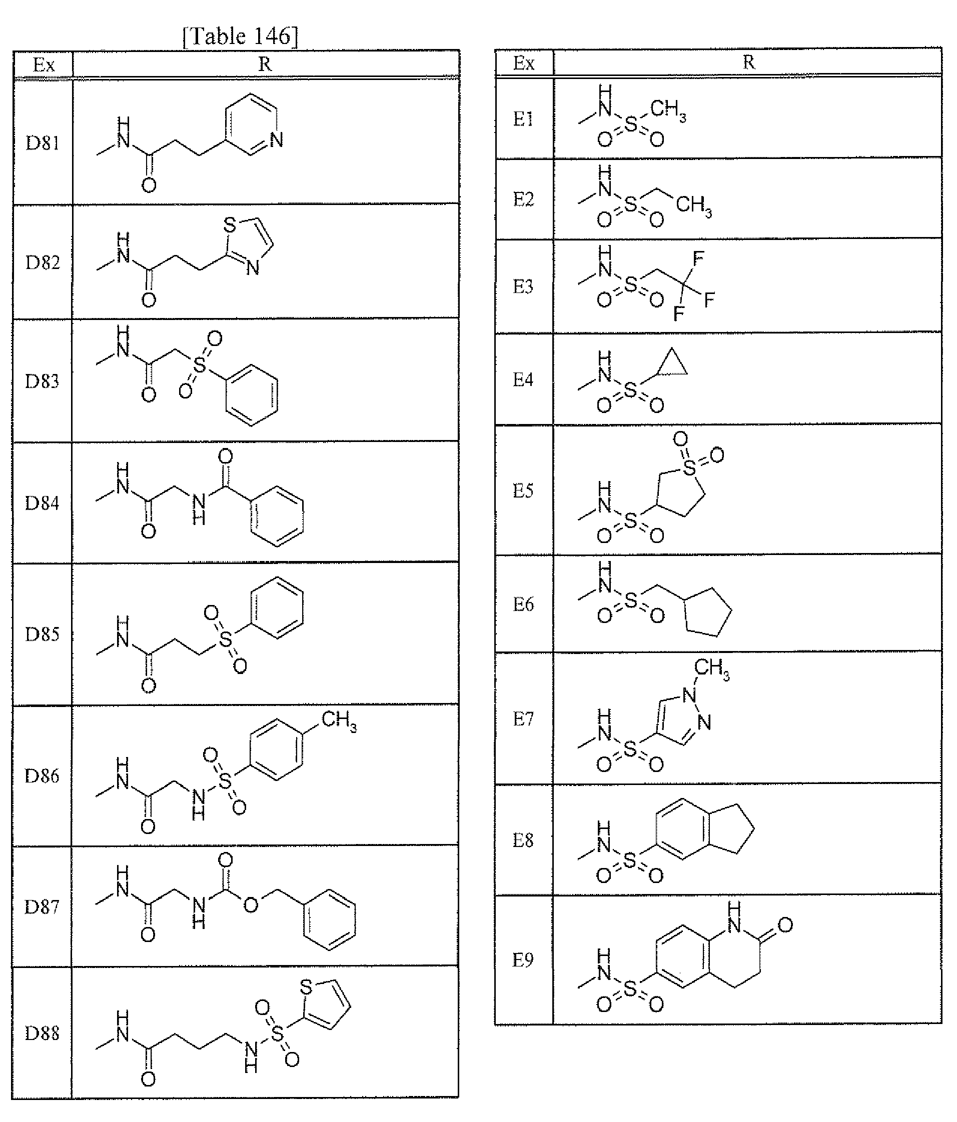

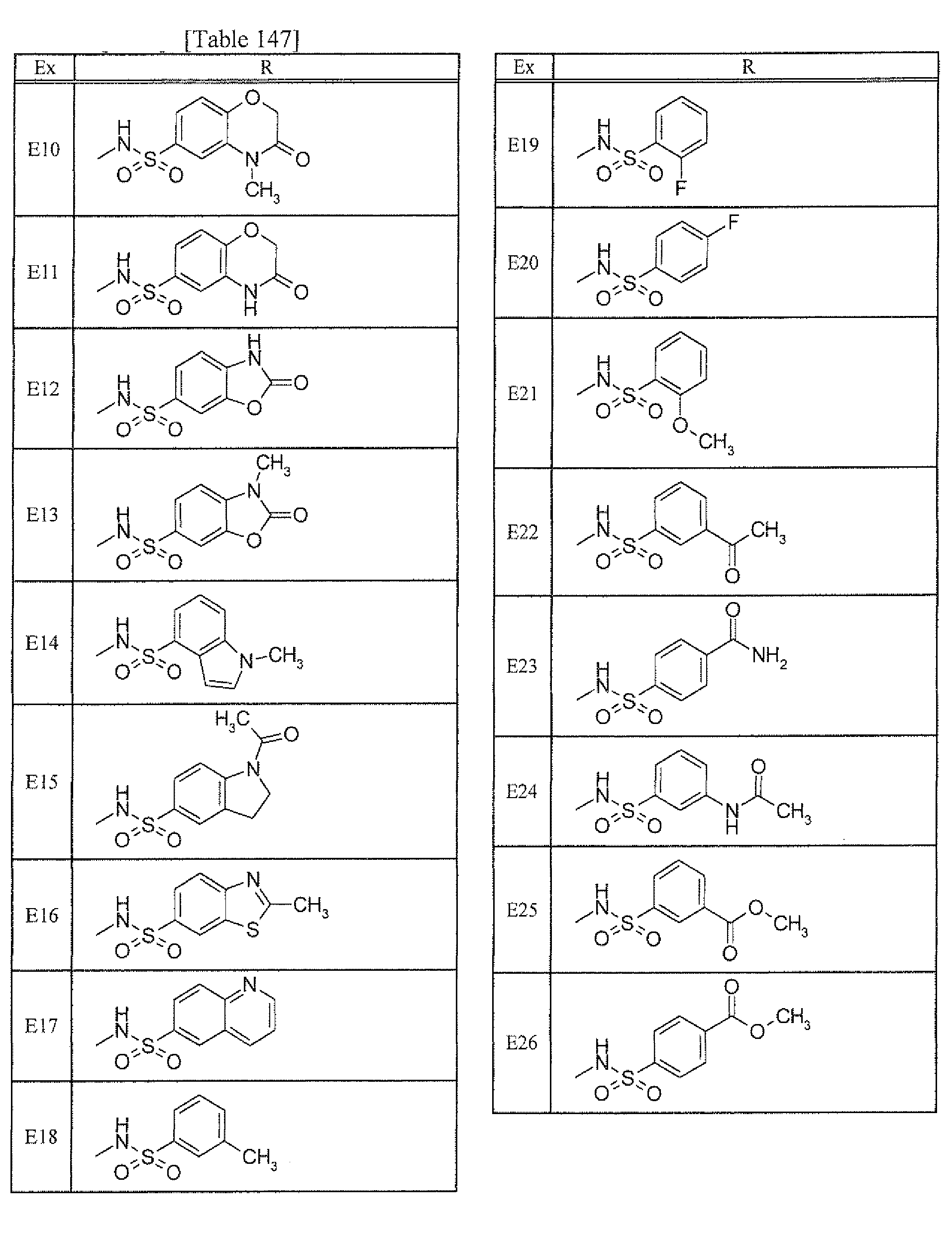

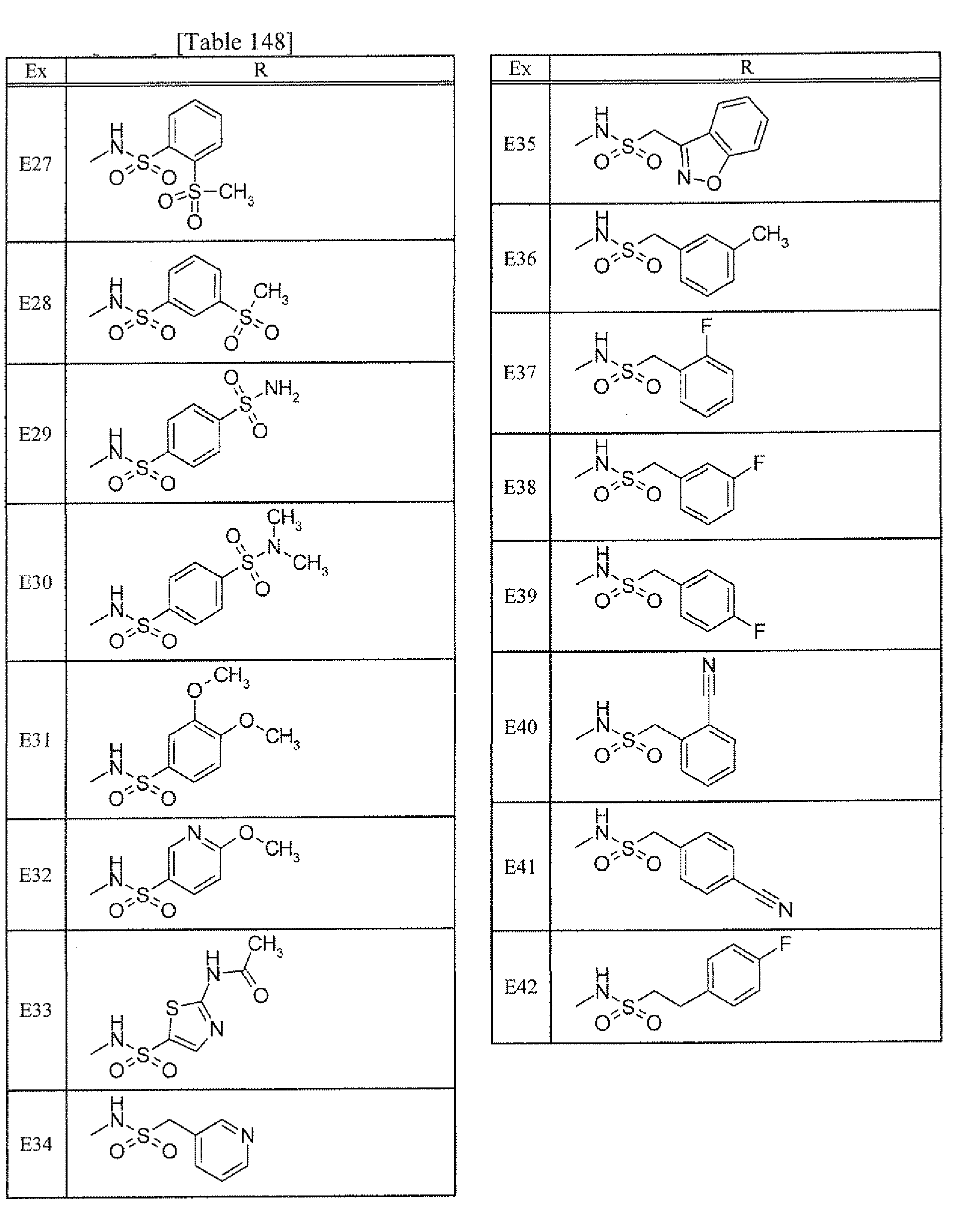

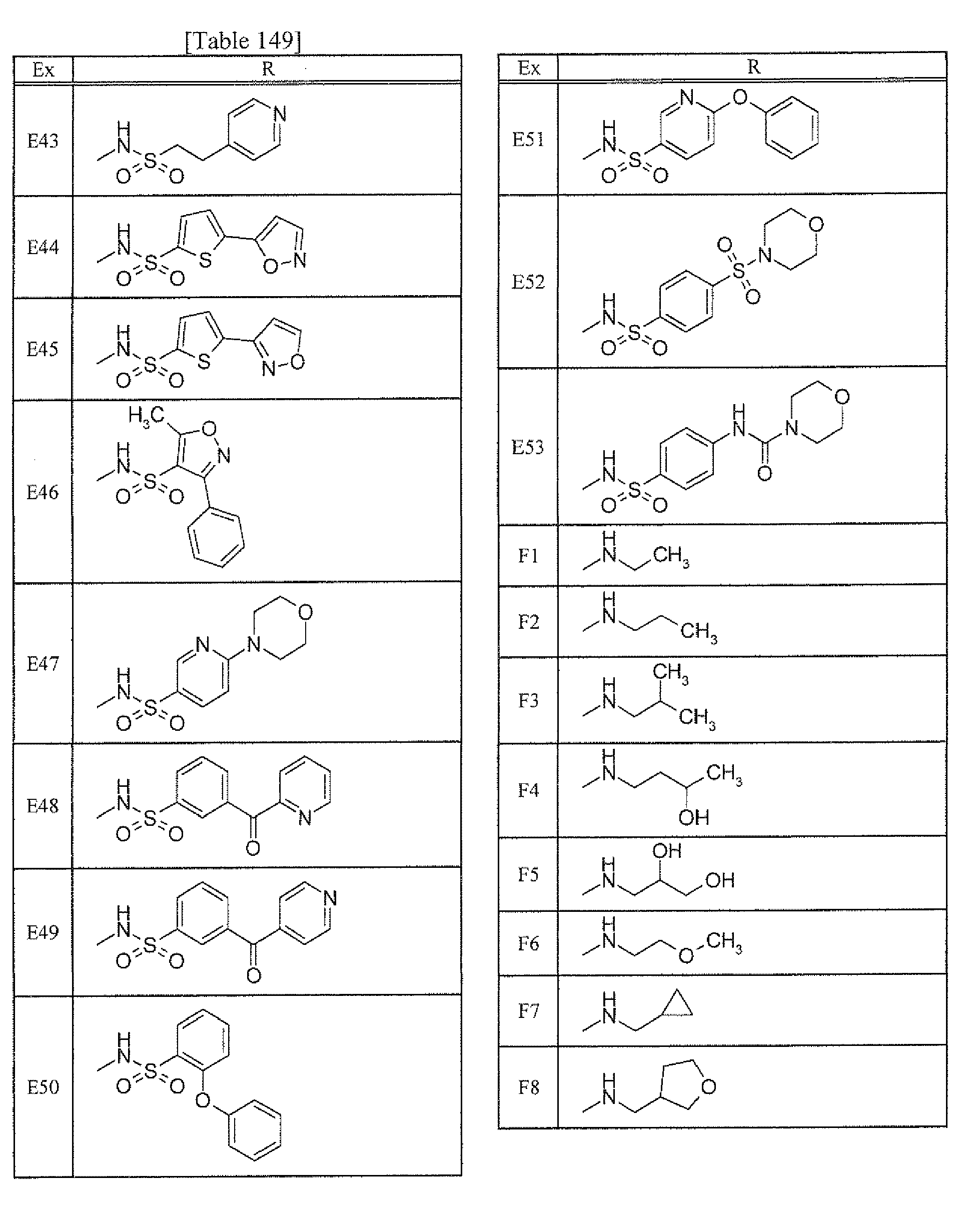

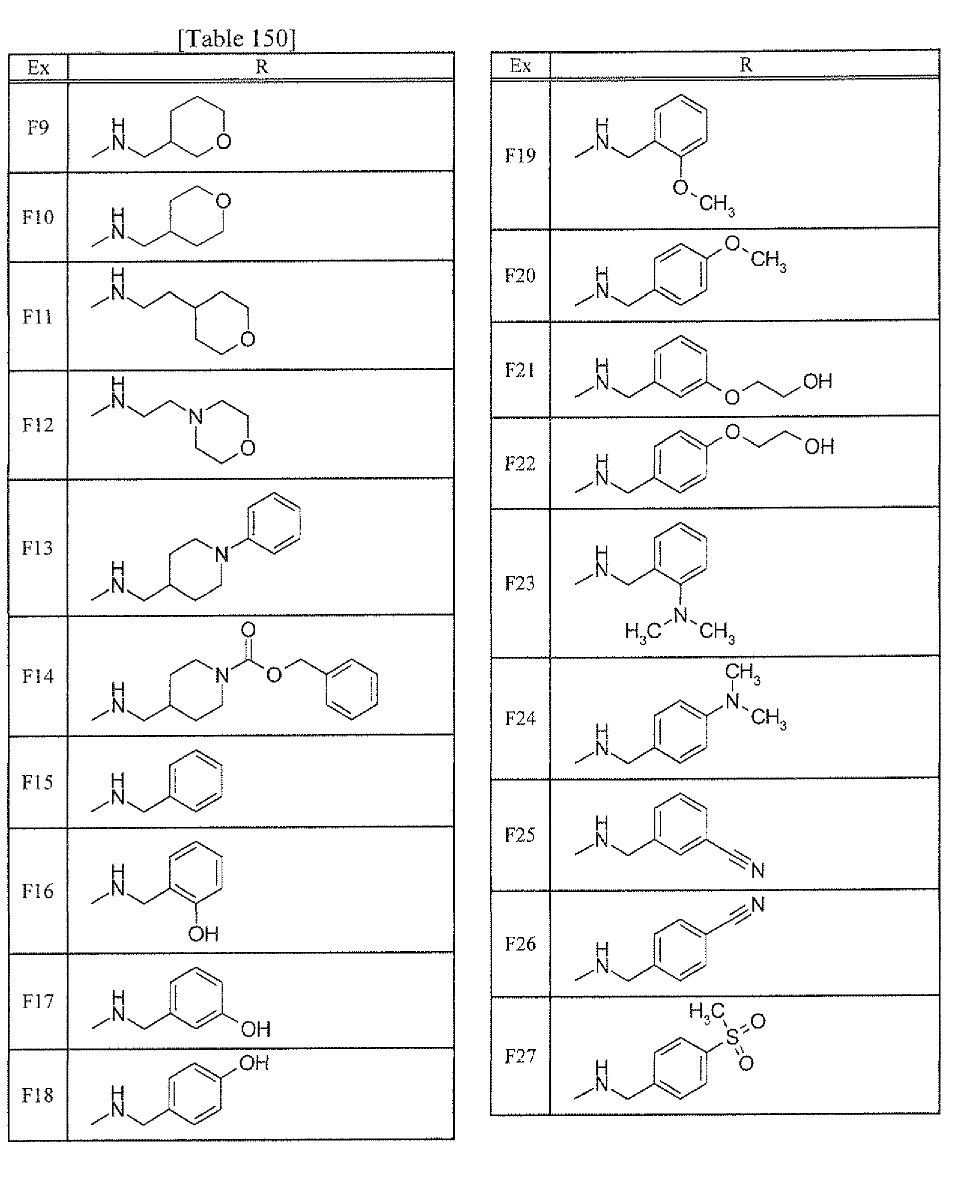

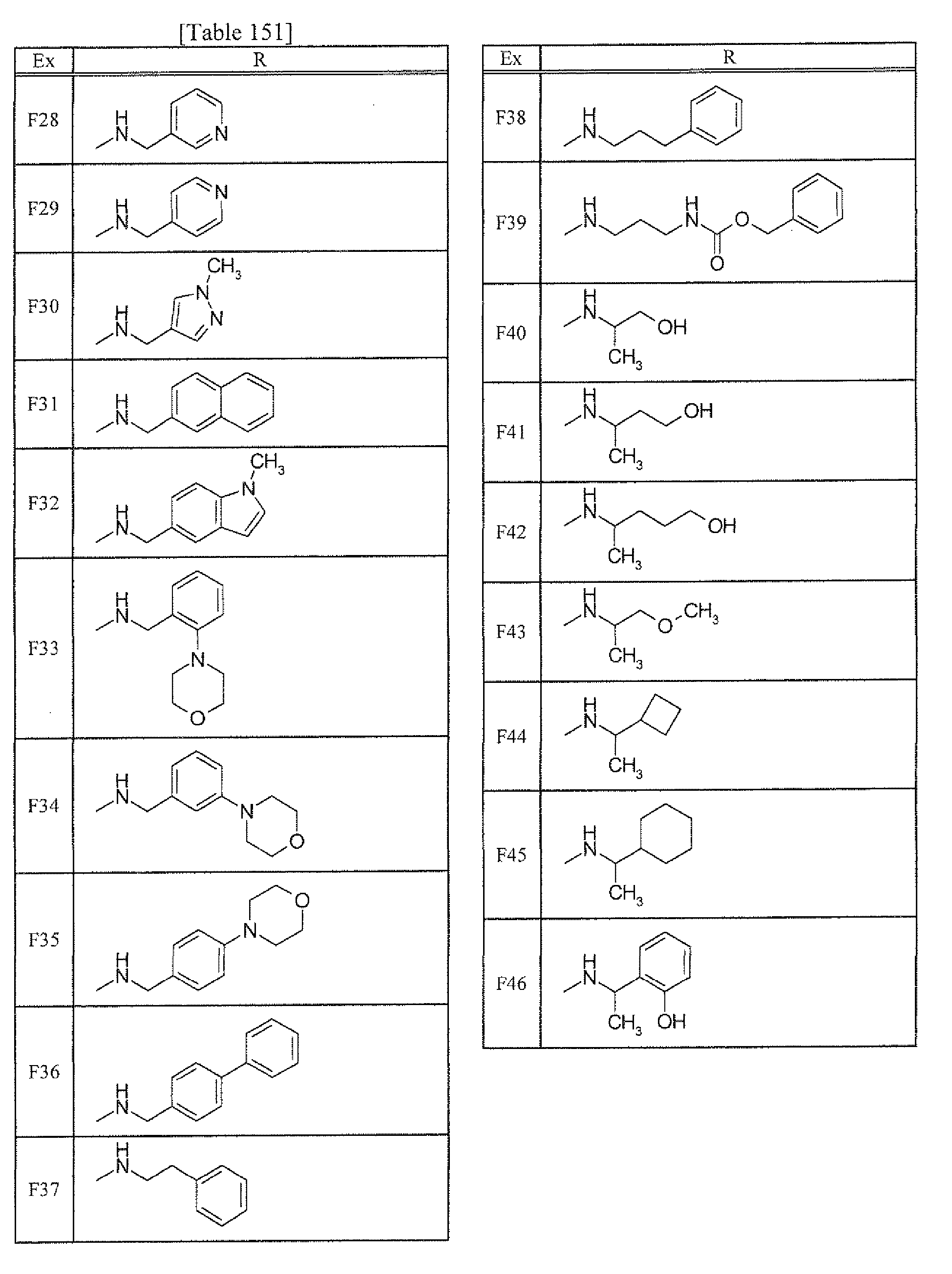

**[Table 152]**

| Ex | R |
|---|---|
| F47 | 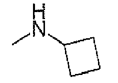 |
| F48 | 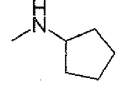 |
| F49 | 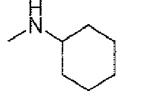 |
| F50 | 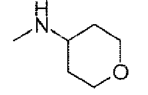 |
| F51 | 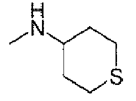 |
| F52 | 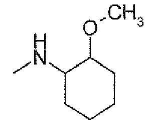 |
| F53 | 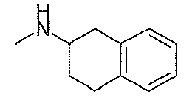 |

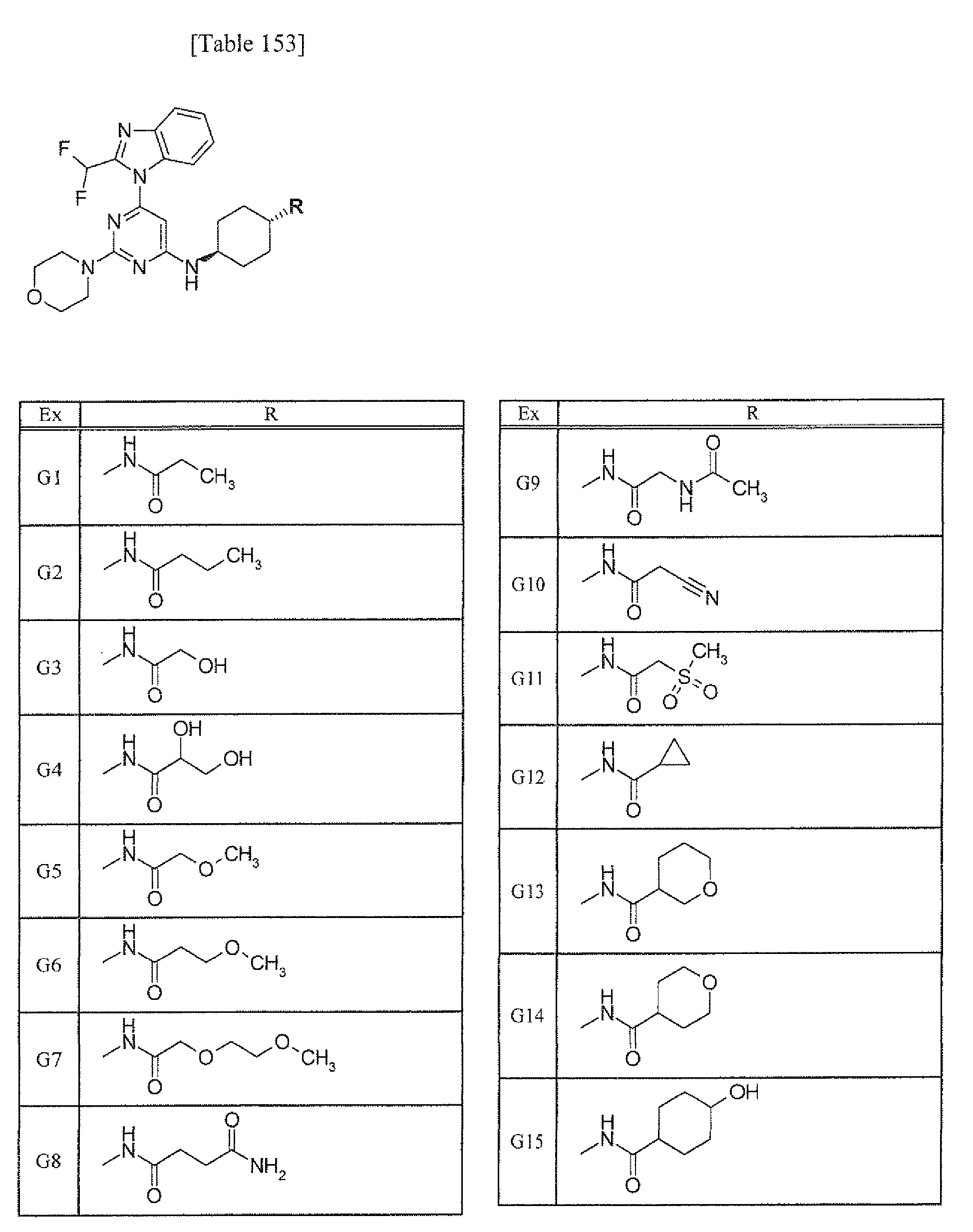

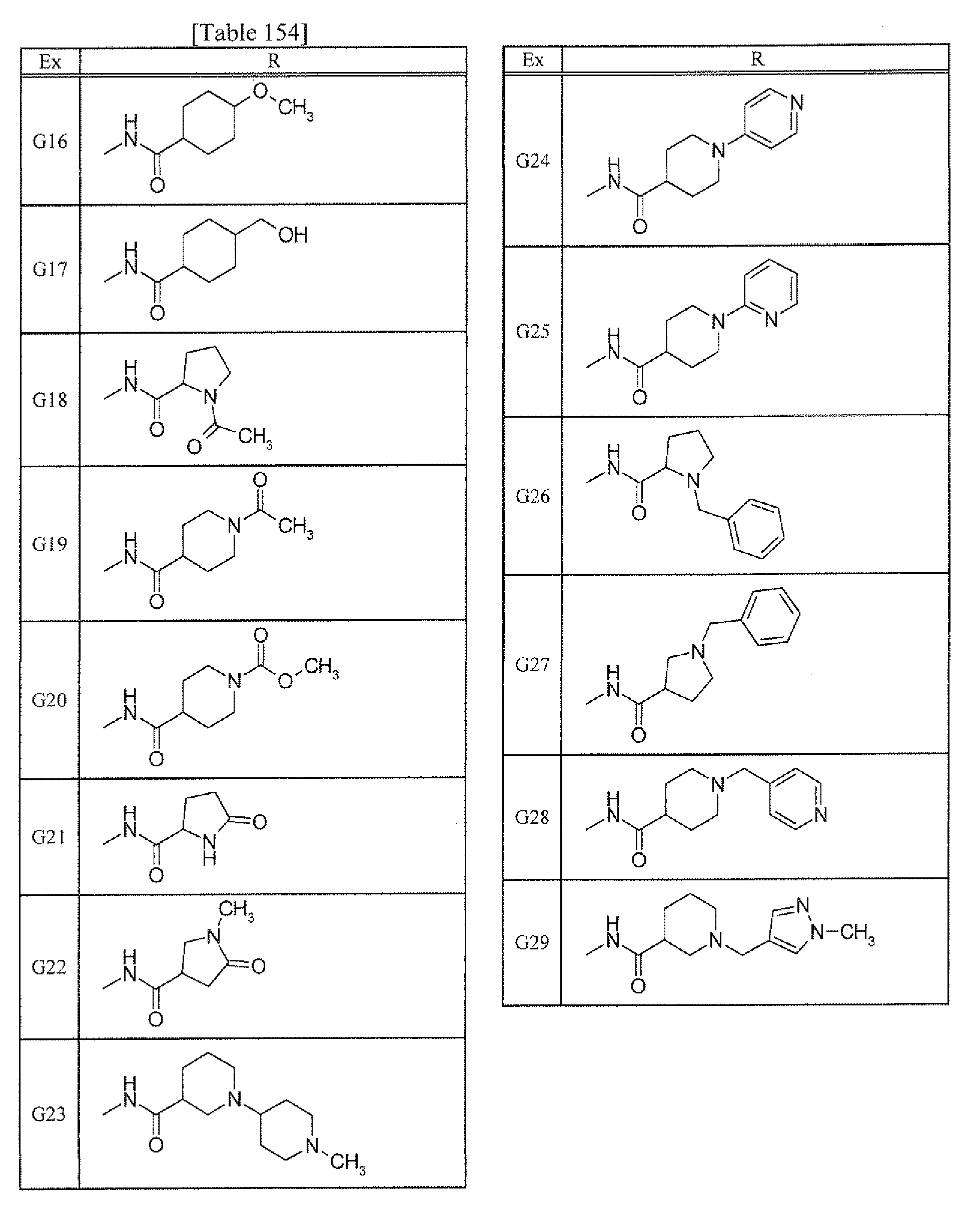

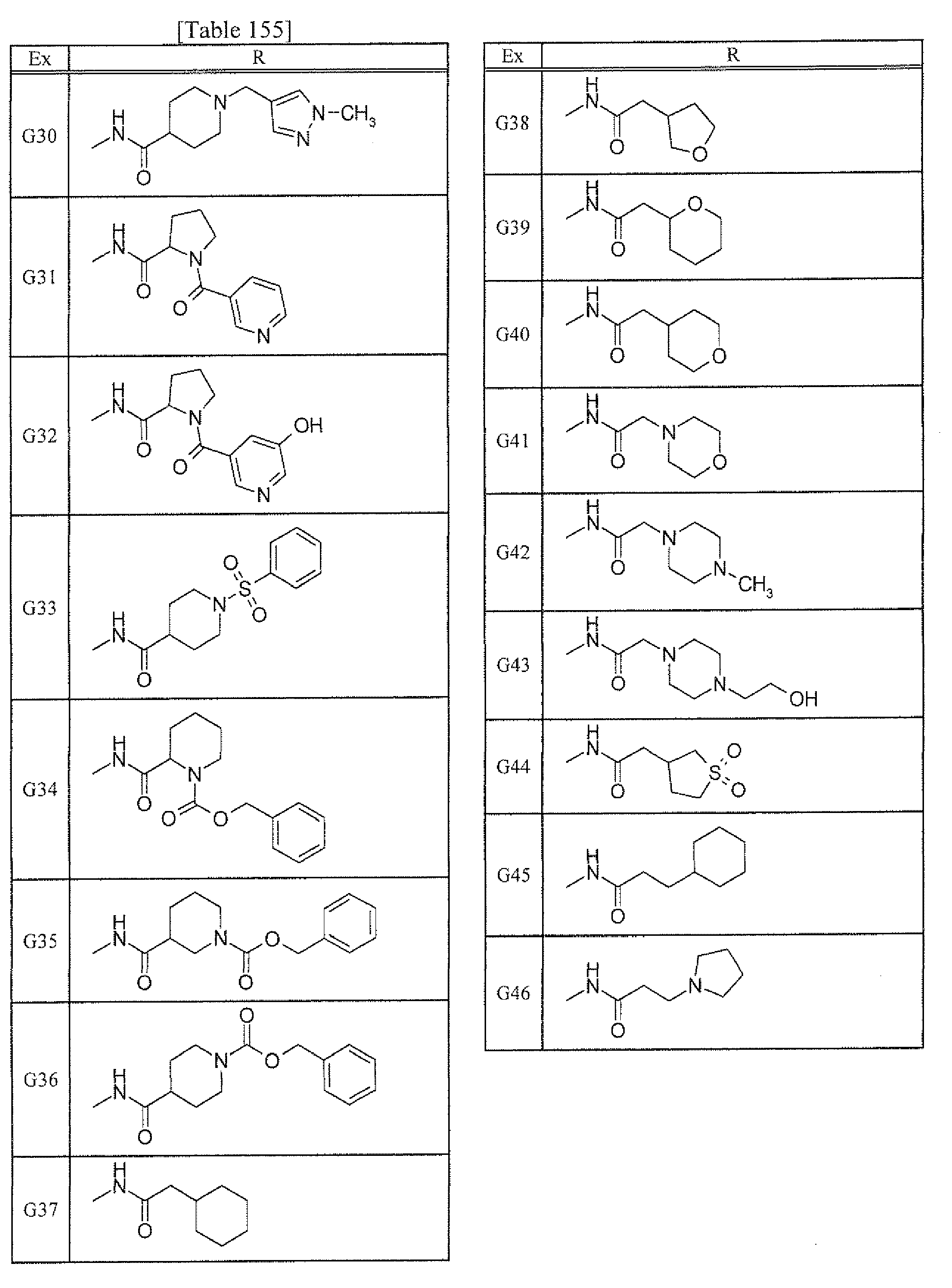

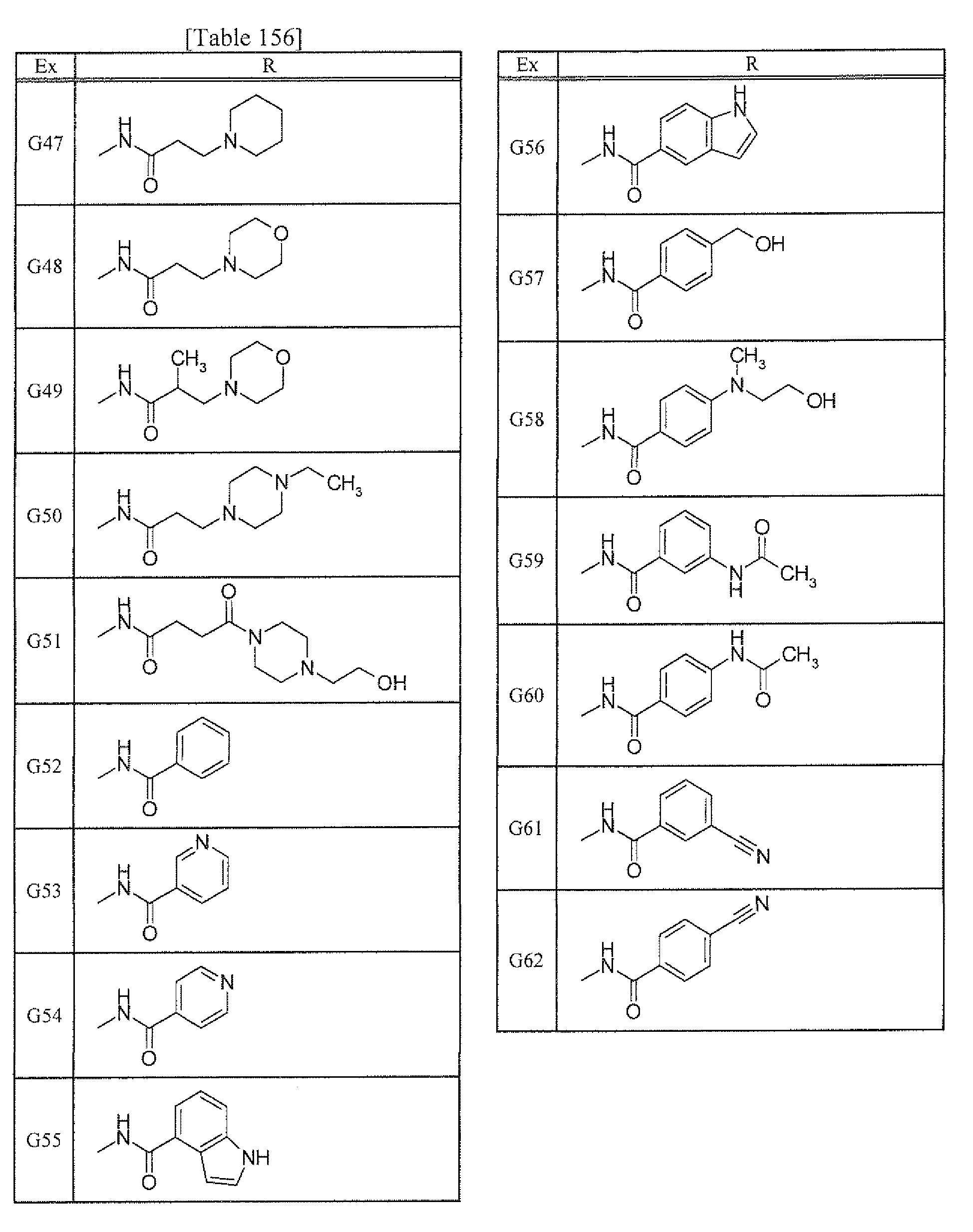

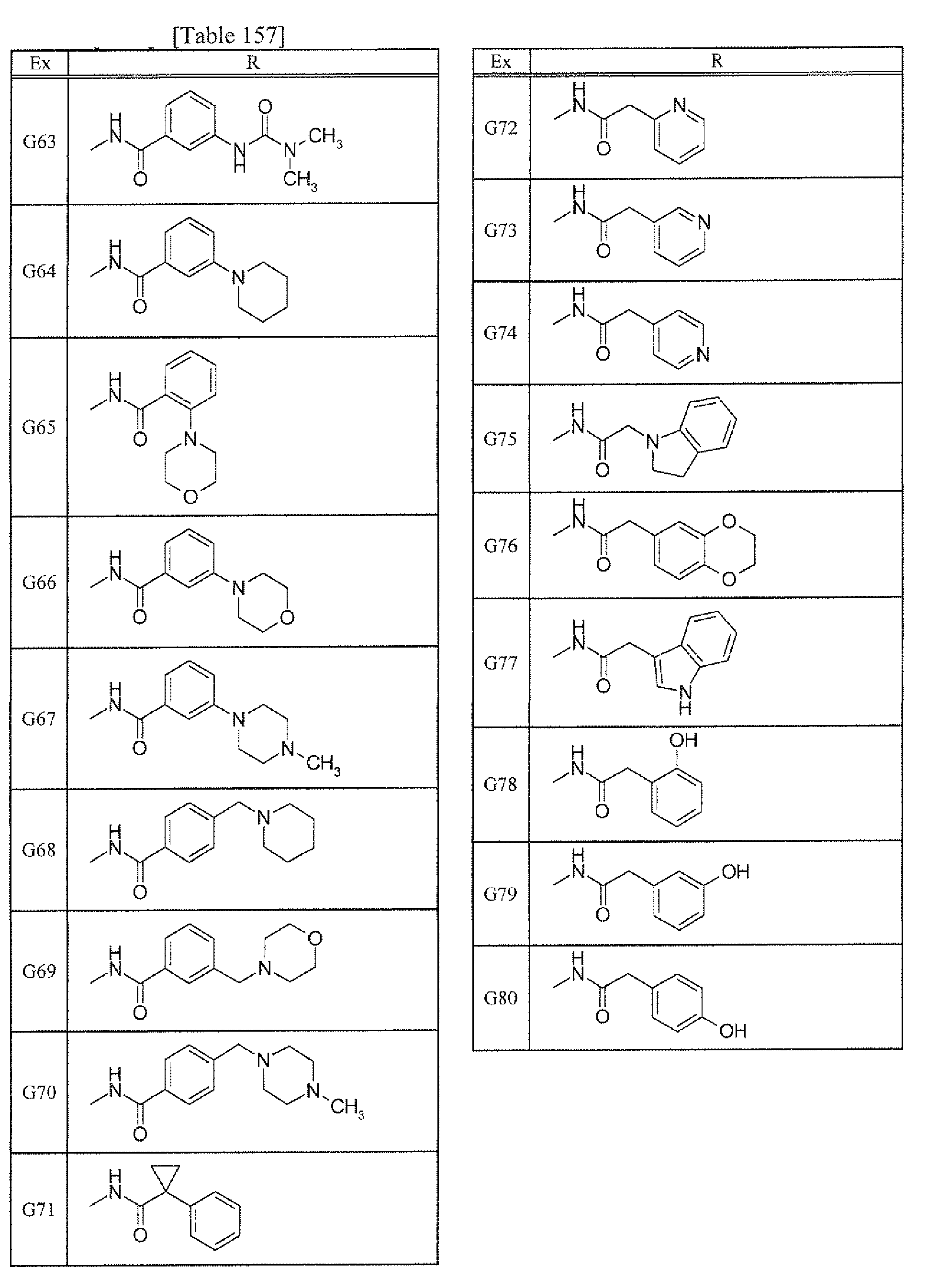

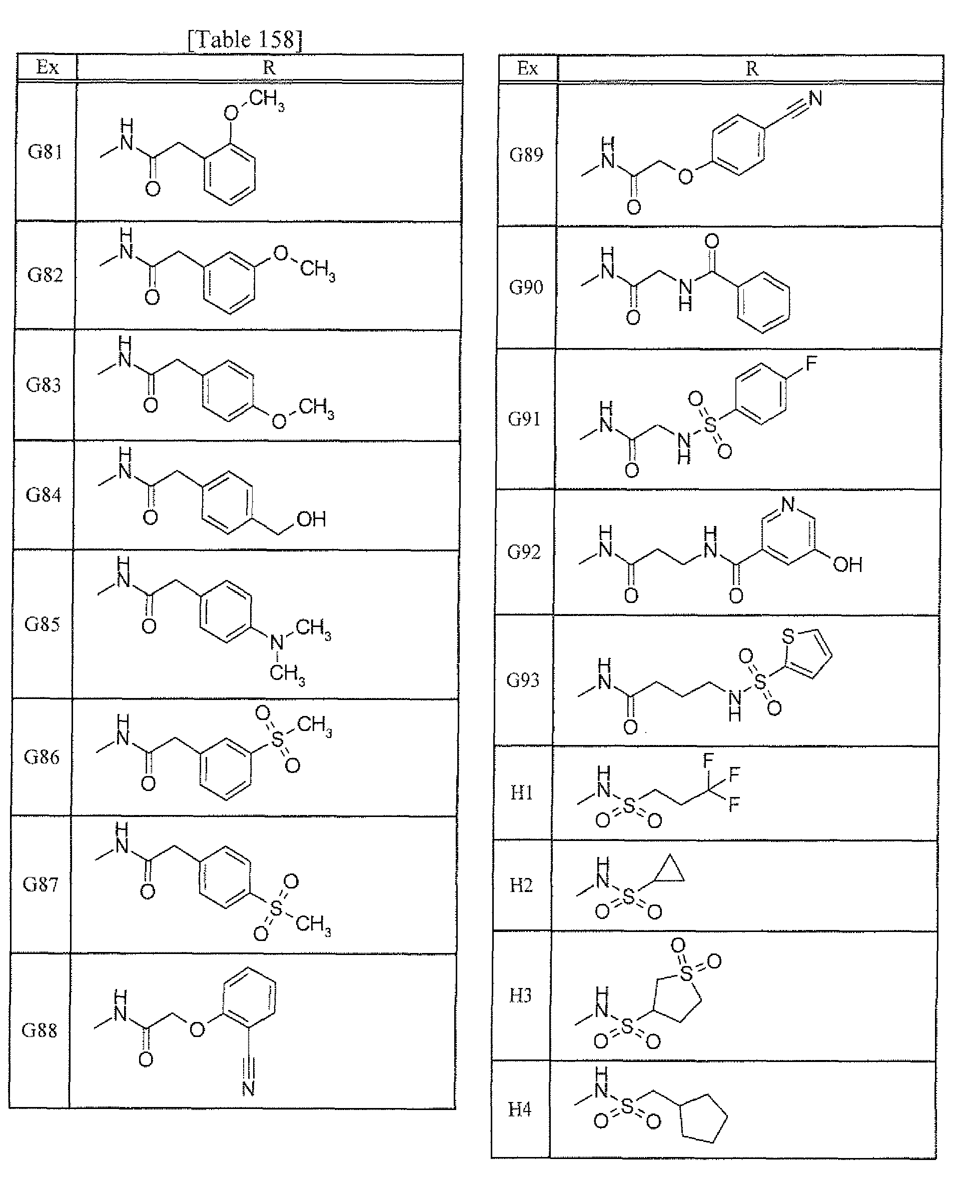

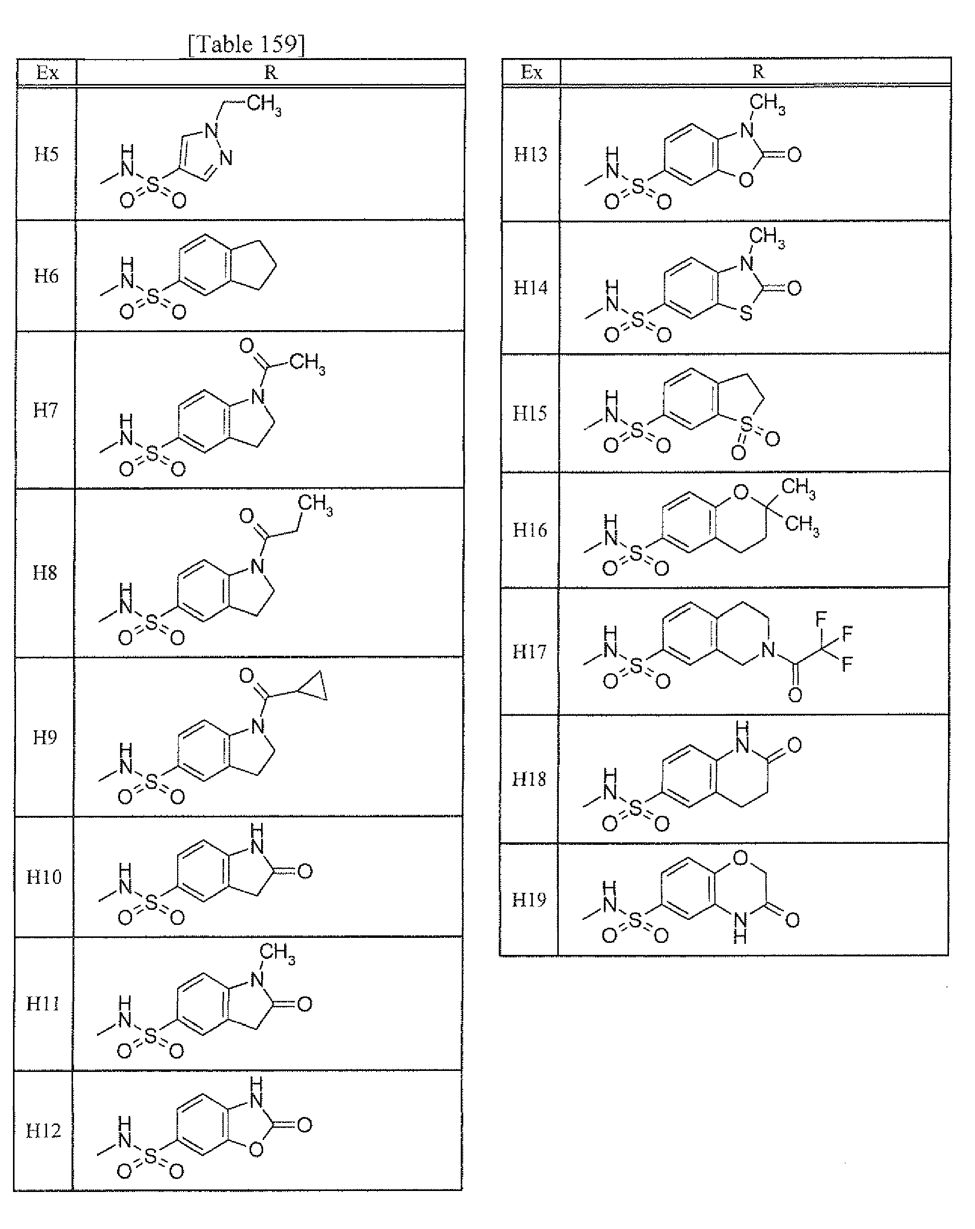

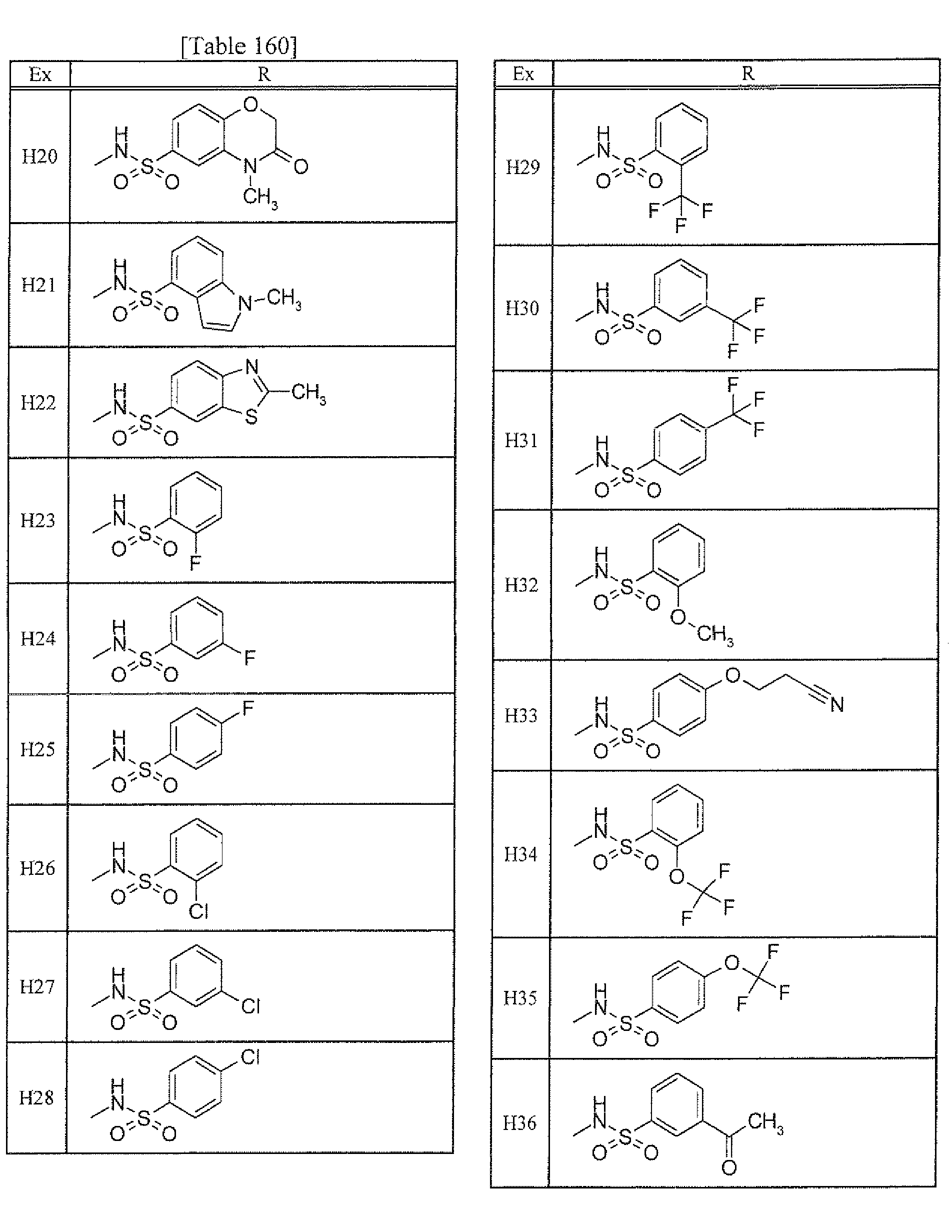

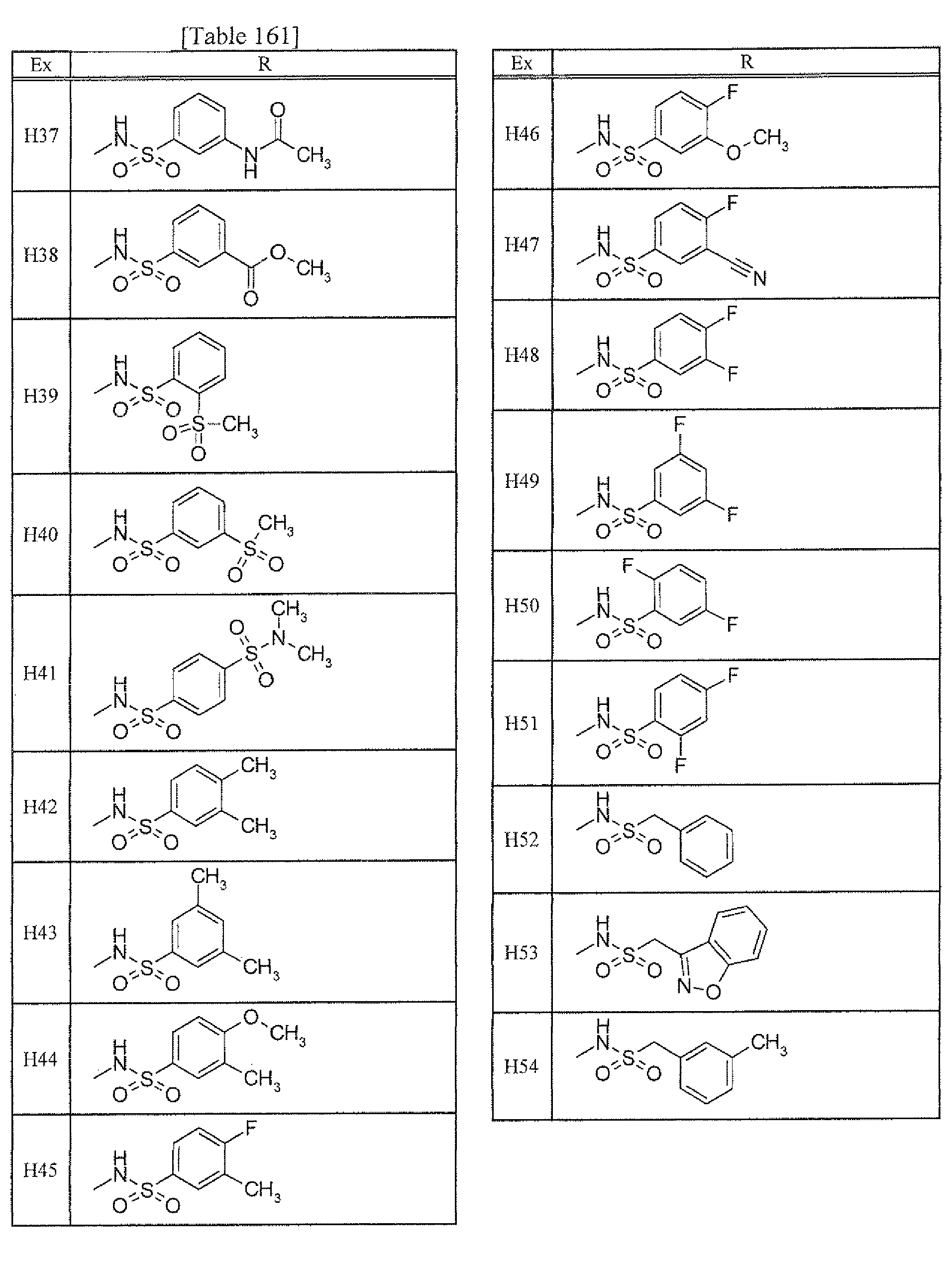

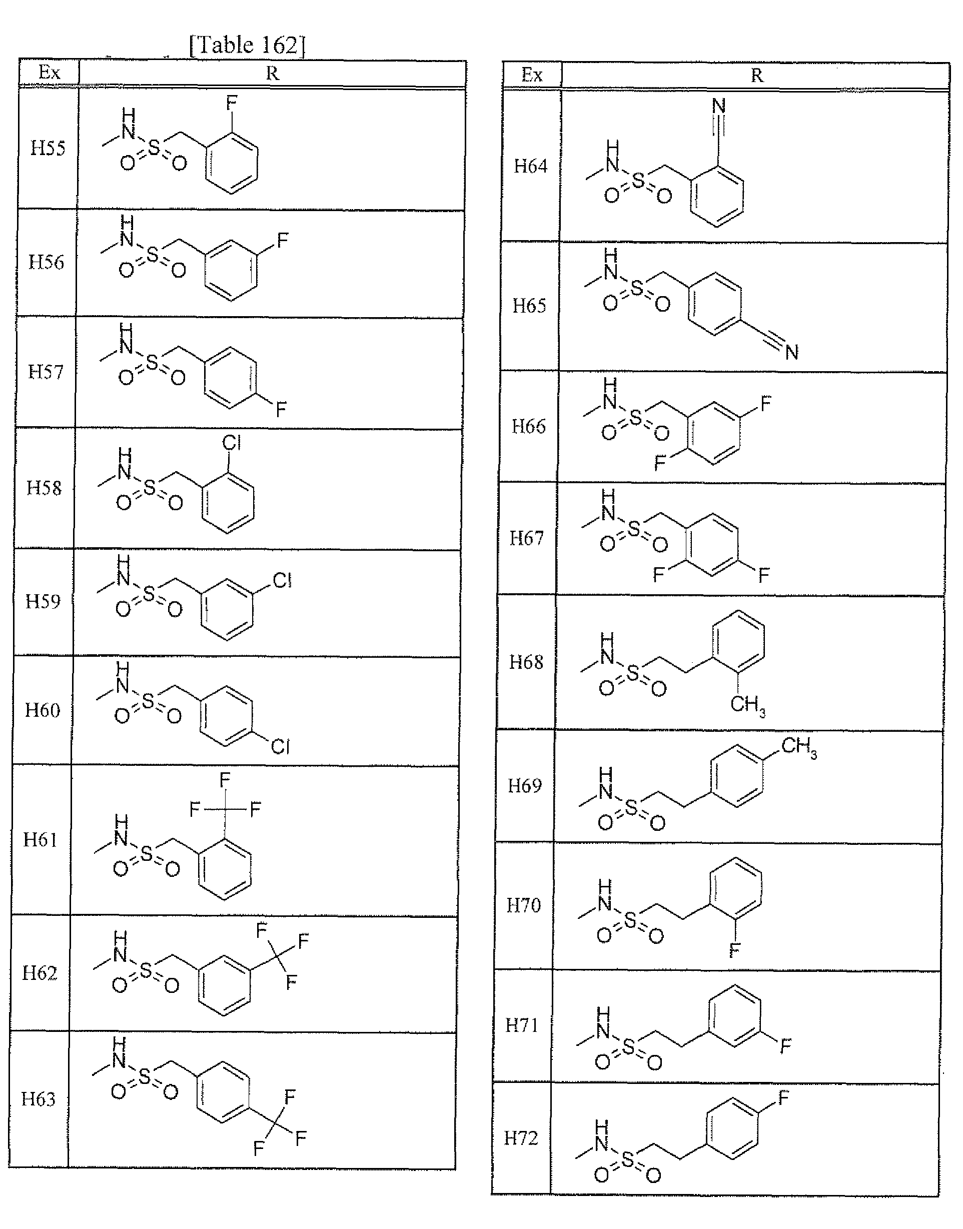

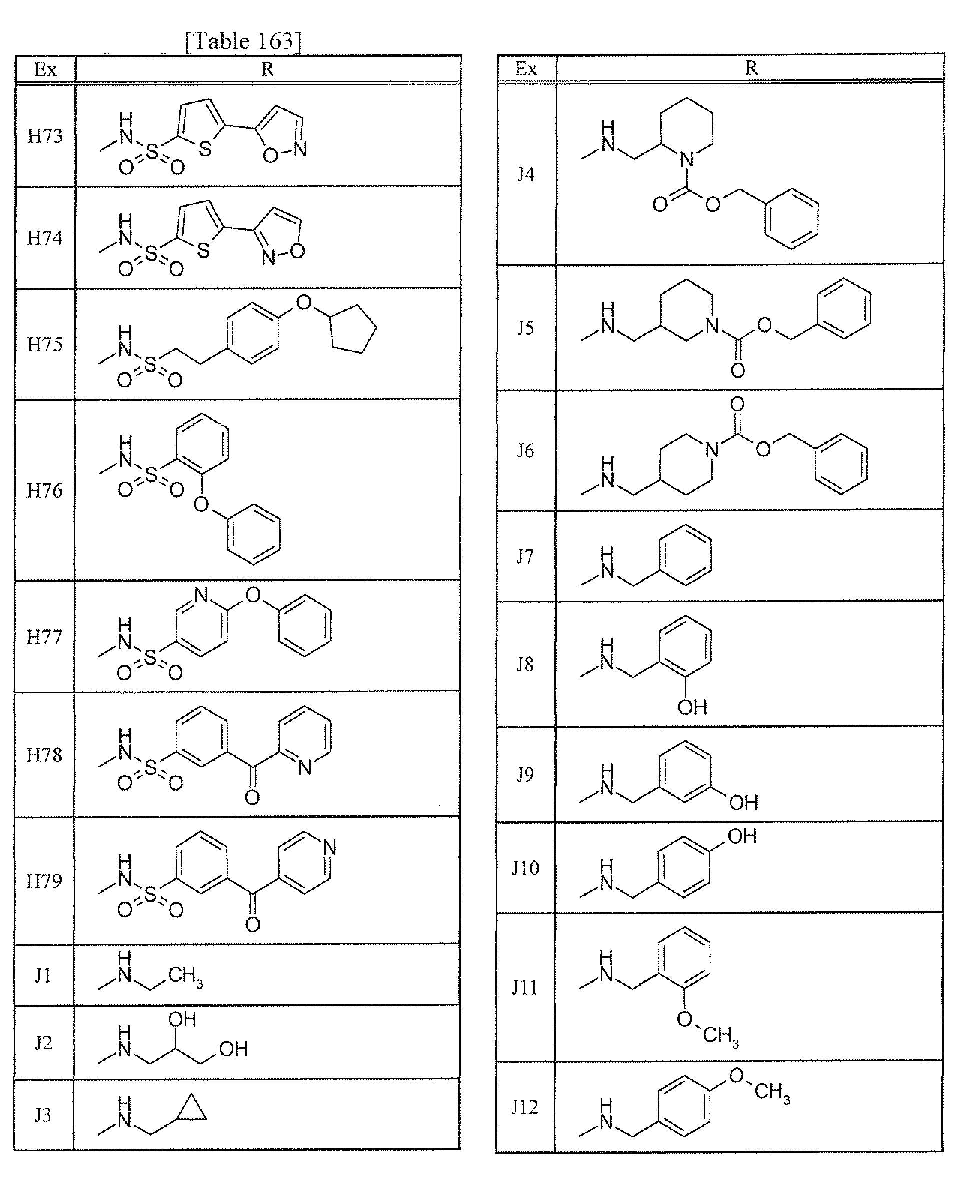

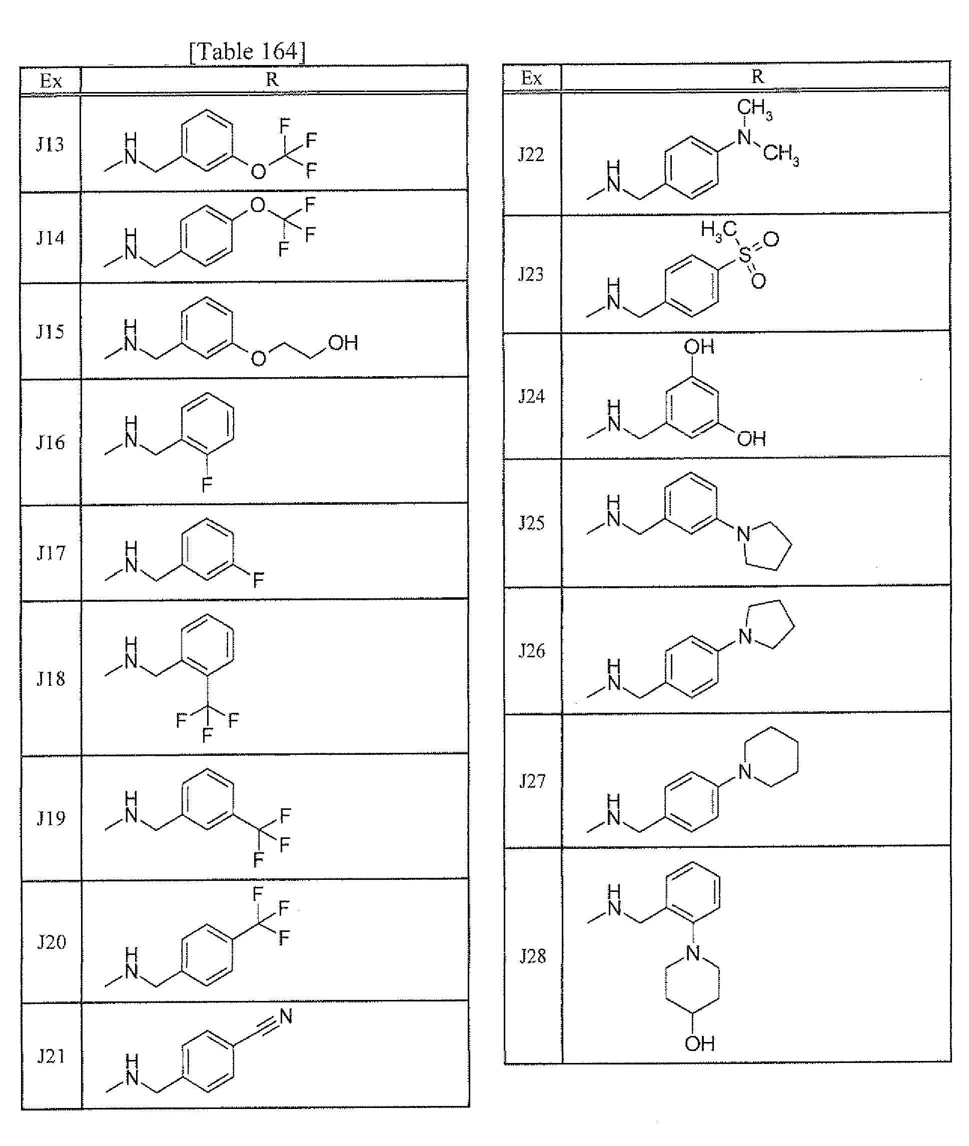

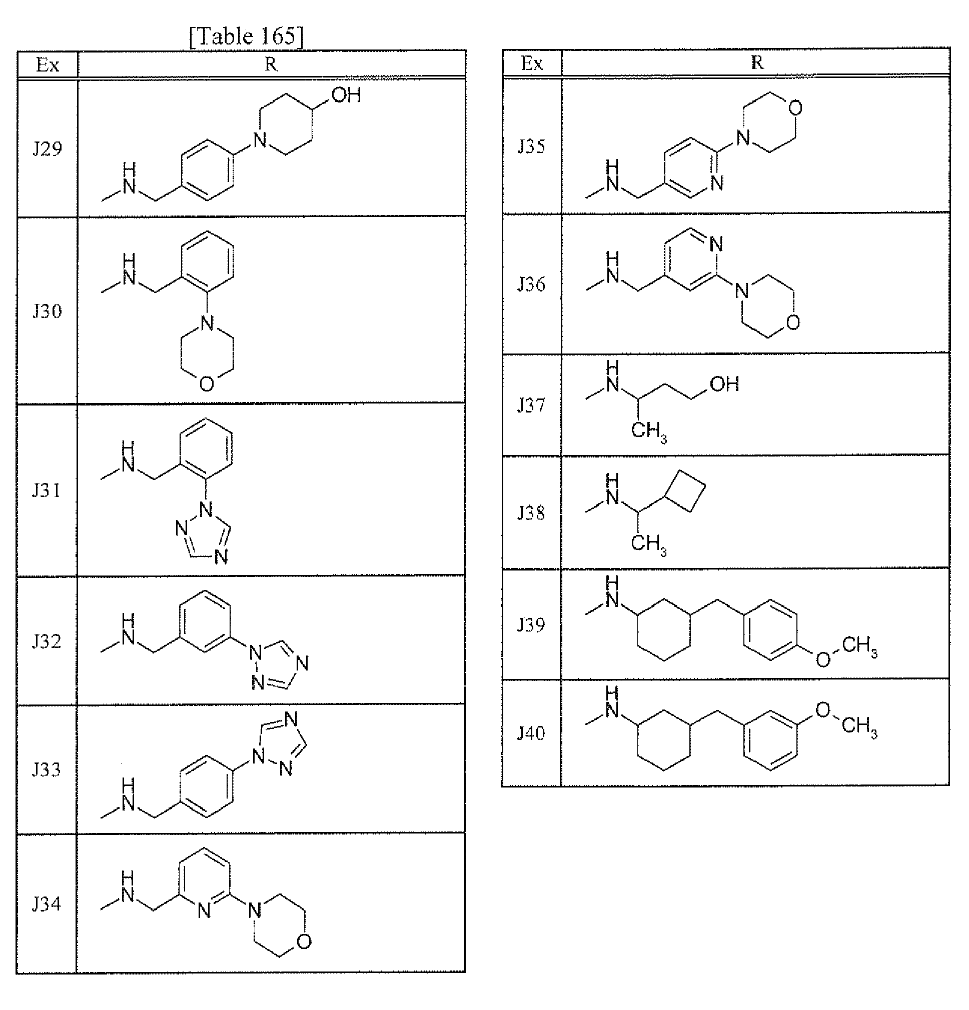

**[Table 166]**

| Ex | ESI+ | RT | | Ex | ESI+ | RT | | Ex | ESI+ | RT |
|---|---|---|---|---|---|---|---|---|---|---|
| A1 | 501 | 3.01 | | A33 | 570 | 2.58 | | A65 | 652 | 3.03 |
| A2 | 515 | 3.16 | | A34 | 572 | 2.49 | | A66 | 684 | 3.37 |
| A3 | 515 | 3.14 | | A35 | 585 | 2.5 | | A67 | 634 | 3.35 |
| A4 | 503 | 2.81 | | A36 | 615 | 2.5 | | A68 | 634 | 3.26 |
| A5 | 517 | 2.99 | | A37 | 620 | 2.79 | | A69 | 648 | 2.68 |
| A6 | 531 | 2.96 | | A38 | 583 | 3.71 | | A70 | 616 | 3.58 |
| A7 | 561 | 3.07 | | A39 | 584 | 2.59 | | A71 | 564 | 2.62 |
| A8 | 533 | 2.73 | | A40 | 586 | 2.51 | | A72 | 564 | 2.57 |
| A9 | 519 | 3.06 | | A41 | 549 | 3.26 | | A73 | 564 | 2.56 |
| A10 | 512 | 2.85 | | A42 | 550 | 3.27 | | A74 | 579 | 3.06 |
| A11 | 530 | 2.49 | | A43 | 550 | 2.9 | | A75 | 579 | 3 |
| A12 | 544 | 2.75 | | A44 | 550 | 2.86 | | A76 | 593 | 3.32 |
| A13 | 544 | 2.85 | | A45 | 588 | 3.17 | | A77 | 593 | 3.28 |
| A14 | 544 | 2.76 | | A46 | 588 | 3.2 | | A78 | 593 | 3.26 |
| A15 | 565 | 2.79 | | A47 | 565 | 3.4 | | A79 | 593 | 3 |
| A16 | 513 | 3.06 | | A48 | 565 | 3.1 | | A80 | 606 | 2.7 |
| A17 | 557 | 3.08 | | A49 | 565 | 3.07 | | A81 | 578 | 2.57 |
| A18 | 557 | 3.01 | | A50 | 579 | 3.36 | | A82 | 584 | 3.04 |
| A19 | 571 | 3.01 | | A51 | 579 | 3.3 | | A83 | 627 | 3.07 |
| A20 | 585 | 3.21 | | A52 | 579 | 3.28 | | A84 | 606 | 3.1 |
| A21 | 585 | 3.1 | | A53 | 579 | 3.05 | | A85 | 656 | 3.21 |
| A22 | 556 | 2.78 | | A54 | 593 | 3.27 | | A86 | 636 | 3.27 |
| A23 | 570 | 2.84 | | A55 | 592 | 2.9 | | A87 | 676 | 3.09 |
| A24 | 570 | 2.58 | | A56 | 592 | 3.11 | | B1 | 537 | 2.88 |
| A25 | 570 | 2.54 | | A57 | 592 | 3.31 | | B2 | 551 | 2.97 |
| A26 | 598 | 2.92 | | A58 | 622 | 3.14 | | B3 | 591 | 2.95 |
| A27 | 614 | 3.13 | | A59 | 607 | 3.32 | | B4 | 549 | 2.89 |
| A28 | 632 | 2.81 | | A60 | 606 | 3.11 | | B5 | 627 | 2.78 |
| A29 | 569 | 3.57 | | A61 | 606 | 3.08 | | B6 | 591 | 3.18 |
| A30 | 557 | 3 | | A62 | 574 | 3.23 | | B7 | 605 | 3.26 |
| A31 | 571 | 3.24 | | A63 | 574 | 3.22 | | B8 | 585 | 3.01 |
| A32 | 571 | 3.05 | | A64 | 635 | 3.13 | | B9 | 589 | 2.8 |

**[Table 167]**

| Ex | ESI+ | RT | | Ex | ESI+ | RT | | Ex | ESI+ | RT |
|---|---|---|---|---|---|---|---|---|---|---|
| B10 | 576 | 2.74 | | B42 | 613 | 3.15 | | C16 | 552 | 2.18 |
| B11 | 643 | 2.99 | | B43 | 614 | 2.55 | | C17 | 540 | 2.23 |
| B12 | 654 | 2.83 | | B44 | 629 | 3.13 | | C18 | 620 | 2.45 |
| B13 | 670 | 2.98 | | B45 | 673 | 3.13 | | C19 | 620 | 2.46 |
| B14 | 656 | 2.92 | | B46 | 661 | 3.26 | | C20 | 641 | 2.69 |
| B15 | 642 | 2.86 | | B47 | 661 | 3.25 | | C21 | 636 | 2.58 |
| B16 | 656 | 2.9 | | B48 | 658 | 2.88 | | C22 | 503 | 2.21 |
| B17 | 636 | 2.96 | | B49 | 666 | 3.2 | | C23 | 517 | 2.23 |
| B18 | 638 | 3 | | B50 | 671 | 2.96 | | C24 | 531 | 2.26 |
| B19 | 638 | 3 | | B51 | 690 | 3.06 | | C25 | 517 | 2.25 |
| B20 | 668 | 2.9 | | B52 | 690 | 3 | | C26 | 517 | 2.3 |
| B21 | 615 | 3 | | B53 | 677 | 3.24 | | C27 | 531 | 2.28 |
| B22 | 627 | 2.97 | | B54 | 677 | 3.26 | | C28 | 527 | 2.48 |
| B23 | 628 | 2.79 | | B55 | 677 | 3.26 | | C29 | 555 | 2.64 |
| B24 | 642 | 2.87 | | B56 | 678 | 3.17 | | C30 | 565 | 2.48 |
| B25 | 643 | 3.05 | | B57 | 734 | 2.97 | | C31 | 527 | 2.48 |
| B26 | 643 | 3.06 | | B58 | 713 | 2.86 | | C32 | 545 | 2.35 |
| B27 | 663 | 3.06 | | C1 | 473 | 2.26 | | C33 | 557 | 2.44 |
| B28 | 663 | 2.85 | | C2 | 501 | 2.4 | | C34 | 603 | 2.7 |
| B29 | 664 | 2.78 | | C3 | 517 | 2.24 | | C35 | 618 | 1.93 |
| B30 | 685 | 2.9 | | C4 | 503 | 2.29 | | D1 | 500 | 2.69 |
| B31 | 699 | 2.99 | | C5 | 529 | 2.26 | | D2 | 514 | 2.8 |
| B32 | 692 | 2.95 | | C6 | 558 | 2.33 | | D3 | 514 | 2.78 |
| B33 | 656 | 2.82 | | C7 | 535 | 2.43 | | D4 | 502 | 2.5 |
| B34 | 645 | 2.95 | | C8 | 551 | 2.33 | | D5 | 516 | 2.68 |
| B35 | 616 | 3.05 | | C9 | 551 | 2.34 | | D6 | 530 | 2.64 |
| B36 | 649 | 2.88 | | C10 | 551 | 2.35 | | D7 | 560 | 2.74 |
| B37 | 599 | 3.07 | | C11 | 565 | 2.47 | | D8 | 532 | 2.43 |
| B38 | 600 | 2.77 | | C12 | 565 | 2.45 | | D9 | 543 | 2.09 |
| B39 | 640 | 3.07 | | C13 | 565 | 2.45 | | D10 | 543 | 2.46 |
| B40 | 624 | 2.97 | | C14 | 595 | 2.34 | | D11 | 543 | 2.55 |
| B41 | 624 | 2.94 | | C15 | 578 | 2.53 | | D12 | 543 | 2.46 |

**[Table 168]**

| Ex | ESI+ | RT | | Ex | ESI+ | RT | | Ex | ESI+ | RT |
|---|---|---|---|---|---|---|---|---|---|---|
| D13 | 511 | 2.53 | | D45 | 583 | 2.17 | | D77 | 563 | 2.45 |
| D14 | 564 | 2.48 | | D46 | 585 | 2.1 | | D78 | 563 | 2.32 |
| D15 | 512 | 2.73 | | D47 | 599 | 2.19 | | D79 | 563 | 2.26 |
| D16 | 554 | 3.03 | | D48 | 548 | 2.89 | | D80 | 601 | 2.85 |
| D17 | 556 | 2.75 | | D49 | 549 | 2.68 | | D81 | 577 | 2.3 |
| D18 | 556 | 2.68 | | D50 | 549 | 2.67 | | D82 | 583 | 2.73 |
| D19 | 570 | 2.68 | | D51 | 587 | 2.8 | | D83 | 626 | 2.7 |
| D20 | 584 | 2.87 | | D52 | 587 | 2.82 | | D84 | 605 | 2.74 |
| D21 | 584 | 2.74 | | D53 | 578 | 2.92 | | D85 | 640 | 2.74 |
| D22 | 569 | 2.15 | | D54 | 578 | 2.7 | | D86 | 655 | 2.82 |
| D23 | 569 | 2.13 | | D55 | 591 | 2.97 | | D87 | 635 | 2.86 |
| D24 | 597 | 2.6 | | D56 | 621 | 2.8 | | D88 | 675 | 2.71 |
| D25 | 613 | 2.77 | | D57 | 606 | 2.95 | | E1 | 522 | 2.53 |
| D26 | 555 | 2.48 | | D58 | 605 | 2.76 | | E2 | 536 | 2.62 |
| D27 | 569 | 2.53 | | D59 | 605 | 2.74 | | E3 | 590 | 2.72 |
| D28 | 652 | 1.8 | | D60 | 573 | 2.86 | | E4 | 548 | 2.64 |
| D29 | 631 | 2.36 | | D61 | 573 | 2.85 | | E5 | 626 | 2.52 |
| D30 | 646 | 2.15 | | D62 | 634 | 2.78 | | E6 | 590 | 2.98 |
| D31 | 646 | 2.59 | | D63 | 651 | 2.71 | | E7 | 588 | 2.54 |
| D32 | 695 | 2.87 | | D64 | 633 | 3 | | E8 | 624 | 3 |
| D33 | 568 | 3.11 | | D65 | 633 | 2.92 | | E9 | 653 | 2.59 |
| D34 | 556 | 2.68 | | D66 | 633 | 2.9 | | E10 | 669 | 2.76 |
| D35 | 570 | 2.87 | | D67 | 647 | 2.29 | | E11 | 655 | 2.69 |
| D36 | 570 | 2.72 | | D68 | 588 | 3.15 | | E12 | 641 | 2.62 |
| D37 | 555 | 2.13 | | D69 | 578 | 2.83 | | E13 | 655 | 2.67 |
| D38 | 569 | 2.18 | | D70 | 578 | 2.69 | | Z14 | 637 | 2.79 |
| D39 | 571 | 2.15 | | D71 | 578 | 2.65 | | E15 | 667 | 2.66 |
| D40 | 584 | 2.1 | | D72 | 592 | 2.93 | | E16 | 655 | 2.83 |
| D41 | 614 | 2.1 | | D73 | 592 | 2.89 | | E17 | 635 | 2.73 |
| D42 | 604 | 2.53 | | D74 | 592 | 2.88 | | E18 | 598 | 2.88 |
| D43 | 619 | 2.5 | | D75 | 592 | 2.66 | | E19 | 602 | 2.79 |
| D44 | 582 | 3.21 | | D76 | 605 | 2.58 | | E20 | 602 | 2.84 |

**[Table 169]**

| Ex | ESI+ | RT | | Ex | ESI+ | RT | | Ex | ESI+ | RT |
|---|---|---|---|---|---|---|---|---|---|---|
| E21 | 614 | 2.78 | | E53 | 712 | 2.63 | | F32 | 587 | 2.65 |
| E22 | 626 | 2.74 | | F1 | 472 | 2.24 | | F33 | 619 | 2.53 |
| E23 | 627 | 2.54 | | F2 | 486 | 2.34 | | F34 | 619 | 2.51 |
| E24 | 641 | 2.64 | | F3 | 500 | 2.42 | | F35 | 619 | 2.51 |
| E25 | 642 | 2.84 | | F4 | 516 | 2.22 | | F36 | 610 | 2.85 |
| E26 | 642 | 2.85 | | F5 | 518 | 2.15 | | F37 | 548 | 2.62 |
| E27 | 662 | 2.84 | | F6 | 502 | 2.27 | | F38 | 562 | 2.7 |
| E28 | 662 | 2.62 | | F7 | 498 | 2.34 | | F39 | 635 | 2.71 |
| E29 | 663 | 2.54 | | F8 | 528 | 2.26 | | F40 | 502 | 2.2 |
| E30 | 691 | 2.73 | | F9 | 542 | 2.35 | | F41 | 516 | 2.2 |
| E31 | 644 | 2.72 | | F10 | 542 | 2.29 | | F42 | 530 | 2.27 |
| E32 | 615 | 2.82 | | F11 | 556 | 2.35 | | F43 | 516 | 2.34 |
| E33 | 648 | 2.64 | | F12 | 557 | 2.39 | | F44 | 526 | 2.28 |
| E34 | 599 | 2.51 | | F13 | 617 | 2.5 | | F45 | 554 | 2.42 |
| E35 | 639 | 2.85 | | F14 | 675 | 2.77 | | F46 | 564 | 2.24 |
| E36 | 612 | 2.95 | | F15 | 534 | 2.47 | | F47 | 498 | 2.15 |
| E37 | 616 | 2.86 | | F16 | 550 | 2.46 | | F48 | 512 | 2.19 |
| E38 | 616 | 2.86 | | F17 | 550 | 2.38 | | F49 | 526 | 2.26 |
| E39 | 616 | 2.86 | | F18 | 550 | 2.38 | | F50 | 528 | 2.02 |
| E40 | 623 | 2.75 | | F19 | 564 | 2.58 | | F51 | 544 | 2.12 |
| E41 | 623 | 2.72 | | F20 | 564 | 2.51 | | F52 | 556 | 2.24 |
| E42 | 630 | 2.94 | | F21 | 594 | 2.39 | | F53 | 574 | 2.42 |
| E43 | 613 | 2.28 | | F22 | 594 | 2.37 | | G1 | 500 | 2.82 |
| E44 | 657 | 2.66 | | F23 | 577 | 2.64 | | G2 | 514 | 2.93 |
| E45 | 657 | 2.66 | | F24 | 577 | 2.64 | | G3 | 502 | 2.65 |
| E46 | 665 | 3 | | F25 | 559 | 2.38 | | G4 | 532 | 2.58 |
| E47 | 670 | 2.72 | | F26 | 559 | 2.35 | | G5 | 516 | 2.82 |
| E48 | 689 | 2.84 | | F27 | 612 | 2.27 | | G6 | 530 | 2.78 |
| E49 | 689 | 2.79 | | F28 | 535 | 2.21 | | G7 | 560 | 2.87 |
| E50 | 676 | 3.06 | | F29 | 535 | 2.21 | | G8 | 543 | 2.6 |
| E51 | 677 | 2.98 | | F30 | 538 | 2.24 | | G9 | 543 | 2.61 |
| E52 | 733 | 2.75 | | F31 | 584 | 2.7 | | G10 | 511 | 2.67 |

**[Table 170]**

| Ex | ESI+ | RT | | Ex | ESI+ | RT | | Ex | ESI+ | RT |
|---|---|---|---|---|---|---|---|---|---|---|
| G11 | 564 | 2.62 | | G43 | 614 | 2.25 | | G75 | 603 | 3.18 |
| G12 | 512 | 2.86 | | G44 | 604 | 2.66 | | G76 | 620 | 2.96 |
| G13 | 556 | 2.89 | | G45 | 582 | 3.31 | | G77 | 601 | 2.97 |
| G14 | 556 | 2.82 | | G46 | 569 | 2.29 | | G78 | 578 | 2.95 |
| G15 | 570 | 2.81 | | G47 | 583 | 2.31 | | G79 | 578 | 2.82 |
| G16 | 584 | 2.99 | | G48 | 585 | 2.26 | | G80 | 578 | 2.78 |
| G17 | 584 | 2.87 | | G49 | 599 | 2.32 | | G81 | 592 | 3.04 |
| G18 | 583 | 2.74 | | G50 | 612 | 2.27 | | G82 | 592 | 3 |
| G19 | 597 | 2.73 | | G51 | 656 | 2.29 | | G83 | 592 | 2.99 |
| G20 | 613 | 2.89 | | G52 | 548 | 3.01 | | G84 | 592 | 2.78 |
| G21 | 555 | 2.62 | | G53 | 549 | 2.82 | | G85 | 605 | 2.76 |
| G22 | 569 | 2.67 | | G54 | 549 | 2.81 | | G86 | 640 | 2.75 |
| G23 | 652 | 1.99 | | G55 | 587 | 2.92 | | G87 | 640 | 2.73 |
| G24 | 632 | 2.38 | | G56 | 587 | 2.94 | | G88 | 603 | 3.01 |
| G25 | 632 | 2.42 | | G57 | 578 | 2.83 | | G89 | 603 | 2.94 |
| G26 | 631 | 2.51 | | G58 | 621 | 2.92 | | G90 | 605 | 2.86 |
| G27 | 631 | 2.48 | | G59 | 605 | 2.88 | | G91 | 659 | 2.89 |
| G28 | 646 | 2.28 | | G60 | 605 | 2.87 | | G92 | 636 | 2.68 |
| G29 | 649 | 2.32 | | G61 | 573 | 2.98 | | G93 | 675 | 2.83 |
| G30 | 649 | 2.3 | | G62 | 573 | 2.97 | | H1 | 604 | 2.82 |
| G31 | 646 | 2.72 | | G63 | 634 | 2.91 | | H2 | 548 | 2.62 |
| G32 | 662 | 2.69 | | G64 | 631 | 3.09 | | H3 | 626 | 2.52 |
| G33 | 695 | 2.97 | | G65 | 633 | 3.1 | | H4 | 590 | 2.99 |
| G34 | 689 | 3.23 | | G66 | 633 | 3.03 | | H5 | 602 | 2.6 |
| G35 | 689 | 3.18 | | G67 | 646 | 2.46 | | H6 | 624 | 3.02 |
| G36 | 689 | 3.13 | | G68 | 645 | 2.44 | | H7 | 667 | 2.65 |
| G37 | 568 | 3.21 | | G69 | 647 | 2.42 | | H8 | 681 | 2.76 |
| G38 | 556 | 2.81 | | G70 | 660 | 2.46 | | H9 | 693 | 2.79 |
| G39 | 570 | 2.99 | | G71 | 588 | 3.26 | | H10 | 639 | 2.53 |
| G40 | 570 | 2.85 | | G72 | 563 | 2.63 | | H11 | 653 | 2.61 |
| G41 | 571 | 2.3 | | G73 | 563 | 2.5 | | H12 | 641 | 2.62 |
| G42 | 584 | 2.25 | | G74 | 563 | 2.41 | | H13 | 655 | 2.66 |

**[Table 171]**

| Ex | ESI+ | RT | | Ex | ESI+ | RT | | Ex | ESI+ | RT |
|---|---|---|---|---|---|---|---|---|---|---|
| H14 | 671 | 2.77 | | H46 | 632 | 2.88 | | H78 | 689 | 2.86 |
| H15 | 674 | 2.58 | | H47 | 627 | 2.83 | | H79 | 689 | 2.79 |
| H16 | 668 | 3.05 | | H48 | 620 | 2.94 | | J1 | 472 | 2.32 |
| H17 | 735 | 2.85 | | H49 | 620 | 2.97 | | J2 | 518 | 2.25 |
| H18 | 653 | 2.58 | | H50 | 620 | 2.87 | | J3 | 498 | 2.41 |
| H19 | 655 | 2.68 | | H51 | 620 | 2.86 | | J4 | 675 | 2.77 |
| H20 | 669 | 2.75 | | H52 | 598 | 2.86 | | J5 | 675 | 2.74 |
| H21 | 637 | 2.78 | | H53 | 639 | 2.87 | | J6 | 675 | 2.74 |
| H22 | 655 | 2.82 | | H54 | 612 | 2.96 | | J7 | 534 | 2.51 |
| H23 | 602 | 2.79 | | H55 | 616 | 2.88 | | J8 | 550 | 2.5 |
| H24 | 602 | 2.79 | | H56 | 616 | 2.87 | | J9 | 550 | 2.43 |
| H25 | 602 | 2.85 | | H57 | 616 | 2.87 | | J10 | 550 | 2.41 |
| H26 | 618 | 2.88 | | H58 | 632 | 2.96 | | J11 | 564 | 2.58 |
| H27 | 618 | 2.97 | | H59 | 632 | 2.97 | | J12 | 564 | 2.58 |
| H28 | 618 | 2.98 | | H60 | 632 | 2.98 | | J13 | 618 | 2.7 |
| H29 | 652 | 2.9 | | H61 | 666 | 3 | | J14 | 618 | 2.66 |
| H30 | 652 | 3 | | H62 | 666 | 2.99 | | J 15 | 594 | 2.43 |
| H31 | 652 | 3.02 | | H63 | 666 | 3 | | J16 | 552 | 2.51 |
| H32 | 614 | 2.78 | | H64 | 623 | 2.74 | | J17 | 552 | 2.54 |
| H33 | 653 | 2.62 | | H65 | 623 | 2.71 | | J18 | 602 | 2.67 |
| H34 | 668 | 2.96 | | H66 | 634 | 2.88 | | J19 | 602 | 2.66 |
| H35 | 668 | 3.04 | | H67 | 634 | 2.91 | | J20 | 602 | 2.64 |
| H36 | 626 | 2.74 | | H68 | 626 | 3.08 | | J21 | 559 | 2.41 |
| H37 | 641 | 2.64 | | H69 | 626 | 3.09 | | J22 | 577 | 2.61 |
| H38 | 642 | 2.85 | | H70 | 630 | 2.99 | | J23 | 612 | 2.33 |
| H39 | 662 | 2.88 | | H71 | 630 | 2.98 | | J24 | 566 | 2.35 |
| H40 | 662 | 2.61 | | H72 | 630 | 2.96 | | J25 | 603 | 2.74 |
| H41 | 691 | 2.73 | | H73 | 657 | 2.66 | | J26 | 603 | 2.78 |
| H42 | 612 | 2.97 | | H74 | 657 | 2.66 | | J27 | 617 | 2.82 |
| H43 | 612 | 3 | | H75 | 668 | 3.16 | | J28 | 633 | 2.49 |
| H44 | 628 | 2.92 | | H76 | 676 | 3.09 | | J29 | 633 | 2.51 |
| H45 | 616 | 2.97 | | H77 | 677 | 3 | | J30 | 619 | 2.56 |

**[Table 172]**

| Ex | ESI+ | RT |
|---|---|---|
| J31 | 601 | 2.38 |
| J32 | 601 | 2.4 |
| J33 | 601 | 2.4 |
| J34 | 620 | 2.54 |
| J35 | 620 | 2.41 |
| J36 | 620 | 2.41 |
| J37 | 516 | 2.31 |
| J38 | 526 | 2.57 |
| J39 | 646 | 2.86 |
| J40 | 646 | 2.84 |

### Industrial Applicability

Since the compound which is an active ingredient of the pharmaceutical of the present invention has a PI3Kδ-selective inhibitory action, and/or an IL-2 production inhibitory action, and/or a B cell proliferation inhibitory action (including an activation inhibitory action), and an excellent pharmacological action based thereon, the pharmaceutical composition of the present invention can be used as an agent for preventing or treating rejection in the transplantation of various organs, allergy diseases (asthma, atopic dermatitis, or the like), autoimmune diseases (rheumatoid arthritis, psoriasis, ulcerative colitis, Crohn's disease, systemic lupus erythematosus, or the like), hematologic tumor (leukemia or the like), and the like.

## Claims

1. A compound of the formula (I) or a salt thereof: 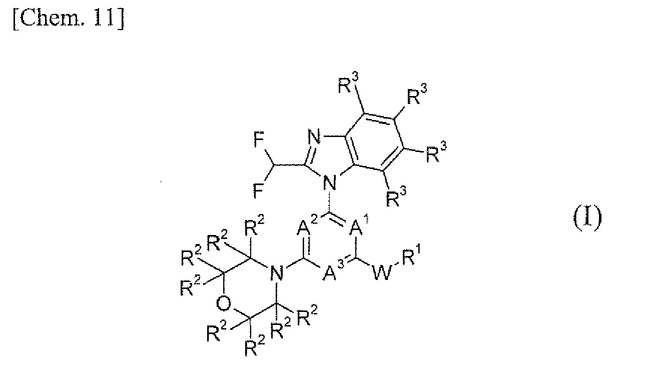 [wherein
A¹, A², and A³: the same as or different from each other, each representing CH or N, provided that at least two of A¹ to A³ are N;
W: NH or O;
R¹: 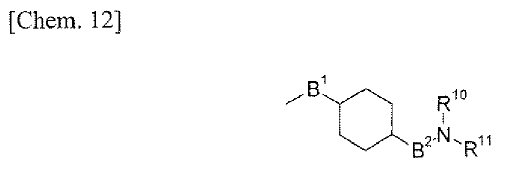 or 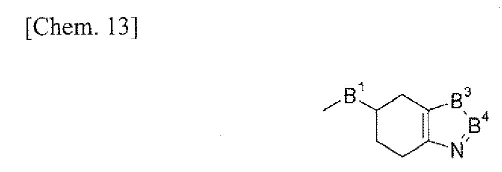
R²: the same as or different from each other, each representing H, or lower alkyl which may be substituted with halogen or -OH;
R³: the same as or different from each other, each representing H or halogen;
B¹: a bond or C₁₋₄ alkylene;
B²: a bond or C₁₋₄ alkylene;
B³: O, S, or NR⁰;
B⁴: CR¹² or N;
R⁰: the same as or different from each other, each representing H or lower alkyl;
R¹⁰: H; lower alkyl, in which the lower alkyl may be substituted with halogen, -C(O)O-lower alkyl, -OH, or -O-lower alkyl; lower alkenyl; lower alkynyl; -lower alkylene-phenyl, in which the phenyl may be substituted with -O-lower alkyl; -lower alkylene-O-lower alkylene-phenyl;
R¹¹: H, R¹⁰⁰, -C(O)R¹⁰¹, -C(O)OR¹⁰², -C(O) NR¹⁰³R¹⁰⁴, or -S(O)₂R¹⁰⁵;
or R¹⁰ and R¹¹ are combined with the N to which they are bonded to form a 3- to 8-membered monocyclic hetero ring group containing 1 to 4 hetero atoms selected from O, S, and N, and the monocyclic hetero ring may be substituted with lower alkyl which may be substituted with halogen, OH, -O-lower alkyl, or a hetero ring, oxo, -C(O)O-lower alkyl, N(R⁰)₂, halogen, -CN, -OH, -O-lower alkyl, -O-C(O)-lower alkyl, -O-lower alkylene-phenyl, or a hetero ring group;
R¹²: R⁰ or amino;
R¹⁰⁰: lower alkyl, in which the lower alkyl may be substituted with group(s) selected from halogen, -C(O)N(R⁰)₂, -C(O)O-lower alkyl, -CN, -OH, -O-lower alkyl, -O-lower alkylene-phenyl, -NHC(O)O-lower alkylene-phenyl, and -S(O)₂-lower alkyl; lower alkenyl; lower alkynyl;
-X-cycloalkyl, in which the cycloalkyl may be substituted with group(s) selected from lower alkyl, phenyl, -lower alkylene-O-lower alkyl, -O-lower alkyl, and -lower alkylene-phenyl, in which the phenyl may be substituted with -O-lower alkyl;
-X-aryl, in which the aryl may be substituted with group(s) selected from lower alkyl, halogen, halogeno-lower alkyl, phenyl, -CN, -OH, -O-lower alkyl, -O-halogeno-lower alkyl, -O-lower alkylene-OH, -O-lower alkylene-phenyl, -S(O)₂-lower alkyl, -N(R⁰)₂, pyrrolidinyl, piperidyl which may be substituted with OH, morpholinyl, and triazolyl; or
-X-hetero ring group, in which the hetero ring group may be substituted with group(s) selected from lower alkyl, halogen, halogeno-lower alkyl, phenyl, morpholinyl, -C(O)O-lower alkylene-phenyl, -OH, -lower alkylene-phenyl, and -lower alkylene-OH;
R¹⁰¹: lower alkyl, in which the lower alkyl may be substituted with group(s) selected from halogen; -C(O)N(R⁰)₂; -C(O)-piperazinyl, in which the piperazinyl may be substituted with -lower alkylene-OH; -CN; -OH; -O-lower alkyl; -O-lower alkylene-phenyl; -O-lower alkylene-O-lower alkyl; -O-(phenyl which may be substituted with -CN); -S(O)₂-lower alkyl; -S(O)₂-phenyl; -N(R⁰)₂; -N(R⁰)-lower alkyl, in which the lower alkyl may be substituted with -O-lower alkyl; -NH-phenyl; -NHC(O)-lower alkyl; -NHC(O)-phenyl; -NHC(O)-(pyridyl which may be substituted with -OH); -N(R⁰)C(O)O-lower alkyl; -NHC(O)O-lower alkylene-phenyl; -NHS(O)₂-phenyl, in which the phenyl may be substituted with group(s) selected from lower alkyl and halogen; and -NHS(O)₂-thienyl;
-X-cycloalkyl, in which the cycloalkyl may be substituted with group(s) selected from phenyl, -CN, -OH, -O-lower alkyl, and -lower alkylene-OH;
-X-phenyl, in which the phenyl may be substituted with group(s) selected from lower alkyl, halogen, halogeno-lower alkyl, -C(O)O-lower alkyl, -CN, -OH, -O-lower alkyl, -N(R⁰)₂, -N(R⁰)-lower alkylene-OH, -N(-lower alkylene-OH)₂, -NHC(O)-lower alkyl, -N(R⁰)C(O)N(R⁰)₂, -S(O)₂-lower alkyl, -S(O)₂N(lower alkyl)₂, -lower alkylene-OH, -lower alkylene-O-lower alkyl, -X-piperidyl, -X-morpholinyl, and -X-(piperazinyl which may be substituted with lower alkyl);
-X-hetero ring group, in which the hetero ring group may be substituted with group(s) selected from lower alkyl, halogen, -OH, halogeno-lower alkyl, phenyl, -C(O)O-lower alkyl, -C(O)O-lower alkylene-phenyl, -C(O)-(pyridyl which may be substituted with -OH), -C(O)-lower alkyl, oxo, -N(R⁰)₂, -N(R⁰)C(O)O-lower alkyl, -S(O)₂-phenyl, piperidyl which may be substituted with lower alkyl, -X-pyridyl, -slower alkylene-phenyl, -slower alkylene-OH, -lower alkylene-O-lower alkyl, and -lower alkylene-(pyrazolyl which may be substituted with lower alkyl); or
-C(O)N(R⁰)₂;
R¹⁰²: lower alkyl;
R¹⁰³: H or lower alkyl;
R¹⁰⁴: lower alkyl, in which the lower alkyl may be substituted with group(s) selected from -CN, -OH, -O-lower alkyl, or -N(R⁰)₂
-X-phenyl, in which the phenyl may be substituted with group(s) selected from lower alkyl, halogen, halogeno-lower alkyl, -CN, -O-lower alkyl, -O-halogeno-lower alkyl, and -N(R⁰)₂; or
-X-hetero ring group;
or R¹⁰³ and R¹⁰⁴ are combined with the N to which they are bonded to form a morpholinyl group;
R¹⁰⁵: lower alkyl, in which the lower alkyl may be substituted with group(s) selected from halogen, and -O-phenyl, in which the phenyl may be substituted with -O-lower alkyl; or hetero ring group;
lower alkenyl;
-X-cycloalkyl;
-X-aryl, in which the aryl may be substituted with group(s) selected from lower alkyl, halogen, halogeno-lower alkyl, phenyl, -C(O)O-lower
alkyl, -C(O)N(R⁰)₂, -CN, -C(O)-lower alkyl, -C(O)-pyridyl, -O-lower alkyl, -O-halogeno-lower alkyl, -O-cycloalkyl, -O-phenyl, -O-lower alkylene-CN, -X-NHC(O)-lower alkyl, -NHC(O)-morpholinyl, -S(O)₂-lower alkyl, -N(R⁰)C(O)N(R⁰)₂, -S(O)₂N(R⁰)₂,
and -S(O)₂-morpholinyl;
-X-hetero ring group, in which the hetero ring group may be substituted with lower alkyl, halogen, halogeno-lower alkyl, phenyl, -C(O)-lower alkyl, -C(O)-halogeno-lower alkyl, -C(O)-cyloalkyl, -O-lower alkyl, -O-phenyl, oxo, -NHC(O)-lower alkyl, morpholinyl, and isoxozolyl; or
-N(R⁰)₂; and
X: a bond or lower alkylene].

2. The compound according to claim 1 or a salt thereof, wherein A¹ is CH and A² and A³ are N, or A² is CH and A¹ and A³ are N.

3. The compound according to claim 2 or a salt thereof, wherein R¹ is:

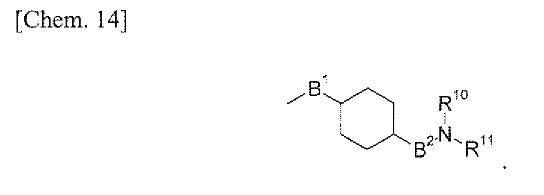

4. The compound according to claim 3 or a salt thereof, wherein B¹ is a bond or methylene, and B² is a bond.

5. The compound according to claim 4 or a salt thereof, wherein R² are the same as or different from each other and represent H or lower alkyl.

6. The compound according to claim 5 or a salt thereof, wherein R³ is H.

7. The compound according to claim 6 or a salt thereof, wherein R¹⁰ is H, lower alkyl which may be substituted with halogen or -OH, -lower alkylene-O-lower alkyl, lower alkenyl, lower alkynyl, -lower alkylene-phenyl, or -lower allcylene-O-lower alkylene-phenyl, in which the phenyl may be substituted with -O-lower alkyl.

8. The compound according to claim 7 or a salt thereof, wherein R¹¹ is R¹⁰⁰ or -C(O)R¹⁰¹.

9. The compound according to claim 6 or a salt thereof, wherein R¹⁰ and R¹¹ are combined with the N to which they are bonded to form a 3- to 8-membered monocyclic hetero ring group containing 1 to 4 hetero atoms selected from O, S, and N, and the monocyclic hetero ring may be substituted with lower alkyl, oxo, halogeno-lower alkyl, -lower alkylene-OH, -C(O)O-lower alkyl, -C(O)NR¹⁰³R¹⁰⁴, -N(R⁰)₂, halogen, -CN, -OH, -O-lower alkyl, -lower alkylene-O-lower alkyl, or a hetero ring group.

10. The compound according to claim 1 or a pharmaceutically acceptable salt thereof, selected from the group consisting of:
N-[trans-4-({6-[2-(difluoromethyl)-1H-benzimidazol-1-yl]-2-morpholin-4-ylpyrimidin-4-yl}oxy)cyclohexyl]-N,N-dimethylglycinamide,
N-[trans-4-({6-[2-(difluoromethyl)-1H-benzimidazol-1-yl]-2-morpholin-4-ylpyrimidin-4-yl}amino)cyclohexyl]-N,N-dimethylglycinamide,
4-[2-(difluoromethyl)-1H-benzimidazol-1-yl]-N-({trans-4-[(2-fluoroethyl)(methyl)amino]cyclohexyl}methyl)-6-morpholin-4-ylpyrimidin-2-amine,
4-[2-(difluoromethyl)-1H-benzimidazol-1-yl]-N-({trans-4-[(2-methoxyethyl)(methyl)amino]cyclohexyl}methyl)-6-morpholin-4-ylpyrimidin-2-amine,
6-[2-(difluoromethyl)-1H-benzimidazol-1-yl]-2-morpholin-4-yl-N-[(trans-4-morpholin-4-ylcyclohexyl)methyl]pyrimidin-4-amine,
1-[{trans-4-[({4-[2-(difuoromethyl)-1H-benzimidazol-1-yl]-6-[(3S)-3-methylmorpholin-4-yl]pyrimidin-2-yl}amino)methyl]cyclohexyl}(methyl)amino]-2-methylpropan-2-ol,
1-[{trans-4-[({4-[2-(difluoromethyl)-1H-benzimidazol-1-yl]-6-[(3S)-3-methylmorpholin-4-yl]pyrimidin-2-yl}amino)methyl]cyclohexyl}(ethyl)amino]-2-methylpropan-2-ol,
4-[2-(difluoromethyl)-1H-benzimidazol-1-yl]-N-({trans-4-[ethyl(1-methoxypropan-2-yl)amino]cydohexyl)methyl)-6-(morpholin-4-yl)pyrimidin-2-amine,
4-[2-(difluoromethyl)-1H-benzimidazol-1-yl]-N-{[trans-4-(dipropylamino)cyclohexyl]methyl}-6-(morpholin-4-yl)pyrimidin-2-amine,
3-[{trans-4-[({4-[2-(difluoromethyl)-1H-benzimidazol-1-yl]-6-(morpholin-4-yl)pyrimidin-2-yl}amino)methyl]cyclohexyl}(methyl)amino]-2-methylbutan-2-oI,
6-[2-(difluoromethyl)-1H-benzimidazol-1-yl]-N-({trans-4-[(3S)-3-fluoropyrrolidin-1-yl]cyclohexyl}methyl)-2-(morpholin-4-yl)pyrimidin-4-amine,
3-[{trans-4-[({(4-[2-(difluoromethyl)-1H-benzimidazol-1-yl]-6-[(3S)-3-methylmorpholin-4-yl]pyrimidin-2-yl}amino)methyl]cyclohexyl}(methyl)amino]-2-methylbutan-2-ol,
3-[{trans-4-[({4-[2-(difluoromethyl}-1H-benzimidazol-1-yl]-6-[(3R)-3-methylmorpholin-4-yl]pyrimidin-2-yl}amino)methyl]cyclohexyl}(methyl)amino]-2-methylbutan-2-ol,
4-[2-(difluoromethyl)-1H-benzimidazol-1-yl]-N-({trans-4-[(1-methoxypropan-2-yl)(methyl)amino]cyclohexyl}methyl)-6-[(3R)-3-methylmorpholin-4-yl]pyrimidin-2-amine,
4-[2-(difluoromethyl)-1H-benzimidazol-1-yl]-N-({trans-4-[ethyl(1-methoxypropan-2-yl)amino]cyclohexyl)methyl)-6-[(3R)-3-methylmorpholin-4-yl]pyrimidin-2-amine,
4-[2-(difluoromethyl)-1H-benzimidazol-1-yl]-N-({trans-4-[(2S)-2-(fluoromethyl)pyrrolidin-1-yl]cyclohexyl} methyl)-6-(morpholin-4-yl)pyrimidin-2-amine, and
6-[2-(difluoromethyl)-1H-benzimidazol-1-yl]-N-({trans-4-[(2S)-2-(fluoromethyl)azetidin-1-yl]cyclohexyl}methyl)-2-(morpholin-4-yl)pyrimidin-4-amine.

11. A pharmaceutical composition comprising the compound according to claim 1 or a salt thereof, and a pharmaceutically acceptable excipient.

12. A pharmaceutical composition for preventing or treating rejection in the transplantation of various organs, an allergy disease, an autoimmune disease, a hematologic tumor, or the like, comprising the compound according to claim 1 or a salt thereof.

13. Use of the compound according to claim 1 or a salt thereof for the manufacture of a pharmaceutical composition for preventing or treating rejection in the transplantation of various organs, an allergy disease, an autoimmune disease, a hematologic tumor, or the like.

14. Use of the compound according to claim 1 or a salt thereof for preventing or treating rejection in the transplantation of various organs, an allergy disease, an autoimmune disease, a hematologic tumor, or the like.

15. A method for preventing or treating rejection in the transplantation of various organs, an allergy disease, an autoimmune disease, a hematologic tumor, or the like, comprising administering to a patient an effective amount of the compound according to claim 1 or a pharmaceutically acceptable salt thereof.
